(19) Europäisches Patentamt / European Patent Office / Office européen des brevets

(11) EP 1 869 038 B1

(12) **EUROPÄISCHE PATENTSCHRIFT**

(45) Veröffentlichungstag und Bekanntmachung des Hinweises auf die Patenterteilung:
**08.07.2009 Patentblatt 2009/28**

(21) Anmeldenummer: **06724098.6**

(22) Anmeldetag: **07.04.2006**

(51) Int Cl.:
***C07D 471/04*** *(2006.01)* ***A61K 31/437*** *(2006.01)*
***A61P 25/00*** *(2006.01)*

(86) Internationale Anmeldenummer:
**PCT/EP2006/003153**

(87) Internationale Veröffentlichungsnummer:
**WO 2006/105971 (12.10.2006 Gazette 2006/41)**

(54) **SUBSTITUIERTE 5,6,7,8-TETRAHYDRO-IMIDAZO[1 ,2-A]PYRIDIN-2-YLAMIN-VERBINDUNGEN UND DEREN VERWENDUNG ZUR HERSTELLUNG VON ARZNEIMITTELN**

SUBSTITUTED 5,6,7,8-TETRAHYDRO-IMIDAZO[1,2-A]PYRIDIN-2-YLAMINE COMPOUNDS AND THEIR USE FOR PRODUCING DRUGS

COMPOSES SUBSTITUES DE 5,6,7,8-TETRAHYDRO-IMIDAZO[1,2-A]PYRIDINE-2-YLAMINE ET LEUR UTILISATION POUR PRODUIRE DES MEDICAMENTS

(84) Benannte Vertragsstaaten:
**AT BE BG CH CY CZ DE DK EE ES FI FR GB GR HU IE IS IT LI LT LU LV MC NL PL PT RO SE SI SK TR**

(30) Priorität: **08.04.2005 DE 102005016547**

(43) Veröffentlichungstag der Anmeldung:
**26.12.2007 Patentblatt 2007/52**

(73) Patentinhaber: **Grünenthal GmbH**
**52078 Aachen (DE)**

(72) Erfinder:
- **FRANK, Robert**
  **52070 Aachen (DE)**
- **SUNDERMANN, Bernd**
  **52074 Aachen (DE)**
- **SUNDERMANN, Corinna**
  **52074 Aachen (DE)**

(74) Vertreter: **Bülle, Jan et al**
**Kutzenberger & Wolff**
**Patentanwaltssozietät**
**Theodor-Heuss-Ring 23**
**50668 Köln (DE)**

(56) Entgegenhaltungen:
**WO-A-20/04074290** **US-A1- 2002 183 327**
**US-A1- 2003 022 914** **US-A1- 2004 204 409**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).



Printed by Jouve, 75001 PARIS (FR)

## Beschreibung

Die vorliegende Erfindung betrifft substituierte 5,6,7,8-Tetrahydro-imidazo[1,2-a]pyridin-2-ylamin-Verbindungen, Verfahren zu ihrer Herstellung, Arzneimittel enthaltend diese Verbindungen und die Verwendung dieser Verbindungen zur Herstellung von Arzneimitteln.

Die Behandlung von Schmerz, insbesondere von neuropathischem Schmerz, hat in der Medizin große Bedeutung. Es besteht ein weltweiter Bedarf an wirksamen Schmerztherapien. Der dringende Handlungsbedarf für eine patientengerechte und zielorientierte Behandlung chronischer und nicht chronischer Schmerzzustände, wobei hierunter die erfolgreiche und zufriedenstellende Schmerzbehandlung für den Patienten zu verstehen ist, dokumentiert sich auch in der großen Anzahl von wissenschaftlichen Arbeiten, die auf dem Gebiet der angewandten Analgetik bzw. der Grundlagenforschung zur Nociception in letzter Zeit erschienen sind.

Einen geeigneten Ansatzpunkt zur Behandlung von Schmerz, insbesondere von neuropathischem Schmerz, stellt der Vanilloid-Rezeptor vom Subtyp 1 (VR1/TRPV1) dar, der häufig auch als Capsaicin-Rezeptor bezeichnet wird. Dieser Rezeptor wird u.a. durch Vanilloide wie z.B. Capsaicin, Hitze und Protonen stimuliert und spielt eine zentrale Rolle bei der Schmerzentstehung. Darüber hinaus ist er für eine Vielzahl weiterer physiologischer und pathophysiologischer Prozesse von Bedeutung wie beispielsweise Migräne; Depressionen; neurodegenerativen Erkrankungen; kognitiven Erkrankungen; Angstzuständen; Epilepsie; Husten; Diarrhöe; Pruritus; Störungen des kardiovaskulären Systems; Störungen der Nahrungsaufnahme; Medikamentenabhängigkeit; Medikamentenmißbrauch und insbesondere Harninkontinzenz.

Eine Aufgabe der vorliegenden Erfindung bestand daher darin, neue Verbindungen zur Verfügung zu stellen, die sich insbesondere als pharmakologische Wirkstoffe in Arzneimitteln eignen, vorzugsweise in Arzneimitteln zur Behandlung von Störungen oder Krankheiten, die zumindest teilweise durch Vanilloid-Rezeptoren 1 (VR1/TRPV1-Rezeptoren) vermittelt werden.

Überraschenderweise wurde nun gefunden, dass substituierte 5,6,7,8-Tetrahydro-imidazo[1,2-a]pyridin-2-ylamin-Verbindungen der nachstehend angegebenen allgemeinen Formel I eine ausgezeichnete Affinität zum Vanilloid-Rezeptor vom Subtyp 1 (VR1/TRPV1-Rezeptor) aufweisen und sich daher insbesondere zur Prophylaxe und/oder Behandlung von Störungen oder Krankheiten eignen, die zumindest teilweise durch Vanilloid-Rezeptoren 1 (VR1/TRPV1) vermittelt werden.

Ein Gegenstand der vorliegenden Erfindung sind daher substituierte 5,6,7,8-Tetrahydro-imidazo[1,2-a]pyridin-2-ylamin-Verbindungen der allgemeinen Formel I,

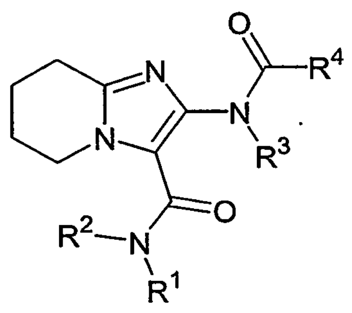

I

worin

$R^1$ und $R^2$, unabhängig voneinander, jeweils für

einen Wasserstoff-Rest;

-C(=O)-OR$^5$;

-(CHR$^6$)-(CH$_2$)$_m$-C(=O)-OR$^7$ mit m = 0, 1, 2, 3, 4 oder 5;

-C(=O)-R$^8$;

-(CH$_2$)$_n$-C(=O)-R$^9$ mit n = 1, 2, 3, 4 oder 5;

-C(=O)-NH-R$^{10}$;

-(CH$_2$)$_o$-C(=O)-NHR$^{11}$ mit o = 0, 1, 2, 3, 4 oder 5;

-C(=O)-NR$^{12}$R$^{13}$;

-(CH$_2$)$_p$-C(=O)-NR$^{14}$R$^{15}$ mit p = 1, 2, 3, 4 oder 5;

$-(CHR^{16})-X_q-(CHR^{17})_r-Y_s-(CHR^{18})_t-Z_u-R^{19}$ mit q = 0 oder 1, r = 0 oder 1, s = 0 oder 1, t = 0 oder 1, u = 0 oder 1, worin X, Y und Z, unabhängig voneinander, jeweils für O, S, NH, $N(CH_3)$, $N(C_2H_5)$ oder $N[CH(CH_3)_2]$ stehen;
für einen linearen oder verzweigten, gesättigten oder ungesättigten, ggf. substituierten $C_{1-10}$ aliphatischen Rest;
für einen ungesättigten oder gesättigten, ggf. substituierten 3-, 4-, 5-, 6-, 7-, 8- oder 9-gliedrigen cycloaliphatischen Rest, der mit einer linearen oder verzweigten, ggf. substituierten $C_{1-5}$-Alkylen-Gruppe überbrückt und/oder mit einem gesättigten, ungesättigten oder aromatischen, ggf. substituierten mono- oder polyzyklischen Ringsystem kondensiert sein kann;
oder für einen ggf. substituierten 5- bis 14-gliedrigen Aryl- oder Heteroaryl-Rest, der mit einem gesättigten oder ungesättigten, ggf. substituierten mono- oder polyzyklischen Ringsystem kondensiert sein kann, stehen;
oder

$R^1$ und $R^2$ zusammen mit dem sie verbindenden Stickstoffatom als Ringglied einen gesättigten oder ungesättigten, ggf. substituierten 4-, 5-, 6-, 7-, 8- oder 9-gliedrigen heterocycloaliphatischen Rest bilden, der mit einem gesättigten, ungesättigten oder aromatischen, ggf. substituierten mono- oder polyzyklischen Ringsystem kondensiert sein kann und/oder über ein gemeinsames Ringatom zusammen mit einem gesättigten oder ungesättigten, ggf. substituierten 5-, 6- oder 7-gliedrigen cycloaliphatischen Rest eine ggf. substituierte Spiro-Verbindung bilden kann,
wobei jeweils der heterocycloaliphatische Rest und ggf. der cycloaliphatische Rest der Spiro-Verbindung mit 1, 2, 3, 4 oder 5 Substituenten unabhängig voneinander ausgewählt aus der Gruppe bestehend aus $R^{20}$, $-(CHR^{21})-(CH_2)_v-(CH_2)_w-R^{22}$ mit v = 0 oder 1 und w = 0 oder 1, $-CH=CH-R^{23}$, $-(CH_2)_x-C(=O)-OR^{24}$ mit x = 0, 1, 2, 3, 4 oder 5; $-(CH_2)_y-C(=O)-R^{25}$ mit y = 0, 1, 2, 3, 4 oder 5; $-(CH_2)_z-C(=O)-NHR^{26}$ mit z = 0, 1, 2, 3, 4 oder 5; $-(CH_2)_{aa}-C(=O)-NR^{27}R^{28}$ mit aa = 0, 1, 2, 3, 4 oder 5; F; Cl; Br; -CN; $-CF_3$; $-NO_2$; Oxo (=O); Thioxo (=S); $-C_{1-5}$-Alkyl; -OH; $-O-C_{1-5}$-Alkyl; -SH; $-S-C_{1-5}$-Alkyl; $-NH_2$; $-NH-C_{1-5}$-Alkyl und $-N(C_{1-5}\text{-Alkyl})_2$ substituiert und/oder jeweils weitere 1, 2, 3, 4 oder 5 Heteroatom(e) unabhängig voneinander ausgewählt aus der Gruppe bestehend aus Sauerstoff, Stickstoff und Schwefel als Ringglied(er) aufweisen kann;

$R^3$ für einen Wasserstoff-Rest;
für einen linearen oder verzweigten, gesättigten oder ungesättigten, ggf. substituierten $C_{1-10}$ aliphatischen Rest,
für einen ungesättigten oder gesättigten, ggf. substituierten 3-, 4-, 5-, 6-, 7-, 8- oder 9-gliedrigen cycloaliphatischen Rest, der über eine lineare oder verzweigte, ggf. substituierte $C_{1-5}$-Alkylen-, $C_{2-5}$-Alkenylen- oder $C_{2-5}$-Alkinylen-Gruppe gebunden sein kann,
oder für einen ggf. substituierten 5- bis 14-gliedrigen Aryl- oder Heteroaryl-Rest, der mit einem gesättigten oder ungesättigten, ggf. substituierten mono- oder polyzyklischen Ringsystem kondensiert und/oder über eine lineare oder verzweigte, ggf. substituierte $C_{1-5}$-Alkylen-, $C_{2-5}$-Alkenylen- oder $C_{2-5}$-Alkinylen- Gruppe gebunden sein kann, steht;

$R^4$ für einen linearen oder verzweigten, gesättigten oder ungesättigten, ggf. substituierten $C_{1-10}$ aliphatischen Rest,
für einen ungesättigten oder gesättigten, ggf. substituierten 3-, 4-, 5-, 6-, 7-, 8- oder 9-gliedrigen cycloaliphatischen Rest, der über eine lineare oder verzweigte, ggf. substituierte $C_{1-5}$-Alkylen-, $C_{2-5}$-Alkenylen- oder $C_{2-5}$-Alkinylen-Gruppe gebunden sein kann,
oder für einen ggf. substituierten 5- bis 14-gliedrigen Aryl- oder Heteroaryl-Rest, der mit einem gesättigten oder ungesättigten, ggf. substituierten mono- oder polyzyklischen Ringsystem kondensiert und/oder über eine lineare oder verzweigte, ggf. substituierte $C_{1-5}$-Alkylen-, $C_{2-5}$-Alkenylen- oder $C_{2-5}$-Alkinylen- Gruppe gebunden sein kann, steht;

$R^5$, $R^7$, $R^8$, $R^9$, $R^{10}$, $R^{11}$, $R^{12}$, $R^{13}$, $R^{14}$, $R^{15}$, $R^{24}$, $R^{25}$, $R^{26}$, $R^{27}$ und $R^{28}$, unabhängig voneinander, jeweils für
einen linearen oder verzweigten, gesättigten oder ungesättigten, ggf. substituierten $C_{1-10}$ aliphatischen Rest,
für einen ungesättigten oder gesättigten, ggf. substituierten 3-, 4-, 5-, 6-, 7-, 8- oder 9-gliedrigen cycloaliphatischen Rest, der mit einem gesättigten, ungesättigten oder aromatischen, ggf. substituierten mono- oder polyzyklischen Ringsystem kondensiert und/oder über eine lineare oder ver-

zweigte, ggf. substituierte $C_{1-5}$-Alkylen-, $C_{2-5}$-Alkenylen- oder $C_{2-5}$-Alkinylen-Gruppe gebunden sein kann,
oder für einen ggf. substituierten 5- bis 14-gliedrigen Aryl- oder Heteroaryl-Rest, der mit einem gesättigten oder ungesättigten, ggf. substituierten mono- oder polyzyklischen Ringsystem kondensiert und/oder über eine lineare oder verzweigte, ggf. substituierte $C_{1-5}$-Alkylen-, $C_{2-5}$-Alkenylen- oder $C_{2-5}$-Alkinylen- Gruppe gebunden sein kann, stehen;

$R^6$ für einen Wasserstoff-Rest;
für einen linearen oder verzweigten, gesättigten oder ungesättigten, ggf. substituierten $C_{1-10}$ aliphatischen Rest, der 1, 2, 3, 4 oder 5 Heteroatom(e) unabhängig voneinander ausgewählt aus der Gruppe bestehend aus Sauerstoff, Schwefel und Stickstoff als Kettenglied(er) aufweisen kann;
oder für einen ggf. substituierten 5- bis 14-gliedrigen Aryl- oder Heteroaryl-Rest, der mit einem gesättigten oder ungesättigten, ggf. substituierten mono- oder polyzyklischen Ringsystem kondensiert und/oder über eine lineare oder verzweigte, ggf. substituierte $C_{1-5}$-Alkylen-, $C_{2-5}$-Alkenylen- oder $C_{2-5}$-Alkinylen- Gruppe gebunden sein kann, steht;

$R^{16}$, $R^{17}$ und $R^{18}$, unabhängig voneinander, jeweils
für einen Wasserstoff-Rest;
für einen linearen oder verzweigten, gesättigten oder ungesättigten, ggf. substituierten $C_{1-10}$ aliphatischen Rest, der 1, 2, 3, 4 oder 5 Heteroatom(e) unabhängig voneinander ausgewählt aus der Gruppe bestehend aus Sauerstoff, Schwefel und Stickstoff als Kettenglied(er) aufweisen kann
oder für einen ggf. substituierten 5- bis 14-gliedrigen Aryl- oder Heteroaryl-Rest, der mit einem gesättigten oder ungesättigten, ggf. substituierten mono- oder polyzyklischen Ringsystem kondensiert sein kann, stehen;

$R^{19}$ für einen ungesättigten oder gesättigten, ggf. substituierten 3-, 4-, 5-, 6-, 7-, 8- oder 9-gliedrigen cycloaliphatischen Rest, der mit 1, 2, 3, 4 oder 5 linearen oder verzweigten, ggf. substituierten $C_{1-5}$-Alkylen-Gruppen überbrückt und/oder mit einem gesättigten, ungesättigten oder aromatischen, ggf. substituierten mono- oder polyzyklischen Ringsystem kondensiert sein kann;
oder für einen ggf. substituierten 5- bis 14-gliedrigen Aryl- oder Heteroaryl-Rest, der mit einem gesättigten oder ungesättigten, ggf. substituierten mono- oder polyzyklischen Ringsystem kondensiert sein kann, steht;

$R^{20}$ für einen linearen oder verzweigten, gesättigten oder ungesättigten, ggf. substituierten $C_{1-10}$ aliphatischen Rest;
für einen ungesättigten oder gesättigten, ggf. substituierten 3-, 4-, 5-, 6-, 7-, 8- oder 9-gliedrigen cycloaliphatischen Rest
oder für einen ggf. substituierten 5- bis 14-gliedrigen Aryl- oder Heteroaryl-Rest, der mit einem gesättigten oder ungesättigten, ggf. substituierten mono- oder polyzyklischen Ringsystem kondensiert sein kann, steht;

$R^{21}$ für einen Wasserstoff-Rest;
für einen linearen oder verzweigten, gesättigten oder ungesättigten, ggf. substituierten $C_{1-10}$ aliphatischen Rest;
für einen ungesättigten oder gesättigten, ggf. substituierten 3-, 4-, 5-, 6-, 7-, 8- oder 9-gliedrigen cycloaliphatischen Rest
oder für einen ggf. substituierten 5- bis 14-gliedrigen Aryl- oder Heteroaryl-Rest, der mit einem gesättigten oder ungesättigten, ggf. substituierten mono- oder polyzyklischen Ringsystem kondensiert sein kann, steht;
und

$R^{22}$ und $R^{23}$, unabhängig voneinander, jeweils
für einen ungesättigten oder gesättigten, ggf. substituierten 3-, 4-, 5-, 6-, 7-, 8- oder 9-gliedrigen cycloaliphatischen Rest
oder für einen ggf. substituierten 5- bis 14-gliedrigen Aryl- oder Heteroaryl-Rest, der mit einem gesättigten oder ungesättigten, ggf. substituierten mono- oder polyzyklischen Ringsystem kondensiert sein kann, stehen;

wobei

die vorstehend genannten $C_{1-10}$ aliphatischen Reste jeweils ggf. mit 1, 2, 3, 4 oder 5 Substituenten unabhängig voneinander ausgewählt aus der Gruppe bestehend aus F, Cl, Br, I, -CN, $-NO_2$, -OH, -SH und $-NH_2$ substituiert sein können;

die vorstehend genannten cycloaliphatischen Reste jeweils ggf. mit 1, 2, 3, 4 oder 5 Substituenten unabhängig voneinander ausgewählt aus der Gruppe bestehend aus Oxo (=O), Thioxo (=S), F, Cl, Br, I, -CN, $-CF_3$, $-SF_5$, -OH, -O-$C_{1-5}$-Alkyl, $-NH_2$, $-NO_2$, -O-$CF_3$, -S-$CF_3$, -SH, -S-$C_{1-5}$-Alkyl, -$C_{1-5}$-Alkyl, -C(=O)-OH, -C(=O)-O-$C_{1-5}$-Alkyl, -NH-$C_{1-5}$-Alkyl, -N($C_{1-5}$-Alkyl)$_2$, -($CH_2$)-Benzo[b]furanyl, -O-Phenyl, -O-Benzyl, Phenyl und Benzyl substituiert sein können, wobei jeweils der zyklische Teil der Reste -O-Phenyl, -O-Benzyl, Phenyl, -($CH_2$)-Benzo[b]furanyl und Benzyl mit 1, 2, 3, 4 oder 5 Substituenten unabhängig voneinander ausgewählt aus der Gruppe bestehend aus F, Cl, Br, -OH, $-CF_3$, $-SF_5$, -CN, $-NO_2$, -$C_{1-5}$-Alkyl, -O-$C_{1-5}$-Alkyl, -O-$CF_3$, -S-$CF_3$, Phenyl und -O-Benzyl substituiert sein kann

und die vorstehend genannten cycloaliphatischen Reste jeweils ggf. 1, 2, 3, 4 oder 5 Heteroatom(e) unabhängig voneinander ausgewählt aus der Gruppe bestehend aus Sauerstoff, Stickstoff und Schwefel als Ringglied(er) aufweisen können;

die vorstehend genannten $C_{1-5}$-Alkylen-, $C_{2-5}$-Alkenylen- oder $C_{2-5}$-Alkinylen-Gruppen jeweils ggf. mit 1, 2, 3, 4 oder 5 Substituenten unabhängig voneinander ausgewählt aus der Gruppe bestehend aus F, Cl, Br, -OH, -SH, $-NH_2$, -CN, $NO_2$ und Phenyl substituiert sein können;

die Ringe der vorstehend genannten mono- oder polyzyklischen Ringsysteme ggf. jeweils mit 1, 2, 3, 4 oder 5 Substituenten unabhängig voneinander ausgewählt aus der Gruppe bestehend aus Oxo (=O), Thioxo (=S), F, Cl, Br, I, -CN, $-CF_3$, $-SF_5$, -OH, -O-$C_{1-5}$-Alkyl, $-NH_2$, $-NO_2$, -O-$CF_3$, -S-$CF_3$, -SH, -S-$C_{1-5}$-Alkyl, -$C_{1-5}$-Alkyl, -C(=O)-OH, -C(=O)-O-$C_{1-5}$-Alkyl, -NH-$C_{1-5}$-Alkyl, -N($C_{1-5}$-Alkyl)$_2$, -($CH_2$)-Benzo[b]furanyl, -O-Phenyl, -O-Benzyl, Phenyl und Benzyl substituiert sein können, wobei jeweils der zyklische Teil der Reste -O-Phenyl, -O-Benzyl, Phenyl, -($CH_2$)-Benzo[b]furanyl und Benzyl mit 1, 2, 3, 4 oder 5 Substituenten unabhängig voneinander ausgewählt aus der Gruppe bestehend aus F, Cl, Br, -OH, $-CF_3$, $-SF_5$, -CN, $-NO_2$, -$C_{1-5}$-Alkyl, -O-$C_{1-5}$-Alkyl, -O-$CF_3$, -S-$CF_3$, Phenyl und -O-Benzyl substituiert sein kann

und die Ringe der vorstehend genannten mono- oder polyzyklischen Ringsysteme jeweils 5-, 6- oder 7-gliedrig sind und jeweils ggf. 1, 2, 3, 4 oder 5 Heteroatom(e) als Ringglied(er) aufweisen können, die unabhängig voneinander aus der Gruppe bestehend aus Sauerstoff, Stickstoff und Schwefel ausgewählt sind;

und die vorstehend genannten Aryl- oder Heteroaryl-Reste jeweils ggf. mit 1, 2, 3, 4 oder 5 Substituenten unabhängig voneinander ausgewählt aus der Gruppe bestehend aus F, Cl, Br, I, -CN, $-CF_3$, $-SF_5$, -OH, -O-$C_{1-5}$-Alkyl, $-NH_2$, $-NO_2$, -O-$CF_3$, -S-$CF_3$, -SH, -S-$C_{1-5}$-Alkyl, -$C_{1-5}$-Alkyl, -C(=O)-OH, -C(=O)-O-$C_{1-5}$-Alkyl, -NH-$C_{1-5}$-Alkyl, -N($C_{1-5}$-Alkyl)$_2$, -NH-C(=O)-O-$C_{1-5}$-Alkyl, -C(=O)-H, -C(=O)-$C_{1-5}$-Alkyl, -C(=O)-$NH_2$, -C(=O)-NH-$C_{1-5}$-Alkyl, C(=O)-N-($C_{1-5}$-Alkyl)$_2$, -($CH_2$)-Benzo[b]furanyl, -O-Phenyl, -O-Benzyl, Phenyl und Benzyl substituiert sein können, wobei jeweils der zyklische Teil der Reste -O-Phenyl, -O-Benzyl, Phenyl, -($CH_2$)-Benzo[b]furanyl und Benzyl mit 1, 2, 3, 4 oder 5 Substituenten unabhängig voneinander ausgewählt aus der Gruppe bestehend aus F, Cl, Br, -OH, $-CF_3$, $-SF_5$, -CN, $-NO_2$, -$C_{1-5}$-Alkyl, -O-$C_{1-5}$-Alkyl, -O-$CF_3$, -S-$CF_3$, Phenyl und -O-Benzyl substituiert sein kann,

und

die vorstehend genannten Heteroaryl-Reste jeweils ggf. 1, 2, 3, 4 oder 5 Heteroatom(e) unabhängig voneinander ausgewählt aus der Gruppe bestehend aus Sauerstoff, Stickstoff und Schwefel als Ringglied(er) aufweisen können;

jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form entsprechender Salze, oder jeweils in Form entsprechender Solvate.

Unter einem mono- oder polyzyklischen Ringsystem werden im Sinne der vorliegenden Erfindung mono- oder polyzyklische Kohlenwasserstoffreste verstanden, die gesättigt, ungesättigt oder aromatisch sein und ggf. 1, 2, 3, 4 oder 5 Heteroatome unabhängig voneinander ausgewählt aus der Gruppe bestehend aus Sauerstoff, Stickstoff und Schwefel als Ringglieder aufweisen können. Ein solches mono- bzw. polyzyklisches Ringsystem kann beispielsweise mit einem cycloaliphatischen Rest, einem Aryl-Rest oder einem Heteroaryl-Rest kondensiert (anneliert) sein.

Sofern ein polyzyklisches Ringsystem wie beispielsweise ein bizyklisches Ringsystem vorliegt, können die verschiedenen Ringe, jeweils unabhängig voneinander, einen unterschiedlichen Sättigungsgrad aufweisen, d.h. gesättigt, ungesättigt oder aromatisch sein. Bevorzugt ist ein polyzyklisches Ringsystem ein bizyklisches Ringsystem.

Aliphatische Reste im Sinne der vorliegenden Erfindung umfassen gesättigte AlkylReste wie auch ungesättigte Alkenyl-Reste mit wenigstens einer C=C-Doppelbindung und ungesättigte Alkinyl-Reste mit wenigstens einer CC-Dreifachbindung. Beispielhaft seien Methyl, Ethyl, n-Propyl, iso-Propyl, n-Butyl, iso-Butyl, sec-Butyl, tert-Butyl, n-Pentyl, sec-Pentyl, 2-Pentyl, 3-Pentyl, iso-Pentyl, neo-Pentyl, n-Hexyl, 2-Hexyl, 3-Hexyl, n-Heptyl, n-Octyl, n-Nonyl, n-Decyl, -C(H)($C_2H_5$)$_2$, -C(H)($CH_3$)-C(H)($CH_3$)$_2$, -($CH_2$)-($CH_2$)-C($CH_3$)$_3$, -C(H)(n-$C_3H_7$)$_2$, -$CH_2$-$CH_2$-C(H)($CH_3$)-($CH_2$)$_3$-$CH_3$, Vinyl, Ethinyl, 1-Propenyl, 2-Propenyl, 1-Propinyl, 2-Propinyl, 1-Butenyl, 2-Butenyl, 3-Butenyl, 1-Butinyl, 2-Butinyl, 3-Butinyl, 1-Pentenyl, 2-Pentenyl, 3-Pentenyl, 4-Pentenyl, 1-Pentinyl, 2-Pentinyl, 3-Pentinyl, 4-Pentinyl, Hexenyl, Hexinyl und -CH=CH-CH=CH-$CH_3$ als aliphatische Reste genannt.

Cycloaliphatische Reste im Sinne der vorliegenden Erfindung umfassen sowohl gesättigte wie auch ungesättigte cyclische Kohlenwasserstoffreste, die jeweils ggf. 1, 2, 3, 4 oder 5 Heteroatom(e) unabhängig voneinander ausgewählt aus der Gruppe bestehend aus Sauerstoff, Stickstoff und Schwefel als Ringglied(er) aufweisen können. Beispielhaft seien die Reste Cyclopropyl, Cyclobutyl, Cyclopentyl, Cyclohexyl, Cycloheptyl, Cyclooctyl, Cyclopentenyl, Cyclohexenyl, Cycloheptenyl, Cyclooctenyl, Imidazolidinyl, Tetrahydrofuranyl (Tetrahydrofuryl), Piperidinyl, Piperazinyl, Morpholinyl, Pyrrolidinyl, Tetrahydrothiophenyl, Tetrahydropyranyl, Thiomorpholinyl, Dioxolanyl, Azepanyl, Diazepanyl und Dithiolanyl genannt.

Als geeignete Aryl-Reste seien beispielsweise Phenyl und Naphthyl (1-Naphtyl und 2-Naphthyl) genannt.

Als geeignete Heteroaryl-Reste seien beispielhaft Pyridinyl, Thiophenyl (Thienyl), Furanyl (Furyl), Pyrazolinyl, Pyrimidinyl, Pyridinyl, 2-Pyridinyl, 3-Pyridinyl, 4-Pyridinyl, 2-Pyrimidinyl, 4-Pyrimidinyl, 5-Pyrimidinyl, Pyridazinyl, 3-Pyridazinyl, 4-Pyridazinyl, Pyrazinyl, 3-Pyrazinyl, Imidazolyl, 2-Imidazolyl, 4-Imidazolyl, Isoxazolyl, 3-Isoxazolyl, 4-Isoxazolyl, 5-Isoxazolyl, Pyrazolyl, 3-Pyrazolyl, 4-Pyrazolyl, 5-Pyrazolyl, Oxazolyl, 2-Oxazolyl, 4-Oxazolyl, 5-Oxazolyl, 1,2,3-Oxathiazolyl, 1,2,3-Oxadiazolyl, 1,2,4-Oxadiazolyl, 1,2,5-Oxadiazolyl, 1,3,4-Oxadiazolyl, Thiazolyl, 2-Thiazolyl, 4-Thiazolyl, 5-Thiazolyl, 3-Isothiazolyl, 4-Isothiazolyl, 5-Isothiazolyl, 2-Thiophenyl, 3-Thiophenyl, Pyrrolyl, 2-Pyrrolyl, 3-Pyrrolyl, 3-Isopyrrolyl, 4-Isopyrrolyl, 5-Isopyrrolyl, 1,2,4-Oxadiazol-3-yl, 1,2,4-Oxadiazol-5-yl, 1,2,4-Thiadiazol-3-yl, 1,2,4-Thiadiazol-5-yl, 1,3,4-Thiadiazol-5-yl, Triazolyl, 1,2,4-Triazol-3-yl, 1,2,4-Triazol-5-yl, 1,2,3,4-Tetrazol-5-yl, 1,2,3,4-Thiatriazolyl, Chinolinyl, Triazinyl, Chinoxalinyl, Pyranyl, Indolyl, Isoindolyl, Benzo[b]furanyl, Benzo[b]thiophenyl, Indazolyl und Isochinolinyl genannt.

Der Fachmann versteht, daß einige der erfindungsgemäßen substituierten 5,6,7,8-Tetrahydro-imidazo[1,2-a]pyridin-2-ylamin-Verbindungen der allgemeinen Formel I in Form von Tautomeren vorliegen können, die ebenfalls Gegenstand der vorliegenden Erfindung sind und jeweils auch als Wirkstoffe in den untenstehend beschriebenen Arzneimitteln vorliegen können.

Bevorzugt sind substituierte 5,6,7,8-Tetrahydro-imidazo[1,2-a]pyridin-2-ylamin-Verbindungen der vorstehend angegebenen allgemeinen Formel I, worin

$R^1$ für einen Wasserstoff-Rest; -C(=O)-OR$^5$; -(CHR$_6$)-(CH$_2$)$_m$-C(=O)-OR mit m = 0, 1, 2, 3, 4 oder 5; -C(=O)-NH-R$^{10}$; -(CH$_2$)$_o$-C(=O)-NHR$^{11}$ mit o = 0, 1, 2, 3, 4 oder 5; -(CHR$^{16}$)-X$_q$-(CHR$^{17}$)$_r$-Y$_s$-(CHR$^{18}$)$_t$-Zu-R$^{19}$ mit q = 0 oder 1, r = 0 oder 1, s = 0 oder 1, t = 0 oder 1, u = 0 oder 1, worin X, Y und Z, unabhängig voneinander, jeweils für O, S, NH und N(CH$_3$) stehen; für einen ggf. substituierten Alkyl-Rest ausgewählt aus der Gruppe bestehend aus Methyl, Ethyl, n-Propyl, iso-Propyl, -(CH$_2$)-(CH$_2$)-(CN), n-Butyl, sec-Butyl, iso-Butyl, tert-Butyl, -C(H)(CH$_3$)-C(H)(CH$_3$)$_2$ und -(CH$_2$)-(CH$_2$)-(C(CH$_3$)$_3$),
für einen Alkenyl-Rest ausgewählt aus der Gruppe bestehend aus Vinyl, 1-Propenyl und 2-Propenyl,
für einen (hetero)cycloaliphatischen Rest ausgewählt aus der Gruppe bestehend aus Cyclopropyl, Cyclobutyl, Cyclopentyl, Cyclohexyl, Cycloheptyl, Cyclopentenyl, Cyclohexenyl, Cycloheptenyl, Imidazolidinyl, Tetrahydrofuranyl, Tetrahydrothiophenyl, Pyrrolidinyl, Piperidinyl, Morpholinyl, Piperazinyl, Thiomorpholinyl, Tetrahydropyranyl, Azepanyl, Diazepanyl, Dithiolanyl, (6,6)-Dimethyl-[3.1.1]-bicycloheptyl, Indanyl, Indenyl, (1,4)-Benzodioxanyl, (1,2,3,4)-Tetrahydronaphthyl, (1,2,3,4)-Tetrahydrochinolinyl und (1,2,3,4)-Tetrahydrochinazolinyl, wobei der (hetero)cycloaliphatische Rest jeweils ggf. mit 1, 2, 3, 4 oder 5 Substituenten unabhängig voneinander ausgewählt aus der Gruppe bestehend aus F, Cl, Br, I, -CN, -CF$_3$, -SF$_5$, -OH, -NH$_2$, -O-CF$_3$, -SH, -O-CH$_3$, -O-C$_2$H$_5$, Methyl, Ethyl, n-Propyl, iso-Propyl, n-Butyl, sec-Butyl, iso-Butyl, tert-Butyl, -S-CH$_3$, -S-C$_2$H$_5$, -C(=O)-O-CH$_3$, -C(=O)-O-C$_2$H$_5$, Oxo (=O), -N(CH$_3$)$_2$, -N(C$_2$H$_5$)$_2$, -N(H)(CH$_3$), -N(H)(C$_2$H$_5$), -NO$_2$, -SCF$_3$, -C(=O)-OH, -(CH$_2$)-Benzo[b]furanyl, -O-Phenyl, -O-Benzyl, Phenyl und Benzyl substituiert sein kann, wobei jeweils der zyklische Teil der -(CH$_2$)-Benzo[b]furanyl, -O-Phenyl, -O-Benzyl-, Benzyl- und Phenyl-Reste mit 1, 2, 3, 4 oder 5 Substituenten unabhängig voneinander ausgewählt aus der Gruppe bestehend aus F, Cl, Br, -OH, -CF$_3$, -SF$_5$, -CN, -NO$_2$, Methyl, Ethyl, iso-Propyl, n-Propyl, -O-CH$_3$, -O-C$_2$H$_5$, -O-CF$_3$, Phenyl und -O-Benzyl substituiert sein kann,
oder für einen Rest ausgewählt aus der Gruppe bestehend aus Phenyl, Naphthyl, (1,3)-Benzodioxolyl, (1,4)-Benzodioxanyl, Thiophenyl, Furanyl, Pyrrolyl, Pyrazolyl, Pyrazinyl, Pyranyl, Triazolyl, Pyridinyl, Imidazolyl, Indolyl, Isoindolyl, Benzo[b]furanyl, Benzo[b]thiophenyl, Thiazolyl, Oxazolyl, Isoxazolyl, Pyridazinyl, Pyrazinyl, Pyrimidinyl, Indazolyl, Chinazolinyl, Chinolinyl und Isochinolinyl, wobei der Rest jeweils ggf. mit 1, 2, 3, 4 oder 5 Substituenten unabhängig voneinander ausgewählt aus der Gruppe bestehend aus F, Cl, Br, I, -CN, -NO$_2$, -OH, -SH, Methyl, Ethyl, n-Propyl, iso-Propyl, n-Butyl, sec-Butyl, iso-Butyl, tert-Butyl, -CF$_3$, -O-CF$_3$, -S-CF$_3$, -SF$_5$, -O-CH$_3$, -O-C$_2$H$_5$, -O-Phenyl, -O-Benzyl, -NH$_2$, -N(CH$_3$)$_2$, -N(C$_2$H$_5$)$_2$, -N(H)(CH$_3$), -N(H)(C$_2$H$_5$), -NH-C(=O)-O-CH$_3$, -NH-C(=O)-O-C$_2$H$_5$ und -NH-C(=O)-O-C(CH$_3$)$_3$ substituiert sein kann, steht,
und jeweils die übrigen Reste $R^1$ und $R^2$ zusammen sowie $R^2$-$R^{28}$ die vorstehend genannte Bedeutung haben, jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form entsprechender Salze, oder jeweils in Form entsprechender Solvate.

Weiterhin bevorzugt sind substituierte 5,6,7,8-Tetrahydro-imidazo[1,2-a]pyridin-2-ylamin-Verbindungen der vorstehend angegebenen allgemeinen Formel I, worin

$R^2$ für einen Wasserstoff-Rest,

für -(CHR$^{16}$)-R$^{19}$,

oder für einen Alkyl-Rest ausgewählt aus der Gruppe bestehend aus Methyl, Ethyl, n-Propyl, iso-Propyl, n-Butyl, sec-Butyl, iso-Butyl und tert-Butyl steht, und jeweils die übrigen Reste R$^1$, R$^1$ und R$^2$ zusammen sowie R$^3$-R$^{28}$ die vorstehend genannte Bedeutung haben, jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form entsprechender Salze, oder jeweils in Form entsprechender Solvate.

Weiterhin bevorzugt sind auch substituierte 5,6,7,8-Tetrahydro-imidazo[1,2-a]pyridin-2-ylamin-Verbindungen der vorstehend angegebenen allgemeinen Formel I, worin R$^1$ und R$^2$ zusammen mit dem sie verbindenden Stickstoffatom als Ringglied einen Rest bilden ausgewählt aus der Gruppe bestehend aus

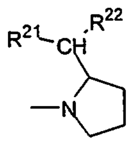 , 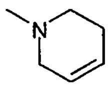 , 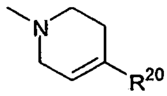 , 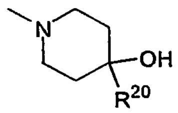 ,

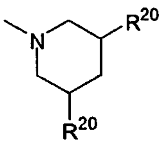 , 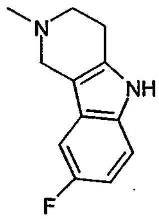 , 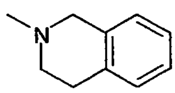 , 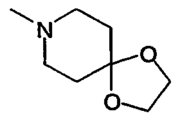 ,

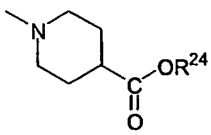 , 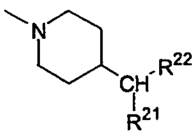 , 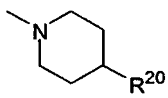 , 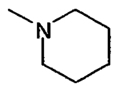 ,

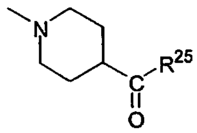 , 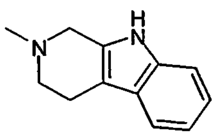 , 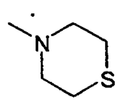 , 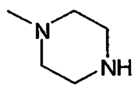 ,

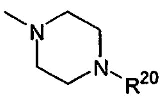 , 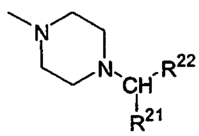 , 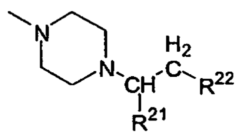 ,

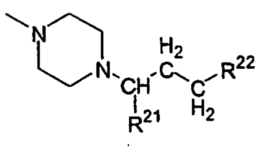 , 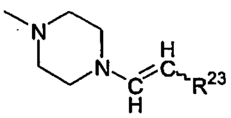 , 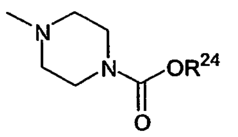 ,

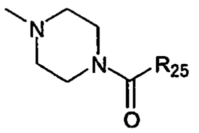 , 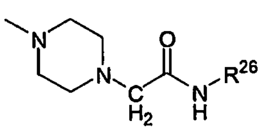 und 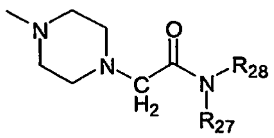 ,

und jeweils die übrigen ResteR$^1$ und $R^2$ separat und $R^3$-$R^{28}$ die vorstehend genannte Bedeutung haben, jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form entsprechender Salze, oder jeweils in Form entsprechender Solvate.

Weiterhin bevorzugt sind auch substituierte 5,6,7,8-Tetrahydro-imidazo[1,2-a]pyridin-2-ylamin-Verbindungen der vorstehend angegebenen allgemeinen Formel I, worin

$R^3$ für einen Wasserstoff-Rest oder für einen Alkyl-Rest ausgewählt aus der Gruppe bestehend aus Methyl, Ethyl, n-Propyl, iso-Propyl, n-Butyl, sec-Butyl, iso-Butyl, tert-Butyl und n-Pentyl steht,

und jeweils die übrigen Reste $R^1$, $R^2$ und $R^4$-$R^{28}$ die vorstehend genannte Bedeutung haben, jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form entsprechender Salze, oder jeweils in Form entsprechender Solvate.

Weiterhin bevorzugt sind auch substituierte 5,6,7,8-Tetrahydro-imidazo[1,2-a]pyridin-2-ylamin-Verbindungen der vorstehend angegebenen allgemeinen Formel I, worin

$R^4$ für einen Phenyl- oder Naphthyl-Rest steht, der jeweils mit 1, 2, 3, 4 oder 5 Substituenten unabhängig voneinander ausgewählt aus der Gruppe bestehend aus Methyl, Ethyl, n-Propyl, iso-Propyl, n-Butyl, iso-Butyl, sec-Butyl, tert-Butyl, F, Cl, Br, -CN, $-SF_5$, $-S-CF_3$, $-NH_2$, $-N(CH_3)_2$, $-N(C_2H_5)_2$, $-N(H)(CH_3)$, $-N(H)(C_2H_5)$, -SH, $-NO_2$, $-CF_3$, $-OCF_3$, -OH, $-O-CH_3$ und $-O-C_2H_5$ substituiert und/oder über eine $-(CH_2)-$, $-(CH_2)_2-$ oder $-(CH_2)_3$-Gruppe gebunden sein kann,

und jeweils die übrigen Reste $R^1$-$R^3$ und $R^5$-$R^{28}$ die vorstehend genannte Bedeutung haben, jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form entsprechender Salze, oder jeweils in Form entsprechender Solvate.

Weiterhin bevorzugt sind auch substituierte 5,6,7,8-Tetrahydro-imidazo[1,2-a]pyridin-2-ylamin-Verbindungen der vorstehend angegebenen allgemeinen Formel I, worin

$R^5$, $R^7$, $R^8$, $R^9$, $R^{10}$, $R^{11}$, $R^{12}$, $R^{13}$, $R^{14}$ und $R^{15}$, unabhängig voneinander, jeweils für einen Alkyl-Rest ausgewählt aus der Gruppe bestehend aus Methyl, Ethyl, n-Propyl, iso-Propyl, n-Butyl, sec-Butyl, iso-Butyl und tert-Butyl, wobei der Alkyl-Rest jeweils ggf. mit 1, 2, 3, 4 oder 5 Substituenten unabhängig voneinander ausgewählt aus der Gruppe bestehend aus F, Cl, Br, I, -CN, $-NO_2$, -OH, -SH und $-NH_2$ substituiert sein kann,

oder für einen Rest ausgewählt aus der Gruppe bestehend aus Phenyl, Naphthyl, (1,3)-Benzodioxolyl, (1,4)-Benzodioxanyl, Thiophenyl, Furanyl, Pyrrolyl, Pyrazolyl, Pyrazinyl, Pyranyl, Triazolyl, Pyridinyl, Imidazolyl, Indolyl, Isoindolyl, Benzo[b]furanyl, Benzo[b]thiophenyl, Thiazolyl, Oxazolyl, Isoxazolyl, Pyridazinyl, Pyrazinyl, Pyrimidinyl, Indazolyl, Chinazolinyl, Chinolinyl und Isochinolinyl, wobei der Rest jeweils über eine $-(CH_2)-$, $-(CH_2)_2-$ oder $-(CH_2)_3$-Gruppe gebunden und/oder ggf. mit 1, 2, 3, 4 oder 5 Substituenten unabhängig voneinander ausgewählt aus der Gruppe bestehend aus F, Cl, Br, I, -CN, $-NO_2$, -OH, -SH, Methyl, Ethyl, n-Propyl, iso-Propyl, n-Butyl, sec-Butyl, iso-Butyl, tert-Butyl, $-CF_3$, $-O-CF_3$, $-S-CF_3$, $-SF_5$, $-O-CH_3$, $-O-C_2H_5$, -O-Phenyl, -O-Benzyl, $-NH_2$, $-N(CH_3)_2$, $-N(C_2H_5)_2$, $-N(H)(CH_3)$, $-N(H)(C_2H_5)$, $-NH-C(=O)-O-CH_3$, $-NH-C(=O)-O-C_2H_5$ und $-NH-C(=O)-O-C(CH_3)_3$ substituiert sein kann, stehen,

und jeweils die übrigen Reste $R^1$-$R^4$, $R^6$ und $R^{16}$-$R^{28}$ die vorstehend genannte Bedeutung haben, jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form entsprechender Salze, oder jeweils in Form entsprechender Solvate.

Weiterhin bevorzugt sind auch substituierte 5,6,7,8-Tetrahydro-imidazo[1,2-a]pyridin-2-ylamin-Verbindungen der vorstehend angegebenen allgemeinen Formel I, worin

$R^6$ für einen Wasserstoff-Rest,
für einen Rest ausgewählt aus der Gruppe bestehend aus Methyl, Ethyl, n-Propyl, iso-Propyl, n-Butyl, sec-Butyl, iso-Butyl, tert-Butyl, n-Pentyl, sec-Pentyl, $-C(H)(CH_3)-C(H)(CH_3)_2$, $-(CH_2)-(CH_2)-(C(CH_3)_3)$, $-C(H)(CH_3)(O(C(CH_3)_3))$ und n-Hexyl, wobei der Rest jeweils ggf. mit 1, 2, 3, 4 oder 5 Substituenten unabhängig voneinander ausgewählt aus der Gruppe bestehend aus F, Cl, Br, I, -CN, $-NO_2$, -OH, -SH und $-NH_2$ substituiert sein kann,
oder für einen Rest ausgewählt aus der Gruppe bestehend aus Phenyl, Naphthyl, (1,3)-Benzodioxolyl, (1,4)-Benzodioxanyl, Thiophenyl, Furanyl, Pyrrolyl, Pyrazolyl, Pyrazinyl, Pyranyl, Triazolyl, Pyridinyl, Imidazolyl, Indolyl, Isoindolyl, Benzo[b]furanyl, Benzo[b]thiophenyl, Thiazolyl, Oxazolyl, Isoxazolyl, Pyridazinyl, Pyrazinyl, Pyrimidinyl, Indazolyl, Chinazolinyl, Chinolinyl und Isochinolinyl, wobei der Rest jeweils über eine $-(CH_2)-$, $-(CH_2)_2-$ oder $-(CH_2)_3$-Gruppe gebunden und/oder ggf. mit 1, 2, 3, 4 oder 5 Substituenten unabhängig voneinander ausgewählt aus der Gruppe bestehend aus F, Cl, Br, I, -CN, $-NO_2$, -OH, -SH, Methyl, Ethyl, n-Propyl, iso-Propyl, n-Butyl, sec-Butyl, iso-Butyl, tert-Butyl, $-CF_3$, $-O-CF_3$, $-S-CF_3$, $-SF_5$, $-O-CH_3$, $-O-C_2H_5$, -O-Phenyl, -O-Benzyl, $-NH_2$, $-N(CH_3)_2$, $-N(C_2H_5)_2$, $-N(H)(CH_3)$, $-N(H)(C_2H_5)$, $-NH-C(=O)-O-CH_3$, $-NH-C(=O)-O-C_2H_5$ und $-NH-C(=O)-O-C(CH_3)_3$ substituiert sein kann, steht,
und jeweils die übrigen Reste $R^1$-$R^5$ und $R^7$-$R^{28}$ die vorstehend genannte Bedeutung haben, jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form entsprechender Salze, oder jeweils in Form entsprechender Solvate.

Weiterhin bevorzugt sind auch substituierte 5,6,7,8-Tetrahydro-imidazo[1,2-a]pyridin-2-ylamin-Verbindungen der vorstehend angegebenen allgemeinen Formel I, worin
$R^{16}$, $R^{17}$ und $R^{18}$, unabhängig voneinander, jeweils für einen Wasserstoff-Rest,
für einen Rest ausgewählt aus der Gruppe bestehend aus Methyl, Ethyl, n-Propyl, iso-Propyl, n-Butyl, sec-Butyl, iso-Butyl, tert-Butyl, n-Pentyl, sec-Pentyl, $-C(H)(CH_3)-C(H)(CH_3)_2$, $-(CH_2)-(CH_2)-(C(CH_3)_3)$, $-(CH_2)-O-(CH_3)$ und n-Hexyl, wobei der Rest jeweils ggf. mit 1, 2, 3, 4 oder 5 Substituenten unabhängig voneinander ausgewählt aus der Gruppe bestehend aus F, Cl, Br, I, -CN, $-NO_2$, -OH, -SH und $-NH_2$ substituiert sein kann,
oder für einen Rest ausgewählt aus der Gruppe bestehend aus Phenyl und Naphthyl, wobei der Rest jeweils mit 1, 2, 3, 4 oder 5 Substituenten unabhängig voneinander ausgewählt aus der Gruppe bestehend aus F, Cl, Br, I, -CN, $-NO_2$, -OH, -SH, Methyl, Ethyl; n-Propyl, iso-Propyl, n-Butyl, sec-Butyl, iso-Butyl, tert-Butyl, $-CF_3$, $-O-CF_3$, $-S-CF_3$; $-SF_5$, $-O-CH_3$, $-O-C_2H_5$, -O-Phenyl, -O-Benzyl, $-NH_2$, $-N(CH_3)_2$, $-N(C_2H_5)_2$, $-N(H)(CH_3)$, $-N(H)(C_2H_5)$, $-NH-C(=O)-O-CH_3$, $-NH-C(=O)-O-C_2H_5$ und $-NH-C(=O)-O-C(CH_3)_3$ substituiert sein kann, stehen,
und jeweils die übrigen Reste $R^1$-$R^{15}$ und $R^{19}$-$R^{28}$ die vorstehend genannte Bedeutung haben, jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form entsprechender Salze, oder jeweils in Form entsprechender Solvate.

Weiterhin bevorzugt sind auch substituierte 5,6,7,8-Tetrahydro-imidazo[1,2-a]pyridin-2-ylamin-Verbindungen der vorstehend angegebenen allgemeinen Formel I, worin
$R^{19}$ für einen (hetero)cycloaliphatischen Rest ausgewählt aus der Gruppe bestehend aus Cyclopropyl, Cyclobutyl, Cyclopentyl, Cyclohexyl, Cycloheptyl, Cyclopentenyl, Cyclohexenyl, Cycloheptenyl, Imidazolidinyl, Tetrahydrofuranyl, Tetrahydrothiophenyl, Pyrrolidinyl, Piperidinyl, Morpholinyl, Piperazinyl, Thiomorpholinyl, Tetrahydropyranyl, Azepanyl, Diazepanyl, Dithiolanyl, (6,6)-Dimethyl-[3.1.1]-bicycloheptyl, Adamantyl (Tricyclo-[3.3.1.1$^{3,7}$]-decanyl), Indanyl, Indenyl, (1,4)-Benzodioxanyl, (1,2,3,4)-Tetrahydronaphthyl, (1,2,3,4)-Tetrahydrochinolinyl und (1,2,3,4)-Tetrahydrochinazolinyl, wobei der (hetero)cycloaliphatische Rest jeweils ggf. mit 1, 2, 3, 4 oder 5 Substituenten unabhängig voneinander ausgewählt aus der Gruppe bestehend aus F, Cl, Br, I, -CN, $-CF_3$, $-SF_5$, -OH, $-NH_2$, $-O-CF_3$, -SH, $-O-CH_3$, $-O-C_2H_5$, Methyl, Ethyl, n-Propyl, iso-Propyl, n-Butyl, sec-Butyl, iso-Butyl, tert-Butyl, $-S-CH_3$, $-S-C_2H_5$, $-C(=O)-O-CH_3$, $-C(=O)-O-C_2H_5$, Oxo (=O), $-N(CH_3)_2$, $-N(C_2H_5)_2$, $-N(H)(CH_3)$, $-N(H)(C_2H_5)$, $-NO_2$, $-SCF_3$, -C(=O)-OH, $-(CH_2)$-Benzo[b]furanyl, -O-Phenyl, -O-Benzyl, Phenyl und Benzyl substituiert sein kann,
oder für einen Rest ausgewählt aus der Gruppe bestehend aus Phenyl, Naphthyl, (1,3)-Benzodioxolyl, (1,4)-Benzodioxanyl, Thiophenyl, Furanyl, Pyrrolyl, Pytazolyl, Pyrazinyl, Pyranyl, Triazolyl, Pyridinyl, Imidazolyl, Indolyl, Isoindolyl, Benzo[b]furanyl, Benzo[b]thiophenyl, Thiazolyl, Oxazolyl, Isoxazolyl, Pyridazinyl, Pyrazinyl, Pyrimidinyl, Indazolyl, Chinazolinyl, Chinolinyl und Isochinolinyl, wobei der Rest jeweils ggf. mit 1, 2, 3, 4 oder 5 Substituenten unabhängig voneinander ausgewählt aus der Gruppe bestehend aus F, Cl, Br, I, -CN, $-NO_2$, -OH, -SH, Methyl, Ethyl, n-Propyl, iso-Propyl, n-Butyl, sec-Butyl, iso-Butyl, tert-Butyl, $-CF_3$, $-O-CF_3$, $-S-CF_3$, $-SF_5$, $-O-CH_3$, $-O-C_2H_5$, -O-Phenyl, -O-Benzyl, $-NH_2$, $-N(CH_3)_2$, $-N(C_2H_5)_2$, $-N(H)(CH_3)$, $-N(H)(C_2H_5)$, $-NH-C(=O)-O-CH_3$, $-NH-C(=O)-O-C_2H_5$ und $-NH-C(=O)-O-C(CH_3)_3$ substituiert sein kann, steht,
und jeweils die übrigen Reste $R^1$-$R^{18}$ und $R^{20}$-$R^{28}$ die vorstehend genannte Bedeutung haben, jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form entsprechender Salze, oder jeweils in Form entsprechender Solvate.

Weiterhin bevorzugt sind auch substituierte 5,6,7,8-Tetrahydro-imidazo[1,2-a]pyridin-2-ylamin-Verbindungen

der vorstehend angegebenen allgemeinen Formel I, worin
$R^{20}$ für einen Alkyl-Rest ausgewählt aus der Gruppe bestehend aus Methyl, Ethyl, n-Propyl, iso-Propyl, n-Butyl, iso-Butyl, sec-Butyl, tert-Butyl und n-Pentyl,
für einen (hetero)cycloaliphatischen Rest ausgewählt aus der Gruppe bestehend aus Cyclopentyl, Cyclohexyl, Piperidinyl und Cycloheptyl, wobei der (hetero)cycloaliphatische Rest jeweils ggf. mit 1, 2, 3, 4 oder 5 Substituenten unabhängig voneinander ausgewählt aus der Gruppe bestehend aus F, Cl, Br, I, -CN, $-CF_3$, $-SF_5$, -OH, $-NH_2$, $-O-CF_3$, -SH, $-O-CH_3$, $-O-C_2H_5$, Methyl, Ethyl, n-Propyl, iso-Propyl, n-Butyl, sec-Butyl, iso-Butyl und tert-Butyl substituiert sein kann,
oder für einen Rest ausgewählt aus der Gruppe bestehend aus Phenyl, Naphthyl, (1,3)-Benzodioxolyl, Thiophenyl, Furanyl, Pyridinyl, Imidazolyl, Indolyl, Benzo[b]furanyl, Benzo[b]thiophenyl, Thlazolyl, Oxazolyl, Isoxazolyl, Pyridazinyl, Pyrazinyl, Pyrimidinyl und Chinolinyl, wobei der Rest jeweils ggf. mit 1, 2, 3, 4 oder 5 Substituenten unabhängig voneinander ausgewählt aus der Gruppe bestehend aus Methyl, Ethyl, n-Propyl, iso-Propyl, n-Butyl, iso-Butyl, sec-Butyl, tert-Butyl, F, Cl, Br, -CN, $-SF_5$, $-O-CF_3$, $-S-CF_3$, $-NH_2$, $-N(CH_3)_2$, $-N(C_2H_5)_2$, $-N(H)(CH_3)$, $-N(H)(C_2H_5)$, -SH, $-NO_2$, $-CF_3$, $-OCF_3$, -OH, $-O-CH_3$ und $-O-C_2H_5$ substituiert sein kann, steht,
und jeweils die übrigen Reste $R^1$-$R^{19}$ und $R^{21}$-$R^{28}$ die vorstehend genannte Bedeutung haben, jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form entsprechender Salze, oder jeweils in Form entsprechender Solvate.

Weiterhin bevorzugt sind auch substituierte 5,6,7,8-Tetrahydro-imidazo[1,2-a]pyridin-2-ylamin-Verbindungen der vorstehend angegebenen allgemeinen Formel I, worin
$R^{21}$ für einen Wasserstoff-Rest
oder für einen Phenyl- oder Naphthyl-Rest, der mit 1, 2 oder 3 Substituenten unabhängig voneinander ausgewählt aus der Gruppe bestehend aus Methyl, Ethyl, n-Propyl, iso-Propyl, n-Butyl, iso-Butyl, sec-Butyl, tert-Butyl, F, Cl und Br substituiert sein kann, steht,
und jeweils die übrigen Reste $R^1$-$R^{20}$ und $R^{22}$-$R^{28}$ die vorstehend genannte Bedeutung haben, jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form entsprechender Salze, oder jeweils in Form entsprechender Solvate.

Weiterhin bevorzugt sind auch substituierte 5,6,7,8-Tetrahydro-imidazo[1,2-a]pyridin-2-ylamin-Verbindungen der vorstehend angegebenen allgemeinen Formel I, worin
$R^{22}$ und $R^{23}$, unabhängig voneinander, jeweils für einen (hetero)cycloaliphatischen Rest ausgewählt aus der Gruppe bestehend aus Pyrrolidinyl, Morpholinyl und Thiomorpholinyl, wobei der (hetero)cycloaliphatische Rest jeweils ggf. mit 1, 2, 3, 4 oder 5 Substituenten unabhängig voneinander ausgewählt aus der Gruppe bestehend aus F, Cl, Br, I, -CN, $-CF_3$, $-SF_5$, -OH, $-NH_2$, $-O-CF_3$, -SH, $-O-CH_3$, $-O-C_2H_5$, Methyl, Ethyl, n-Propyl, iso-Propyl, n-Butyl, sec-Butyl, iso-Butyl und tert-Butyl substituiert sein kann,
oder für einen Rest ausgewählt aus der Gruppe bestehend aus Phenyl, Naphthyl, (1,3)-Benzodioxolyl, Thiophenyl, Furanyl, Pyridinyl, Imidazolyl, Indolyl, Benzo[b]furanyl, Benzo[b]thiophenyl, Thiazolyl, Oxazolyl, Isoxazolyl, Pyridazinyl, Pyrazinyl, Pyrimidinyl und Chinolinyl, wobei der Rest jeweils ggf. mit 1, 2, 3, 4 oder 5 Substituenten unabhängig voneinander ausgewählt aus der Gruppe bestehend aus Methyl, Ethyl, n-Propyl, iso-Propyl, n-Butyl, iso-Butyl, sec-Butyl, tert-Butyl, F, Cl, Br, -CN, $-SF_5$, $-O-CF_3$, $-S-CF_3$, $-NH_2$, $-N(CH_3)_2$, $-N(C_2H_5)_2$, $-N(H)(CH_3)$, $-N(H)(C_2H_5)$, -SH, $-NO_2$, $-CF_3$, $-OCF_3$, -OH, $-O-CH_3$ und $-O-C_2H_5$ substituiert sein kann, stehen,
und jeweils die übrigen Reste $R^1$-$R^{21}$ und $R^{24}$-$R^{28}$ die vorstehend genannte Bedeutung haben, jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form entsprechender Salze, oder jeweils in Form entsprechender Solvate.

Weiterhin bevorzugt sind auch substituierte 5,6,7,8-Tetrahydro-imidazo[1,2-a]pyridin-2-ylamin-Verbindungen der vorstehend angegebenen allgemeinen Formel I, worin
$R^{24}$ für einen Alkyl-Rest ausgewählt aus der Gruppe bestehend aus Methyl, Ethyl, n-Propyl, iso-Propyl, n-Butyl, iso-Butyl, sec-Butyl und tert-Butyl steht,
$R^{25}$ für einen Rest ausgewählt aus der Gruppe bestehend aus Phenyl, Naphthyl, Furanyl, Pyrazinyl und Pyrimidinyl, der über eine $-(CH_2)-$, $-(CH_2)_2-$ oder $-(CH_2)_3-$Gruppe gebunden und/oder mit 1, 2 oder 3 Substituenten unabhängig voneinander ausgewählt aus der Gruppe bestehend aus Methyl, Ethyl, n-Propyl und iso-Propyl substituiert sein kann, steht,
$R^{26}$ für einen Alkyl-Rest ausgewählt aus der Gruppe bestehend aus Methyl, Ethyl, n-Propyl und iso-Propyl steht,
$R^{27}$ für einen Alkyl-Rest ausgewählt aus der Gruppe bestehend aus Methyl, Ethyl, iso-Propyl und n-Propyl oder für einen Phenyl-Rest steht,
und
$R^{28}$ für einen Alkyl-Rest ausgewählt aus der Gruppe bestehend aus Methyl, Ethyl, iso-Propyl und n-Propyl oder für einen Phenyl-Rest steht,
und jeweils die übrigen Reste $R^1$-$R^{23}$ die vorstehend genannte Bedeutung haben, jeweils ggf. in Form eines ihrer reinen

Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form entsprechender Salze, oder jeweils in Form entsprechender Solvate.

Besonders bevorzugt sind substituierte 5,6,7,8-Tetrahydro-imidazo[1,2-a]pyridin-2-ylamin-Verbindungen der vorstehend angegebenen allgemeinen Formel I, worin

$R^1$ für $-C(=O)-OR^5$; $-(CHR^6)-C(=O)OR^7$; $-C(=O)-NHR^{11}$; $-(CH_2)-C(=O)-NHR^{11}$; $-(CHR^{16})-R^{19}$; $-(CHR^{16})-(CHR^{17})-R^{19}$; $-(CHR^{16})-(CHR^{17})-O-R^{19}$; $-(CHR^{16})-(CHR^{17})-(CHR^{18})-R^{19}$; $-(CHR^{16})-(CHR^{17})-S-(CHR^{18})-R^{19}$; $-(CHR^{16})-(CHR^{17})-(CHR^{18})-N(CH_3)-R^{19}$,
für einen ggf. substituierten Alkyl-Rest ausgewählt aus der Gruppe bestehend aus Methyl, Ethyl, n-Propyl, iso-Propyl, $-CH_2-CH_2-CN$, n-Butyl, sec-Butyl, iso-Butyl, tert-Butyl, $-CH(CH_3)-CH(CH_3)_2$ und $-CH_2-CH_2-C(CH_3)_3$,
für einen Alkenyl-Rest ausgewählt aus der Gruppe bestehend aus 1-Propenyl und 2-Propenyl,
für einen Rest ausgewählt aus der Gruppe bestehend aus Cyclopentyl, Cyclohexyl, Cycloheptyl, (6,6)-Dimethyl-[3.1.1]-bicycloheptyl, Indanyl und Indenyl, wobei der Rest jeweils mit 1, 2 oder 3 Substituenten unabhängig voneinander ausgewählt aus der Gruppe bestehend aus F, Cl, Br, Methyl, Ethyl, iso-Propyl, n-Propyl und -O-Benzyl substituiert sein kann,
für einen Pyrrolidinyl-Rest, der mit einem $-(CH_2)$-Benzo[b]furanyl- oder Benzyl-Rest substituiert sein kann, wobei der zyklische Teil des Benzyl-Rests jeweils mit 1, 2 oder 3 Substituenten unabhängig voneinander ausgewählt aus der Gruppe bestehend aus F, Cl, Br, -OH, Methyl, Ethyl, iso-Propyl, n-Propyl, $-CF_3$, $-O-CH_3$, $-O-C_2H_5$, Phenyl und -O-Benzyl substituiert sein kann,
oder für einen Rest ausgewählt aus der Gruppe bestehend aus Phenyl, Naphthyl, (1,3)-Benzodioxolyl und (1,4)-Benzodioxanyl steht, wobei der Rest jeweils mit 1, 2 oder 3 Substituenten unabhängig voneinander ausgewählt aus der Gruppe bestehend aus F, Cl, Br, I, Methyl, Ethyl, n-Propyl, iso-Propyl, n-Butyl, sec-Butyl, iso-Butyl, tert-Butyl, -O-Phenyl, -O-Benzyl, $-NH-C(=O)-O-CH_3$, $-NH-C(=O)-O-C_2H_5$ und $-NH-C(=O)-O-C(CH_3)_3$ substituiert sein kann;

$R^2$ für einen Wasserstoff-Rest,
$-(CHR^{16})-R^{19}$,
oder für einen Alkyl-Rest ausgewählt aus der Gruppe bestehend aus Methyl, Ethyl, n-Propyl, iso-Propyl, n-Butyl, sec-Butyl, iso-Butyl und tert-Butyl steht;
oder

$R^1$ und $R^2$ zusammen mit dem sie verbindenden Stickstoffatom als Ringglied einen Rest bilden, der ausgewählt ist aus der Gruppe bestehend aus

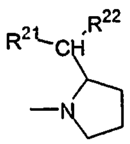 , 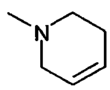 , 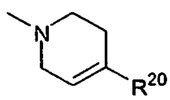 , 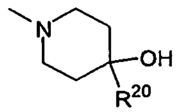 ,

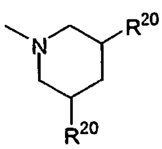 , 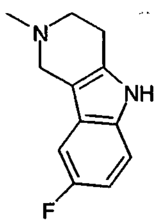 , 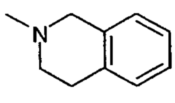 , 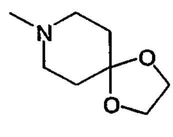 ,

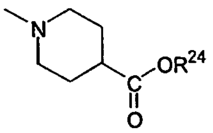 , 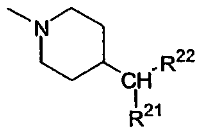 , 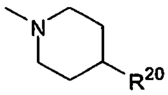 , 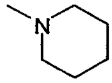 ,

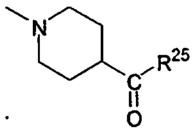 , 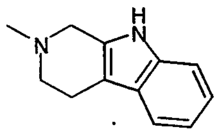 , 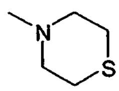 , 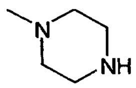 ,

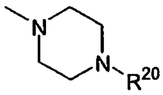 , 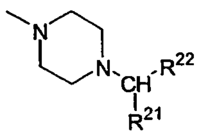 , 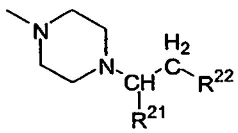 ,

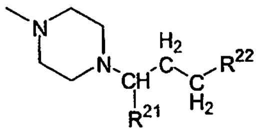 , 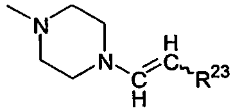 , 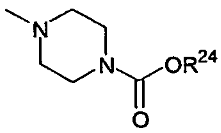 ,

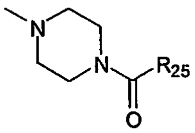 , 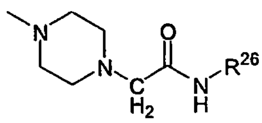 und 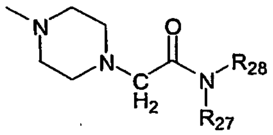 ,

$R^3$ für einen Alkyl-Rest ausgewählt aus der Gruppe bestehend aus Methyl, Ethyl, n-Propyl, iso-Propyl, n-Butyl, sec-Butyl, iso-Butyl und tert-Butyl;

$R^4$ für einen Rest steht, der ausgewählt ist aus der Gruppe bestehend aus

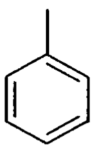 , 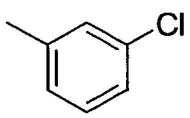 , 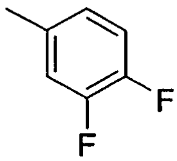 , 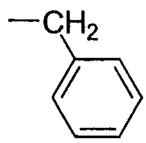

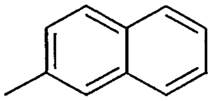 und 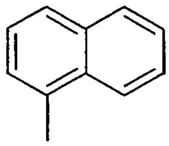 ,

$R^5$, $R^7$ und $R^{11}$, unabhängig voneinander, jeweils für einen Alkyl-Rest ausgewählt aus der Gruppe bestehend aus Methyl, Ethyl, n-Propyl, iso-Propyl, n-Butyl, sec-Butyl, iso-Butyl und tert-Butyl; oder für einen Benzyl- oder Naphthyl-Rest stehen;

$R^6$ für einen Wasserstoff-Rest, für einen Rest ausgewählt aus der Gruppe bestehend aus Methyl, Ethyl, n-Propyl, iso-Propyl, n-Butyl, sec-Butyl, iso-Butyl, tert-Butyl, n-Pentyl, sec-Pentyl, neo-Pentyl und $-C(H)(CH_3)(O(C(CH_3)_3))$, oder für einen Indolyl-Rest, der über eine $-(CH_2)$-Gruppe gebunden ist, steht;

$R^{16}$ für einen Wasserstoff-Rest, für einen Rest ausgewählt aus der Gruppe bestehend aus Methyl, Ethyl, iso-Propyl, n-Propyl, n-Butyl, sec-Butyl, iso-Butyl, tert.-Butyl und $-(CH_2)-O-(CH_3)$, oder für einen Phenyl-Rest steht;

$R^{17}$ für einen Wasserstoff-Rest, für einen Alkyl-Rest ausgewählt aus der Gruppe bestehend aus Methyl, Ethyl, iso-Propyl, n-Propyl, n-Butyl, iso-Butyl, sec-Butyl und tert.-Butyl oder für einen Phenyl-Rest steht;

$R^{18}$ für einen Wasserstoff-Rest oder für einen Phenyl-Rest steht;

$R^{19}$ für einen (hetero)cycloaliphatischen Rest ausgewählt aus der Gruppe bestehend aus Cyclopentyl, Cyclohexyl, Cycloheptyl, Tetrahydrofuranyl, Tetrahydrothiophenyl, Pyrrolidinyl, Piperidinyl, Adamantyl (Tricyclo-[3.3.1.1$^{3,7}$]-decanyl) und (1,4)-Benzodioxanyl, wobei der (hetero)cycloaliphatische Rest jeweils mit 1 oder 2 Substituenten unabhängig voneinander ausgewählt aus der Gruppe bestehend Methyl, Ethyl, iso-Propyl und n-Propyl substituiert sein kann, oder für einen Rest ausgewählt aus der Gruppe bestehend aus Phenyl, Naphthyl, (1,3)-Benzodioxolyl, (1,4)-Benzodioxanyl, Thiophenyl, Furanyl, Pyridinyl, Imidazolyl, Indolyl und Isoindolyl, wobei der Rest jeweils ggf. mit 1, 2, 3, 4 oder 5 Substituenten unabhängig voneinander ausgewählt aus der Gruppe bestehend aus F, Cl, Br, I, Methyl, Ethyl, n-Propyl, iso-Propyl, n-Butyl, sec-Butyl, iso-Butyl, tert-Butyl, $-CF_3$, $-O-CH_3$, $-O-C_2H_5$, $-O-CF_3$, -O-Phenyl, -O-Benzyl, $-NH_2$, $-N(CH_3)_2$, $-N(C_2H_5)_2$, $-N(H)(CH_3)$ und $-N(H)(C_2H_5)$ substituiert sein kann, steht;

$R^{20}$ für einen Methyl- oder Ethyl-Rest, für einen (hetero)cycloaliphatischen Rest ausgewählt aus der Gruppe bestehend aus Cyclopentyl, Cyclohexyl, Piperidinyl und Cycloheptyl, oder für einen Phenyl- oder Pyridinyl-Rest, der mit der mit 1, 2 oder 3 Substituenten unabhängig voneinander ausgewählt aus der Gruppe bestehend aus Methyl, Ethyl, n-Propyl, iso-Propyl, n-Butyl, iso-Butyl, sec-Butyl, tert-Butyl, F, Cl, Br, $-CF_3$, -OH, $-O-CH_3$ und $-O-C_2H_5$ substituiert sein kann, steht;

$R^{21}$ für einen Wasserstoff-Rest oder für einen Phenyl-Rest, der mit 1, 2 oder 3 Substituenten unabhängig voneinander ausgewählt aus der Gruppe bestehend aus Methyl, Ethyl, n-Propyl, iso-Propyl, n-Butyl, iso-Butyl, sec-Butyl, tert-Butyl, F, Cl und Br substituiert sein kann, steht;

$R^{22}$ für einen Pyrrolidinyl- oder Morpholinyl-Rest oder für einen Rest ausgewählt aus der Gruppe bestehend aus Phenyl, Naphthyl, (1,3)-Benzodioxolyl, Thiophenyl, Benzo[b]furanyl, Benzo[b]thiophenyl und Chinolinyl, wobei der Rest jeweils mit 1, 2 oder 3 Substituenten unabhängig voneinander ausgewählt aus der Gruppe bestehend aus Methyl, Ethyl,

n-Propyl, F, Cl, Br, -OH, $-O-CH_3$ und $-O-C_2H_5$ substituiert sein kann, steht;

$R^{23}$ für einen Phenyl-Rest steht;

$R^{24}$ für einen Alkyl-Rest ausgewählt aus der Gruppe bestehend aus Methyl, Ethyl, n-Propyl, iso-Propyl, n-Butyl, iso-Butyl, sec-Butyl und tert-Butyl steht;

$R^{25}$ für einen Rest ausgewählt aus der Gruppe bestehend aus Phenyl, Naphthyl, Furanyl, Pyrazinyl und Pyrimidinyl steht, der über eine $-(CH_2)$-Gruppe gebunden und/oder mit 1, 2 oder 3 Substituenten unabhängig voneinander ausgewählt aus der Gruppe bestehend aus Methyl, Ethyl, n-Propyl und iso-Propyl substituiert sein kann;

$R^{26}$ für einen Alkyl-Rest ausgewählt aus der Gruppe bestehend aus Methyl, Ethyl, n-Propyl und iso-Propyl steht;

$R^{27}$ für einen Alkyl-Rest ausgewählt aus der Gruppe bestehend aus Methyl, Ethyl, iso-Propyl und n-Propyl steht
und

$R^{28}$ für einen Phenyl-Rest steht;

jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form entsprechender Salze, oder jeweils in Form entsprechender Solvate.

Ganz besonders bevorzugt sind substituierte 5,6,7,8-Tetrahydro-imidazo[1,2-a]pyridin-2-ylamin Verbindungen der vorstehend angegebenen allgemeinen Formel I, worin

$R^1$ für $-C(=O)-O-CH_3$, $-C(=O)-O-C_2H_5$,
für einen Rest ausgewählt aus der Gruppe bestehend aus

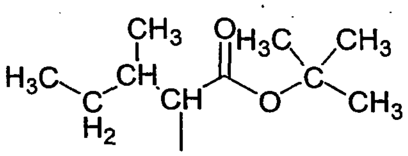 , 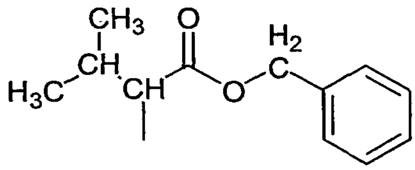 ,

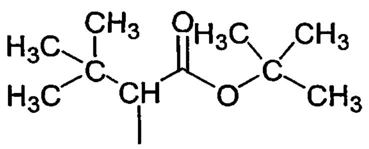 , 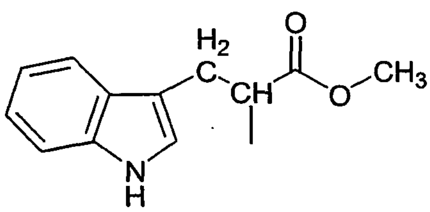 ,

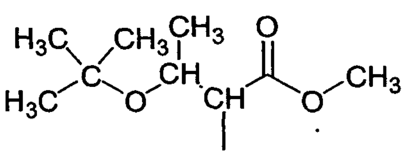 und 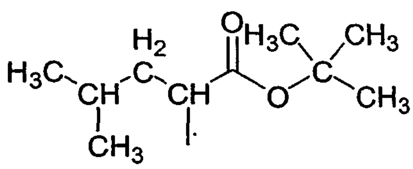 ,

für den folgenden Rest

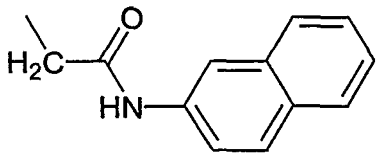,

für einen ggf. substituierten Alkyl-Rest ausgewählt aus der Gruppe bestehend aus Methyl, Ethyl, n-Propyl, iso-Propyl, $-CH_2-CH_2-CN$, n-Butyl, sec-Butyl, iso-Butyl, tert-Butyl, $-CH(CH_3)-CH(CH_3)_2$ und $-CH_2-CH_2-C(CH_3)_3$,
für einen Alkenyl-Rest ausgewählt aus der Gruppe bestehend aus 1-Propenyl und 2-Propenyl,
für einen cycloaliphatischen Rest ausgewählt aus der Gruppe bestehend aus Cyclopentyl, Cyclohexyl, (6,6)-Dimethyl-[3.1.1]-bicycloheptyl, Adamantyl (Tricyclo-[3.3.1.1$^{3,7}$]-decanyl), Indanyl und Indenyl, wobei der cycloaliphatische Rest jeweils mit 1, 2 oder 3 Substituenten unabhängig voneinander ausgewählt aus der Gruppe bestehend aus F, Cl, Br, Methyl, Ethyl, n-Propyl und -O-Benzyl substituiert sein kann,
für einen Pyrrolidinyl-Rest, der mit einem $-(CH_2)$-Benzo[b]furanyl- oder Benzyl-Rest substituiert sein kann, wobei der zyklische Teil des Benzyl-Rests mit 1, 2 oder 3 Substituenten unabhängig voneinander ausgewählt aus der Gruppe bestehend aus F, Cl, Br, -OH, $-CF_3$, $-O-CH_3$, $-O-C_2H_5$, Phenyl und -O-Benzyl substituiert sein kann,
für einen Rest ausgewählt aus der Gruppe bestehend aus Phenyl, Naphthyl, (1,3)-Benzodioxolyl und (1,4)-Benzodioxanyl, wobei der Rest jeweils mit 1, 2 oder 3 Substituenten unabhängig voneinander ausgewählt aus der Gruppe bestehend aus F, Cl, Br, I, Methyl, Ethyl, n-Propyl, iso-Propyl, n-Butyl, sec-Butyl, iso-Butyl, tert-Butyl, -O-Phenyl, -O-Benzyl, $-NH-C(=O)-O-CH_3$, $-NH-C(=O)-O-C_2H_5$ und $-NH-C(=O)-O-C(CH_3)_3$ substituiert sein kann,
für einen Rest ausgewählt aus der Gruppe bestehend aus

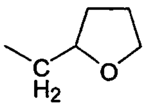, 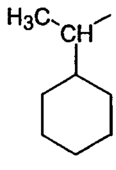, 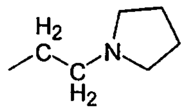,

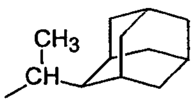, 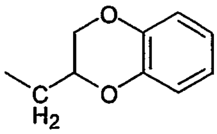 und 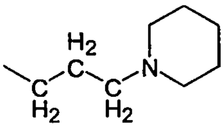,

wobei jeweils der (hetero)cycloaliphatische Teil mit 1 oder 2 Substituenten unabhängig voneinander ausgewählt aus der Gruppe bestehend Methyl, Ethyl, iso-Propyl und n-Propyl substituiert sein kann,
oder für einen Rest ausgewählt aus der Gruppe bestehend aus

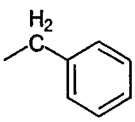, 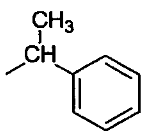, 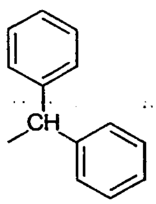, 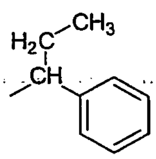, 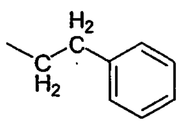,

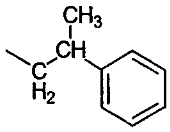 , 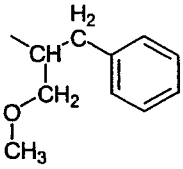 , 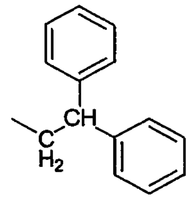 , 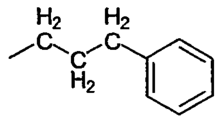 ,

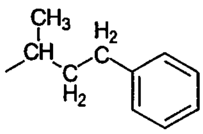 , 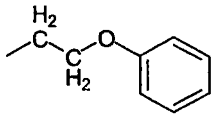 , 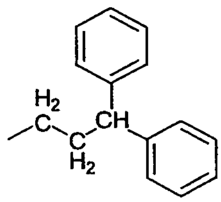 , 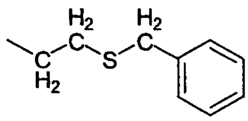 ,

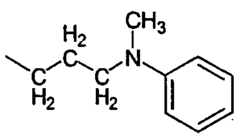 , 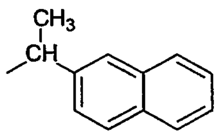 , 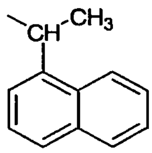 , 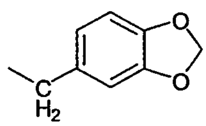 ,

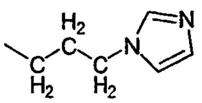 , 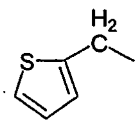 , 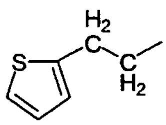 , 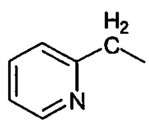 ,

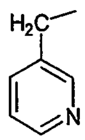 , 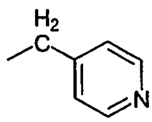 , 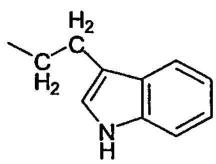 und 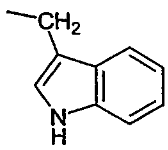 ,

wobei jeweils der (hetero)aromatische Teil mit 1, 2, 3, 4 oder 5 Substituenten unabhängig voneinander ausgewählt aus der Gruppe bestehend aus F, Cl, Br, I, Methyl, Ethyl, n-Propyl, iso-Propyl, n-Butyl, sec-Butyl, iso-Butyl, tert-Butyl, $-CF_3$, $-O-CH_3$, $-O-C_2H_5$, $-O-CF_3$, -O-Phenyl, -O-Benzyl, $-NH_2$, $-N(CH_3)_2$, $-N(C_2H_5)_2$, $-N(H)(CH_3)$ und $-N(H)(C_2H_5)$ substituiert sein kann, steht

$R^2$ für einen Wasserstoff-Rest,
für einen Benzyl-Rest, der 1-, 2- oder 3-fach mit $-O-CH_3$ substituiert sein kann,
oder für einen Alkyl-Rest ausgewählt aus der Gruppe bestehend aus Methyl, Ethyl, n-Propyl, iso-Propyl, n-Butyl, sec-Butyl, iso-Butyl, tert-Butyl steht, oder

$R^1$ und $R^2$ zusammen mit dem sie verbindenden Stickstoffatom als Ringglied einen Rest bilden ausgewählt aus der Gruppe aus

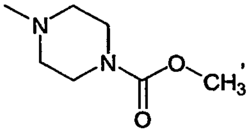, 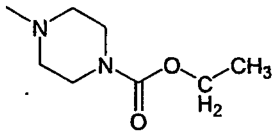, 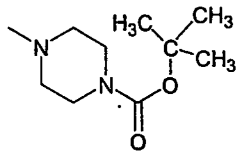,

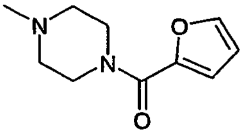, 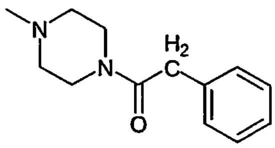, 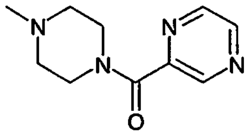,

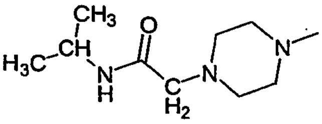, 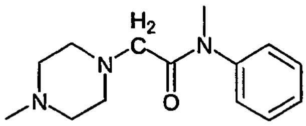, 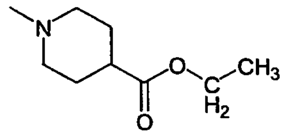

und

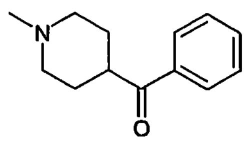,

wobei jeweils ggf. der (hetero)aromatische Teil der vorstehend genannten Reste mit 1, 2 oder 3 Substituenten unabhängig voneinander ausgewählt aus der Gruppe bestehend aus Methyl, Ethyl, n-Propyl und iso-Propyl substituiert sein kann,
oder $R^1$ und $R^2$ zusammen mit dem sie verbindenden Stickstoffatom als Ringglied einen Rest bilden ausgewählt aus der folgenden Gruppe

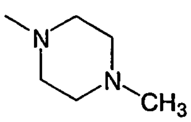, 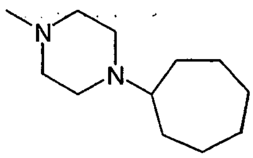, 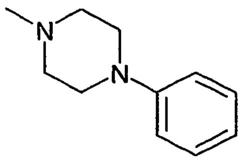, 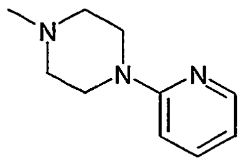

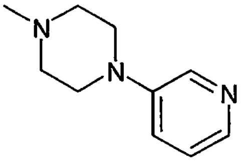, 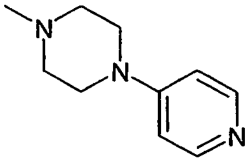, 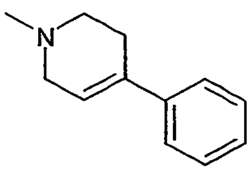,

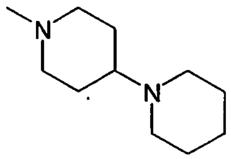 , 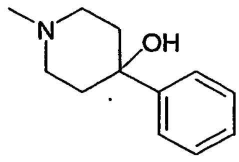 und 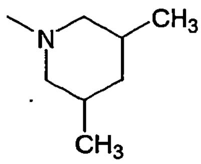 ,

wobei jeweils der (hetero)aromatische Teil der vorstehend genannten Reste mit 1, 2 oder 3 Substituenten unabhängig voneinander ausgewählt aus der Gruppe bestehend aus Methyl, Ethyl, n-Propyl, iso-Propyl, n-Butyl, iso-Butyl, sec-Butyl, tert-Butyl, F, Cl, Br, $-CF_3$, -OH, $-O-CH_3$ und $-O-C_2H_5$ substituiert sein kann,
oder $R^1$ und $R^2$ zusammen mit dem sie verbindenden Stickstoffatom als Ringglied einen Rest bilden ausgewählt aus der Gruppe bestehend aus

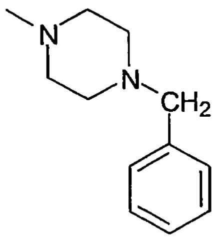 , 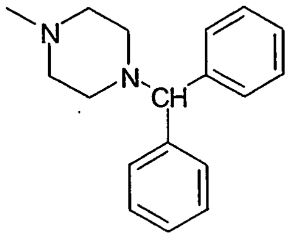 , 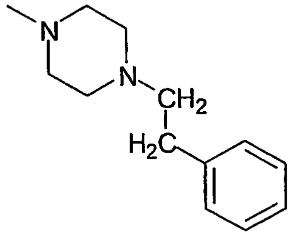 ,

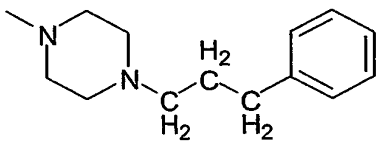 , 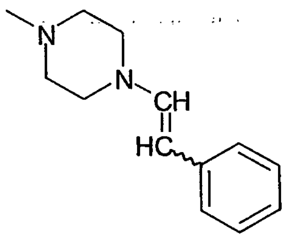 , 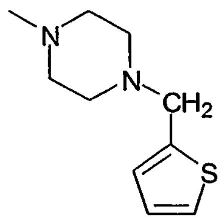

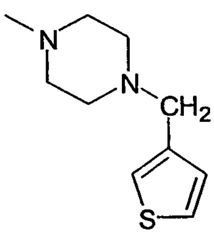 , 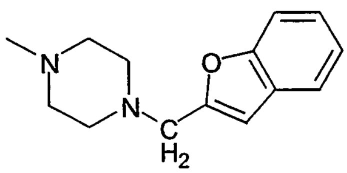 , 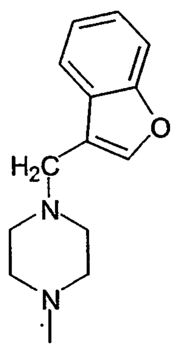 ,

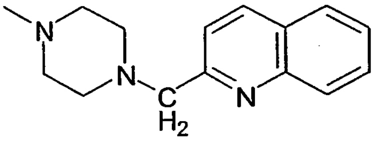 , 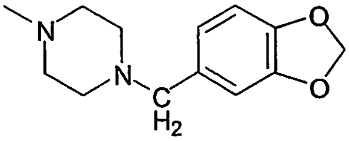 , 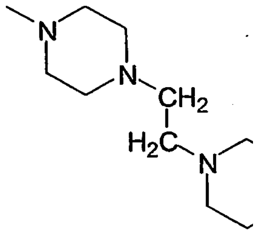

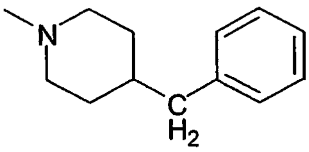 und 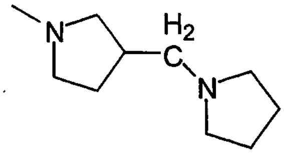 ,

wobei jeweils der (hetero)aromatische Teil der vorstehend genannten Reste mit 1, 2 oder 3 Substituenten unabhängig voneinander ausgewählt aus der Gruppe bestehend aus Methyl, Ethyl, n-Propyl, F, Cl, Br, -OH, $-O-CH_3$ und $-O-C_2H_5$ substituiert sein kann,

oder $R^1$ und $R^2$ zusammen mit dem sie verbindenden Stickstoffatom als Ringglied einen Rest bilden ausgewählt aus der Gruppe bestehend aus

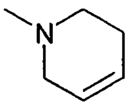 , 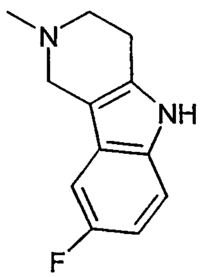 , 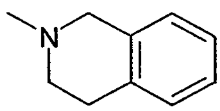 , 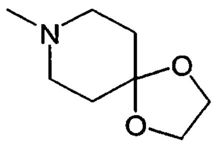 ,

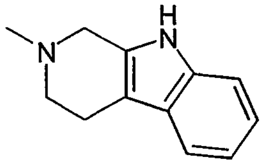 , 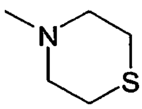 , 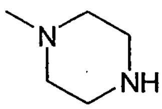 und 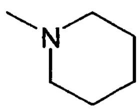

$R^3$ für einen Alkyl-Rest ausgewählt aus der Gruppe bestehend aus Methyl, Ethyl, n-Propyl, iso-Propyl, n-Butyl, sec-Butyl, iso-Butyl und tert-Butyl steht;

$R^4$ für einen Rest steht, der ausgewählt ist aus der Gruppe bestehend aus

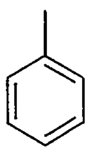 , 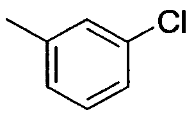 , 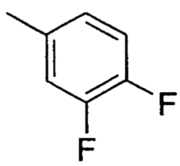 , 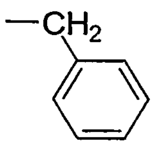

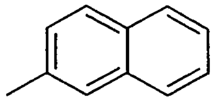 und 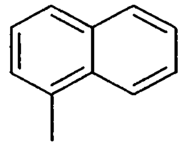 ,

jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form entsprechender Salze, oder jeweils in Form entsprechender Solvate.

Noch weiter bevorzugt sind substituierte 5,6,7,8-Tetrahydro-imidazo[1,2-a]pyridin-2-ylamin-Verbindungen der allgemeinen Formel I ausgewählt aus der Gruppe bestehend aus

[1] 2-[(3-Chlor-benzoyl)-methyl-amino]-5,6,7,8-tetrahydro-imidazo[1,2-a]pyridin-3-carbonsäure (2-thiophen-2-yl-ethyl)-amid,

[2] 2-[Butyl-(3-chlor-benzoyl)-amino]-5,6,7,8-tetrahydro-imidazo[1,2-a]pyridin-3-carbonsäure [2-(2,5-dimethoxy-phenyl)-ethyl]-amid,

[3] N-{3-[4-(2-Ethyl-phenyl)-piperazin-1-carbonyl]-5,6,7,8-tetrahydro-imidazo[1,2-a]pyridin-2-yl}-N-methyl-benzamid,

[4] 2-(Methyl-phenylacetyl-amino)-5,6,7,8-tetrahydro-imidazo[1,2-a]pyridin-3-carbonsäure [3-(2-methyl-piperidin-1-yl)-propyl]-amid,

[5] 2-[(3-Chlor-benzoyl)-methyl-amino]-5,6,7,8-tetrahydro-imidazo[1,2-a]pyridin-3-carbonsäure (2-phenyl-propyl)-amid,

[6] 2-[(3-Chlor-benzoyl)-methyl-amino]-5,6,7,8-tetrahydro-imidazo[1,2-a]pyridin-3-carbonsäure (2-pyrrolidin-1-yl-ethyl)-amid,

[7] 2-[Methyl-(naphthalen-1-carbonyl)-amino]-5,6,7,8-tetrahydro-imidazo[1,2-a]pyridin-3-carbonsäure ethyl-pyridin-4-ylmethyl-amid,

[8] 2-(Benzoyl-methyl-amino)-5,6,7,8-tetrahydro-imidazo[1,2-a]pyridin-3-carbonsäure (2-thiophen-2-yl-ethyl)-amid,

[9] 2-[Butyl-(3-chlor-benzoyl)-amino]-5,6,7,8-tetrahydro-imidazo[1,2-a]pyridin-3-carbonsäure (3-imidazol-1-yl-propyl)-amid,

[10] 3-Chlor-N-{3-[4-(3-chlor-phenyl)-piperazin-1-carbonyl]-5,6,7,8-tetrahydro-imidazo[1,2-a]pyridin-2-yl}-N-methyl-benzamid,

[11] 3-Chlor-N-methyl-N-[3-(4-phenyl-piperazin-1-carbonyl)-5,6,7,8-tetrahydro-imidazo[1,2-a]pyridin-2-yl]-benzamid,

[12] 2-[Butyl-(3,4-difluor-benzoyl)-amino]-5,6,7,8-tetrahydro-imidazo[1,2-a]pyridin-3-carbonsäure indan-1-ylamid,

[13] N-Butyl-3-chlor-N-{3-[4-(2-morpholin-4-yl-ethyl)-piperazin-1-carbonyl]-5,6,7,8-tetrahydro-imidazo[1,2-a]pyridin-2-yl}-benzamid,

[14] 3-Chlor-N-methyl-N-{3-[4-(3-trifluormethyl-phenyl)-piperazin-1-carbonyl]-5,6,7,8-tetrahydro-imidazo[1,2-a]pyridin-2-yl}-benzamid,

[15] 2-[Methyl-(naphthalen-1-carbonyl)-amino]-5,6,7,8-tetrahydro-imidazo[1,2-a]pyridin-3-carbonsäure 3,5-bis-trifluormethyl-benzylamid,

[16] 2-[Butyl-(3,4-difluor-benzoyl)-amino]-5,6,7,8-tetrahydro-imidazo[1,2-a]pyridin-3-carbonsäure (4-methyl-cyclohexyl)-amid,

[17] 2-(Benzoyl-methyl-amino)-5,6,7,8-tetrahydro-imidazo[1,2-a]pyridin-3-carbonsäure 3-methoxy-benzylamid,

[18] 2-[Methyl-(naphthalen-1-carbonyl)-amino]-5,6,7,8-tetrahydro-imidazo[1,2-a]pyridin-3-carbonsäure (1-p-tolyl-ethyl)-amid,

[19] 2-[Butyl-(3-chlor-benzoyl)-amino]-5,6,7,8-tetrahydro-imidazo[1,2-a]pyridin-3-carbonsäure (1,2-dimethyl-propyl)-amid,

[20] 2-(Benzoyl-methyl-amino)-5,6,7,8-tetrahydro-imidazo[1,2-a]pyridin-3-carbonsäure (4-methyl-cyclohexyl)-amid,

[21] 2-(Benzoyl-methyl-amino)-5,6,7,8-tetrahydro-imidazo[1,2-a]pyridin-3-carbonsäure (2-phenyl-propyl)-amid,

[22] 4-[2-(Benzoyl-methyl-amino)-5,6,7,8-tetrahydro-imidazo[1,2-a]pyridin-3-carbonyl]-piperazin-1-carbonsäure ethyl ester,

[23] 3-Chlor-N-methyl-N-(3-{4-[(methyl-phenyl-carbamoyl)-methyl]-piperazin-1-carbonyl}-5,6,7,8-tetrahydro-imidazo[1,2-a]pyridin-2-yl)-benzamid,

[24] 3-Chlor-N-{3-[4-(furan-2-carbonyl)-piperazin-1-carbonyl]-5,6,7,8-tetrahydro-imidazo[1,2-a]pyridin-2-yl}-N-methyl-benzamid,

[25] N-Methyl-N-[3-(4-p-tolyl-piperazin-1-carbonyl)-5,6,7,8-tetrahydro-imidazo[1,2-a]pyridin-2-yl]-benzamid,

[26] N-Butyl-3-chlor-N-{3-[4-(3-phenyl-propyl)-piperazin-1-carbonyl]-5,6,7,8-tetrahydro-imidazo[1,2-a]pyridin-2-yl}-benzamid,

[27] 2-[(3-Chlor-benzoyl)-methyl-amino]-5,6,7,8-tetrahydro-imidazo[1,2-a]pyridin-3-carbonsäure (2,6,6-trimethyl-bicyclo[3.1.1]hept-3-yl)-amid,

[28] 2-[Butyl-(3,4-difluor-benzoyl)-amino]-5,6,7,8-tetrahydro-imidazo[1,2-a]pyridin-3-carbonsäure (3-phenyl-propyl)-amid,

[29] Naphthalen-1-carbonsäure {3-[4-(4-methoxy-phenyl)-piperazin-1-carbonyl]-5,6,7,8-tetrahydro-imidazo[1,2-a]pyridin-2-yl}-methyl-amid,

[30] 2-[Methyl-(naphthalen-1-carbonyl)-amino]-5,6,7,8-tetrahydro-imidazo[1,2-a]pyridin-3-carbonsäure [1-(4-trifluormethyl-benzyl)-pyrrolidin-3-yl]-amid,

[31] 2-[(3-Chlor-benzoyl)-methyl-amino]-5,6,7,8-tetrahydro-imidazo[1,2-a]pyridin-3-carbonsäure (4-ethyl-phenyl)-amid,

[32] 2-[Methyl-(naphthalen-1-carbonyl)-amino]-5,6,7,8-tetrahydro-imidazo[1,2-a]pyridin-3-carbonsäure (3-methoxy-benzyl)-(tetrahydro-furan-2-ylmethyl)-amid,

[33] 2-[Methyl-(naphthalen-1-carbonyl)-amino]-5,6,7,8-tetrahydro-imidazo[1,2-a]pyridin-3-carbonsäure 2,3-dichlor-benzylamid,

[34] 2-[Butyl-(3,4-difluor-benzoyl)-amino]-5,6,7,8-tetrahydro-imidazo[1,2-a]pyridin-3-carbonsäure (1-benzofuran-2-ylmethyl-pyrrolidin-3-yl)-methyl-amid,

[35] 3-Chlor-N-{3-[4-(4-chlor-phenyl)-piperazin-1-carbonyl]-5,6,7,8-tetrahydro-imidazo[1,2-a]pyridin-2-yl}-N-methyl-benzamid,

[36] 2-({2-[(3-Chlor-benzoyl)-methyl-amino]-5,6,7,8-tetrahydro-imidazo[1,2-a]pyridin-3-carbonyl}-amino)-3-(1H-indol-3-yl)-propionsäure methyl ester,

[37] N-Butyl-3-chlor-N-[3-(4-phenylacetyl-piperazin-1-carbonyl)-5,6,7,8-tetrahydro-imidazo[1,2-a]pyridin-2-yl]-benzamid,

[38] 2-({2-[(3-Chlor-benzoyl)-methyl-amino]-5,6,7,8-tetrahydro-imidazo[1,2-a]pyridin-3-carbonyl}-amino)-3-methyl-pentansäure-tert-butyl ester,

[39] 2-[Butyl-(3-chlor-benzoyl)-amino]-5,6,7,8-tetrahydro-imidazo[1,2-a]pyridin-3-carbonsäure (1-naphthalen-1-yl-ethyl)-amid,

[40] 2-[Methyl-(naphthalen-1-carbonyl)-amino]-5,6,7,8-tetrahydro-imidazo[1,2-a]pyridin-3-carbonsäure allyl-methyl-amid,

[41] N-Butyl-N-[3-(3,6-dihydro-2H-pyridin-1-carbonyl)-5,6,7,8-tetrahydro-imidazo[1,2-a]pyridin-2-yl]-3,4-difluor-benzamid,

[42] 2-({2-[(3-Chlor-benzoyl)-methyl-amino]-5,6,7,8-tetrahydro-imidazo[1,2-a]pyridin-3-carbonyl}-amino)-4-methyl-pentansäure-benzyl ester,

[43] 2-[Butyl-(3-chlor-benzoyl)-amino]-5,6,7,8-tetrahydro-imidazo[1,2-a]pyridin-3-carbonsäure 2-ethoxy-benzylamid,

[44] N-Butyl-3-chlor-N-{3-[4-(5-methyl-pyrazin-2-carbonyl)-piperazin-1-carbonyl]-5,6,7,8-tetrahydro-imidazo[1,2-a]pyridin-2-yl}-benzamid,

[45] N-[3-(4-Benzo[1,3]dioxol-5-ylmethyl-piperazin-1-carbonyl)-5,6,7,8-tetrahydro-imidazo[1,2-a]pyridin-2-yl]-3-chlor-N-methyl-benzamid,

[46] 2-[Methyl-(naphthalen-1-carbonyl)-amino]-5,6,7,8-tetrahydro-imidazo[1,2-a]pyridin-3-carbonsäure (2,2-diphenyl-ethyl)-amid,

[47] 2-[Butyl-(3-chlor-benzoyl)-amino]-5,6,7,8-tetrahydro-imidazo[1,2-a]pyridin-3-carbonsäure 2,4-difluor-benzylamid,

[48] N-Butyl-3-chlor-N-{3-[4-(2-fluor-phenyl)-piperazin-1-carbonyl]-5,6,7,8-tetrahydro-imidazo[1,2-a]pyridin-2-yl}-benzamid,

[49] 2-[Methyl-(naphthalen-1-carbonyl)-amino]-5,6,7,8-tetrahydro-imidazo[1,2-a]pyridin-3-carbonsäure (2-p-tolyl-ethyl)-amid,

[50] 2-[Methyl-(naphthalen-1-carbonyl)-amino]-5,6,7,8-tetrahydro-imidazo[1,2-a]pyridin-3-carbonsäure (pyridin-2-ylmethyl)-amid,

[51] 2-[Butyl-(3-chlor-benzoyl)-amino]-5,6,7,8-tetrahydro-imidazo[1,2-a]pyridin-3-carbonsäure 2-trifluormethyl-benzylamid,

[52] 2-[Butyl-(3-chlor-benzoyl)-amino]-5,6,7,8-tetrahydro-imidazo[1,2-a]pyridin-3-carbonsäure indan-1-ylamid,

[53] 3-Chlor-N-methyl-N-[3-(4-chinolin-2-ylmethyl-piperazin-1-carbonyl)-5,6,7,8-tetrahydro-imidazo[1,2-a]pyridin-2-yl]-benzamid,

[54] N-Butyl-3,4-difluor-N-[3-(4-methyl-piperazin-1-carbonyl)-5,6,7,8-tetrahydro-imidazo[1,2-a]pyridin-2-yl]-benzamid,

[55] 2-[(3-Chlor-benzoyl)-methyl-amino]-5,6,7,8-tetrahydro-imidazo[1,2-a]pyridin-3-carbonsäure 2,4-difluor-benzylamid,

[56] N-[3-(4-Benzhydryl-piperazin-1-carbonyl)-5,6,7,8-tetrahydro-imidazo[1,2-a]pyridin-2-yl]-N-butyl-3,4-difluor-benzamid,

[57] 4-Methyl-2-({2-[methyl-(naphthalen-1-carbonyl)-amino]-5,6,7,8-tetrahydro-imidazo[1,2-a]pyridin-3-carbonyl}-amino)-pentansäure- benzyl ester,

[58] 2-[Butyl-(3-chlor-benzoyl)-amino]-5,6,7,8-tetrahydro-imidazo[1,2-a]pyridin-3-carbonsäure (2-pyrrolidin-1-yl-ethyl)-amid,

[59] 2-[(3-Chlor-benzoyl)-methyl-amino]-5,6,7,8-tetrahydro-imidazo[1,2-a]pyridin-3-carbonsäure [1-(2-benzyloxy-benzyl)-pyrrolidin-3-yl]-amid,

[60] 2-[Butyl-(3,4-difluor-benzoyl)-amino]-5,6,7,8-tetrahydro-imidazo[1,2-a]pyridin-3-carbonsäure (pyridin-3-ylmethyl)-amid,

[61] 3-Chlor-N-{3-[4-(4-fluor-phenyl)-3,6-dihydro-2H-pyridin-1-carbonyl]-5,6,7,8-tetrahydro-imidazo[1,2-a]pyridin-2-yl}-N-methyl-benzamid,

[62] 2-[(3-Chlor-benzoyl)-methyl-amino]-5,6,7,8-tetrahydro-imidazo[1,2-a]pyridin-3-carbonsäure cyclohexylamid,

[63] N-[3-(4-Cycloheptyl-piperazin-1-carbonyl)-5,6,7,8-tetrahydro-imidazo[1,2-a]pyridin-2-yl]-N-methyl-benzamid,

[64] Naphthalen-1-carbonsäure methyl-[3-(4-thiophen-3-ylmethyl-piperazin-1-carbonyl)-5,6,7,8-tetrahydro-imidazo [1,2-a]pyridin-2-yl]-amid,

[65] 2-[Butyl-(3-chlor-benzoyl)-amino]-5,6,7,8-tetrahydro-imidazo[1,2-a]pyridin-3-carbonsäure 2,4-dimethoxy-benzylamid,

[66] 1-[2-(Benzoyl-methyl-amino)-5,6,7,8-tetrahydro-imidazo[1,2-a]pyridin-3-carbonyl]-piperidin-4-carbonsäure ethyl ester,

[67] 2-[(3-Chlor-benzoyl)-methyl-amino]-5,6,7,8-tetrahydro-imidazo[1,2-a]pyridin-3-carbonsäure (2-benzyloxy-cyclohexyl)-amid,

[68] 2-[Methyl-(naphthalen-1-carbonyl)-amino]-5,6,7,8-tetrahydro-imidazo[1,2-a]pyridin-3-carbonsäure [2-(4-phenoxy-phenyl)-ethyl]-amid,

[69] 2-[Butyl-(3-chlor-benzoyl)-amino]-5,6,7,8-tetrahydro-imidazo[1,2-a]pyridin-3-carbonsäure [2-(7-methyl-1H-indol-3-yl)-ethyl]-amid,

[70] Naphthalen-1-carbonsäure methyl-[3-(4-phenyl-piperazin-1-carbonyl)-5,6,7,8-tetrahydro-imidazo[1,2-a]pyridin-2-yl]-amid,

[71] 2-[(3-Chlor-benzoyl)-methyl-amino]-5,6,7,8-tetrahydro-imidazo[1,2-a]pyridin-3-carbonsäure [2-(3-trifluormethyl-phenyl)-ethyl]-amid,

[72] N-Butyl-3-chlor-N-[3-(4-pyridin-2-yl-piperazin-1-carbonyl)-5,6,7,8-tetrahydro-imidazo[1,2-a]pyridin-2-yl]-benzamid,

[73] 2-[Methyl-(naphthalen-1-carbonyl)-amino]-5,6,7,8-tetrahydro-imidazo[1,2-a]pyridin-3-carbonsäure 3-methoxy-benzylamid,

[74] 2-[Butyl-(3,4-difluor-benzoyl)-amino]-5,6,7,8-tetrahydro-imidazo[1,2-a]pyridin-3-carbonsäure 2,4-difluor-benzylamid,

[75] 3-Chlor-N-methyl-N-[3-(4-pyridin-2-yl-piperazin-1-carbonyl)-5,6,7,8-tetrahydro-imidazo[1,2-a]pyridin-2-yl]-benzamid,

[76] 2-[Methyl-(naphthalen-1-carbonyl)-amino]-5,6,7,8-tetrahydro-imidazo[1,2-a]pyridin-3-carbonsäure (1-biphenyl-4-ylmethyl-pyrrolidin-3-yl)-methyl-amid,

[77] 2-[Butyl-(3-chlor-benzoyl)-amino]-5,6,7,8-tetrahydro-imidazo[1,2-a]pyridin-3-carbonsäure (2,3-dihydro-benzo [1,4]dioxin-2-ylmethyl)-amid,

[78] 2-[Butyl-(3-chlor-benzoyl)-amino]-5,6,7,8-tetrahydro-imidazo[1,2-a]pyridin-3-carbonsäure [2-(1H-indol-3-yl)-ethyl]-methyl-amid,

[79] N-Butyl-3-chlor-N-{3-[4-(3-trifluormethyl-phenyl)-piperazin-1-carbonyl]-5,6,7,8-tetrahydro-imidazo[1,2-a]pyridin-2-yl}-benzamid,

[80] 2-(Benzoyl-methyl-amino)-5,6,7,8-tetrahydro-imidazo[1,2-a]pyridin-3-carbonsäure [2-(1H-indol-3-yl)-ethyl]-methyl-amid,

[81] 3-Chlor-N-{3-[4-hydroxy-4-(3-trifluormethyl-phenyl)-piperidin-1-carbonyl]-5,6,7,8-tetrahydro-imidazo[1,2-a]pyridin-2-yl}-N-methyl-benzamid,

[82] 2-[Butyl-(3,4-difluor-benzoyl)-amino]-5,6,7,8-tetrahydro-imidazo[1,2-a]pyridin-3-carbonsäure (1-methoxymethyl-2-phenyl-ethyl)-amid,

[83] 2-[(3-Chlor-benzoyl)-methyl-amino]-5,6,7,8-tetrahydro-imidazo[1,2-a]pyridin-3-carbonsäure [1-(2,6-dichlor-benzyl)-pyrrolidin-3-yl]-amid,

[84] 2-[(3-Chlor-benzoyl)-methyl-amino]-5,6,7,8-tetrahydro-imidazo[1,2-a]pyridin-3-carbonsäure [1-(2-brom-4,5-dimethoxy-benzyl)-pyrrolidin-3-yl]-amid,

[85] 2-(Benzoyl-methyl-amino)-5,6,7,8-tetrahydro-imidazo[1,2-a]pyridin-3-carbonsäure benzo[1,3]dioxol-5-ylamid,

[86] 2-[Butyl-(3-chlor-benzoyl)-amino]-5,6,7,8-tetrahydro-imidazo[1,2-a]pyridin-3-carbonsäure (1-methoxymethyl-2-phenyl-ethyl)-amid,

[87] 2-[Butyl-(3-chlor-benzoyl)-amino]-5,6,7,8-tetrahydro-imidazo[1,2-a]pyridin-3-carbonsäure [1-(2,6-dichlor-benzyl)-pyrrolidin-3-yl]-methyl-amid,

[88] 2-[Butyl-(3,4-difluor-benzoyl)-amino]-5,6,7,8-tetrahydro-imidazo[1,2-a]pyridin-3-carbonsäure 2-trifluormethoxy-benzylamid,

[89] N-Butyl-3,4-difluor-N-{3-[4-(isopropylcarbamoyl-methyl)-piperazin-1-carbonyl]-5,6,7,8-tetrahydro-imidazo[1,2-a]pyridin-2-yl}-benzamid,

[90] 2-[(3-Chlor-benzoyl)-methyl-amino]-5,6,7,8-tetrahydro-imidazo[1,2-a]pyridin-3-carbonsäure (1-methyl-3-phenyl-propyl)-amid,

[91] 2-({2-[Butyl-(3-chlor-benzoyl)-amino]-5,6,7,8-tetrahydro-imidazo[1,2-a]pyridin-3-carbonyl}-amino)-3-methyl-buttersäurebenzylester,

[92] 2-[(3-Chlor-benzoyl)-methyl-amino]-5,6,7,8-tetrahydro-imidazo[1,2-a]pyridin-3-carbonsäure (thiophen-2-ylmethyl)-amid,

[93] 2-[Butyl-(3,4-difluor-benzoyl)-amino]-5,6,7,8-tetrahydro-imidazo[1,2-a]pyridin-3-carbonsäure (2-methyl-cyclohexyl)-amid,

[94] 2-(Benzoyl-methyl-amino)-5,6,7,8-tetrahydro-imidazo[1,2-a]pyridin-3-carbonsäure benzyl-methyl-amid,

[95] 2-[Butyl-(3-chlor-benzoyl)-amino]-5,6,7,8-tetrahydro-imidazo[1,2-a]pyridin-3-carbonsäure [2-(2,6-dichlor-benzylsulfanyl)-ethyl]-amid,

[96] 2-[Butyl-(3,4-difluor-benzoyl)-amino]-5,6,7,8-tetrahydro-imidazo[1,2-a]pyridin-3-carbonsäure (2,3-dihydro-benzo[1,4]dioxin-2-ylmethyl)-amid,

[97] 2-[(3-Chlor-benzoyl)-methyl-amino]-5,6,7,8-tetrahydro-imidazo[1,2-a]pyridin-3-carbonsäure 4-trifluormethyl-benzylamid,

[98] N-[3-(4-Benzyl-piperidin-1-carbonyl)-5,6,7,8-tetrahydro-imidazo[1,2-a]pyridin-2-yl]-N-methyl-benzamid,

[99] 2-[Methyl-(naphthalen-1-carbonyl)-amino]-5,6,7,8-tetrahydro-imidazo[1,2-a]pyridin-3-carbonsäure 2-trifluormethoxy-benzylamid,

[100] 3-Chlor-N-{3-[4-(2,5-dimethyl-phenyl)-piperazin-1-carbonyl]-5,6,7,8-tetrahydro-imidazo[1,2-a]pyridin-2-yl}-N-methyl-benzamid,

[101] 2-(Benzoyl-methyl-amino)-5,6,7,8-tetrahydro-imidazo[1,2-a]pyridin-3-carbonsäure [1-(2,6-dichlor-benzyl)-pyrrolidin-3-yl]-amid,

[102] 2-[Butyl-(3,4-difluor-benzoyl)-amino]-5,6,7,8-tetrahydro-imidazo[1,2-a]pyridin-3-carbonsäure 2-chlor-6-methyl-benzylamid,

[103] N-Butyl-3-chlor-N-[3-(3,5-dimethyl-piperidin-1-carbonyl)-5,6,7,8-tetrahydro-imidazo[1,2-a]pyridin-2-yl]-benzamid,

[104] 2-({2-[(3-Chlor-benzoyl)-methyl-amino]-5,6,7,8-tetrahydro-imidazo[1,2-a]pyridin-3-carbonyl}-amino)-3,3-dimethyl-buttersäure tert-butylester,

[105] 2-[Methyl-(naphthalen-1-carbonyl)-amino]-5,6,7,8-tetrahydro-imidazo[1,2-a]pyridin-3-carbonsäure (1-cyclohexyl-ethyl)-amid,

[106] 3-Chlor-N-methyl-N-[3-(4-phenethyl-piperazin-1-carbonyl)-5,6,7,8-tetrahydro-imidazo[1,2-a]pyridin-2-yl]-benzamid,

[107] 2-[Butyl-(3-chlor-benzoyl)-amino]-5,6,7,8-tetrahydro-imidazo[1,2-a]pyridin-3-carbonsäure (2-phenoxy-ethyl)-amid,

[108] Naphthalen-1-carbonsäure [3-(4-benzofuran-2-ylmethyl-piperazin-1-carbonyl)-5,6,7,8-tetrahydro-imidazo [1,2-a]pyridin-2-yl]-methyl-amid,

[109] N-[3-([1,4']Bipiperidinyl-1'-carbonyl)-5,6,7,8-tetrahydro-imidazo[1,2-a]pyridin-2-yl]-N-butyl-3-chlor-benzamid,

[110] N-Butyl-3-chlor-N-[3-(8-fluor-1,3,4,5-tetrahydro-pyrido[4,3-b]indol-2-carbonyl)-5,6,7,8-tetrahydro-imidazo [1,2-a]pyridin-2-yl]-benzamid,

[111] 2-[Methyl-(naphthalen-1-carbonyl)-amino]-5,6,7,8-tetrahydro-imidazo[1,2-a]pyridin-3-carbonsäure 4-dimethylamino-benzylamid,

[112] 2-[Methyl-(naphthalen-1-carbonyl)-amino]-5,6,7,8-tetrahydro-imidazo[1,2-a]pyridin-3-carbonsäure (1-methyl-3-phenyl-propyl)-amid,

[113] 2-[Butyl-(3-chlor-benzoyl)-amino]-5,6,7,8-tetrahydro-imidazo[1,2-a]pyridin-3-carbonsäure 3-methyl-benzylamid,

[114] 2-[(3-Chlor-benzoyl)-methyl-amino]-5,6,7,8-tetrahydro-imidazo[1,2-a]pyridin-3-carbonsäure (2,3-dihydro-benzo[1,4]dioxin-6-yl)-amid,

[115] Naphthalen-1-carbonsäure methyl-[3-(4-chinolin-2-ylmethyl-piperazin-1-carbonyl)-5,6,7,8-tetrahydro-imidazo [1,2-a]pyridin-2-yl]-amid,

[116] 2-[Butyl-(3,4-difluor-benzoyl)-amino]-5,6,7,8-tetrahydro-imidazo[1,2-a]pyridin-3-carbonsäure (2-cyano-ethyl)-methyl-amid,

[117] 2-(Methyl-phenylacetyl-amino)-5,6,7,8-tetrahydro-imidazo[1,2-a]pyridin-3-carbonsäure (2-p-tolyl-ethyl)-amid,

[118] 2-[Butyl-(3,4-difluor-benzoyl)-amino]-5,6,7,8-tetrahydro-imidazo[1,2-a]pyridin-3-carbonsäure [1-(4-fluor-phenyl)-ethyl]-amid,

[119] N-Butyl-N-{3-[4-(5-chlor-2-methyl-phenyl)-piperazin-1-carbonyl]-5,6,7,8-tetrahydro-imidazo[1,2-a]pyridin-2-yl}-3,4-difluor-benzamid,

[120] 2-[Butyl-(3-chlor-benzoyl)-amino]-5,6,7,8-tetrahydro-imidazo[1,2-a]pyridin-3-carbonsäure cyclohexylamid,

[121] 2-[(3-Chlor-benzoyl)-methyl-amino]-5,6,7,8-tetrahydro-imidazo[1,2-a]pyridin-3-carbonsäure (benzo[1,3]dioxol-5-ylmethyl)-amid,

[122] 2-[Butyl-(3,4-difluor-benzoyl)-amino]-5,6,7,8-tetrahydro-imidazo[1,2-a]pyridin-3-carbonsäure 3-methoxy-benzylamid,

[123] 3-tert-Butoxy-2-({2-[(3-chlor-benzoyl)-methyl-amino]-5,6,7,8-tetrahydro-imidazo[1,2-a]pyridin-3-carbonyl}-amino)-buttersäure methyl ester,

[124] 2-[Butyl-(3-chlor-benzoyl)-amino]-5,6,7,8-tetrahydro-imidazo[1,2-a]pyridin-3-carbonsäure (2-benzyloxy-cyclohexyl)-amid,

[125] N-{3-[4-(4-Fluor-phenyl)-piperazin-1-carbonyl]-5,6,7,8-tetrahydro-imidazo[1,2-a]pyridin-2-yl}-N-methyl-benzamid,

[126] 2-(Benzoyl-methyl-amino)-5,6,7,8-tetrahydro-imidazo[1,2-a]pyridin-3-carbonsäure (naphthalen-2-ylcarbamoylmethyl)-amid,

[127] 2-[Butyl-(3-chlor-benzoyl)-amino]-5,6,7,8-tetrahydro-imidazo[1,2-a]pyridin-3-carbonsäure 4-trifluormethoxy-benzylamid,

[128] 2-[(3-Chlor-benzoyl)-methyl-amino]-5,6,7,8-tetrahydro-imidazo[1,2-a]pyridin-3-carbonsäure (3,3-diphenyl-propyl)-amid,

[129] 2-[(3-Chlor-benzoyl)-methyl-amino]-5,6,7,8-tetrahydro-imidazo[1,2-a]pyridin-3-carbonsäure (4-benzyloxy-phenyl)-amid,

[130] N-{3-[4-(5-Brom-2-ethoxy-benzyl)-piperazin-1-carbonyl]-5,6,7,8-tetrahydro-imidazo[1,2-a]pyridin-2-yl}-N-methyl-2-phenyl-acetamid,

[131] N-[3-(3,4-Dihydro-1H-isochinolin-2-carbonyl)-5,6,7,8-tetrahydro-imidazo[1,2-a]pyridin-2-yl]-N-methyl-benzamid,

[132] 2-[Butyl-(3-chlor-benzoyl)-amino]-5,6,7,8-tetrahydro-imidazo[1,2-a]pyridin-3-carbonsäure (1-phenyl-propyl)-amid,

[133] 2-[Butyl-(3-chlor-benzoyl)-amino]-5,6,7,8-tetrahydro-imidazo[1,2-a]pyridin-3-carbonsäure 2,3-dimethyl-benzylamid,

[134] 2-[Butyl-(3-chlor-benzoyl)-amino]-5,6,7,8-tetrahydro-imidazo[1,2-a]pyridin-3-carbonsäure [2-(4-phenoxy-phenyl)-ethyl]-amid,

[135] N-{3-[4-(4-Ethoxy-phenyl)-piperazin-1-carbonyl]-5,6,7,8-tetrahydro-imidazo[1,2-a]pyridin-2-yl}-N-methyl-2-phenyl-acetamid,

[136] Naphthalen-1-carbonsäure {3-[4-(2-ethyl-phenyl)-piperazin-1-carbonyl]-5,6,7,8-tetrahydro-imidazo[1,2-a]pyridin-2-yl}-methyl-amid,

[137] N-[3-(4-Benzo[1,3]dioxol-5-ylmethyl-piperazin-1-carbonyl)-5,6,7,8-tetrahydro-imidazo[1,2-a]pyridin-2-yl]-N-butyl-3,4-difluor-benzamid,

[138] N-Butyl-N-{3-[4-(2,5-dimethyl-phenyl)-piperazin-1-carbonyl]-5,6,7,8-tetrahydro-imidazo[1,2-a]pyridin-2-yl}-3,4-difluor-benzamid,

[139] 2-[Methyl-(naphthalen-1-carbonyl)-amino]-5,6,7,8-tetrahydro-imidazo[1,2-a]pyridin-3-carbonsäure [(4-chlor-phenyl)-phenyl-methyl]-amid,

[140] N-Butyl-3-chlor-N-{3-[4-(4-chlor-benzyl)-piperazin-1-carbonyl]-5,6,7,8-tetrahydro-imidazo[1,2-a]pyridin-2-yl}-benzamid,

[141] 2-[Butyl-(3-chlor-benzoyl)-amino]-5,6,7,8-tetrahydro-imidazo[1,2-a]pyridin-3-carbonsäure (1-naphthalen-2-yl-ethyl)-amid,

[142] 2-(Methyl-phenylacetyl-amino)-5,6,7,8-tetrahydro-imidazo[1,2-a]pyridin-3-carbonsäure 2-fluor-benzylamid,

[143] N-[3-(1,4-Dioxa-8-aza-spiro[4.5]decane-8-carbonyl)-5,6,7,8-tetrahydro-imidazo[1,2-a]pyridin-2-yl]-N-methyl-2-phenyl-acetamid,

[144] Naphthalen-1-carbonsäure methyl-[3-(1,3,4,9-tetrahydro-b-carbolin-2-carbonyl)-5,6,7,8-tetrahydro-imidazo[1,2-a]pyridin-2-yl]-amid,

[145] 2-[Butyl-(3-chlor-benzoyl)-amino]-5,6,7,8-tetrahydro-imidazo[1,2-a]pyridin-3-carbonsäure (1-methyl-3-phenyl-propyl)-amid,

[146] 3-Chlor-N-methyl-N-{3-[4-(2,4,6-trimethoxy-benzyl)-piperazin-1-carbonyl]-5,6,7,8-tetrahydro-imidazo[1,2-a]pyridin-2-yl}-benzamid,

[147] 2-[Butyl-(3-chlor-benzoyl)-amino]-5,6,7,8-tetrahydro-imidazo[1,2-a]pyridin-3-carbonsäure benzhydryl-amid,

[148] Naphthalen-1-carbonsäure {3-[4-(2-chlor-phenyl)-piperazin-1-carbonyl]-5,6,7,8-tetrahydro-imidazo[1,2-a]pyridin-2-yl}-methyl-amid,

[149] 2-[Butyl-(3-chlor-benzoyl)-amino]-5,6,7,8-tetrahydro-imidazo[1,2-a]pyridin-3-carbonsäure (thiophen-2-ylmethyl)-amid,

[150] N-Butyl-N-{3-[4-(4-chlor-benzyl)-piperazin-1-carbonyl]-5,6,7,8-tetrahydro-imidazo[1,2-a]pyridin-2-yl}-3,4-difluor-benzamid,

[151] 2-(Benzoyl-methyl-amino)-5,6,7,8-tetrahydro-imidazo[1,2-a]pyridin-3-carbonsäure (3-phenyl-propyl)-amid,

[152] 2-[Butyl-(3-chlor-benzoyl)-amino]-5,6,7,8-tetrahydro-imidazo[1,2-a]pyridin-3-carbonsäure (4-tert-butyl-phenyl)-amid,

[153] 2-[Butyl-(3-chlor-benzoyl)-amino]-5,6,7,8-tetrahydro-imidazo[1,2-a]pyridin-3-carbonsäure (4-ethyl-phenyl)-amid,

[154] 2-[Methyl-(naphthalen-1-carbonyl)-amino]-5,6,7,8-tetrahydro-imidazo[1,2-a]pyridin-3-carbonsäure cyclohexylamid,

[155] 2-[Methyl-(naphthalen-1-carbonyl)-amino]-5,6,7,8-tetrahydro-imidazo[1,2-a]pyridin-3-carbonsäure [3-(methyl-phenyl-amino)-propyl]-amid

[156] 2-(Methyl-phenylacetyl-amino)-5,6,7,8-tetrahydro-imidazo[1,2-a]pyridin-3-carbonsäure [2-(3-trifluormethyl-phenyl)-ethyl]-amid,

[157] 2-(Benzoyl-methyl-amino)-5,6,7,8-tetrahydro-imidazo[1,2-a]pyridin-3-carbonsäure 4-chlor-benzylamid,

[158] 2-[Butyl-(3-chlor-benzoyl)-amino]-5,6,7,8-tetrahydro-imidazo[1,2-a]pyridin-3-carbonsäure 3-fluor-benzylamid,

[159] 2-(Methyl-phenylacetyl-amino)-5,6,7,8-tetrahydro-imidazo[1,2-a]pyridin-3-carbonsäure p-tolylamid,

[160] 2-(Benzoyl-methyl-amino)-5,6,7,8-tetrahydro-imidazo[1,2-a]pyridin-3-carbonsäure (2-phenoxy-ethyl)-amid,

[161] 2-[Butyl-(3,4-difluor-benzoyl)-amino]-5,6,7,8-tetrahydro-imidazo[1,2-a]pyridin-3-carbonsäure (2-benzyloxy-cyclohexyl)-amid,

[162] Naphthalen-1-carbonsäure {3-[4-(4-fluor-phenyl)-piperazin-1-carbonyl]-5,6,7,8-tetrahydro-imidazo[1,2-a]pyridin-2-yl}-methyl-amid,

[163] 2-[Methyl-(naphthalen-1-carbonyl)-amino]-5,6,7,8-tetrahydro-imidazo[1,2-a]pyridin-3-carbonsäure [2-(4-chlor-phenyl)-propyl]-amid,

[164] N-Butyl-3,4-difluor-N-[3-(thiomorpholin-4-carbonyl)-5,6,7,8-tetrahydro-imidazo[1,2-a]pyridin-2-yl]-benzamid,

[165] 2-[(3-Chlor-benzoyl)-methyl-amino]-5,6,7,8-tetrahydro-imidazo[1,2-a]pyridin-3-carboxylsäure (1-adamantan-1-yl-ethyl)-amid,

[166] 2-(Benzoyl-methyl-amino)-5,6,7,8-tetrahydro-imidazo[1,2-a]pyridin-3-carbonsäure [(4-chlor-phenyl)-phenyl-methyl]-amid,

[167] N-Methyl-N-{3-[4-(4-trifluormethyl-phenyl)-piperazin-1-carbonyl]-5,6,7,8-tetrahydro-imidazo[1,2-a]pyridin-2-yl}-benzamid,

[168] 2-(Methyl-phenylacetyl-amino)-5,6,7,8-tetrahydro-imidazo[1,2-a]pyridin-3-carbonsäure (1-biphenyl-4-ylmethyl-pyrrolidin-3-yl)-methyl-amid,

[169] N-[3-(4-Benzoyl-piperidin-1-carbonyl)-5,6,7,8-tetrahydro-imidazo[1,2-a]pyridin-2-yl]-3-chlor-N-methyl-benzamid,

[170] 2-[Methyl-(naphthalen-1-carbonyl)-amino]-5,6,7,8-tetrahydro-imidazo[1,2-a]pyridin-3-carbonsäure (4-tert-butyl-phenyl)-amid,

[171] 2-[Butyl-(3,4-difluor-benzoyl)-amino]-5,6,7,8-tetrahydro-imidazo[1,2-a]pyridin-3-carbonsäure (3,3-dimethyl-butyl)-amid,

[172] 2-[Butyl-(3-chlor-benzoyl)-amino]-5,6,7,8-tetrahydro-imidazo[1,2-a]pyridin-3-carbonsäure [1-(2-brom-4,5-dimethoxy-benzyl)-pyrrolidin-3-yl]-amid,

[173] Naphthalen-1-carbonsäure methyl-{3-[4-(3-phenyl-allyl)-piperazin-1-carbonyl]-5,6,7,8-tetrahydro-imidazo[1,2-a]pyridin-2-yl}-amid,

[174] Naphthalen-1-carbonsäure methyl-[3-(4-phenethyl-piperazin-1-carbonyl)-5,6,7,8-tetrahydro-imidazo[1,2-a]pyridin-2-yl]-amid,

[175] N-Butyl-3-chlor-N-{3-[4-hydroxy-4-(3-trifluormethyl-phenyl)-piperidin-1-carbonyl]-5,6,7,8-tetrahydro-imidazo[1,2-a]pyridin-2-yl}-benzamid,

[176] N-Methyl-2-phenyl-N-{3-[4-(3-phenyl-propyl)-piperazin-1-carbonyl]-5,6,7,8-tetrahydro-imidazo[1,2-a]pyridin-2-yl}-acetamid,

[177] 2-(Benzoyl-methyl-amino)-5,6,7,8-tetrahydro-imidazo[1,2-a]pyridin-3-carbonsäure 3-fluor-benzylamid,

[178] N-Butyl-N-{3-[4-(2-chlor-phenyl)-piperazin-1-carbonyl]-5,6,7,8-tetrahydro-imidazo[1,2-a]pyridin-2-yl}-3,4-difluor-benzamid,

[179] 2-[Methyl-(naphthalen-1-carbonyl)-amino]-5,6,7,8-tetrahydro-imidazo[1,2-a]pyridin-3-carbonsäure benzo[1,3]dioxol-5-ylamid,

[180] 2-[Butyl-(3,4-difluor-benzoyl)-amino]-5,6,7,8-tetrahydro-imidazo[1,2-a]pyridin-3-carbonsäure (3,3-diphenyl-propyl)-amid,

[181] 2-[Butyl-(3-chlor-benzoyl)-amino]-5,6,7,8-tetrahydro-imidazo[1,2-a]pyridin-3-carbonsäure (2,3-dihydro-benzo[1,4]dioxin-6-yl)-amid,

[182] 2-(Benzoyl-methyl-amino)-5,6,7,8-tetrahydro-imidazo[1,2-a]pyridin-3-carbonsäure [2-(3,4-dimethoxy-phenyl)-ethyl]-amid,

[183] Naphthalen-1-carbonsäure [3-(4-benzoyl-piperidin-1-carbonyl)-5,6,7,8-tetrahydro-imidazo[1,2-a]pyridin-2-yl]-methyl-amid,

[184] 4-Methyl-2-({2-[methyl-(naphthalen-1-carbonyl)-amino]-5,6,7,8-tetrahydro-imidazo[1,2-a]pyridin-3-carbonyl}-amino)-valeriansäure tert-butylester,

[185] 2-[Methyl-(naphthalen-1-carbonyl)-amino]-5,6,7,8-tetrahydro-imidazo[1,2-a]pyridin-3-carbonsäure (4-phenoxy-phenyl)-amid,

[186] N-Methyl-2-phenyl-N-[3-(4-phenyl-piperazin-1-carbonyl)-5,6,7,8-tetrahydro-imidazo[1,2-a]pyridin-2-yl]-acetamid,

[187] N-Butyl-3,4-difluor-N-{3-[4-(5-trifluormethyl-pyridin-2-yl)-piperazin-1-carbonyl]-5,6,7,8-tetrahydro-imidazo[1,2-a]pyridin-2-yl}-benzamid,

[188] 2-(Benzoyl-methyl-amino)-5,6,7,8-tetrahydro-imidazo[1,2-a]pyridin-3-carbonsäure 2,4-dichlor-6-methyl-benzylamid,

[189] [4-({2-[Butyl-(3-chlor-benzoyl)-amino]-5,6,7,8-tetrahydro-imidazo[1,2-a]pyridin-3-carbonyl}-amino)-phenyl]-carbaminsäure tert-butyl ester,

[190] N-Methyl-2-phenyl-N-[3-(2-pyrrolidin-1-ylmethyl-pyrrolidin-1-carbonyl)-5,6,7,8-tetrahydro-imidazo[1,2-a]pyridin-2-yl]-acetamid und

[191] 4-[2-(Methyl-phenylacetyl-amino)-5,6,7,8-tetrahydro-imidazo[1,2-a]pyridin-3-carbonyl]-piperazin-1-carbonsäure tert-butyl ester,

jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form entsprechender Salze, oder jeweils in Form entsprechender Solvate.

Ebenfalls bevorzugt sind erfindungsgemäße substituierte 5,6,7,8-Tetrahydro-imidazo[1,2-a]pyridin-2-ylamin-Verbindungen der allgemeinen Formel I, die im FLIPR-Assay in einer Konzentration von 10 μM eine Hemmung des $Ca^{2+}$-Ionen-Einstroms in Dorsalwurzelganglien von Ratten von wenigstens 30 %, bevorzugt von wenigstens 50 %, besonders bevorzugt von wenigstens 70 %, ganz besonders bevorzugt von wenigstens 80 %, noch weiter bevorzugt von wenigstens 90 %, im Vergleich zur maximal erreichbaren Hemmung des $Ca^{2+}$-Ionen-Einstroms mit Capsaicin in einer Konzentration von 10 μM aufweisen.

Dabei wird Im FLIPR-Assay der $Ca^{2+}$-Einstrom mit Hilfe eines $Ca^{2+}$-sensitiven Farbstoffs (Typ Fluo-4, Molecular Probes Europe BV, Leiden Niederlande) im Fluorescent Imaging Plate Reader (FLIPR, Molecular Devices, Sunnyvale, USA) quantifiziert, wie untenstehend beschrieben.

Ein weiterer Gegenstand der vorliegenden Erfindung ist ein Verfahren zur Herstellung von erfindungsgemäßen Verbindungen der vorstehend angegebenen allgemeinen Formel I gemäß dem wenigstens eine Verbindung der allgemeinen Formel II,

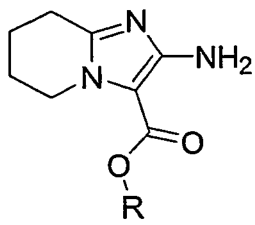

II

worin R für einen linearen oder verzweigten $C_{1-6}$-Alkyl-Rest, vorzugsweise für einen Methyl- oder Ethyl-Rest steht, in einem Reaktionsmedium in Gegenwart wenigstens eines Reduktionsmittels, ggf. in Gegenwart wenigstens einer organischen Säure, vorzugsweise in Gegenwart von Essigsäure, mit wenigstens einer Verbindung der allgemeinen Formel $R^3$-C(=O)-H, worin $R^3$ die vorstehend genannte Bedeutung mit Ausnahme des Wasserstoff-Restes hat, zu einer Verbindung der allgemeinen Formel III,

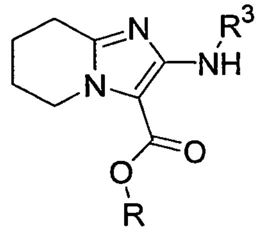

III

worin R die vorstehend genannte Bedeutung und $R^3$ die vorstehend genannte Bedeutung mit Ausnahme des Wasserstoff-Restes haben, umgesetzt wird und diese ggf. gereinigt und/oder isoliert wird
und wenigstens eine Verbindung der allgemeinen Formel III in einem Reaktionsmedium ggf. in Gegenwart wenigstens einer Base mit wenigstens einer Verbindung der allgemeinen Formel $R^4$-C(=O)-X, worin $R^4$ die vorstehend genannte Bedeutung hat und X für eine Abgangsgruppe, vorzugsweise für einen Halogen-Rest, besonders bevorzugt für ein Chlor- oder Bromatom steht, oder in einem Reaktionsmedium in Gegenwart wenigstens eines Kupplungsreagenzes ggf. in Gegenwart wenigstens einer Base mit einer Verbindung der allgemeinen Formel $R^4$-C(=O)-OH, worin $R^4$ die vorstehend genannte Bedeutung hat, zu einer Verbindung der allgemeinen Formel IV,

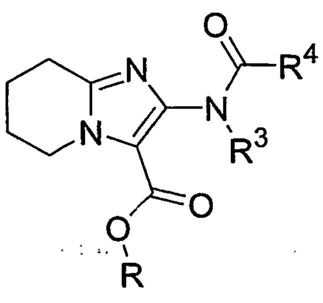

IV

worin R und $R^4$ die vorstehend genannte Bedeutung haben und $R^3$ die vorstehend genannte Bedeutung mit Ausnahme des Wasserstoff-Restes hat, umgesetzt wird und diese ggf. gereinigt und/oder isoliert wird,
oder

wenigstens eine Verbindung der allgemeinen Formel II, worin R die vorstehend genannte Bedeutung hat, in einem Reaktionsmedium ggf. in Gegenwart wenigstens einer Base mit Wenigstens einer Verbindung der allgemeinen Formel $R^4$-C(=O)-X, worin $R^4$ die vorstehend genannte Bedeutung hat und X für eine Abgangsgruppe, vorzugsweise für einen Halogen-Rest, besonders bevorzugt für ein Chlor- oder Bromatom steht, oder in einem Reaktionsmedium in Gegenwart wenigstens eines Kupplungsreagenzes ggf. in Gegenwart wenigstens einer Base mit einer Verbindung der allgemeinen Formel $R^4$-C(=O)-OH, worin $R^4$ die vorstehend genannte Bedeutung hat, zu einer Verbindung der allgemeinen Formel V,

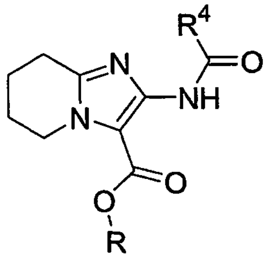

V

worin R und $R^4$ die vorstehend genannte Bedeutung haben, umgesetzt wird und diese ggf. gereinigt und/oder isoliert wird und wenigstens eine Verbindung der allgemeinen Formel V in einem Reaktionsmedium in Gegenwart wenigstens einer Base, vorzugsweise wenigstens eines Metallhydrids, mit wenigstens einer Verbindung der allgemeinen Formel $R^3$-X, worin $R^3$ die vorstehend genannte Bedeutung mit Ausnahme des Wasserstoff-Restes hat und X für eine Abgangsgruppe, vorzugsweise für einen Halogen-Rest, besonders bevorzugt für ein Chlor- oder Bromatom steht, zu einer Verbindung der allgemeinen Formel IV, worin R, $R^3$ und $R^4$ die vorstehend genannte Bedeutung haben wobei $R^3$ nicht gleich Wasserstoff ist, umgesetzt wird und diese ggf. gereinigt und/oder isoliert wird,
und wenigstens eine Verbindung der allgemeinen Formel IV in einem Reaktionsmedium in Gegenwart wenigstens einer Base, vorzugsweise in Gegenwart wenigstens eines Metallhydroxids, zu einer Verbindung der allgemeinen Formel VI,

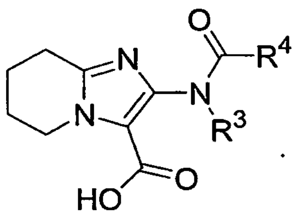

VI

worin $R^3$ die vorstehend genannte Bedeutung mit Ausnahme des Wasserstoff-Restes hat und $R^4$ die vorstehend genannte Bedeutung hat, umgesetzt wird und diese ggf. gereinigt und/oder isoliert wird und wenigstens eine Verbindung der allgemeinen Formel VI in einem Reaktionsmedium in Gegenwart wenigstens eines Kupplungsreagenzes ggf. in Gegenwart wenigstens einer Base mit wenigstens einer Verbindung der allgemeinen Formel $HNR^1R^2$, worin $R^1$ die vorstehend genannte Bedeutung hat und $R^2$ für Wasserstoff steht, zu einer Verbindung der allgemeinen Formel I,

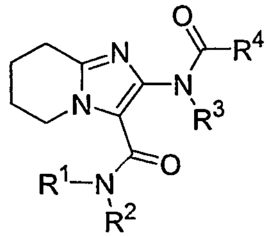

I

worin $R^3$ die vorstehend genannte Bedeutung mit Ausnahme des Wasserstoff-Restes hat, $R^1$ und $R^4$ die vorstehend

genannte Bedeutung haben und $R^2$ für Wasserstoff steht, umgesetzt wird und diese ggf. gereinigt und/oder isoliert wird und ggf. wenigstens eine Verbindung der allgemeinen Formel I, worin $R^3$ die vorstehend genannte Bedeutung mit Ausnahme des Wasserstoff-Restes hat, $R^1$ und $R^4$ die vorstehend genannte Bedeutung haben und $R^2$ für Wasserstoff steht, in einem Reaktionsmedium in Gegenwart wenigstens einer Base, vorzugsweise eines Metallhydrids, mit wenigstens einer Verbindung der allgemeinen Formel $R^2$-X, worin $R^2$ die vorstehend genannte Bedeutung mit Ausnahme des Wasserstoff-Restes hat und X für eine Abgangsgruppe, vorzugsweise einen Halogen-Rest, besonders bevorzugt für ein Chlor- oder Bromatom steht, zu einer Verbindung der allgemeinen Formel I, worin $R^1$ und $R^4$ die vorstehend genannte Bedeutung haben sowie $R^2$ und $R^3$ die vorstehend genannte Bedeutung mit Ausnahme des Wasserstoff-Restes haben, umgesetzt wird und diese ggf. gereinigt und/oder isoliert wird
oder
wenigstens eine Verbindung der allgemeinen Formel VI in einem Reaktionsmedium in Gegenwart wenigstens eines Kupplungsreagenzes ggf. in Gegenwart wenigstens einer Base mit wenigstens einer Verbindung der allgemeinen Formel $HNR^1R^2$, worin $R^1$ und $R^2$ die vorstehend genannte Bedeutung mit Ausnahme des Wasserstoff-Restes haben, zu einer Verbindung der allgemeinen Formel I, worin $R^1$, $R^2$ und $R^3$ und die vorstehend genannte Bedeutung mit Ausnahme des Wasserstoff-Restes haben und $R^4$ die vorstehend genannte Bedeutung hat, umgesetzt wird und diese ggf. gereinigt und/oder isoliert wird.

Ein weiterer Gegenstand der vorliegenden Erfindung ist ein Verfahren zur Herstellung von Verbindungen der vorstehend angegebenen allgemeinen Formel I gemäß dem wenigstens eine Verbindung der allgemeinen Formel II,

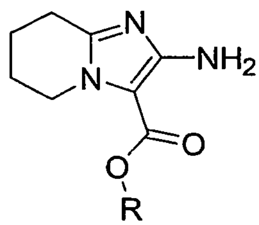

II

worin R für einen linearen oder verzweigten $C_{1-6}$-Alkyl-Rest, vorzugsweise für einen Methyl- oder Ethyl-Rest steht, in einem Reaktionsmedium ggf. in Gegenwart wenigstens einer Base mit wenigstens einer Verbindung der allgemeinen Formel $R^4$-C(=O)-X, worin $R^4$ die Bedeutung die vorstehend genannte Bedeutung hat und X für eine Abgangsgruppe, vorzugsweise für einen Halogen-Rest, besonders bevorzugt für ein Chlor- oder Bromatom steht, oder in einem Reaktionsmedium in Gegenwart wenigstens eines Kupplungsreagenzes ggf. in Gegenwart wenigstens einer Base mit einer Verbindung der allgemeinen Formel $R^4$-C(=O)-OH, worin $R^4$ die Bedeutung vorstehend genannte Bedeutung hat, zu einer Verbindung der allgemeinen Formel IV,

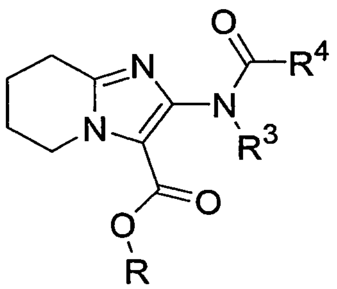

IV

worin R und und $R^4$ die vorstehend genannte Bedeutung haben und $R^3$ gleich Wasserstoff ist, umgesetzt wird und diese ggf. gereinigt und/oder isoliert wird,
und wenigstens eine Verbindung der allgemeinen Formel IV in einem Reaktionsmedium in Gegenwart wenigstens einer Base, vorzugsweise in Gegenwart wenigstens eines Metallhydroxids, zu einer Verbindung der allgemeinen Formel VI,

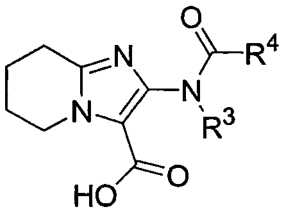

VI

worin $R^4$ die vorstehend genannte Bedeutung hat und $R^3$ gleich Wasserstoff ist, umgesetzt wird und diese ggf. gereinigt und/isoliert wird, und

wenigstens eine Verbindung der allgemeinen Formel VI in einem Reaktionsmedium in Gegenwart wenigstens eines Kupplungsreagenzes ggf. in Gegenwart wenigstens einer Base mit wenigstens einer Verbindung der allgemeinen Formel $HNR^1R^2$, worin $R^1$ und $R^2$ die vorstehend genannte Bedeutung haben, zu einer Verbindung der allgemeinen Formel I,

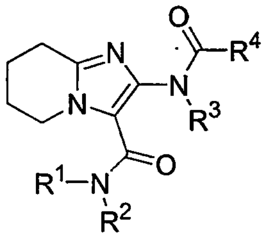

I

worin $R^1$, $R^2$ und $R^4$ die vorstehend genannte Bedeutung haben und $R^3$ gleich Wasserstoff ist, umgesetzt wird und diese ggf. gereinigt und/oder isoliert wird.

Die erfindungsgemäßen Verfahren zur Herstellung substituierter 5,6,7,8-Tetrahydro-imidazo[1,2-a]pyridin-2-ylamin-Verbindungen der vorstehend angegebenen allgemeinen Formel I sind auch in den nachfolgenden Schemata 1 bis 3 wiedergegeben.

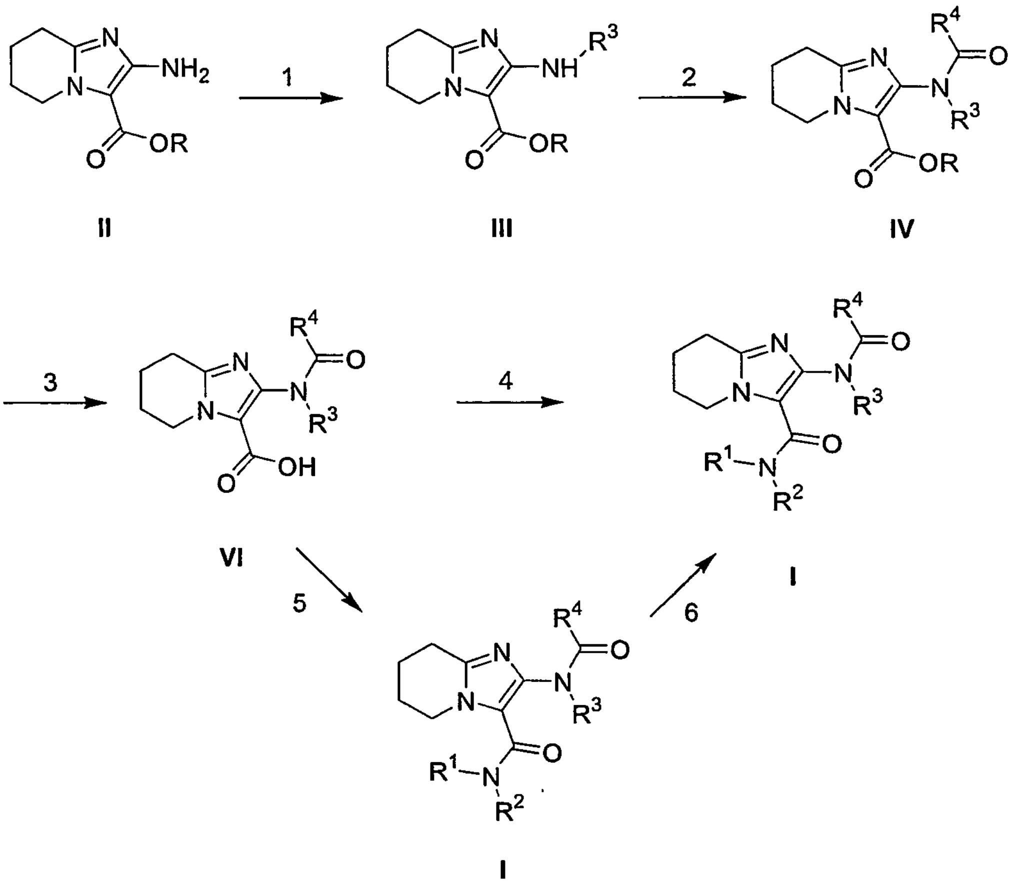


II III IV

VI I

I

Schema 1.

In Stufe 1 werden Verbindungen der vorstehend angegebenen allgemeinen Formel II mit Aldehyden der allgemeinen Formel $R^3$-C(=O)-H, worin $R^3$ die vorstehend genannte Bedeutung mit Ausnahme des Wasserstoff-Restes hat, in einem Reaktionsmedium, vorzugsweise ausgewählt aus der Gruppe bestehend aus Diethylether, Tetrahydrofuran, Methanol, Ethanol, Dichlormethan, Dichlorethan, Chloroform, Toluol und entsprechenden Mischungen, unter Zusatz eines Reduktionsmittels, vorzugsweise ausgewählt aus der Gruppe bestehend aus Natriumborhydrid, Natriumacetoxyborhydrid oder Natriumcyanoborhydrid, ggf. in Gegenwart wenigstens einer organischen Säure, vorzugsweise in Gegenwart von Essigsäure, bei Temperaturen von vorzugsweise -70°C bis 100°C zu Verbindungen der allgemeinen Formel III umgesetzt.

In Stufe 2 werden Verbindungen der vorstehend angegebenen allgemeinen Formel III mit Carbonsäuren der allgemeinen Formel $R^4$-C(=O)-OH, worin $R^4$ die vorstehend genannte Bedeutung hat, in einem Reaktionsmedium, vorzugsweise ausgewählt aus der Gruppe bestehend aus Diethylether, Tetrahydrofuran, Acetonitril, Methanol, Ethanol, Dimethylformamid, Dichlormethan und entsprechenden Mischungen, ggf. in Gegenwart wenigstens eines Kupplungsreagenzes, vorzugsweise ausgewählt aus der Gruppe bestehend aus 1-Benzotriazolyloxy-tris-(dimethylamino)-phosphonium hexafluorophosphat (BOP), Dicyclohexylcarbodiimid (DCC), N'-(3-Dimethylaminopropyl)-N-ethylcarbodiimid (EDCI), N-[(Dimethyamino)-1H-1, 2, 3-triazolo[4, 5-b]pyridino-1-ylmethylen]-N-methylmethanaminium hexafluorophosphat N-oxid (HATU), O-(Benzotriazol-1-yl)-N,N,N',N'-tetramethyluroniom hexafluorophosphat (HBTU) und 1-Hydroxy-7-azabenzotriazol (HOAt), ggf. in Gegenwart wenigstens einer anorganischen Base, vorzugsweise ausgewählt aus der Gruppe bestehend aus Kaliumcarbonat und Cäsiumcarbonat, oder wenigstens einer organischen Base, vorzugsweise ausgewählt aus der Gruppe bestehend aus Triethylamin, Pyridin, Dimethylaminopyridin und Diisopropylethylamin vorzugsweise bei Temperaturen von -70°C bis 100°C zu Verbindungen der allgemeinen Formel IV umgesetzt.

Alternativ werden Verbindungen der allgemeinen Formel III mit Carbonsäurederivaten bzw. Kohlensäurederivaten der allgemeinen Formel $R^4$-C(=O)-X, wobei $R^4$ die vorstehend genannte Bedeutung hat und X für einen Halogen-Rest, vorzugsweise Chlor oder Brom, steht, in einem Reaktionsmedium, vorzugsweise ausgewählt aus der Gruppe bestehend aus Diethylether, Tetrahydrofuran, Acetonitril, Methanol, Ethanol, Dimethylformamid, Dichlormethan und

entsprechenden Mischungen, ggf. in Gegenwart wenigstens einer organischen Base, vorzugsweise ausgewählt aus der Gruppe bestehend aus Triethylamin, Dimethylaminopyridin, Pyridin und Diisopropylamin, oder wenigstens einer anorganischen Base bei Temperaturen von vorzugsweise -70°C bis 100°C zu Verbindungen der allgemeinen Formel IV umgesetzt.

Ebenfalls können Verbindungen der allgemeinen Formel II mit Carbonsäuren der allgemeinen Formel $R^4$-C(=O)-OH oder Verbindungen der allgemeinen Formel $R^4$-C(=O)-X, wie vorstehend in Stufe 2 beschrieben, zu Verbindungen der allgemeinen Formel IV, worin R und $R^4$ die vorstehend genannte Bedeutung haben und $R^3$ gleich Wasserstoff ist, umgesetzt werden.

In Stufe 3 werden Verbindungen der allgemeinen Formel IV in einem geeigneten Reaktionsmedium, vorzugsweise ausgewählt aus der Gruppe bestehend aus Dioxan, Tetrahydrofuran, Diethylether, Methanol, Ethanol, Isopropanol, Wasser und entsprechenden Mischungen, unter Zusatz wenigstens einer anorganischen Base, vorzugsweise unter Zusatz wengistens eines Metallhydroxids, beispielsweise Natriumhydroxid, Kaliumhydroxid oder Lithiumhydroxid, bei Temperaturen von vorzugsweise 0°C bis 30°C zu Verbindungen der allgemeinen Formel VI umgesetzt. Vorzugsweise erfolgt die Reaktion in einem Reaktionsmedium, das aus Methanol, Dioxan und einer 4 M Natriumhydroxid-Lösung in Wasser mit einem Verhältnis der entsprechenden Volumina von 15:4:1 besteht.

In Stufe 4 werden Verbindungen der vorstehend angegebenen allgemeinen Formel VI mit Aminen der allgemeinen Formel $HNR^1R^2$, worin $R^1$ und $R^2$ die vorstehend genannte Bedeutung haben, in einem Reaktionsmedium, vorzugsweise ausgewählt aus der Gruppe bestehend aus Diethylether, Tetrahydrofuran, Acetonitril, Methanol, Ethanol, Dimethylformamid, Dichlormethan und entsprechenden Mischungen, ggf. in Gegenwart wenigstens eines Kupplungsreagenzes, vorzugsweise ausgewählt aus der Gruppe bestehend aus 1-Benzotriazolyloxy-tris-(dimethylamino)-phosphonium hexafluorophosphat (BOP), Dicyclohexylcarbodiimid (DCC), N'-(3-Dimethylaminopropyl)-N-ethylcarbodiimid (EDCI), N-[(Dimethyamino)-1H-1, 2, 3-triazolo[4, 5-b]pyridino-1-ylmethylen]-N-methylmethanaminium hexafluorophosphat N-oxid (HATU), O-(Benzotriazol-1-yl)-N,N,N',N'-tetramethyluroniom hexafluorophosphat (HBTU) und 1-Hydroxy-7-azabenzotriazol (HOAt), ggf. in Gegenwart wenigstens einer anorganischen Base, vorzugsweise ausgewählt aus der Gruppe bestehend aus Kaliumcarbonat und Cäsiumcarbonat, oder einer organischen Base, vorzugsweise ausgewählt aus der Gruppe bestehend aus Triethylamin, Pyridin, Dimethylaminopyridin und Diisopropylethylamin vorzugsweise bei Temperaturen von -70°C bis 100°C zu Verbindungen der allgemeinen Formel I umgesetzt.

In Stufe 5 werden Verbindungen der vorstehend angegebenen allgemeinen Formel VI mit Aminen der allgemeinen Formel $HNR^1R^2$, worin $R^1$ die vorstehend genannte Bedeutung hat und $R^2$ für Wasserstoff steht, mit den Methoden wie vorstehend in Schema 1, Stufe 4 beschrieben zu Verbindungen der allgemeinen Formel I, worin $R^1$, $R^3$ und $R^4$ die vorstehend genannte Bedeutung haben, $R^3$ nicht gleich Wasserstoff ist und $R^2$ für Wasserstoff steht, umgesetzt.

In Stufe 6 werden Verbindungen der allgemeinen Formel I, worin $R^1$, $R^3$ und $R^4$ die vorstehend genannte Bedeutung haben, $R^3$ nicht gleich Wasserstoff ist und $R^2$ für Wasserstoff steht, mit Verbindungen der allgemeinen Formel $R^2$-X, worin $R^2$ die vorstehend genannte Bedeutung hat und nicht gleich Wasserstoff ist und X für einen Halogen-Rest, bevorzugt Chlor, steht, in einem Reaktionsmedium, vorzugsweise ausgewählt aus der Gruppe bestehend aus Dimethylformamid, Heptan, Hexan, Toluol, Tetrahydrofuran, Diethylether und entsprechenden Mischungen, unter Zusatz wenigstens eines Metallhydrids, vorzugsweise unter Zusatz wenigstens eines Metallhydridsalzes ausgewählt aus der Gruppe bestehend aus Natriumhydrid, Kaliumhydrid und Lithiumhydrid, bei Temperaturen von vorzugsweise 0 °C bis 40 °C zu Verbindungen der allgemeinen Formel I, worin $R^1$, $R^2$, $R^3$ und $R^4$ die vorstehend genannte Bedeutung haben wobei $R^2$ und $R^3$ nicht gleich Wasserstoff sind, umgesetzt.

Die Verbindungen der allgemeinen Formel IV lassen sich ebenfalls wie in Schema 2 beschrieben erhalten.

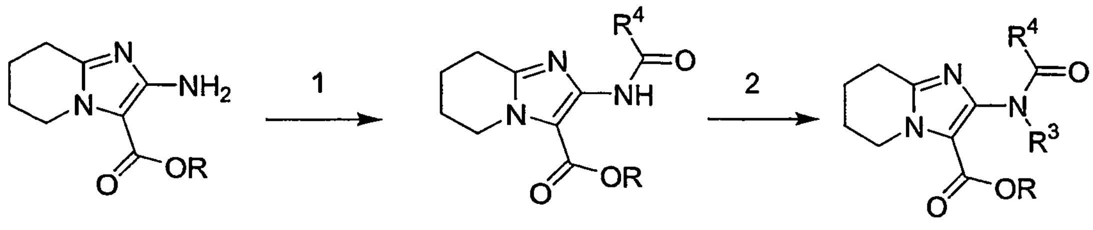


II V IV

Schema 2.

In Stufe 1 werden Verbindungen der vorstehend angegebenen allgemeinen Formel II mit Carbonsäuren der allgemeinen Formel $R^4$-C(=O)-OH, worin $R^4$ die vorstehend genannte Bedeutung hat, oder mit Carbonsäurederivaten

bzw. Kohlensäurederivaten der allgemeinen Formel $R^4$-C(=O)-X, wobei $R^4$ die vorstehend genannte Bedeutung hat und X für einen Halogen-Rest, vorzugsweise Chlor oder Brom, steht, mit den gleichen Methoden, wie unter Schema 1, Stufe 2, beschrieben, zu Verbindungen der allgemeinen Formel V umgesetzt.

In Stufe 2 werden Verbindungen der allgemeinen Formel V mit Verbindungen der allgemeinen Formel $R^3$-X, worin $R^3$ die vorstehend genannte Bedeutung hat und nicht gleich Wasserstoff ist und X für einen Halogen- Rest, bevorzugt Chlor, steht, mit den gleichen Methoden, wie unter Schema 1, Stufe 6, beschrieben, zu Verbindungen der allgemeinen Formel IV umgesetzt.

Die Verbindungen der allgemeinen Formel II lassen sich wie in Schema 3 beschrieben erhalten.

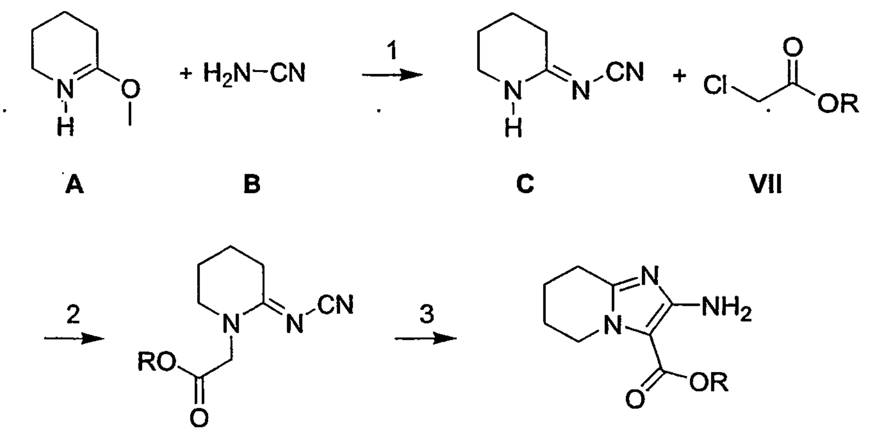

A B C VII

VIII II

Schema 3.

In Stufe 1 wird 6-Methoxy-2,3,4-5-tetrahydropyridin (A) in einem Reaktionsmedium, vorzugsweise ausgewählt aus der Gruppe bestehend aus Methanol, Ethanol und Isopropanol, mit Cyanamid B vorzugsweise bei einer Temperatur von 0°C bis 30 °C zur gewünschten Verbindung Piperidin-2-yliden-cyanamid (C) umgesetzt. 6-Methoxy-2,3,4-5-tetrahydropyridin (A) wurde gemäß den Referenzen "Product class 18: pyridopyridazines; Sako, M.; Science of Synthesis 2004, 1109-1153"; "Synthesis of pyrido[4,3-d]pyrimidin-5(6H)-ones via anionic cycloaddition of methyl-2,4-dimethoxy-6-methyl-5-pyrimidinecarboxylate with imines; Wada, A. et al.; Chemical and Pharmaceutical Bulletin 1991, 1189-1192" und "Reaction of lactim ethers with 2-(carbethoxymethyl)-piperidines; Takahata, H. et al. Fukusokan Kagaku Toronkai Koen Yoshishu, 12th (1979), 296-300" erhalten. Die entsprechenden Teile der Referenzen gelten hiermit als Teil der Offenbarung.

Die Verbindung C aus Stufe 1 wird ohne weitere Aufreinigung in Stufe 2 mit einer Verbindung der allgemeinen Formel VII, worin R für einen linearen oder verzweigten $C_{1-6}$-Alkyl-Rest, vorzugsweise für einen Methyl- oder Ethyl-Rest steht, in einem Reaktionsmedium, vorzugsweise ausgewählt aus der Gruppe bestehend aus Acetonitril, Dichlormethan, Chloroform, Dimethylformamid, Dimethylacetamid und Dimethylsulfoxid, unter Zusatz einer anorganischen Base, vorzugsweise ausgewählt aus der Gruppe bestehend aus Kaliumcarbonat, Natriumcarbonat, Lithiumcarbonat und Magnesiumcarbonat, vorzugsweise bei einer Temperatur von 50°C bis 150°C zu einer Verbindung der allgemeinen Formel VIII, worin R die vorstehend genannte Bedeutung hat, umgesetzt.

In Stufe 3 wird eine Verbindung der allgemeinen Formel VIII in einem Reaktionsmedium, vorzugsweise ausgewählt aus der Gruppe bestehend aus Methanol, Ethanol und Isopropanol, unter Zusatz eines Alkalimetallalkoholat-Salzes, vorzugsweise ausgewählt aus der Gruppe bestehend aus Natriummethanolat, Natriumethanolat, Kaliummethanolat und Kaliumethanolat, vorzugsweise bei einer Temperatur von 50°C bis 120 °C zu einer Verbindung der allgemeinen Formel II umgesetzt.

Die Verbindungen der vorstehend angegebenen Formeln $R^3$-C(=O)-H, $R^4$-C(=O)-OH, $R^4$-C(=O)-X, $HNR^1R^2$, $R^2$-X, $R^3$-X,B und VII sind jeweils am Markt käuflich erhältlich und können auch nach üblichen, dem Fachmann bekannten Verfahren hergestellt werden.

Die vorstehend beschriebenen Umsetzungen können jeweils unter üblichen, dem Fachmann geläufigen Bedingungen, beispielsweise in Hinblick auf Druck oder Reihenfolge der Zugabe der Komponenten durchgeführt werden. Ggf. kann die unter den jeweiligen Bedingungen optimale Verfahrensführung vom Fachmann durch einfache Vorversuche ermittelt werden. Die nach den vorstehend beschriebenen Umsetzungen erhaltenen Zwischen- und Endprodukte können jeweils, falls gewünscht und/oder erforderlich, nach üblichen, dem Fachmann bekannten Methoden gereinigt und/oder

isoliert werden. Geeignete Reinigungsverfahren sind beispielsweise Extraktionsverfahren und chromatographische Verfahren wie Säulenchromatographie oder präparative Chromatographie. Sämtliche der vorstehend beschriebenen Verfahrensschritte sowie jeweils auch die Reinigung und/oder Isolierung von Zwischen- oder Endprodukten können teilweise oder vollständig unter einer Inertgasatmossphäre, vorzugsweise unter Stickstoffatmossphäre, durchgeführt werden.

Die erfindungsgemäßen substituierten 5,6,7,8-Tetrahydro-imidazo[1,2-a]pyridin-2-ylamin-Verbindungen der vorstehend genannten allgemeinen Formel I sowie entsprechende Stereoisomere können sowohl in Form ihrer freien Basen, ihrer freien Säuren wie auch in Form entsprechender Salze, insbesondere physiologisch verträglicher Salze, isoliert werden. Die freien Basen der jeweiligen erfindungsgemäßen substituierten 5,6,7,8-Tetrahydro-imidazo[1,2-a] pyridin-2-ylamin-Verbindungen der vorstehend genannten allgemeinen Formel I sowie entsprechender Stereoisomere können beispielsweise durch Umsetzung mit einer anorganischen oder organischen Säure, vorzugsweise mit Salzsäure, Bromwasserstoffsäure, Schwefelsäure, Phosphorsäure, Methansulfonsäure, p-Toluolsulfonsäure, Kohlensäure, Ameisensäure, Essigsäure, Oxalsäure, Bernsteinsäure, Weinsäure, Mandelsäure, Fumarsäure, Milchsäure, Citronensäure, Glutaminsäure oder Asparaginsäure, in die entsprechenden Salze, vorzugsweise physiologisch verträglichen Salze, überführt werden. Die freien Basen der jeweiligen substituierten 5,6,7,8-Tetrahydro-imidazo[1,2-a]pyridin-2-ylamin-Verbindungen der vorstehend genannten allgemeinen Formel I und entsprechender Stereoisomere können ebenfalls mit der freien Säure oder einem Salz eines Zuckerersatzstoffes, wie z.B. Saccharin, Cyclamat oder Acesulfam, in die entsprechenden physiologisch verträglichen Salze überführt werden.

Entsprechend können die freien Säuren der substituierten 5,6,7,8-Tetrahydro-imidazo[1,2-a]pyridin-2-ylamin-Verbindungen der vorstehend genannten allgemeinen Formel I und entsprechender Stereoisomere durch Umsetzung mit einer geeigneten Base in die entsprechenden physiologisch verträglichen Salze überführt werden. Beispielhaft seien die Alkalimetallsalze, Erdalkalimetallsalze oder Ammoniumsalze $[NH_xR_{4-x}]^+$, worin x = 0, 1, 2, 3 oder 4 ist und R für einen linearen oder verzweigten $C_{1-4}$-Alkyl-Rest steht, genannt.

Die erfindungsgemäßen substituierten 5,6,7,8-Tetrahydro-imidazo[1,2-a]pyridin-2-ylamin-Verbindungen der vorstehend genannten allgemeinen Formel I und entsprechende Stereoisomere können ggf., ebenso wie die entsprechenden Säuren, die entsprechenden Basen oder Salze dieser Verbindungen, nach üblichem, dem Fachmann bekannten Methoden auch in Form ihrer Solvate, vorzugsweise in Form ihrer Hydrate, erhalten werden.

Sofern die erfindungsgemäßen substituierten 5,6,7,8-Tetrahydro-imidazo[1,2-a]pyridin-2-ylamin-Verbindungen der vorstehend genannten allgemeinen Formel I nach ihrer Herstellung in Form einer Mischung ihrer Stereoisomeren, vorzugsweise in Form ihrer Racemate oder anderer Mischungen ihrer verschiedenen Enantiomeren und/oder Diastereomeren erhalten werden, können diese nach üblichen, dem Fachmann bekannten Verfahren getrennt und ggf. isoliert werden. Beispielhaft seien chromatographische Trennverfahren, insbesondere Flüssigkeitschromatographie-Verfahren unter Normaldruck oder unter erhöhtem Druck, bevorzugt MPLC- und HPLC-Verfahren, sowie Verfahren der fraktionierten Kristallisation genannt. Dabei können insbesondere einzelne Enantiomeren, z.B. mittels HPLC an chiraler stationärer Phase oder mittels Kristallisation mit chiralen Säuren, etwa (+)-Weinsäure, (-)-Weinsäure oder (+)-10-Camphersulfonsäure, gebildete diastereomere Salze voneinander getrennt werden.

Die erfindungsgemäßen substituierten 5,6,7,8-Tetrahydro-imidazo[1,2-a]pyridin-2-ylamin-Verbindungen der vorstehend genannten allgemeinen Formel I und entsprechende Stereoisomere sowie jeweils die entsprechenden Säuren, Basen, Salze und Solvate sind toxikologisch unbedenklich und eignen sich daher als pharmazeutische Wirkstoffe in Arzneimitteln.

Ein weiterer Gegenstand der vorliegenden Erfindung ist daher ein Arzneimittel enthaltend wenigstens eine erfindungsgemäße 5,6,7,8-Tetrahydro-imidazo[1,2-a]pyridin-2-ylamin-Verbindung der vorstehend angegebenen allgemeinen Formel I, jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form eines entsprechenden Salzes, oder jeweils in Form eines entsprechenden Solvates, sowie ggf. einen oder mehrere pharmazeutisch verträgliche Hilfsstoffe.

Diese erfindungsgemäßen Arzneimittel eignen sich insbesondere zur Vanilloid-Rezeptor 1-(VR1/TRPV1)-Regulation, vorzugsweise zur Vanilloid-Rezeptor 1-(VR1/TRPV1)-Hemmung und/oder zur Batrachotoxin-(BTX)-Rezeptor-Regulation, vorzugsweise zur Batrachotoxin-(BTX)-Rezeptor-Hemmung und/oder zur Opioid-Rezeptor-Regulation, vorzugsweise zur $\mu$-Opioid-Rezeptor-Regulation.

Ebenfalls bevorzugt eignen sich die erfindungsgemäßen Arzneimittel zur Prophylaxe und/oder Behandlung von Störungen oder Krankheiten, die zumindest teilweise durch Vanilloid-Rezeptoren 1 und/oder durch Batrachotoxin-Rezeptoren und/oder durch Opioid-Rezeptoren, insbesondere durch $\mu$-Opioid-Rezeptoren, vermittelt werden.

Bevorzugt eignet sich das erfindungsgemäße Arzneimittel daher zur Behandlung und/oder Prophylaxe von Schmerz, vorzugsweise von Schmerz ausgewählt aus der Gruppe bestehend aus akutem Schmerz, chronischem Schmerz und neuropathischem Schmerz; zur Prophylaxe und/oder Behandlung von einer oder mehreren Erkrankungen ausgewählt aus der Gruppe bestehend aus Migräne; Depressionen; Harninkontinenz; Husten; neurodegenerativen Erkrankungen, vorzugsweise ausgewählt aus der Gruppe bestehend aus Morbus Parkinson, Morbus Huntington, Morbus Alzheimer und multipler Sklerose; Störungen der Nahrungsmittelaufnahme, vorzugsweise ausgewählt aus der Gruppe

bestehend aus Bulimie, Anorexie, Fettsucht und Kachexie; Angstzuständen; kognitiven Dysfunktionen, vorzugsweise Gedächtnisstörungen; kognitive Mangelzustände (attention deficit syndrom, ADS); Epilepsie; Diarrhoe und Pruritus; zur Prophylaxe und/oder Behandlung von Alkohol- und/oder Drogen- und/oder Medikamentenmißbrauch und Alkohol- und/oder Drogen- und/oder Medikamentenabhängigkeit, vorzugsweise zur Prophylaxe und/oder Verminderung von Entzugserscheinungen bei Alkohol- und/oder Drogen- und/oder Medikamentenabhängigkeit; zur Prophylaxe und/oder Verminderung einer Toleranzentwicklung gegenüber Medikamenten, insbesondere Medikamenten auf Basis von Opioiden; zur Regulation der Nahrungsaufnahme; zur Modulation der Bewegungsaktivität; zur Regulation des kardiovaskulären Systems; zur Lokalanästhesie; zur Vigilanzsteigerung; zur Libidosteigerung; zur Diurese und/oder zur Antinatriurese.

Besonders bevorzugt eignet sich das erfindungsgemäße Arzneimittel daher zur Behandlung und/oder Prophylaxe von Schmerz, vorzugsweise von Schmerz ausgewählt aus der Gruppe bestehend aus akutem Schmerz, chronischem Schmerz und neuropathischem Schmerz; zur Prophylaxe und/oder Behandlung von einer oder mehreren Erkrankungen ausgewählt aus der Gruppe bestehend aus Migräne; Depressionen; neurodegenerativen Erkrankungen, vorzugsweise ausgewählt aus der Gruppe bestehend aus Morbus Parkinson, Morbus Huntington, Morbus Alzheimer und multipler Sklerose; Angstzuständen; kognitiven Dysfunktionen, vorzugsweise Gedächtnisstörungen; kognitive Mangelzustände (attention deficit syndrom, ADS); Epilepsie; zur Prophylaxe und/oder Behandlung von Alkohol- und/oder Drogen- und/oder Medikamentenmißbrauch und Alkohol- und/oder Drogen- und/oder Medikamentenabhängigkeit, vorzugsweise zur Prophylaxe und/oder Verminderung von Entzugserscheinungen bei Alkohol- und/oder Drogen- und/oder Medikamentenabhängigkeit; zur Prophylaxe und/oder Verminderung einer Toleranzentwicklung gegenüber Medikamenten, insbesondere Medikamenten auf Basis von Opioiden.

Ganz besonders bevorzugt eignet sich das erfindungsgemäße Arzneimittel zur Behandlung und/oder Prophylaxe von Schmerz, vorzugsweise von Schmerz ausgewählt aus der Gruppe bestehend aus akutem Schmerz, chronischem Schmerz und neuropathischem Schmerz.

Ein weiterer Gegenstand der vorliegenden Erfindung ist die Verwendung wenigstens einer erfindungsgemäßen 5,6,7,8-Tetrahydro-imidazo[1,2-a]pyridin-2-ylamin-Verbindung der vorstehend angegebenen allgemeinen Formel I, jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form eines entsprechenden Salzes, oder jeweils in Form eines entsprechenden Solvates, sowie ggf. eines oder mehrerer pharmazeutisch verträglicher Hilfsstoffe zur Herstellung eines Arzneimittels zur Vanilloid-Rezeptor 1-(VR1/TRPV1)-Regulation, vorzugsweise zur Vanilloid-Rezeptor 1-(VR1/TRPV1)-Hemmung, und/oder zur Batrachotoxin-(BTX)-Rezeptor-Regulation, vorzugsweise zur Batrachotoxin-(BTX)-Rezeptor-Hemmung und/oder zur Opioid-Rezeptor-Regulation, vorzugsweise zur $\mu$-Opioid-Rezeptor-Regulation.

Bevorzugt ist die Verwendung wenigstens einer substituierten 5,6,7,8-Tetrahydro-imidazo[1,2-a]pyridin-2-ylamin-Verbindung der vorstehend angegebenen allgemeinen Formel I, jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form eines entsprechenden Salzes, oder jeweils in Form eines entsprechenden Solvates, sowie ggf. eines oder mehrerer pharmazeutisch verträglicher Hilfsstoffe zur Herstellung eines Arzneimittels zur Prophylaxe und/oder Behandlung von Störungen oder Krankheiten, die zumindest teilweise durch Vanilloid-Rezeptoren 1 und/oder durch Batrachotoxin-Rezeptoren und/oder durch Opioid-Rezeptoren vermittelt werden.

Besonders bevorzugt ist die Verwendung wenigstens einer substituierten 5,6,7,8-Tetrahydro-imidazo[1,2-a]pyridin-2-ylamin-Verbindung der vorstehend angegebenen allgemeinen Formel I, jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form eines entsprechenden Salzes, oder jeweils in Form eines entsprechenden Solvates, sowie ggf. eines oder mehrerer pharmazeutisch verträglicher Hilfsstoffe zur Herstellung eines Arzneimittels zur Behandlung und/oder Prophylaxe von Schmerz, vorzugsweise von Schmerz ausgewählt aus der Gruppe bestehend aus akutem Schmerz, chronischem Schmerz und neuropathischem Schmerz; zur Prophylaxe und/oder Behandlung von einer oder mehreren Erkrankungen ausgewählt aus der Gruppe bestehend aus Migräne; Depressionen; Harninkontinenz; Husten; neurodegenerativen Erkrankungen, vorzugsweise ausgewählt aus der Gruppe bestehend aus Morbus Parkinson, Morbus Huntington, Morbus Alzheimer und multipler Sklerose; Störungen der Nahrungsmittelaufnahme, vorzugsweise ausgewählt aus der Gruppe bestehend aus Bulimie, Anorexie, Fettsucht und Kachexie; Angstzuständen; kognitiven Dysfunktionen, vorzugsweise Gedächtnisstörungen; kognitive Mangelzustände (attention deficit syndrom, ADS); Epilepsie; Diarrhoe und Pruritus; zur Prophylaxe und/oder Behandlung von Alkohol- und/oder Drogen- und/oder Medikamentenmißbrauch und Alkohol- und/oder Drogen- und/oder Medikamentenabhängigkeit, vorzugsweise zur Prophylaxe und/oder Verminderung von Entzugserscheinungen bei Alkohol- und/oder Drogen- und/oder Medikamentenabhängigkeit; zur Prophylaxe und/oder Verminderung einer Toleranzentwicklung gegenüber Medikamenten, insbesondere Medikamenten auf Basis von Opioiden; zur Regulation der Nahrungsaufnahme; zur Modulation der Bewegungsaktivität; zur Regulation des kardiovaskulären Systems; zur Lokalanästhesie; zur Vigilanzsteigerung; zur Libidosteigerung; zur Diurese und/oder zur Antinatriurese.

Ganz besonders bevorzugt ist die Verwendung wenigstens einer substituierten 5,6,7,8-Tetrahydro-imidazo [1,2-a]pyridin-2-ylamin-Verbindung der vorstehend angegebenen allgemeinen Formel I, jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form eines entsprechenden Salzes, oder jeweils in Form eines entsprechenden Solvates, sowie ggf. eines oder mehrerer pharmazeutisch verträglicher Hilfsstoffe zur Herstellung eines Arzneimittels zur Behandlung und/oder Prophylaxe von Schmerz, vorzugsweise von Schmerz ausgewählt aus der Gruppe bestehend aus akutem Schmerz, chronischem Schmerz und neuropathischem Schmerz; zur Prophylaxe und/oder Behandlung von einer oder mehreren Erkrankungen ausgewählt aus der Gruppe bestehend aus Migräne; Depressionen; neurodegenerativen Erkrankungen, vorzugsweise ausgewählt aus der Gruppe bestehend aus Morbus Parkinson, Morbus Huntington, Morbus Alzheimer und multipler Sklerose; Angstzuständen; kognitiven Dysfunktionen, vorzugsweise Gedächtnisstörungen; kognitive Mangelzustände (attention deficit syndrom, ADS); Epilepsie; zur Prophylaxe und/oder Behandlung von Alkohol- und/oder Drogen- und/oder Medikamentenmißbrauch und Alkohol- und/oder Drogen- und/oder Medikamentenabhängigkeit, vorzugsweise zur Prophylaxe und/oder Verminderung von Entzugserscheinungen bei Alkohol- und/oder Drogen- und/oder Medikamentenabhängigkeit; zur Prophylaxe und/oder Verminderung einer Toleranzentwicklung gegenüber Medikamenten, insbesondere Medikamenten auf Basis von Opioiden.

Noch weiter bevorzugt ist die Verwendung wenigstens einer substituierten 5,6,7,8-Tetrahydro-imidazo[1,2-a] pyridin-2-ylamin-Verbindung der vorstehend angegebenen allgemeinen Formel I, jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form eines entsprechenden Salzes, oder jeweils in Form eines entsprechenden Solvates, sowie ggf. eines oder mehrerer pharmazeutisch verträglicher Hilfsstoffe zur Herstellung eines Arzneimittels zur Behandlung und/oder Prophylaxe von Schmerz, vorzugsweise von Schmerz ausgewählt aus der Gruppe bestehend aus akutem Schmerz, chronischem Schmerz und neuropathischem Schmerz.

Das erfindungsgemäße Arzneimittel eignet sich zur Verabreichung an Erwachsene und Kinder einschließlich Kleinkindern und Säuglingen. Das erfindungsgemäße Arzneimittel kann als flüssige, halbfeste oder feste Arzneiform, beispielsweise in Form von Injektionslösungen, Tropfen, Säften, Sirupen, Sprays, Suspensionen, Tabletten, Patches, Kapseln, Pflastern, Zäpfchen, Salben, Cremes, Lotionen, Gelen, Emulsionen, Aerosolen oder in multipartikulärer Form, beispielsweise in Form von Pellets oder Granulaten, ggf. zu Tabletten verpreßt, in Kapseln abgefüllt oder in einer Flüssigkeit suspendiert, vorliegen und als solche auch verabreicht werden.

Neben wenigstens einer substituierten 5,6,7,8-Tetrahydro-imidazo[1,2-a]pyridin-2-ylamin-Verbindung der vorstehend angegebenen allgemeinen Formel I, ggf. in Form eines ihrer reinen Stereoisomeren, insbesondere Enantiomeren oder Diastereomeren, ihres Racemates oder in Form von Mischungen der Stereoisomeren, insbesondere der Enantiomeren oder Diastereomeren, in einem beliebigen Mischungsverhältnis, oder ggf. in Form eines entsprechenden Salzes oder jeweils in Form eines entsprechendes Solvates, enthält das erfindungsgemäße Arzneimittel üblicherweise weitere physiologisch verträgliche pharmazeutische Hilfsstoffe, die beispielsweise ausgewählt werden können aus der Gruppe bestehend aus Trägermaterialien, Füllstoffen, Lösungsmitteln, Verdünnungsmitteln, oberflächenaktiven Stoffen, Farbstoffen, Konservierungsstoffen, Sprengmitteln, Gleitmitteln, Schmiermitteln, Aromen und Bindemitteln.

Die Auswahl der physiologisch verträglichen Hilfsstoffe sowie die einzusetzenden Mengen derselben hängt davon ab, ob das Arzneimittel oral, subkutan, parenteral, intravenös, intraperitoneal, intradermal, intramuskulär, intranasal, buccal, rectal oder örtlich, zum Beispiel auf Infektionen an der Haut, der Schleimhäute und an den Augen, appliziert werden soll. Für die orale Applikation eignen sich bevorzugt Zubereitungen in Form von Tabletten, Dragees, Kapseln, Granulaten, Pellets, Tropfen, Säften und Sirupen, für die parenterale, topische und inhalative Applikation Lösungen, Suspensionen, leicht rekonstituierbare Trockenzubereitungen sowie Sprays. Die in dem erfindungsgemäßen Arzneimittel zum Einsatz kommenden erfindungsgemäßen substituierten 5,6,7,8-Tetrahydro-imidazo[1,2-a]pyridin-2-ylamin-Verbindungen in einem Depot in gelöster Form oder in einem Pflaster, gegebenenfalls unter Zusatz von die Hautpenetration fördernden Mitteln, sind geeignete perkutane Applikationszubereitungen. Oral oder perkutan anwendbare Zubereitungsformen können die jeweilige erfindungsgemäße substituierte 5,6,7,8-Tetrahydro-imidazo[1,2-a]pyridin-2-ylamin-Verbindung auch verzögert freisetzen.

Die Herstellung der erfindungsgemäßen Arzneimittel erfolgt mit Hilfe von üblichen, aus dem Stande der Technik bekannten Mitteln, Vorrichtungen, Methoden und Verfahren, wie sie beispielsweise in "Remington's Pharmaceutical Sciences", Herausgeber A.R. Gennaro, 17. Auflage, Mack Publishing Company, Easton, Pa, 1985, insbesondere in Teil 8, Kapitel 76 bis 93 beschrieben sind. Die an den Patienten zu verabreichende Menge der jeweiligen erfindungsgemäßen substituierten 5,6,7,8-Tetrahydro-imidazo[1,2-a]pyridin-2-ylamin-Verbindungen der vorstehend angegebenen allgemeinen Formel I kann variieren und ist beispielsweise abhängig vom Gewicht oder Alter des Patienten sowie von der Applikationsart, der Indikation und dem Schweregrad der Erkrankung. Üblicherweise werden 0,001 bis 100 mg/kg,

vorzugsweise 0,05 bis 75 mg/kg, besonders bevorzugt 0,05 bis 50 mg/kg, Körpergewicht des Patienten wenigstens einer solchen erfindungsgemäßen Verbindung appliziert.

**Pharmakologische Methoden:**

**I. Funktionelle Untersuchung am Vanilloid-Rezeptor 1 (VRI/TRPV1-Rezeptor)**

Die agonistische bzw. antagonistische Wirkung der zu untersuchenden Substanzen am Vanilloid-Rezeptor 1 (VR1/TRPV1) der Spezies Ratte kann mit folgendem Assay bestimmt werden. Gemäß diesem Assay wird der $Ca^{2+}$-Einstrom durch den Rezeptorkanal mit Hilfe eines $Ca^{2+}$-sensitiven Farbstoffs (Typ Fluo-4, Molecular Probes Europe BV, Leiden Niederlande) im Fluorescent Imaging Plate Reader (FLIPR, Molecular Devices, Sunnyvale, USA) quantifiziert.

**Methode:**

Komplett-Medium: 50 mL HAMS F12 Nutrient Mixture (Gibco Invitrogen GmbH, Karlsruhe, Deutschland) mit 10 Vol-% FCS (fetal calf serum, Gibco Invitrogen GmbH, Karlsruhe, Deutschland, hitzeinaktiviert);
2mM L-Glutamin (Sigma, München, Deutschland);
1 Gew-% AA-Lösung (Antibiotika/Antimykotika-Lösung, PAA, Pasching, Österreich)
und 25 ng/ml Medium NGF (2.5 S, Gibco Invitrogen GmbH, Karlsruhe, Deutschland)

Zellkultur-Platte: Poly-D-Lysin-beschichtete, schwarze 96-Loch-Platten mit klarem Boden (96 well black/clear plate, BD Biosciences, Heidelberg, Deutschland) werden zusätzlich mit Laminin (Gibco Invitrogen GmbH, Karlsruhe, Deutschland) beschichtet, indem Laminin auf eine Konzentration 100 μg/mL mit PBS (Ca-Mg-free PBS, Gibco Invitrogen GmbH, Karlsruhe, Deutschland) verdünnt wird. Es werden Aliquots mit einer Konzentration von 100 μg/mL an Laminin entnommen und bei -20 °C gelagert. Die Aliquots werden mit PBS im Verhältnis 1:10 auf 10 μg/mL Laminin verdünnt und jeweils 50 μL der Lösung in eine Vertiefung der Zellkultur-Platte pipettiert. Die Zellkultur-Platten werden mindestens zwei Stunden bei 37 °C inkubiert, die überstehende Lösung abgesaugt und die Vertiefungen werden jeweils zweimal mit PBS gewaschen. Die beschichteten Zellkultur-Platten werden mit überstehendem PBS aufbewahrt und dieses erst direkt vor der Aufgabe der Zellen entfernt.

**Präparation der Zellen:**

Enthaupteten Ratten wird die Wirbelsäule entnommen und diese direkt in kalten, d. h. in einem Eisbad befindlichen, HBSS-Puffer (Hank's buffered saline solution, Gibco Invitrogen GmbH, Karlsruhe, Deutschland) versetzt mit 1 Vol-% (Volumenprozent) einer AA-Lösung (Antibiotika/Antimykotika-Lösung, PAA, Pasching, Österreich) gelegt. Die Wirbelsäule wird längs durchtrennt und zusammen mit Fascien dem Wirbelkanal entnommen. Anschließend werden die Dorsalwurzelganglien (DRGs; dorsal root ganglia) entnommen und wiederum in kaltem HBSS-Puffer versetzt mit 1 Vol-% einer AA-Lösung aufbewahrt. Die vollständig von Blutresten und Spinalnerven befreiten DRGs werden jeweils in 500 μL kalte Collagenase Typ 2 (PAA, Pasching, Österreich) überführt und 35 Minuten bei 37 °C inkubiert. Nach Zugabe von 2.5 Vol-% Trypsin (PAA, Pasching, Österreich) wird weitere 10 Minuten bei 37 °C inkubiert. Nach der vollständigen Inkubation wird die Enzymlösung vorsichtig ab pipettiert und die zurückgebliebenen DRGs werden jeweils mit 500 μL Komplett-Medium versetzt. Die DRGs werden jeweils mehrfach suspendiert, mittels einer Spritze durch Kanülen Nr. 1, Nr. 12 und Nr. 16 gezogen und in 50 mL Falcon-Röhrchen überführt und dieses mit Komplett-Medium auf 15 mL aufgefüllt. Der Inhalt jedes Falcon-Röhrchen wird jeweils durch einen 70 μm Falcon-Filtereinsatz filtriert und 10 Minuten bei 1200 Umdrehungen und Raumtemperatur zentrifugiert. Das resultierende Pellet wird jeweils in 250 μL Komplett-Medium aufgenommen und die Zellzahl ermittelt.

Die Anzahl der Zellen in der Suspension wird auf 3 mal $10^5$ pro mL eingestellt und jeweils 150 μL dieser Suspension in eine Vertiefung der wie vorstehend beschrieben beschichteten Zellkultur-Platten gegeben. Im Brutschrank werden die Platten bei 37 °C, 5 Vol-% $CO_2$ und 95 % relativer Luftfeuchtigkeit zwei bis drei Tage stehen gelassen.

Anschließend werden die Zellen mit 2 μM Fluo-4 und 0,01 Vol-% Pluronic F127 (Molecular Probes Europe BV, Leiden Niederlande) in HBSS-Puffer (Hank's buffered saline solution, Gibco Invitrogen GmbH, Karlsruhe, Deutschland) für 30 min bei 37 °C beladen, 3 x mit HBSS-Puffer gewaschen und nach einer weiteren Inkubation von 15 Minuten bei Raumtemperatur zur $Ca^{2+}$-Messung im FLIPR-Assay eingesetzt. Die $Ca^{2+}$-abhängige Fluoreszenz wird dabei vor und nach Zugabe von Substanzen gemessen ($\lambda ex$ = 488 nm, $\lambda em$ = 540 nm). Die Quantifizierung erfolgt durch die Messung der höchsten Fluoreszenzintensität (FC, Fluorescence Counts) über die Zeit.

**FLIPR-Assay:**

Das FLIPR-Protokoll besteht aus 2 Substanzzugaben. Zunächst werden die zu testenden Verbindungen (10

μM) auf die Zellen pipettiert und der $Ca^{2+}$-Einstrom mit der Kontrolle (Capsaicin 10 μM) verglichen. Daraus ergibt sich die Angabe in % Aktivierung bezogen auf das $Ca^{2+}$-Signal nach Zugabe von 10 μM Capsaicin (CP). Nach 5 Minuten Inkubation werden 100 nM Capsaicin appliziert und ebenfalls der Einstrom von $Ca^{2+}$ ermittelt.

Desensitisierende Agonisten und Antagonisten führen zu einer Unterdrückung des $Ca^{2+}$-Einstroms. Es werden % Inhibierung im Vergleich zu der maximal erreichbaren Inhibierung mit 10 μM Capsaicin berechnet.

Es werden Dreifach-Bestimmungen (n=3) durchgeführt und diese in mindestens 3 unabhängigen Experimenten wiederholt (N=4).

**II. Methode zur Bestimmung der Affinität zur Batrachotoxin-(BTX)-Bindungsstelle des Natriumkanals:**

Die Bindungsstelle 2 des Natriumkanals ist die sogenannte Batrachotoxin-(BTX) Bindungsstelle. Als Ligand wird [$^3$H]-Batrachotoxinin A20 α-Benzoat (10 nM im Ansatz) eingesetzt. Die Ionenkanal-Partikel (Synaptosomen) werden aus dem Ratten Cerebrocortex angereichert, wie in der Veröffentlichung von Gray und Whittaker, 1962, J. Anat. 76, 79-88 beschrieben. Als unspezifische Bindung ist die Radioaktivität definiert, die in Gegenwart von Veratridin ($3 \times 10^{-4}$ M im Ansatz) gemessen wird.

Die Assaybedingungen werden entsprechend der Veröffentlichung von Pauwels, Leysen und Laduron durchgeführt, wie in Eur. J. Pharmacol. 124, 291-298 beschrieben.

Abweichend von dieser Vorschrift wird der Gesamtansatz auf 250 μl verkleinert, so daß der Assay auf 96-well Mikrotiterplatten durchgeführt werden kann. Die Inkubationszeit in diesen Mikrotiterplatten beträgt zwei Stunden bei Raumtemperatur (ca. 20-25°C).

Folgende Kenndaten wurden für den $K_D$-Wert der Bindungsstelle ermittelt:

$$K_D: 24{,}63 \pm 1{,}56 \text{ nM.}$$

**III. Methode zur Bestimmung der Affinität zum humanen μ-Opioidrezeptor**

Die Rezeptoraffinität zum humanen μ-Opioidrezeptor wird in einem homogenen Ansatz in Mikrotiterplatten bestimmt. Hierzu werden Verdünnungsreihen der jeweils zu prüfenden substituierten 5,6,7,8-Tetrahydro-imidazo[1,2-a]pyridin-2-ylamin-Verbindung der allgemeinen Formel I mit einer Rezeptormembranpräparation (15-40 μg Protein pro 250 μl Inkubationsansatz) von CHO-K1-Zellen, welche den humanen μ-Opioidrezeptor (μ-Opioat-Rezeptor) exprimieren (RB-HOM-Rezeptormembran-Präparation der Firma NEN, Zaventem, Belgien) in Gegenwart von 1 nmol/l des radioaktiven Liganden [$^3$H]-Naloxon (NET719, Firma NEN, Zaventem, Belgien) sowie von 1 mg WGA-SPA-Beads (Wheat germ agglutinin SPA Beads der Firma Amersham/Pharmacia, Freiburg, Deutschland) in einem Gesamtvolumen von 250 μl für 90 Minuten bei Raumtemperatur inkubiert. Als Inkubationspuffer wird 50 mmol/l Tris-HCl supplementiert mit 0,05 Gew.-% Natriumazid und mit 0,06 Gew.-% bovinem Serumalbumin verwendet. Zur Bestimmung der unspezifischen Bindung wird zusätzlich 25 μmol/l Naloxon zugegeben. Nach Beendigung der neunzigminütigen Inkubationszeit werden die Mikrotiterplatten für 20 Minuten bei 1000 g ab zentrifugiert und die Radioaktivität in einem β-Counter (Microbeta-Trilux, Firma PerkinElmer Wallac, Freiburg, Deutschland) vermessen. Es wird die prozentuale Verdrängung des radioaktiven Liganden aus seiner Bindung zum humanen μ-Opiatrezeptor bei einer Konzentration der zu prüfenden Verbindungen von 1 μmol/l bestimmt und als Prozent Hemmung der spezifischen Bindung angegeben.

Im folgenden wird die Erfindung anhand von Beispielen erläutert. Diese Erläuterungen sind lediglich beispielhaft und schränken den allgemeinen Erfindungsgedanken nicht ein.

**Beispiele:**

Die Ausbeuten der hergestellten Verbindungen sind nicht optimiert.

Alle Temperaturen sind unkorrigiert.

**Abkürzungen:**

| | |
|---|---|
| aq. | wäßrig |
| Äq. | Stoffmengenäquivalent |
| BOP | 1-Benzotriazolyloxy-tris-(dimethylamino)-phosphonium hexafluorophosphat |
| DCC | Dicyclohexylcarbodiimid |

| | |
|---|---|
| DCE | Dichlorethan |
| DCM | Dichlormethan |
| DIPEA | Diisopropylethylamin |
| DMF | Dimethylformamid |
| EDCI | N'-(3-Dimethylaminopropyl)-N-ethylcarbodiimid |
| EtOAc | Ethylacetat |
| ges. | gesättigt |
| HATU | N-[(Dimethyamino)-1H-1, 2, 3-triazolo[4, 5-b]pyridino-1-ylmethylen]-N-methylmethanaminium hexafluorophosphat N-oxid |
| HBTU | O-(Benzotriazol-1-yl)-N,N,N',N'-tetramethyluroniom hexafluorophosphat |
| HOAt | 1-Hydroxy-7-azabenzotriazol |
| HPTLC | high performance thin layer chromatography |
| MeOH | Methanol |
| NMR | Kernresonanzspektroskopie |
| RT | Raumtemperatur |

Die eingesetzten Chemikalien und Lösungsmittel wurden kommerziell bei den herkömmlichen Anbietern (Acros, Avocado, Aldrich, Bachem, Fluka, Lancaster, Maybridge, Merck, Sigma, TCI, etc.) bezogen oder nach den für den Fachmann bekannten Methoden synthetisiert.

Als stationäre Phase für die Säulenchromathographie wurde Kieselgel 60 (0.040 - 0.063 mm) der Firma E. Merck, Darmstadt, eingesetzt.
Die dünnschicht-chromatographischen Untersuchungen wurden mit HPTLC-Fertigplatten, Kieselgel 60 F 254, der Firma E. Merck, Darmstadt, durchgeführt. Die Mischungsverhältnisse von Lösungsmitteln, Laufmitteln oder für chromatographische Untersuchungen sind stets in Volumen/Volumen angegeben. Die Analytik erfolgte durch Massenspektroskopie und NMR.

**Allgemeine Vorschriften für die Herstellung beispielgemäßer substituierter 5,6,7,8-Tetrahydro-imidazo[1,2-a] pyridin-2-ylamin-Verbindungen Allgemeines Syntheseschema 1:**

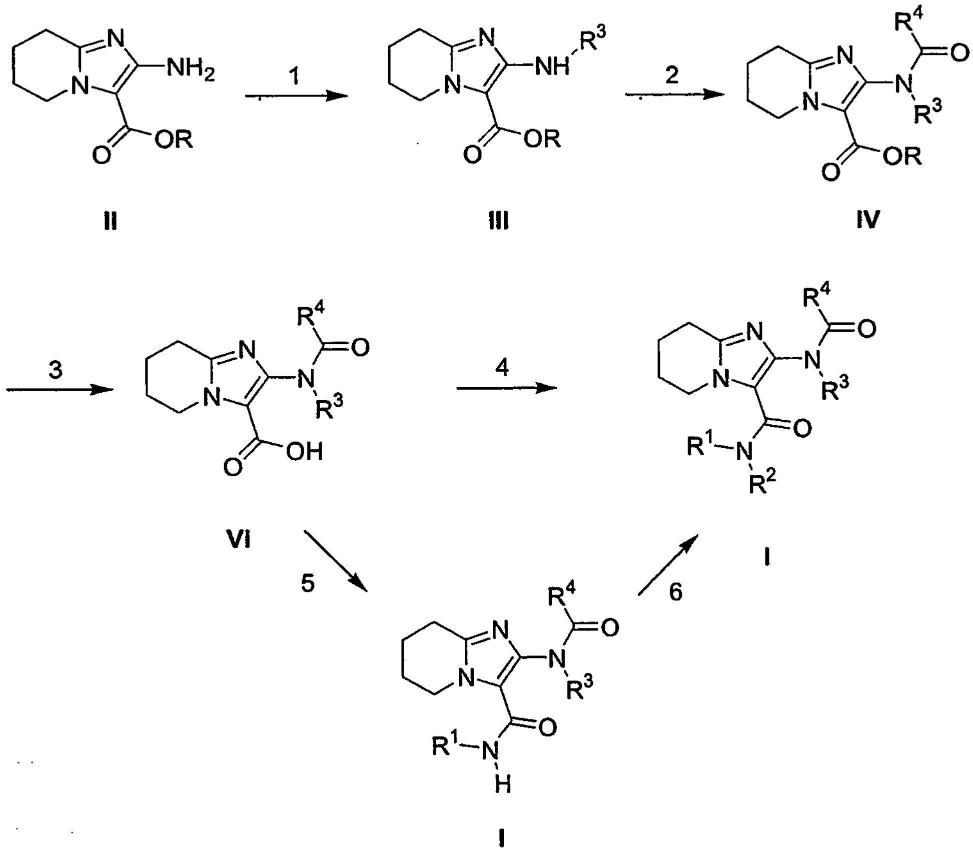


II III IV

VI I

I

In Stufe 1 wurde die Umsetzung von Verbindungen der allgemeinen Formel II mit Aldehyden der allgemeinen Formel $R^3$-C(=O)-H in organischen Lösungsmitteln oder Lösungsmittelgemischen, beispielsweise von Diethylether, Tetrahydrofuran, Methanol, Ethanol, Dichlormethan, Dichlorethan, Chloroform und Toluol, unter Zusatz eines Reduktionsmittels, beispielsweise unter Zusatz von Natriumborhydrid, Natriacetoxyborhydrid oder Natriumcyanoborhydrid, ggf. unter Zusatz einer organischen Säure, beispielsweise unter Zusatz von Essigsäure, bei Temperaturen von -70°C bis 100°C zu Verbindungen der allgemeinen Formel III durchgeführt.

In Stufe 2 wurde die Umsetzung von Verbindungen der allgemeinen Formel III mit Carbonsäuren der allgemeinen Formel $R^4$-C(=O)-OH in organischen Lösungsmitteln oder Lösungsmittelgemischen, beispielsweise von Diethylether, Tetrahydrofuran, Acetonitril, Methanol, Ethanol, Dimethylformamid und Dichlormethan, ggf. unter Zusatz wenigstens eines Kupplungsreagenzes, beispielsweise unter Zusatz von BOP, DCC, EDCI, HATU, HBTU oder HOAt, ggf. unter Zusatz wenigstens einer anorganischen Base, beispielsweise unter Zusatz von Kaliumcarbonat oder Cäsiumcarbonat, oder einer organischen Base, beispielsweise unter Zusatz von Triethylamin, Pyridin, Dimethylaminopyridin oder Diisopropylethylamin bei Temperaturen von -70°C bis 100°C zu Verbindungen der allgemeinen Formel IV durchgeführt.

Alternativ wurden Verbindungen der allgemeinen Formel III mit Carbonsäurederivaten bzw. Kohlensäurederivaten der allgemeinen Formel $R^4$-C(=O)-X, wobei X für einen Halogen-Rest steht, in organischen Lösungsmitteln oder Lösungsmittelgemischen, beispielsweise von Diethylether, Tetrahydrofuran, Acetonitril, Methanol, Ethanol, Dimethylformamid und Dichlormethan, mit oder ohne Zusatz einer organischen Base, beispielsweise unter Zusatz von Triethylamin, Dimethylaminopyridin, Pyridin oder Diisopropylamin bei Temperaturen von -70°C bis 100°C zu Verbindungen der allgemeinen Formel IV umgesetzt.

In Stufe 3 wurde die Umsetzung von Verbindungen der allgemeinen Formel IV in organischen Lösungsmitteln oder Lösungsmittelgemischen, beispielsweise von Dioxan, Tetrahydrofuran, Diethylether, Methanol, Ethanol, Isopropanol und Wasser, unter Zusatz einer anorganischen Base, beispielsweise unter Zusatz von Natriumhydroxid, Kaliumhydroxid oder Lithiumhydroxid, bei Temperaturen von 0°C bis 30°C zu Verbindungen der allgemeinen Formel VI durchgeführt. Vorzugsweise erfolgte die Umsetzung in einem Lösungsmittelgemisch, das aus Methanol, Dioxan und einer 4 M Natriumhydroxid-Lösung in Wasser mit einem Verhältnis der entsprechenden Volumina von 15:4:1 bestand ("Tesser Base").

In Stufe 4 wurde die Umsetzung von Verbindungen der allgemeinen Formel I mit Aminen der allgemeinen Formel $HNR^1R^2$ in organischen Lösungsmitteln oder Lösungsmittelgemischen, beispielsweise von Diethylether, Tetrahydrofuran, Acetonitril, Methanol, Ethanol, Dimethylformamid und Dichlormethan, ggf. unter Zusatz wenigstens eines

Kupplungsreagenzes, beispielsweise unter Zusatz von BOP, DCC, EDCI, HATU, HBTU oder HOAt, ggf. unter Zusatz wenigstens einer anorganischen Base, beispielsweise unter Zusatz von Kaliumcarbonat oder Cäsiumcarbonat, oder einer organischen Base, beispielsweise unter Zusatz von Triethylamin, Pyridin, Dimethylaminopyridin oder Diisopropylethylamin bei Temperaturen von -70°C bis 100°C zu Verbindungen der allgemeinen Formel I durchgeführt.

In Stufe 5 wurden Verbindungen der vorstehend angegebenen allgemeinen Formel VI mit Aminen der allgemeinen Formel $HNR^1R^2$, worin $R^2$ für Wasserstoff steht, mit den Methoden wie vorstehend im Allgemeinen Syntheseschema 1, Stufe 4 beschrieben zu Verbindungen der allgemeinen Formel I umgesetzt.

In Stufe 6 wurden Verbindungen der allgemeinen Formel Ia mit Verbindungen der allgemeinen Formel $R^2$-X, worin X für einen Halogen-Rest steht, in organischen Lösungsmitteln oder Lösungsmittelgemischen, beispielsweise von Dimethylformamid, Heptan, Hexan, Toluol, Tetrahydrofuran und Diethylether, unter Zusatz eines Metallhydridsalzes, beispielsweise unter Zusatz von Natriumhydrid, Kaliumhydrid oder Lithiumhydrid, bei Temperaturen von 0 °C bis 40 °C zu Verbindungen der allgemeinen Formel I umgesetzt.

**Allgemeines Syntheseschema 2:**

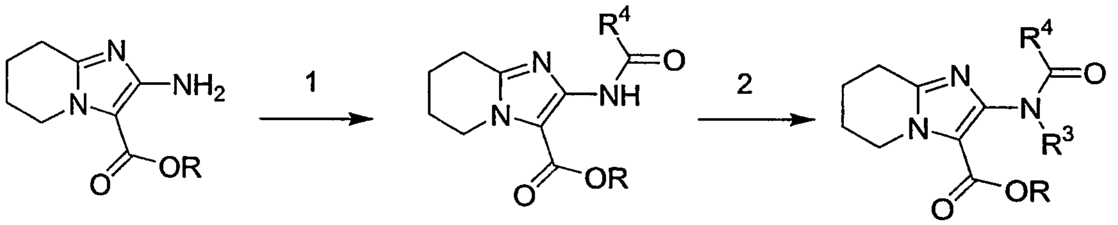


II V IV

Die Verbindungen der allgemeinen Formel II lassen sich mit den gleichen Methoden, wie im Allgemeinen Syntheseschema 1, Stufe 2, beschrieben, zu Verbindungen der allgemeinen Formel V umsetzen.

Die Verbindungen der allgemeinen Formel V lassen sich mit den gleichen Methoden, wie im Allgemeinen Syntheseschema 1, Stufe 6, beschrieben, zu Verbindungen der allgemeinen Formel IV umsetzen.

**Allgemeines Syntheseschema 3:**

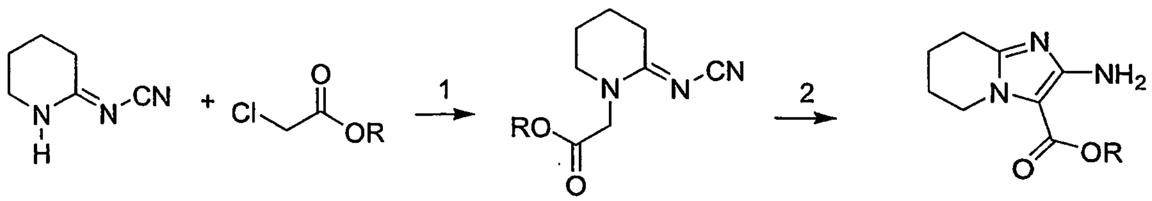


C VII VIII II

In Stufe 1 erfolgte die Umsetzung von Piperidin-2-yliden-cyanamid (C) mit einer Verbindung der allgemeinen Formel VII, worin R für einen linearen oder verzweigten $C_{1-6}$-Alkyl-Rest steht, in organischen Lösungsmitteln oder Lösungsmittelgemischen, beispielsweise von Acetonitril, Dichlormethan, Chloroform, Dimethylformamid, Dimethylacetamid und Dimethylsulfoxid, unter Zusatz einer anorganischen Base, beispielsweise unter Zusatz von Kaliumcarbonat, Natriumcarbonat, Lithiumcarbonat oder Magnesiumcarbonat, bei einer Temperatur von 50°C bis 150°C zu einer Verbindung der allgemeinen Formel VIII.

In Stufe 2 wurde eine Verbindung der allgemeinen Formel VIII in organischen Lösungsmitteln oder Lösungsmittelgemischen, beispielsweise von Methanol, Ethanol und Isopropanol, unter Zusatz eines Alkalimetallalkoholat-Salzes, beispielsweise von Natriummethanolat, Natriumethanolat, Kaliummethanolat und Kaliumethanolat, bei einer Temperatur von 50°C bis 120 °C zu einer Verbindung der allgemeinen Formel II umgesetzt.

Im folgenden werden die vorstehend beschriebenen Vorschriften für die Herstellung substituierter 5,6,7,8-Tetrahydro-imidazo[1,2-a]pyridin-2-ylamin-Verbindungen anhand einiger Beispielverbindungen detailliert erläutert:

**a) Synthese von Piperidin-2-yliden-cyanamid**

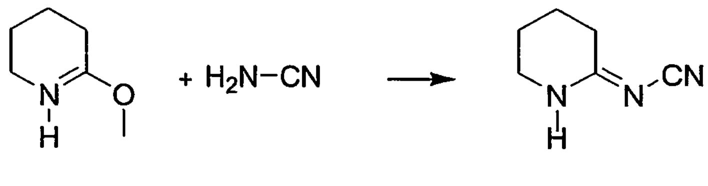

**A** **B** **C**

6-Methoxy-2,3,4-5-tetrahydropyridin (A) (9.01 g, 79.6 mmol) wurde in MeOH (90 mL) gelöst und anschließend wurde Cyanamid (B) (3.35 g, 79.6 mmol, 1 Äq.) langsam hinzu gefügt. Nach 5 Minuten wurde ein weißer Niederschlag beobachtet. Die resultierende Suspension wurde für weitere 72 Stunden bei RT gerührt und das Lösungsmittel im Vakuum entfernt. Piperidin-2-yliden-cyanamid (C) wurde als weißes Pulver erhalten, das direkt weiter verwendet wurde.

**b) Synthese von (2-Cyanoimino-piperidin-1-yl)-essigsäureethylester**

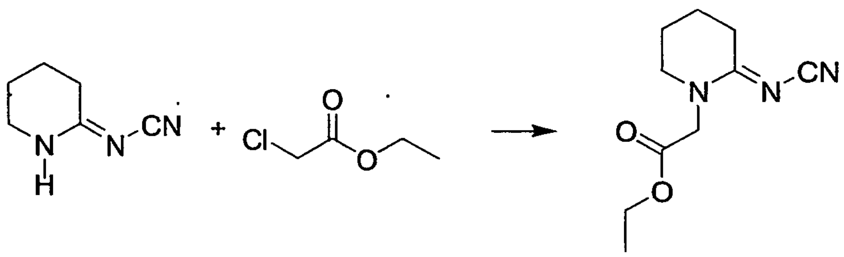

**C** **D**

Piperidin-2-yliden-cyanamid (C) (9.67 g, 78.5 mmol) wurde unter leichtem Erwärmen in Acetonitril (150 mL) gelöst. Anschließend wurden Kaliumcarbonat (13.0 g, 94.2 mmol, 1.2 Äq.) und Ethylchloroacetat (11.7 mL, 109.9 mmol, 1.4 Äq.) hinzu gegeben und die resultierende Suspension wurde für 16 Stunden auf 85°C erwärmt. Anschließend wurde ein weitere Menge Ethylchloroacetat (1.67 mL, 15.7 mmol, 0.2 Äq.) hinzu gegeben. Die Reaktionsmischung wurde 6 Stunden unter Rückfluß erhitzt. Die abgekühlte Suspension wurde filtriert und der feste Rückstand mit DCM gewaschen. Das Filtrat wurde im Vakuum eingeengt und nach säulenchromatographischer Reinigung ($SiO_2$, Heptan/EtOAc 2:3) wurden 16.16 g (98 %) des gewünschten Produkts (2-Cyanoimino-piperidin-1-yl)-essigsäureethylester (D) erhalten.

**c) Synthese von 2-Amino-5,6,7,8-tetrahydro-imidazo[1,2-a]-pyridin-3-carboxylsäureethylester**

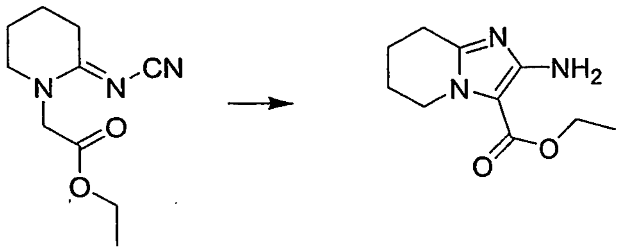

D E

Zu einer Lösung aus Natriumethanolat (5.20 g, 76.7 mmol, 1.0 Äq.) in Ethanol (500 mL) wurde Verbindung D (16.05 g, 76.7 mmol) gegeben und die resultierende Reaktionsmischung wurde 30 Minuten unter Rückfluß erhitzt. Das Lösungsmittel wurde im Vakuum entfernt und das Rohprodukt wurde mittels Säulenchromatographie (Hydromatrix als Adsorbent, $SiO_2$, DCM/ 3 % MeOH → DCM/ 5 % MeOH) gereinigt. Es wurden 9.27 g des gewünschten Produkts E erhalten. Die Säule wurde mit Methanol gewaschen, das Lösungsmittel im Vakuum entfernt und der Rückstand aus Heptan umkristallisiert, um weitere 3.26 g des gewünschten Produkts 2-Amino-5,6,7,8-tetrahydro-imidazo[1,2-a]-pyridin-3-carboxyl-säureethylester (E) zu erhalten. Insgesamt wurden 12.53 g (78 %) des gewünschten Produkts 2-Amino-5,6,7,8-tetrahydro-imidazo[1,2-a]-pyridin-3-carboxyl-säureethylester (E) erhalten.

**d) Synthese von 2-Butylamino-5,6,7,8-tetrahydro-imidazo[1,2-a]pyridin-3-carboxylsäureethylester**

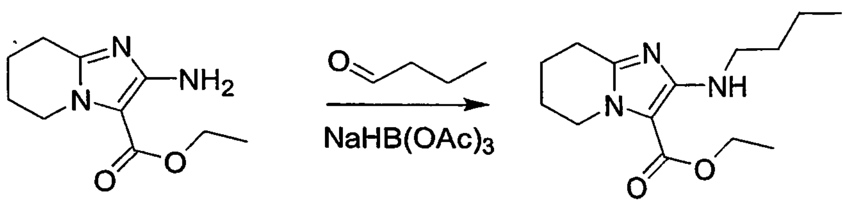


E F

Verbindung E wurde in DCE (150 mL) gelöst und n-Butylaldehyd (49.7 mmol, 4.4 mL, 1.5 Äq.) wurde hinzu gegeben. Anschließend wurde Natriumtriacetoxyborhydrid (11.93 g, 56.3 mmol, 1.7 Äq.) portionsweise hinzu gefügt und die Reaktionsmischung wurde 4 Stunden bei RT gerührt. Die Reaktionsmischung wurde mit DCM (500 mL) verdünnt und mit ges. aq. $NaHCO_3$-Lösung (500 mL) gewaschen. Die wäßrigen Phasen wurden mit DCM (100 mL) extrahiert und die vereinigten organischen Phasen wurden mit ges. aq. NaCl-Lösung (500 mL) gewaschen, über Natriumsulfat getrocknet und das Lösungsmittel im Vakuum entfernt. Das Rohprodukt wurde mittels Säulenchromatographie ($SiO_2$, Heptan/EtOAc 4:1 → 3:1) gereinigt und es wurden 5.7 g (65 %) des gewünschten Produkts 2-Butylamino-5,6,7,8-tetrahydro-imidazo[1,2-a]pyridin-3-carboxylsäureethylester (F) erhalten.

**e) Synthese der Beispielverbindungen 43, 91 und 133**

**1) Synthese von Verbindung 2-[Butyl-(3-chlor-benzoyl)-amino]-5,6,7,8-tetrahydro-imidazo[1,2-a]pyridin-3-carbonsäureethylester**

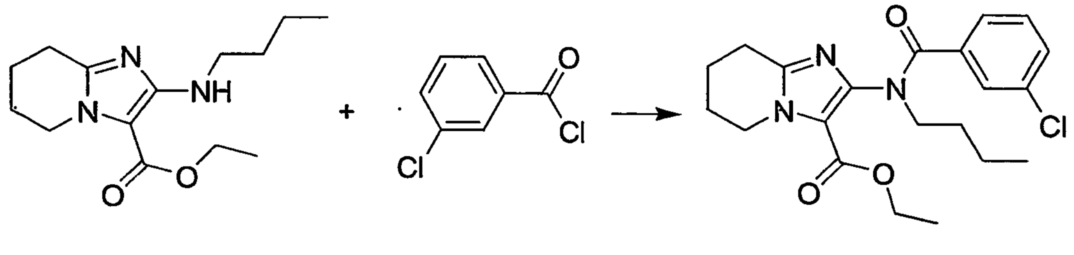

F

G

Verbindung F (3.1 g, 11.7 mmol) und Triethylamin (2.46 ml, 17.5 mmol, 1.5 Äq.) wurden in DCM (70 mL) gelöst und die Reaktionsmischung wurde in einem Eisbad gekühlt. Anschließend wurde 3-Chlorbenzyolchlorid (1.65 mL, 12.85 mmol, 1.1 Äq.) tropfenweise hinzu gegeben. Nach 90 Minuten wurde die Reaktionsmischung mit DCM (130 mL) verdünnt und anschließend mehrfach mit 0.5 M $KHSO_4$ in Wasser (200 mL), ges. aq. $NaHCO_3$-Lösung (200 mL) und ges. aq. NaCl-Lösung (200 mL) gewaschen. Die organische Phase wurde über Natriumsulfat getrocknet und das Lösungsmittel im Vakuum entfernt. Das Rohprodukt 2-[Butyl-(3-chlor-benzoyl)-amino]-5,6,7,8-tetrahydro-imidazo[1,2-a]pyridin-3-carbonsäureethylester wurde mittels Säulenchromatographie ($SiO_2$, DCM → DCM/ 5 % MeOH) gereinigt und direkt in der nächsten Stufe eingesetzt.

**2) Synthese von 2-[Butyl-(3-chlor-benzoyl)-amino]-5,6,7,8-tetrahydro-imidazo[1,2-a]pyridin-3-carbonsäure**

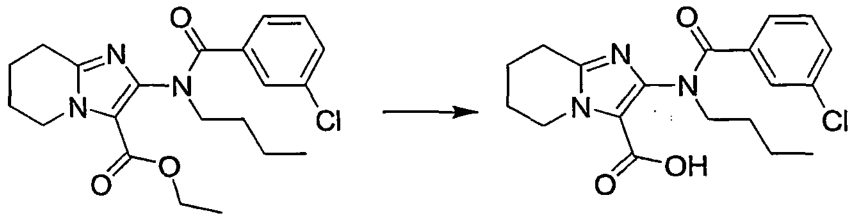

G

H

2-[Butyl-(3-chlor-benzoyl)-amino]-5,6,7,8-tetrahydro-imidazo[1,2-a]pyridin-3-carbonsäureethylester (11.7 mmol) wurde in einer Lösung aus 190 mL MeOH/Dioxan/ 4 M NaOH in Wasser im Verhältnis 15/4/1 gelöst und die Lösung wurde über Nacht bei RT gerührt. Das Lösungsmittel wurde im Vakuum entfernt, EtOAc (700 mL) wurde hinzu gefügt und die organische Phase wurde mit 0.5 M $KHSO_4$ in Wasser (700 mL) gewaschen. Die wäßrige Phase wurde mit EtOAc extrahiert (300 mL) und die vereinigten organischen Phasen wurden mit ges. aq. NaCl-Lösung (700 mL) gewaschen, über Natriumsulfat getrocknet und das Lösungsmittel wurde im Vakuum entfernt. Es wurden 4.13 g (94 % über zwei Stufen) des gewünschten Produkts 2-[Butyl-(3-chlor-benzoyl)-amino]-5,6,7,8-tetrahydro-imidazo[1,2-a]pyridin-3-carbonsäure erhalten.

**3) Synthese von Beispielverbindung 43: 2-[Butyl-(3-chlor-benzoyl)-amino]-5,6,7,8-tetrahydro-imidazo[1,2-a]pyridin-3-carbonsäure 2-ethoxy-benzylamid**

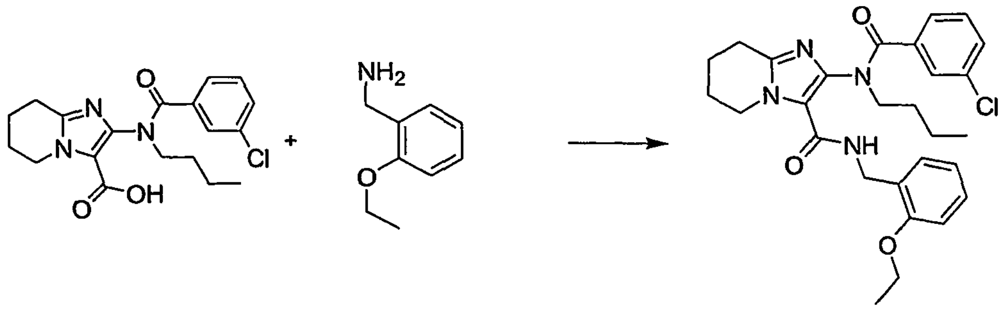

H

43

2-[Butyl-(3-chlor-benzoyl)-amino]-5,6,7,8-tetrahydro-imidazo[1,2-a]pyridin-3-carbonsäure (130 mg, 0.45 mmol), EDCI (72.9 mg, 0.38 mmol, 1.0 Äq.) und HOAt (4.7 mg, 0.035 mmol, 0.1 Äq.) wurden in DCM (3.5 mL) gelöst. *ortho*-Phenetidin (51.5 μL, 0.35 mmol) wurde hinzu gegeben und die Lösung wurde 16 Stunden bei RT gerührt. Das Lösungsmittel wurde im Vakuum entfernt, EtOAc (30 mL) wurde hinzu gegeben und die organische Phase wurde mehrfach mit einer 0.5 M Lösung von $KHSO_4$ in Wasser (30 mL) und ges. aq. $NaHCO_3$-Lösung (35 mL) gewaschen. Die wäßrigen Phasen wurden mehrfach mit Ethylacetat ausgeschüttelt. Die vereinigten organischen Phasen wurden mit ges. aq. NaCl-Lösung (40 mL) gewaschen, über Natriumsulfat getrocknet und das Lösungsmittel wurde im Vakuum entfernt. Das Rohprodukt wurde mittels Säulenchromatographie ($SiO_2$, DCM → DCM/MeOH 98/2) gereinigt. Es wurden 130 mg (73 %) des gewünschten Produkts 2-[Butyl-(3-chlor-benzoyl)-amino]-5,6,7,8-tetrahydro-imidazo[1,2-a]pyridin-3-carbonsäure 2-ethoxy-benzylamid erhalten.
MS: $[M^+]$ 509.6

**4) Synthese von Beispielverbindung 91: 2-({2-[Butyl-(3-chlor-benzoyl)-amino]-5,6,7,8-tetrahydro-imidazo [1,2-a]pyridin-3-carbonyl}-amino)-3-(*S*)-methyl-buttersäurebehzylester**

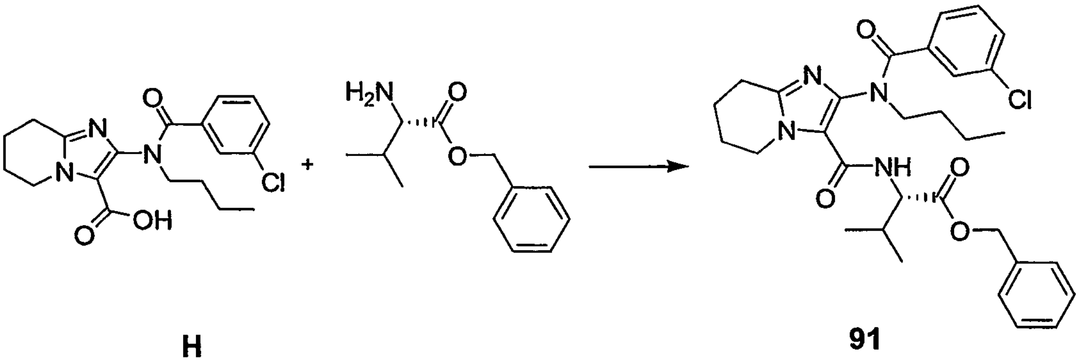

H

91

2-[Butyl-(3-chlor-benzoyl)-amino]-5,6,7,8-tetrahydro-imidazo[1,2-a]pyridin-3-carbonsäure (0.50 g, 1.33 mmol), L-Valinbenzylesterhydrochlorid (324 mg, 1.33 mmol), DIPEA (172 mg, 1.33 mmol) und HOAt (18 mg, 0.13 mmol) wurden in 10 mL DCM gelöst. Die Lösung wurde auf 0 °C gekühlt und EDCI (280 mg, 1.46 mmol) wurde hinzu gegeben.

Anschließend wurde die Lösung 1 Stunde bei 0 °C und über Nacht bei RT gerührt. DCM (50 mL) und ges. aq. NaCl-Lösung (50 mL) wurden hinzu gegeben und die wäßrige Phase wurde mit DCM (50 mL) extrahiert. Die vereinigten organischen Phasen wurden über Natriumsulfat getrocknet und das Lösungsmittel im Vakuum entfernt. Das Rohprodukt wurde mittels Säulenchromatographie ($SiO_2$, DCM/MeOH 98/2) gereinigt. Es wurden 473 mg (63 %) des gewünschten Produkts 2-({2-[Butyl-(3-chlor-benzoyl)-amino]-5,6,7,8-tetrahydro-imidazo[1,2-a]pyridin-3-carbonyl}-amino)-3-(*S*)-methylbuttersäurebenzylester erhalten.

**5) Synthese von Beispielverbindung 132: 2-[Butyl-(3-chlor-benzoyl)-amino]-5,6,7,8-tetrahydro-imidazo[1,2-a] pyridin-3-carbonsäure (1-(*R*)-phenyl-propyl)-amid**

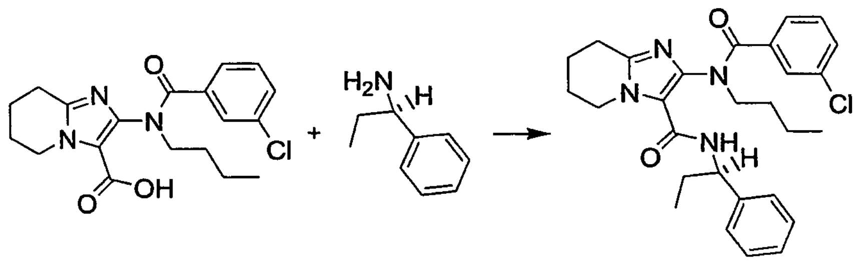

H 132

2-[Butyl-(3-chlor-benzoyl)-amino]-5,6,7,8-tetrahydro-imidazo[1,2-a]pyridin-3-carbonsäure (0.50 g, 1.33 mmol), R-(+)-1-Phenylpropylamin (180 mg, 1.33 mmol) und HOAt (18 mg, 0.13 mmol) wurden in 5 mL DCM gelöst. Die Lösung wurde auf 0 °C gekühlt und EDCI (280 mg, 1.46 mmol) wurde hinzu gegeben. Die Reaktionsmischung wurde 1 Stunde bei 0 °C und anschließend bei RT über Nacht gerührt. Es wurden DCM (50 mL) und ges. aq. NaCl-Lösung (50 mL) hinzu gegeben und die wäßrige Phase wurde mit 50 mL DCM extrahiert. Die vereinigten organischen Phasen wurden über Natriumsulfat getrocknet und das Lösungsmittel wurde im Vakuum entfernt. Das Rohprodukt wurde mittels Säulenchromatographie ($SiO_2$, DCM/MeOH 98/2) gereinigt und es wurden 492 mg (75 %) des gewünschten Produkts 2-[Butyl-(3-chlor-benzoyl)-amino]-5,6,7,8-tetrahydro-imidazo[1,2-a]pyridin-3-carbonsäure (1-(*R*)-phenyl-propyl)-amid erhalten.

**f) Synthese der Beispielverbindungen 16, 93 und 180**

**1) Synthese von 2-[Butyl-(3,4-difluor-benzoyl)-amino]-5,6,7,8-tetrahydroimidazo[1,2-a]pyridin-3-carbonsäure-ethylester**

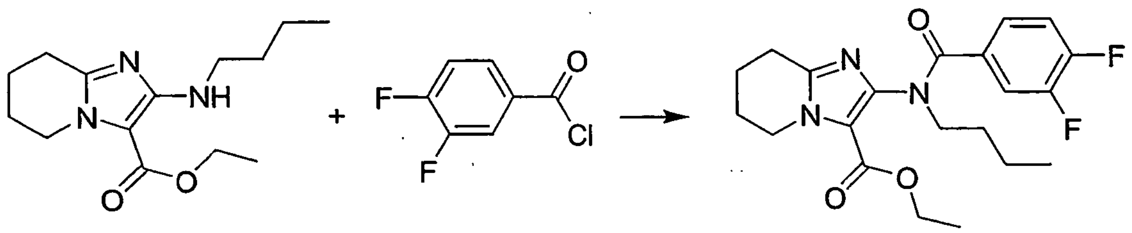

F J

Verbindung F (3.2 g, 12.1 mmol) und Triethylamin (2.54 mL, 18.1 mmol, 1.5 Äq.) wurden in DCM (70 mL) gelöst und die Lösung wurde auf 0 °C gekühlt. 3,4-Difluorbenzoylchlorid (1.66 mL, 13.3 mmol, 1.1 Äq.) wurde tropfenweise hinzu gefügt. Nach 90 Minuten wurde die Reaktionsmischung mit DCM (130 mL) verdünnt und mit 0.5 M $KHSO_4$ in Wasser (200 mL), ges. aq. $NaHCO_3$-Lösung (200 mL) und ges. aq. NaCl-Lösung (200 mL) gewaschen. Die vereinigten organischen Phasen wurden über Natriumsulfat getrocknet und das Lösungsmittel im Vakuum entfernt und das Rohprodukt 2-[Butyl-(3,4-difluorbenzoyl)-amino]-5,6,7,8-tetrahydro-imidazo[1,2-a]pyridin-3-carbonsäureethylester wurde direkt in der nächsten Stufe eingesetzt.

**2) Synthese von 2-[Butyl-(3,4-difluor-benzoyl)-amino]-5,6,7,8-tetrahydro-imidazo[1,2-a]pyridin-3-carbonsäure**

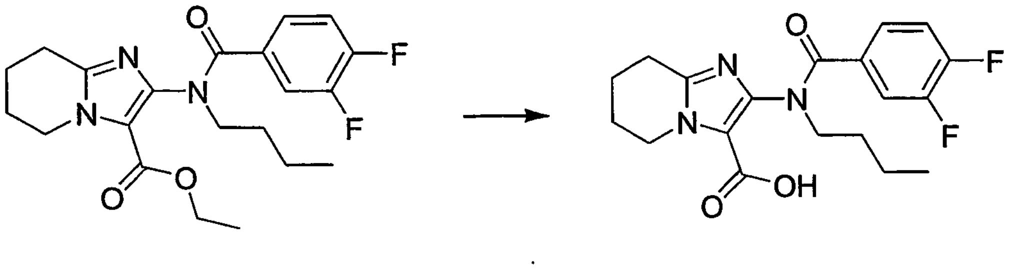

J K

Verbindung J (12.1 mmol) wurde in einer Lösung aus 200 mL MeOH/Dioxan/ 4 M NaOH in Wasser im Verhältnis 15/4/1 gelöst und die Lösung wurde über Nacht bei RT gerührt. Das Lösungsmittel wurde im Vakuum entfernt, EtOAc (700 mL) wurde hinzu gefügt und die organische Phase wurde mit 0.5 M $KHSO_4$ in Wasser (700 mL) gewaschen. Die wäßrige Phase wurde mit EtOAc extrahiert (300 mL) und die vereinigten organischen Phasen wurden mit ges. aq. NaCl-Lösung (700 mL) gewaschen, über Natriumsulfat getrocknet und das Lösungsmittel wurde im Vakuum entfernt. Es wurden 4.38 g (96 % über zwei Stufen) des gewünschten Produkts 2-[Butyl-(3,4-difluor-benzoyl)-amino]-5,6,7,8-tetrahydro-imidazo[1,2-a]pyridin-3-carbonsäure erhalten.

**3) Synthese von Beispielverbindung 16: 2-[Butyl-(3,4-difluor-benzoyl)-amino]-5,6,7,8-tetrahydro-imidazo[1,2-a] pyridin-3-carbonsäure (4-methyl-cyclohexyl)-amid**

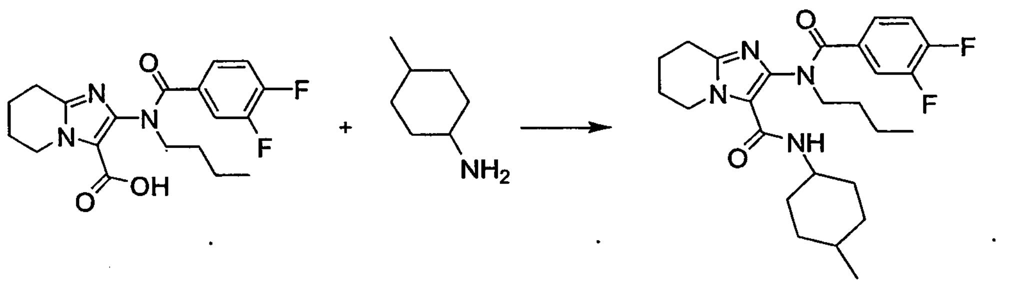

K 16

Verbindung K (0.50 g, 1.33 mmol), 4-Methyl-cyclohexylamin (149 mg, 1.33 mmol, Mischung von cis- und trans-Isomeren), DIPEA (172 mg, 1.33 mmol) und HOAt (18 mg, 0.13 mmol) wurden in 10 mL DCM gelöst. Die Lösung wurde auf 0 °C gekühlt und EDCI (280 mg, 1.46 mmol) wurde hinzu gegeben. Anschließend wurde die Lösung 1 Stunde bei 0 °C und über Nacht bei RT gerührt. DCM (50 mL) und ges. aq. NaCl-Lösung (50 mL) wurden hinzu gegeben und die wäßrige Phase wurde mit DCM (50 mL) extrahiert. Die vereinigten organischen Phasen wurden über Natriumsulfat getrocknet und das Lösungsmittel im Vakuum entfernt. Das Rohprodukt wurde mittels Säulenchromatographie ($SiO_2$, DCM/MeOH 98/2) gereinigt. Es wurden 418 mg (67 %) des gewünschten Produkts 2-[Butyl-(3,4-difluor-benzoyl)-amino]-5,6,7,8-tetrahydro-imidazo[1,2-a]pyridin-3-carbonsäure (4-methyl-cyclohexyl)-amid erhalten.

**4) Synthese von Beispielverbindung 93: 2-[Butyl-(3,4-difluor-benzoyl)-amino]-5,6,7,8-tetrahydro-imidazo[1,2-a] pyridin-3-carbonsäure (2-methyl-cyclohexyl)-amid**

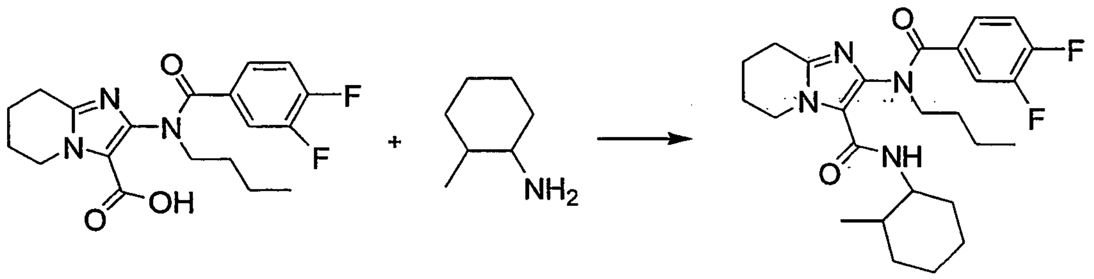

K

93

Verbindung K (0.50 g, 1.33 mmol), 2-Methyl-cyclohexylamin (149 mg, 1.33 mmol, Mischung von cis- und trans-Isomeren), DIPEA (172 mg, 1.33 mmol) und HOAt (18 mg, 0.13 mmol) wurden in 10 mL DCM gelöst. Die Lösung wurde auf 0 °C gekühlt und EDCI (280 mg, 1.46 mmol) wurde hinzu gegeben. Anschließend wurde die Lösung 1 Stunde bei 0 °C und über Nacht bei RT gerührt. DCM (50 mL) und ges. aq. NaCl-Lösung (50 mL) wurden hinzu gegeben und die wäßrige Phase wurde mit DCM (50 mL) extrahiert. Die vereinigten organischen Phasen wurden über Natriumsulfat getrocknet und das Lösungsmittel im Vakuum entfernt. Das Rohprodukt wurde mittels Säulenchromatographie ($SiO_2$, DCM/MeOH 98/2) gereinigt. Es wurden 272 mg (43 %) des gewünschten Produkts 2-[Butyl-(3,4-difluor-benzoyl)-amino]-5,6,7,8-tetrahydro-imidazo[1,2-a]pyridin-3-carbonsäure (2-methyl-cyclohexyl)-amid erhalten.

**5) Synthese von Beispielverbindung 180: 2-[(3-Chlor-benzoyl)-methyl-amino]-5,6,7,8-tetrahydro-imidazo[1,2-a] pyridin-3-carbonsäure (3,3-diphenyl-propyl)-amid**

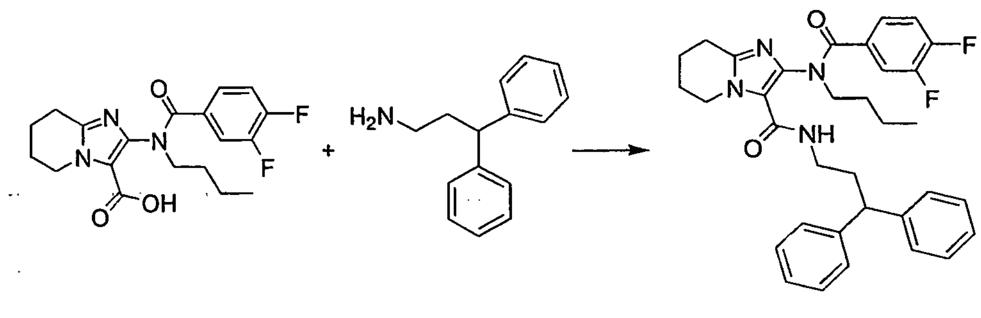

K

180

Verbindung K (0.50 g, 1.32 mmol), 3,3-Diphenylpropylamin (279 mg, 1.32 mmol) und HOAt (18 mg, 0.13 mmol) wurden in 10 mL DCM gelöst. Die Lösung wurde auf 0 °C gekühlt und EDCI (278 mg, 1.45 mmol) wurde hinzu gegeben. Die Reaktionsmischung wurde 1 Stunde bei 0 °C und bei RT über Nacht gerührt. DCM (50 mL) und ges. aq. NaCl-Lösung (50 mL) wurden hinzu gegeben und die wäßrige Phase wurde mit DCM (50 mL) extrahiert. Die vereinigten organischen Phasen wurden über Natriumsulfat getrocknet und das Lösungsmittel wurde im Vakuum entfernt. Das Rohprodukt wurde mittels Säulenchromatographie ($SiO_2$, DCM/MeOH 98/2) gereinigt. Es wurden 507 mg (67 %) des gewünschten Produkts 2-[(3-Chlor-benzoyl)-methyl-amino]-5,6,7,8-tetrahydro-imidazo[1,2-a]pyridin-3-carbonsäure (3,3-diphenyl-propyl)-amid erhalten.

**g) Synthese der Beispielverbindung 121**

**1) Synthese von 2-(3-Chlorbenzoylamino)-5,6,78-tetrahydro-imidazo[1,2-a]-pyridin-3-carboxylsäureethylester**

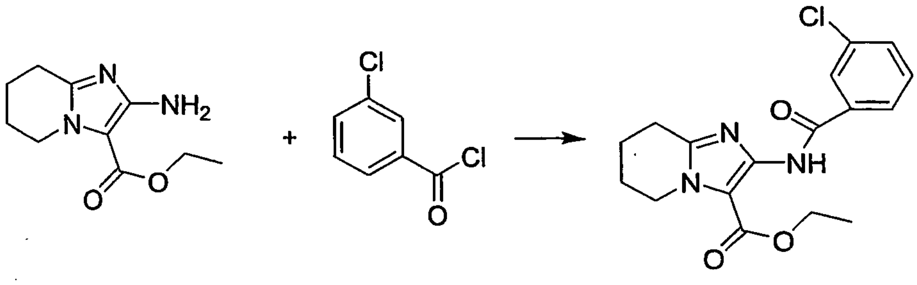

F L

Verbindung F (521 mg, 2.5 mmol) wurde in Dioxan (10 mL) gelöst und anschließend tropfenweise mit 3-Chlorbenzoylchlorid (320 µL, 2.5 mmol) versetzt. Die Reaktionsmischung wurde über Nacht bei RT gerührt und es wurden zusätzliche Mengen des Säurechlorid (16 µL, 0.12 mmol) hinzu gegeben. Nach einer Stunde wurde die Reaktionsmischung mit EtOAc (250 mL) verdünnt und anschließend nacheinander mit ges. aq. $NaHCO_3$-Lösung (250 mL) und 0.5 M $KHSO_4$ in Wasser (250 mL) gewaschen. Die wäßrigen Phasen wurden mit EtOAc (50 mL) gewaschen und die vereinigten organischen Phasen wurden wiederum mit ges. aq. NaCl-Lösung (250 mL) gewaschen, über Natriumsulfat getrocknet und das Lösungsmittel wurde im Vakuum entfernt. Es wurden 481.6 mg (55 %) des gewünschten Produkts 2-(3-Chlorbenzoylamino)-5,6,78-tetrahydro-imidazo[1,2-a]-pyridin-3-carboxylsäureethylester (L) erhalten.

**2) Synthese von 2-[(3-Chlor-benzoyl)-methyl-amino]-5,6,7,8-tetrahydro-imidazo[1,2-a]pyridin-3-carbonsäureethylester und 2-[(3-Chlor-benzoyl)-methyl-amino]-5,6,7,8-tetrahydro-imidazo[1,2-a]pyridin-3-carbonsäuremethylester**

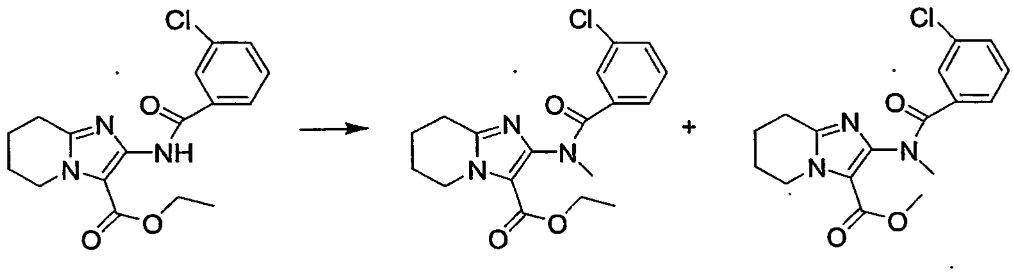

L M N

Natriumhydrid (60%-ige Suspension in Paraffinöl, 148.9 mg, 3.7 mmol, 3.7 Äq.) wurde zwei Mal mit Heptan gewaschen und in DMF aufgenommen. Eine Lösung von Verbindung L (350 mg, 1.0 mmol) in DMF wurde tropfenweise hinzu gegeben. Nach 25 Minuten bei RT wurde eine Lösung von Methyliodid in DMF (1.6 M, 1.9 mL, 3 mmol, 3 Äq.) langsam hinzu getropft und die Reaktionsmischung wurde bei RT über Nacht gerührt. Es wurde weiteres Methyliodid (62 µL, 1.0 mmol, 1 Äq.) hinzu gegeben und nochmals über Nacht gerührt. Die Reaktionsmischung wurde mit EtOAc (300 mL) verdünnt und in 0.5 M $KHSO_4$ in Wasser (300 mL) gegossen. Die Phasen wurden getrennt und die wäßrige Phase wurde mit EtOAc (300 mL) extrahiert. Die vereinigten organischen Phasen wurden mit ges. aq. NaCl-Lösung (70 mL) gewaschen, über Natriumsulfat getrocknet und das Lösungsmittel wurde im Vakuum entfernt. Es wurden 0.294 g einer Mischung von 2-[(3-Chlor-benzoyl)-methyl-amino]-5,6,7,8-tetrahydro-imidazo[1,2-a]pyridin-3-carbonsäureethylester und 2-[(3-Chlor-benzoyl)-methyl-amino]-5,6,7,8-tetrahydro-imidazo[1,2-a]pyridin-3-carbonsäuremethylester erhalten, die direkt in der nächsten Stufe eingesetzt wurde.

**3) Synthese von 2-[(3-Chlor-benzoyl)-methyl-amino]-5,6,7,8-tetrahydro-imidazo[1,2-a]pyridin-3-carbonsäure**

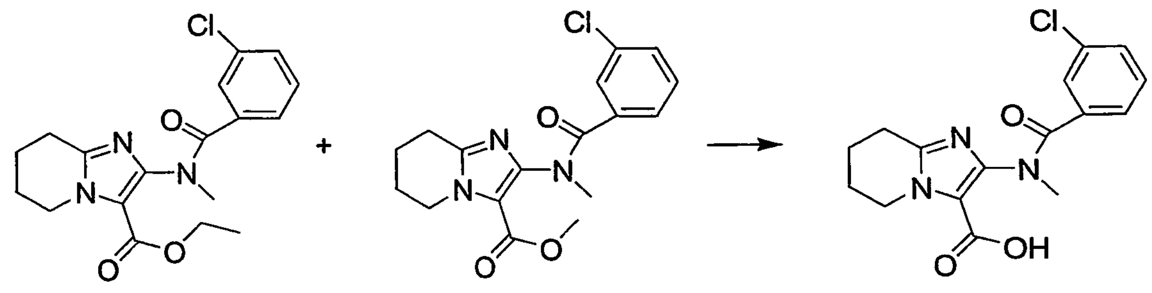

M N O

2-[(3-Chlor-benzoyl)-methyl-amino]-5,6,7,8-tetrahydro-imidazo[1,2-a]pyridin-3-carbonsäureethylester und 2-[(3-Chlor-benzoyl)-methyl-amino]-5,6,7,8-tetrahydro-imidazo[1,2-a]pyridin-3-carbonsäuremethylester (zusammen 1.0 mmol) wurden in einer Lösung aus 16 mL MeOH/Dioxan/ 4 M NaOH in Wasser im Verhältnis 15/4/1 gelöst und die Lösung wurde über Nacht bei RT gerührt. Es wurde weiteres NaOH in Wasser (1.5 mL, 6 mmol) hinzu gegeben. Nach 45 Minuten wurde das Lösungsmittel im Vakuum entfernt, EtOAc (50 mL) wurde hinzu gefügt und die organische Phase wurde mit 0.5 M $KHSO_4$ in Wasser (50 mL) gewaschen. Die wäßrige Phase wurde mit EtOAc (60 mL) extrahiert und die vereinigten organischen Phasen wurden mit ges. aq. NaCl-Lösung gewaschen (80 mL), über Natriumsulfat getrocknet und das Lösungsmittel wurde im Vakuum entfernt. Es wurden 234 mg (70 % über zwei Stufen) des gewünschten Produkts 2-[(3-Chlor-benzoyl)-methyl-amino]-5,6,7,8-tetrahydro-imidazo[1,2-a]pyridin-3-carbonsäure erhalten.

**4) Synthese von Beispielverbindung 121: 2-[(3-Chlor-benzoyl)-methyl-amino]-5,6,7,8-tetrahydro-imidazo[1,2-a]pyridin-3-carbonsäure (benzo[1,3]dioxol-5-ylmethyl)-amid**

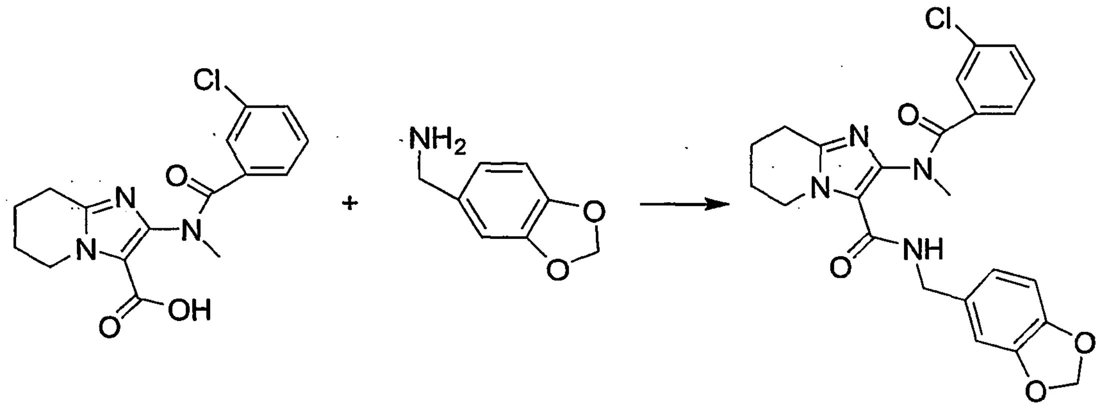

O 121

2-[(3-Chlor-benzoyl)-methyl-amino]-5,6,7,8-tetrahydro-imidazo[1,2-a]pyridin-3-carbonsäure (232 mg, 0.70 mmol), EDCI (146.6 mg, 0.76 mmol, 1.1 Äq.) und HOAt (9.5 mg, 0.07 mmol, 0.1 Äq.) wurden in DCM (10 mL) gelöst. Anschließend wurde Piperonylamin (96 $\mu$L, 0.77 mmol, 1.1 Äq.) hinzu gegeben. Die Reaktionsmischung wurde über Nacht gerührt und anschließend das Lösungsmittel entfernt. EtOAc (70 mL) wurde hinzu gegeben und die Reaktionsmischung wurde nacheinander mit 0.5 M $KHSO_4$ in Wasser (70 mL) und ges. aq. $NaHCO_3$-Lösung (70 mL) gewaschen. Die wäßrigen Phasen wurden mit EtOAc (30 mL) extrahiert. Die vereinigten organischen Phasen wurden mit ges. aq. NaCl-Lösung (70 mL) gewaschen, über Natriumsulfat getrocknet und das Lösungsmittel wurde im Vakuum entfernt. Das Rohprodukt wurde mittels Säulenchromatographie ($SiO_2$, DCM $\rightarrow$ DCM/ 2 % MeOH) gereinigt. Es wurden 116.8 mg (36 %) des gewünschten Produkts 2-[(3-Chlor-benzoyl)-methyl-amino]-5,6,7,8-tetrahydro-imidazo[1,2-a]pyridin-3-carbonsäure (benzo[1,3]dioxol-5-ylmethyl)-amid erhalten.
MS: $[M+H^+]$ 467.6

Die zuvor nicht detailliert beschriebene Herstellung der übrigen Verbindungen gemäß den nachstehend angegebenen Beispielen erfolgte ebenfalls analog den vorstehend angegebenen Herstellungsvorschriften, wobei dem Fachmann aufgrund dieser Vorschriften die jeweils eingesetzten Edukte bekannt sind.

| Beispiel | Name |
|---|---|
| 1 | 2-[(3-Chlor-benzoyl)-methyl-amino]-5,6,7,8-tetrahydro-imidazo[1,2-a]pyridin-3-carbonsäure (2-thiophen-2-yl-ethyl)-amid |
| 2 | 2-[Butyl-(3-chlor-benzoyl)-amino]-5,6,7,8-tetrahydro-imidazo[1,2-a]pyridin-3-carbonsäure [2-(2,5-dimethoxy-phenyl)-ethyl]-amid |
| 3 | N-{3-[4-(2-Ethyl-phenyl)-piperazin-1-carbonyl]-5,6,7,8-tetrahydro-imidazo[1,2-a]pyridin-2-yl}-N-methyl-benzamid |
| 4 | 2-(Methyl-phenylacetyl-amino)-5,6,7,8-tetrahydro-imidazo[1,2-a]pyridin-3-carbonsäure [3-(2-methyl-piperidin-1-yl)-propyl]-amid |
| 5 | 2-[(3-Chlor-benzoyl)-methyl-amino]-5,6,7,8-tetrahydro-imidazo[1,2-a]pyridin-3-carbonsäure (2-phenyl-propyl)-amid |
| 6 | 2-[(3-Chlor-benzoyl)-methyl-amino]-5,6,7,8-tetrahydro-imidazo[1,2-a]pyridin-3-carbonsäure (2-pyrrolidin-1-yl-ethyl)-amid |
| 7 | 2-[Methyl-(naphthalen-1-carbonyl)-amino]-5,6,7,8-tetrahydro-imidazo[1,2-a]pyridin-3-carbonsäure ethyl-pyridin-4-ylmethyl-amid |
| 8 | 2-(Benzoyl-methyl-amino)-5,6,7,8-tetrahydro-imidazo[1,2-a]pyridin-3-carbonsäure (2-thiophen-2-yl-ethyl)-amid |
| 9 | 2-[Butyl-(3-chlor-benzoyl)-amino]-5,6,7,8-tetrahydro-imidazo[1,2-a]pyridin-3-carbonsäure (3-imidazol-1-yl-propyl)-amid |
| 10 | 3-Chlor-N-{3-[4-(3-chlor-phenyl)-piperazin-1-carbonyl]-5,6,7,8-tetrahydro-imidazo[1,2-a]pyridin-2-yl}-N-methyl-benzamid |
| 11 | 3-Chlor-N-methyl-N-[3-(4-phenyl-piperazin-1-carbonyl)-5,6,7,8-tetrahydro-imidazo[1,2-a]pyridin-2-yl]-benzamid |
| 12 | 2-[Butyl-(3,4-difluor-benzoyl)-amino]-5,6,7,8-tetrahydro-imidazo[1,2-a]pyridin-3-carbonsäure indan-1-ylamid |
| 13 | N-Butyl-3-chlor-N-{3-[4-(2-morpholin-4-yl-ethyl)-piperazin-1-carbonyl]-5,6,7,8-tetrahydro-imidazo[1,2-a]pyridin-2-yl}-benzamid |
| 14 | 3-Chlor-N-methyl-N-{3-[4-(3-trifluormethyl-phenyl)-piperazin-1-carbonyl]-5,6,7,8-tetrahydro-imidazo [1,2-a]pyridin-2-yl}-benzamid |
| 15 | 2-[Methyl-(naphthalen-1-carbonyl)-amino]-5,6,7,8-tetrahydro-imidazo[1,2-a]pyridin-3-carbonsäure 3,5-bis-trifluormethyl-benzylamid |
| 16 | 2-[Butyl-(3,4-difluor-benzoyl)-amino]-5,6,7,8-tetrahydro-imidazo[1,2-a]pyridin-3-carbonsäure (4-methyl-cyclohexyl)-amid |
| 17 | 2-(Benzoyl-methyl-amino)-5,6,7,8-tetrahydro-imidazo[1,2-a]pyridin-3-carbonsäure 3-methoxy-benzylamid |
| 18 | 2-[Methyl-(naphthalen-1-carbonyl)-amino]-5,6,7,8-tetrahydro-imidazo[1,2-a]pyridin-3-carbonsäure (1-p-tolyl-ethyl)-amid |
| 19 | 2-[Butyl-(3-chlor-benzoyl)-amino]-5,6,7,8-tetrahydro-imidazo[1,2-a]pyridin-3-carbonsäure (1,2-dimethyl-propyl)-amid |
| 20 | 2-(Benzoyl-methyl-amino)-5,6,7,8-tetrahydro-imidazo[1,2-a]pyridin-3-carbonsäure (4-methyl-cyclohexyl)-amid |
| 21 | 2-(Benzoyl-methyl-amino)-5,6,7,8-tetrahydro-imidazo[1,2-a]pyridin-3-carbonsäure (2-phenyl-propyl)-amid |
| 22 | 4-[2-(Benzoyl-methyl-amino)-5,6,7,8-tetrahydro-imidazo[1,2-a]pyridin-3-carbonyl]-piperazin-1-carbonsäure ethyl ester |

(fortgesetzt)

| Beispiel | Name |
|---|---|
| **23** | 3-Chlor-N-methyl-N-(3-{4-[(methyl-phenyl-carbamoyl)-methyl]-piperazin-1-carbonyl}-5,6,7,8-tetrahydro-imidazo[1,2-a]pyridin-2-yl)-benzamid |
| **24** | 3-Chlor-N-{3-[4-(furan-2-carbonyl)-piperazin-1-carbonyl]-5,6,7,8-tetrahydro-imidazo[1,2-a]pyridin-2-yl}-N-methyl-benzamid |
| **25** | N-Methyl-N-[3-(4-p-tolyl-piperazin-1-carbonyl)-5,6,7,8-tetrahydro-imidazo[1,2-a]pyridin-2-yl]-benzamid |
| **26** | N-Butyl-3-chlor-N-{3-[4-(3-phenyl-propyl)-piperazin-1-carbonyl]-5,6,7,8-tetrahydro-imidazo[1,2-a]pyridin-2-yl}-benzamid |
| **27** | 2-[(3-Chlor-benzoyl)-methyl-amino]-5,6,7,8-tetrahydro-imidazo[1,2-a]pyridin-3-carbonsäure (2,6,6-trimethyl-bicyclo[3.1.1]hept-3-yl)-amid |
| **28** | 2-[Butyl-(3,4-difluor-benzoyl)-amino]-5,6,7,8-tetrahydro-imidazo[1,2-a]pyridin-3-carbonsäure (3-phenyl-propyl)-amid |
| **29** | Naphthalen-1-carbonsäure {3-[4-(4-methoxy-phenyl)-piperazin-1-carbonyl]-5,6,7,8-tetrahydro-imidazo [1,2-a]pyridin-2-yl}-methyl-amid |
| **30** | 2-[Methyl-(naphthalen-1-carbonyl)-amino]-5,6,7,8-tetrahydro-imidazo[1,2-a]pyridin-3-carbonsäure [1-(4-trifluormethyl-benzyl)-pyrrolidin-3-yl]-amid |
| **31** | 2-[(3-Chlor-benzoyl)-methyl-amino]-5,6,7,8-tetrahydro-imidazo[1,2-a]pyridin-3-carbonsäure (4-ethyl-phenyl)-amid |
| **32** | 2-[Methyl-(naphthalen-1-carbonyl)-amino]-5,6,7,8-tetrahydro-imidazo[1,2-a]pyridin-3-carbonsäure (3-methoxy-benzyl)-(tetrahydro-furan-2-ylmethyl)-amid |
| **33** | 2-[Methyl-(naphthalen-1-carbonyl)-amino]-5,6,7,8-tetrahydro-imidazo[1,2-a]pyridin-3-carbonsäure 2,3-dichlor-benzylamid |
| **34** | 2-[Butyl-(3,4-difluor-benzoyl)-amino]-5,6,7,8-tetrahydro-imidazo[1,2-a]pyridin-3-carbonsäure (1-benzofuran-2-ylmethyl-pyrrolidin-3-yl)-methyl-amid |
| **35** | 3-Chlor-N-{3-[4-(4-chlor-phenyl)-piperazin-1-carbonyl]-5,6,7,8-tetrahydro-imidazo[1,2-a]pyridin-2-yl}-N-methyl-benzamid |
| **36** | 2-({2-[(3-Chlor-benzoyl)-methyl-amino]-5,6,7,8-tetrahydro-imidazo[1,2-a]pyridin-3-carbonyl}-amino)-3-(1H-indol-3-yl)-propionsäure methyl ester |
| **37** | N-Butyl-3-chlor-N-[3-(4-phenylacetyl-piperazin-1-carbonyl)-5,6,7,8-tetrahydro-imidazo[1,2-a]pyridin-2-yl]-benzamid |
| **38** | 2-({2-[(3-Chlor-benzoyl)-methyl-amino]-5,6,7,8-tetrahydro-imidazo[1,2-a]pyridin-3-carbonyl}-amino)-3-methyl-pentansäure tert-butyl ester |
| **39** | 2-[Butyl-(3-chlor-benzoyl)-amino]-5,6,7,8-tetrahydro-imidazo[1,2-a]pyridin-3-carbonsäure (1-naphthalen-1-yl-ethyl)-amid |
| **40** | 2-[Methyl-(naphthalen-1-carbonyl)-amino]-5,6,7,8-tetrahydro-imidazo[1,2-a]pyridin-3-carbonsäure allyl-methyl-amid |
| **41** | N-Butyl-N-[3-(3,6-dihydro-2H-pyridin-1-carbonyl)-5,6,7,8-tetrahydro-imidazo[1,2-a]pyridin-2-yl]-3,4-difluor-benzamid |
| **42** | 2-({2-[(3-Chlor-benzoyl)-methyl-amino]-5,6,7,8-tetrahydro-imidazo[1,2-a]pyridin-3-carbonyl}-amino)-4-methyl-pentansäure benzyl ester |
| **43** | 2-[Butyl-(3-chlor-benzoyl)-amino]-5,6,7,8-tetrahydro-imidazo[1,2-a]pyridin-3-carbonsäure 2-ethoxy-benzylamid |
| **44** | N-Butyl-3-chlor-N-{3-[4-(5-methyl-pyrazin-2-carbonyl)-piperazin-1-carbonyl]-5,6,7,8-tetrahydro-imidazo [1,2-a]pyridin-2-yl}-benzamid |

(fortgesetzt)

| Beispiel | Name |
|---|---|
| **45** | N-[3-(4-Benzo[1,3]dioxol-5-ylmethyl-piperazin-1-carbonyl)-5,6,7,8-tetrahydro-imidazo[1,2-a]pyridin-2-yl]-3-chlor-N-methyl-benzamid |
| **46** | 2-[Methyl-(naphthalen-1-carbonyl)-amino]-5,6,7,8-tetrahydro-imidazo[1,2-a]pyridin-3-carbonsäure (2,2-diphenyl-ethyl)-amid |
| **47** | 2-[Butyl-(3-chlor-benzoyl)-amino]-5,6,7,8-tetrahydro-imidazo[1,2-a]pyridin-3-carbonsäure 2,4-difluor-benzylamid |
| **48** | N-Butyl-3-chlor-N-{3-[4-(2-fluor-phenyl)-piperazin-1-carbonyl]-5,6,7,8-tetrahydro-imidazo[1,2-a]pyridin-2-yl}-benzamid |
| **49** | 2-[Methyl-(naphthalen-1-carbonyl)-amino]-5,6,7,8-tetrahydro-imidazo[1,2-a]pyridin-3-carbonsäure (2-p-tolyl-ethyl)-amid |
| **50** | 2-[Methyl-(naphthalen-1-carbonyl)-amino]-5,6,7,8-tetrahydro-imidazo[1,2-a]pyridin-3-carbonsäure (pyridin-2-ylmethyl)-amid |
| **51** | 2-[Butyl-(3-chlor-benzoyl)-amino]-5,6,7,8-tetrahydro-imidazo[1,2-a]pyridin-3-carbonsäure 2-trifluormethyl-benzylamid |
| **52** | 2-[Butyl-(3-chlor-benzoyl)-amino]-5,6,7,8-tetrahydro-imidazo[1,2-a]pyridin-3-carbonsäure indan-1-ylamid |
| **53** | 3-Chlor-N-methyl-N-[3-(4-quinolin-2-ylmethyl-piperazin-1-carbonyl)-5,6,7,8-tetrahydro-imidazo[1,2-a]pyridin-2-yl]-benzamid |
| **54** | N-Butyl-3,4-difluor-N-[3-(4-methyl-piperazin-1-carbonyl)-5,6,7,8-tetrahydro-imidazo[1,2-a]pyridin-2-yl]-benzamid |
| **55** | 2-[(3-Chlor-benzoyl)-methyl-amino]-5,6,7,8-tetrahydro-imidazo[1,2-a]pyridin-3-carbonsäure 2,4-difluor-benzylamid |
| **56** | N-[3-(4-Benzhydryl-piperazin-1-carbonyl)-5,6,7,8-tetrahydro-imidazo[1,2-a]pyridin-2-yl]-N-butyl-3,4-difluor-benzamid |
| **57** | 4-Methyl-2-({2-[methyl-(naphthalen-1-carbonyl)-amino]-5,6,7,8-tetrahydro-imidazo[1,2-a]pyridin-3-carbonyl}-amino)-pentansäure benzyl ester |
| **58** | 2-[Butyl-(3-chlor-benzoyl)-amino]-5,6,7,8-tetrahydro-imidazo[1,2-a]pyridin-3-carbonsäure (2-pyrrolidin-1-yl-ethyl)-amid |
| **59** | 2-[(3-Chlor-benzoyl)-methyl-amino]-5,6,7,8-tetrahydro-imidazo[1,2-a]pyridin-3-carbonsäure [1-(2-benzyloxy-benzyl)-pyrrolidin-3-yl]-amid |
| **60** | 2-[Butyl-(3,4-difluor-benzoyl)-amino]-5,6,7,8-tetrahydro-imidazo[1,2-a]pyridin-3-carbonsäure (pyridin-3-ylmethyl)-amid |
| **61** | 3-Chlor-N-{3-[4-(4-fluor-phenyl)-3,6-dihydro-2H-pyridin-1-carbonyl]-5,6,7,8-tetrahydro-imidazo[1,2-a]pyridin-2-yl}-N-methyl-benzamid |
| **62** | 2-[(3-Chlor-benzoyl)-methyl-amino]-5,6,7,8-tetrahydro-imidazo[1,2-a]pyridin-3-carbonsäure cyclohexylamid |
| **63** | N-[3-(4-Cycloheptyl-piperazin-1-carbonyl)-5,6,7,8-tetrahydro-imidazo[1,2-a]pyridin-2-yl]-N-methyl-benzamid |
| **64** | Naphthalen-1-carbonsäure methyl-[3-(4-thiophen-3-ylmethyl-piperazin-1-carbonyl)-5,6,7,8-tetrahydro-imidazo[1,2-a]pyridin-2-yl]-amid |
| **65** | 2-[Butyl-(3-chlor-benzoyl)-amino]-5,6,7,8-tetrahydro-imidazo[1,2-a]pyridin-3-carbonsäure 2,4-dimethoxy-benzylamid |
| **66** | 1-[2-(Benzoyl-methyl-amino)-5,6,7,8-tetrahydro-imidazo[1,2-a]pyridin-3-carbonyl]-piperidin-4-carbonsäure ethyl ester |

(fortgesetzt)

| Beispiel | Name |
|---|---|
| **67** | 2-[(3-Chlor-benzoyl)-methyl-amino]-5,6,7,8-tetrahydro-imidazo[1,2-a]pyridin-3-carbonsäure (2-benzyloxy-cyclohexyl)-amid |
| **68** | 2-[Methyl-(naphthalen-1-carbonyl)-amino]-5,6,7,8-tetrahydro-imidazo[1,2-a]pyridin-3-carbonsäure [2-(4-phenoxy-phenyl)-ethyl]-amid |
| **69** | 2-[Butyl-(3-chlor-benzoyl)-amino]-5,6,7,8-tetrahydro-imidazo[1,2-a]pyridin-3-carbonsäure [2-(7-methyl-1 H-indol-3-yl)-ethyl]-amid |
| **70** | Naphthalen-1-carbonsäure methyl-[3-(4-phenyl-piperazin-1-carbonyl)-5,6,7,8-tetrahydro-imidazo[1,2-a] pyridin-2-yl]-amid |
| **71** | 2-[(3-Chlor-benzoyl)-methyl-amino]-5,6,7,8-tetrahydro-imidazo[1,2-a]pyridin-3-carbonsäure [2-(3-trifluormethyl-phenyl)-ethyl]-amid |
| **72** | N-Butyl-3-chlor-N-[3-(4-pyridin-2-yl-piperazin-1-carbonyl)-5,6,7,8-tetrahydro-imidazo[1,2-a]pyridin-2-yl]-benzamid |
| **73** | 2-[Methyl-(naphthalen-1-carbonyl)-amino]-5,6,7,8-tetrahydro-imidazo[1,2-a]pyridin-3-carbonsäure 3-methoxy-benzylamid |
| **74** | 2-[Butyl-(3,4-difluor-benzoyl)-amino]-5,6,7,8-tetrahydro-imidazo[1,2-a]pyridin-3-carbonsäure 2,4-difluor-benzylamid |
| **75** | 3-Chlor-N-methyl-N-[3-(4-pyridin-2-yl-piperazin-1-carbonyl)-5,6,7,8-tetrahydro-imidazo[1,2-a]pyridin-2-yl]-benzamid |
| **76** | 2-[Methyl-(naphthalen-1-carbonyl)-amino]-5,6,7,8-tetrahydro-imidazo[1,2-a]pyridin-3-carbonsäure (1-biphenyl-4-ylmethyl-pyrrolidin-3-yl)-methyl-amid |
| **77** | 2-[Butyl-(3-chlor-benzoyl)-amino]-5,6,7,8-tetrahydro-imidazo[1,2-a]pyridin-3-carbonsäure (2,3-dihydro-benzo[1,4]dioxin-2-ylmethyl)-amid |
| **78** | 2-[Butyl-(3-chlor-benzoyl)-amino]-5,6,7,8-tetrahydro-imidazo[1,2-a]pyridin-3-carbonsäure [2-(1 H-indol-3-yl)-ethyl]-methyl-amid |
| **79** | N-Butyl-3-chlor-N-{3-[4-(3-trifluormethyl-phenyl)-piperazin-1-carbonyl]-5,6,7,8-tetrahydro-imidazo[1,2-a]pyridin-2-yl}-benzamid |
| **80** | 2-(Benzoyl-methyl-amino)-5,6,7,8-tetrahydro-imidazo[1,2-a]pyridin-3-carbonsäure [2-(1H-indol-3-yl)-ethyl]-methyl-amid |
| **81** | 3-Chlor-N-{3-[4-hydroxy-4-(3-trifluormethyl-phenyl)-piperidin-1-carbonyl]-5,6,7,8-tetrahydro-imidazo [1,2-a]pyridin-2-yl}-N-methyl-benzamid |
| **82** | 2-[Butyl-(3,4-difluor-benzoyl)-amino]-5,6,7,8-tetrahydro-imidazo[1,2-a]pyridin-3-carbonsäure (1-methoxymethyl-2-phenyl-ethyl)-amid |
| **83** | 2-[(3-Chlor-benzoyl)-methyl-amino]-5,6,7,8-tetrahydro-imidazo[1,2-a]pyridin-3-carbonsäure [1-(2,6-dichlor-benzyl)-pyrrolidin-3-yl]-amid |
| **84** | 2-[(3-Chlor-benzoyl)-methyl-amino]-5,6,7,8-tetrahydro-imidazo[1,2-a]pyridin-3-carbonsäure [1-(2-brom-4,5-dimethoxy-benzyl)-pyrrolidin-3-yl]-amid |
| **85** | 2-(Benzoyl-methyl-amino)-5,6,7,8-tetrahydro-imidazo[1,2-a]pyridin-3-carbonsäure benzo[1,3]dioxol-5-ylamid |
| **86** | 2-[Butyl-(3-chlor-benzoyl)-amino]-5,6,7,8-tetrahydro-imidazo[1,2-a]pyridin-3-carbonsäure (1-methoxymethyl-2-phenyl-ethyl)-amid |
| **87** | 2-[Butyl-(3-chlor-benzoyl)-amino]-5,6,7,8-tetrahydro-imidazo[1,2-a]pyridin-3-carbonsäure [1-(2,6-dichlor-benzyl)-pyrrolidin-3-yl]-methyl-amid |
| **88** | 2-[Butyl-(3,4-difluor-benzoyl)-amino]-5,6,7,8-tetrahydro-imidazo[1,2-a]pyridin-3-carbonsäure 2-trifluormethoxy-benzylamid |

(fortgesetzt)

| Beispiel | Name |
|---|---|
| **89** | N-Butyl-3,4-difluor-N-{3-[4-(isopropylcarbamoyl-methyl)-piperazin-1-carbonyl]-5,6,7,8-tetrahydro-imidazo[1,2-a]pyridin-2-yl}-benzamid |
| **90** | 2-[(3-Chlor-benzoyl)-methyl-amino]-5,6,7,8-tetrahydro-imidazo[1,2-a]pyridin-3-carbonsäure (1-methyl-3-phenyl-propyl)-amid |
| **91** | 2-({2-[Butyl-(3-chlor-benzoyl)-amino]-5,6,7,8-tetrahydro-imidazo[1,2-a]pyridin-3-carbonyl}-amino)-3-methyl-buttersäurebenzylester |
| **92** | 2-[(3-Chlor-benzoyl)-methyl-amino]-5,6,7,8-tetrahydro-imidazo[1,2-a]pyridin-3-carbonsäure (thiophen-2-ylmethyl)-amid |
| **93** | 2-[Butyl-(3,4-difluor-benzoyl)-amino]-5,6,7,8-tetrahydro-imidazo[1,2-a]pyridin-3-carbonsäure (2-methyl-cyclohexyl)-amid |
| **94** | 2-(Benzoyl-methyl-amino)-5,6,7,8-tetrahydro-imidazo[1,2-a]pyridin-3-carbonsäure benzyl-methyl-amid |
| **95** | 2-[Butyl-(3-chlor-benzoyl)-amino]-5,6,7,8-tetrahydro-imidazo[1,2-a]pyridin-3-carbonsäure [2-(2,6-dichlor-benzylsulfanyl)-ethyl]-amid |
| **96** | 2-[Butyl-(3,4-difluor-benzoyl)-amino]-5,6,7,8-tetrahydro-imidazo[1,2-a]pyridin-3-carbonsäure (2,3-dihydro-benzo[1,4]dioxin-2-ylmethyl)-amid |
| **97** | 2-[(3-Chlor-benzoyl)-methyl-amino]-5,6,7,8-tetrahydro-imidazo[1,2-a]pyridin-3-carbonsäure 4-trifluormethyl-benzylamid |
| **98** | N-[3-(4-Benzyl-piperidin-1-carbonyl)-5,6,7,8-tetrahydro-imidazo[1,2-a]pyridin-2-yl]-N-methyl-benzamid |
| **99** | 2-[Methyl-(naphthalen-1-carbonyl)-amino]-5,6,7,8-tetrahydro-imidazo[1,2-a]pyridin-3-carbonsäure 2-trifluormethoxy-benzylamid |
| **100** | 3-Chlor-N-{3-[4-(2,5-dimethyl-phenyl)-piperazin-1-carbonyl]-5,6,7,8-tetrahydro-imidazo[1,2-a]pyridin-2-yl}-N-methyl-benzamid |
| **101** | 2-(Benzoyl-methyl-amino)-5,6,7,8-tetrahydro-imidazo[1,2-a]pyridin-3-carbonsäure [1-(2,6-dichlor-benzyl)-pyrrolidin-3-yl]-amid |
| **102** | 2-[Butyl-(3,4-difluor-benzoyl)-amino]-5,6,7,8-tetrahydro-imidazo[1,2-a]pyridin-3-carbonsäure 2-chlor-6-methyl-benzylamid |
| **103** | N-Butyl-3-chlor-N-[3-(3,5-dimethyl-piperidin-1-carbonyl)-5,6,7,8-tetrahydro-imidazo[1,2-a]pyridin-2-yl]-benzamid |
| **104** | 2-({2-[(3-Chlor-benzoyl)-methyl-amino]-5,6,7,8-tetrahydro-imidazo[1,2-a]pyridin-3-carbonyl}-amino)-3,3-dimethyl-buttersäure tert-butylester |
| **105** | 2-[Methyl-(naphthalen-1-carbonyl)-amino]-5,6,7,8-tetrahydro-imidazo[1,2-a]pyridin-3-carbonsäure (1-cyclohexyl-ethyl)-amid |
| **106** | 3-Chlor-N-methyl-N-[3-(4-phenethyl-piperazin-1-carbonyl)-5,6,7,8-tetrahydro-imidazo[1,2-a]pyridin-2-yl]-benzamid |
| **107** | 2-[Butyl-(3-chlor-benzoyl)-amino]-5,6,7,8-tetrahydro-imidazo[1,2-a]pyridin-3-carbonsäure (2-phenoxy-ethyl)-amid |
| **108** | Naphthalen-1-carbonsäure [3-(4-benzofuran-2-ylmethyl-piperazin-1-carbonyl)-5,6,7,8-tetrahydro-imidazo[1,2-a]pyridin-2-yl]-methyl-amid |
| **109** | N-[3-([1,4']Bipiperidinyl-1'-carbonyl)-5,6,7,8-tetrahydro-imidazo[1,2-a]pyridin-2-yl]-N-butyl-3-chlor-benzamid |
| **110** | N-Butyl-3-chlor-N-[3-(8-fluor-1,3,4,5-tetrahydro-pyrido[4,3-b]indole-2-carbonyl)-5,6,7,8-tetrahydro-imidazo[1,2-a]pyridin-2-yl]-benzamid |
| **111** | 2-[Methyl-(naphthalen-1-carbonyl)-amino]-5,6,7,8-tetrahydro-imidazo[1,2-a]pyridin-3-carbonsäure 4-dimethylamino-benzylamid |

(fortgesetzt)

| Beispiel | Name |
|---|---|
| **112** | 2-[Methyl-(naphthalen-1-carbonyl)-amino]-5,6,7,8-tetrahydro-imidazo[1,2-a]pyridin-3-carbonsäure (1-methyl-3-phenyl-propyl)-amid |
| **113** | 2-[Butyl-(3-chlor-benzoyl)-amino]-5,6,7,8-tetrahydro-imidazo[1,2-a]pyridin-3-carbonsäure 3-methyl-benzylamid |
| **114** | 2-[(3-Chlor-benzoyl)-methyl-amino]-5,6,7,8-tetrahydro-imidazo[1,2-a]pyridin-3-carbonsäure (2,3-dihydro-benzo[1,4]dioxin-6-yl)-amid |
| **115** | Naphthalen-1-carbonsäure methyl-[3-(4-quinolin-2-ylmethyl-piperazin-1-carbonyl)-5,6,7,8-tetrahydro-imidazo[1,2-a]pyridin-2-yl]-amid |
| **116** | 2-[Butyl-(3,4-difluor-benzoyl)-amino]-5,6,7,8-tetrahydro-imidazo[1,2-a]pyridin-3-carbonsäure (2-cyano-ethyl)-methyl-amid |
| **117** | 2-(Methyl-phenylacetyl-amino)-5,6,7,8-tetrahydro-imidazo[1,2-a]pyridin-3-carbonsäure (2-p-tolyl-ethyl)-amid |
| **118** | 2-[Butyl-(3,4-difluor-benzoyl)-amino]-5,6,7,8-tetrahydro-imidazo[1,2-a]pyridin-3-carbonsäure [1-(4-fluor-phenyl)-ethyl]-amid |
| **119** | N-Butyl-N-{3-[4-(5-chlor-2-methyl-phenyl)-piperazin-1-carbonyl]-5,6,7,8-tetrahydro-imidazo[1,2-a]pyridin-2-yl}-3,4-difluor-benzamid |
| **120** | 2-[Butyl-(3-chlor-benzoyl)-amino]-5,6,7,8-tetrahydro-imidazo[1,2-a]pyridin-3-carbonsäure cyclohexylamid |
| **121** | 2-[(3-Chlor-benzoyl)-methyl-amino]-5,6,7,8-tetrahydro-imidazo[1,2-a]pyridin-3-carbonsäure (benzo[1,3]dioxol-5-ylmethyl)-amid |
| **122** | 2-[Butyl-(3,4-difluor-benzoyl)-amino]-5,6,7,8-tetrahydro-imidazo[1,2-a]pyridin-3-carbonsäure 3-methoxy-benzylamid |
| **123** | 3-tert-Butoxy-2-({2-[(3-chlor-benzoyl)-methyl-amino]-5,6,7,8-tetrahydro-imidazo[1,2-a]pyridin-3-carbonyl}-amino)-buttersäure methyl ester |
| **124** | 2-[Butyl-(3-chlor-benzoyl)-amino]-5,6,7,8-tetrahydro-imidazo[1,2-a]pyridin-3-carbonsäure (2-benzyloxy-cyclohexyl)-amid |
| **125** | N-{3-[4-(4-Fluor-phenyl)-piperazin-1-carbonyl]-5,6,7,8-tetrahydro-imidazo[1,2-a]pyridin-2-yl}-N-methyl-benzamid |
| **126** | 2-(Benzoyl-methyl-amino)-5,6,7,8-tetrahydro-imidazo[1,2-a]pyridin-3-carbonsäure (naphthalen-2-ylcarbamoylmethyl)-amid |
| **127** | 2-[Butyl-(3-chlor-benzoyl)-amino]-5,6,7,8-tetrahydro-imidazo[1,2-a]pyridin-3-carbonsäure 4-trifluormethoxy-benzylamid |
| **128** | 2-[(3-Chlor-benzoyl)-methyl-amino]-5,6,7,8-tetrahydro-imidazo[1,2-a]pyridin-3-carbonsäure (3,3-diphenyl-propyl)-amid |
| **129** | 2-[(3-Chlor-benzoyl)-methyl-amino]-5,6,7,8-tetrahydro-imidazo[1,2-a]pyridin-3-carbonsäure (4-benzyloxy-phenyl)-amid |
| **130** | N-{3-[4-(5-Brom-2-ethoxy-benzyl)-piperazin-1-carbonyl]-5,6,7,8-tetrahydro-imidazo[1,2-a]pyridin-2-yl}-N-methyl-2-phenyl-acetamid |
| **131** | N-[3-(3,4-Dihydro-1H-isochinolin-2-carbonyl)-5,6,7,8-tetrahydro-imidazo[1,2-a]pyridin-2-yl]-N-methyl-benzamid |
| **132** | 2-[Butyl-(3-chlor-benzoyl)-amino]-5,6,7,8-tetrahydro-imidazo[1,2-a]pyridin-3-carbonsäure (1-phenyl-propyl)-amid |
| **133** | 2-[Butyl-(3-chlor-benzoyl)-amino]-5,6,7,8-tetrahydro-imidazo[1,2-a]pyridin-3-carbonsäure 2,3-dimethyl-benzylamid |

(fortgesetzt)

| Beispiel | Name |
|---|---|
| **134** | 2-[Butyl-(3-chlor-benzoyl)-amino]-5,6,7,8-tetrahydro-imidazo[1,2-a]pyridin-3-carbonsäure [2-(4-phenoxy-phenyl)-ethyl]-amid |
| **135** | N-{3-[4-(4-Ethoxy-phenyl)-piperazin-1-carbonyl]-5,6,7,8-tetrahydro-imidazo[1,2-a]pyridin-2-yl}-N-methyl-2-phenyl-acetamid |
| **136** | Naphthalen-1-carbonsäure {3-[4-(2-ethyl-phenyl)-piperazin-1-carbonyl]-5,6,7,8-tetrahydro-imidazo[1,2-a]pyridin-2-yl}-methyl-amid |
| **137** | N-[3-(4-Benzo[1,3]dioxol-5-ylmethyl-piperazin-1-carbonyl)-5,6,7,8-tetrahydro-imidazo[1,2-a]pyridin-2-yl]-N-butyl-3,4-difluor-benzamid |
| **138** | N-Butyl-N-{3-[4-(2,5-dimethyl-phenyl)-piperazin-1-carbonyl]-5,6,7,8-tetrahydro-imidazo[1,2-a]pyridin-2-yl}-3,4-difluor-benzamid |
| **139** | 2-[Methyl-(naphthalen-1-carbonyl)-amino]-5,6,7,8-tetrahydro-imidazo[1,2-a]pyridin-3-carbonsäure [(4-chlor-phenyl)-phenyl-methyl]-amid |
| **140** | N-Butyl-3-chlor-N-{3-[4-(4-chlor-benzyl)-piperazin-1-carbonyl]-5,6,7,8-tetrahydro-imidazo[1,2-a]pyridin-2-yl}-benzamid |
| **141** | 2-[Butyl-(3-chlor-benzoyl)-amino]-5,6,7,8-tetrahydro-imidazo[1,2-a]pyridin-3-carbonsäure (1-naphthalen-2-yl-ethyl)-amid |
| **142** | 2-(Methyl-phenylacetyl-amino)-5,6,7,8-tetrahydro-imidazo[1,2-a]pyridin-3-carbonsäure 2-fluor-benzylamid |
| **143** | N-[3-(1,4-Dioxa-8-aza-spiro[4.5]decane-8-carbonyl)-5,6,7,8-tetrahydro-imidazo[1,2-a]pyridin-2-yl]-N-methyl-2-phenyl-acetamid |
| **144** | Naphthalen-1-carbonsäure methyl-[3-(1,3,4,9-tetrahydro-b-carboline-2-carbonyl)-5,6,7,8-tetrahydro-imidazo[1,2-a]pyridin-2-yl]-amid |
| **145** | 2-[Butyl-(3-chlor-benzoyl)-amino]-5,6,7,8-tetrahydro-imidazo[1,2-a]pyridin-3-carbonsäure (1-methyl-3-phenyl-propyl)-amid |
| **146** | 3-Chlor-N-methyl-N-{3-[4-(2,4,6-trimethoxy-benzyl)-piperazin-1-carbonyl]-5,6,7,8-tetrahydro-imidazo [1,2-a]pyridin-2-yl}-benzamid |
| **147** | 2-[Butyl-(3-chlor-benzoyl)-amino]-5,6,7,8-tetrahydro-imidazo[1,2-a]pyridin-3-carbonsäure benzhydryl-amid |
| **148** | Naphthalen-1-carbonsäure {3-[4-(2-chlor-phenyl)-piperazin-1-carbonyl]-5,6,7,8-tetrahydro-imidazo[1,2-a]pyridin-2-yl}-methyl-amid |
| **149** | 2-[Butyl-(3-chlor-benzoyl)-amino]-5,6,7,8-tetrahydro-imidazo[1,2-a]pyridin-3-carbonsäure (thiophen-2-ylmethyl)-amid |
| **150** | N-Butyl-N-{3-[4-(4-chlor-benzyl)-piperazin-1-carbonyl]-5,6,7,8-tetrahydro-imidazo[1,2-a]pyridin-2-yl}-3,4-difluor-benzamid |
| **151** | 2-(Benzoyl-methyl-amino)-5,6,7,8-tetrahydro-imidazo[1,2-a]pyridin-3-carbonsäure (3-phenyl-propyl)-amid |
| **152** | 2-[Butyl-(3-chlor-benzoyl)-amino]-5,6,7,8-tetrahydro-imidazo[1,2-a]pyridin-3-carbonsäure (4-tert-butyl-phenyl)-amid |
| **153** | 2-[Butyl-(3-chlor-benzoyl)-amino]-5,6,7,8-tetrahydro-imidazo[1,2-a]pyridin-3-carbonsäure (4-ethyl-phenyl)-amid |
| **154** | 2-[Methyl-(naphthalen-1-carbonyl)-amino]-5,6,7,8-tetrahydro-imidazo[1,2-a]pyridin-3-carbonsäure cyclohexylamid |
| **155** | 2-[Methyl-(naphthalen-1-carbonyl)-amino]-5,6,7,8-tetrahydro-imidazo[1,2-a]pyridin-3-carbonsäure [3-(methyl-phenyl-amino)-propyl]-amid |

(fortgesetzt)

| Beispiel | Name |
|---|---|
| **156** | 2-(Methyl-phenylacetyl-amino)-5,6,7,8-tetrahydro-imidazo[1,2-a]pyridin-3-carbonsäure [2-(3-trifluormethyl-phenyl)-ethyl]-amid |
| **157** | 2-(Benzoyl-methyl-amino)-5,6,7,8-tetrahydro-imidazo[1,2-a]pyridin-3-carbonsäure 4-chlor-benzylamid |
| **158** | 2-[Butyl-(3-chlor-benzoyl)-amino]-5,6,7,8-tetrahydro-imidazo[1,2-a]pyridin-3-carbonsäure 3-fluor-benzylamid |
| **159** | 2-(Methyl-phenylacetyl-amino)-5,6,7,8-tetrahydro-imidazo[1,2-a]pyridin-3-carbonsäure p-tolylamid |
| **160** | 2-(Benzoyl-methyl-amino)-5,6,7,8-tetrahydro-imidazo[1,2-a]pyridin-3-carbonsäure (2-phenoxy-ethyl)-amid |
| **161** | 2-[Butyl-(3,4-difluor-benzoyl)-amino]-5,6,7,8-tetrahydro-imidazo[1,2-a]pyridin-3-carbonsäure (2-benzyloxy-cyclohexyl)-amid |
| **162** | Naphthalen-1-carbonsäure {3-[4-(4-fluor-phenyl)-piperazin-1-carbonyl]-5,6,7,8-tetrahydro-imidazo[1,2-a]pyridin-2-yl}-methyl-amid |
| **163** | 2-[Methyl-(naphthalen-1-carbonyl)-amino]-5,6,7,8-tetrahydro-imidazo[1,2-a]pyridin-3-carbonsäure [2-(4-chlor-phenyl)-propyl]-amid |
| **164** | N-Butyl-3,4-difluor-N-[3-(thiomrpholine-4-carbonyl)-5,6,7,8-tetrahydro-imidazo[1,2-a]pyridin-2-yl]-benzamid |
| **165** | 2-[(3-Chlor-benzoyl)-methyl-amino]-5,6,7,8-tetrahydro-imidazo[1,2-a]pyridine-3-carboxylsäure (1-adamantan-1-yl-ethyl)-amid |
| **166** | 2-(Benzoyl-methyl-amino)-5,6,7,8-tetrahydro-imidazo[1,2-a]pyridin-3-carbonsäure [(4-chlor-phenyl)-phenyl-methyl]-amid |
| **167** | N-Methyl-N-{3-[4-(4-trifluormethyl-phenyl)-piperazin-1-carbonyl]-5,6,7,8-tetrahydro-imidazo[1,2-a] pyridin-2-yl}-benzamid |
| **168** | 2-(Methyl-phenylacetyl-amino)-5,6,7,8-tetrahydro-imidazo[1,2-a]pyridin-3-carbonsäure (1-biphenyl-4-ylmethyl-pyrrolidin-3-yl)-methyl-amid |
| **169** | N-[3-(4-Benzoyl-piperidin-1-carbonyl)-5,6,7,8-tetrahydro-imidazo[1,2-a]pyridin-2-yl]-3-chlor-N-methyl-benzamid |
| **170** | 2-[Methyl-(naphthalen-1-carbonyl)-amino]-5,6,7,8-tetrahydro-imidazo[1,2-a]pyridin-3-carbonsäure (4-tert-butyl-phenyl)-amid |
| **171** | 2-[Butyl-(3,4-difluor-benzoyl)-amino]-5,6,7,8-tetrahydro-imidazo[1,2-a]pyridin-3-carbonsäure (3,3-dimethyl-butyl)-amid |
| **172** | 2-[Butyl-(3-chlor-benzoyl)-amino]-5,6,7,8-tetrahydro-imidazo[1,2-a]pyridin-3-carbonsäure [1-(2-brom-4,5-dimethoxy-benzyl)-pyrrolidin-3-yl]-amid |
| **173** | Naphthalen-1-carbonsäure methyl-{3-[4-(3-phenyl-allyl)-piperazin-1-carbonyl]-5,6,7,8-tetrahydro-imidazo[1,2-a]pyridin-2-yl}-amid |
| **174** | Naphthalen-1-carbonsäure methyl-[3-(4-phenethyl-piperazin-1-carbonyl)-5,6,7,8-tetrahydro-imidazo [1,2-a]pyridin-2-yl]-amid |
| **175** | N-Butyl-3-chlor-N-{3-[4-hydroxy-4-(3-trifluormethyl-phenyl)-piperidin-1-carbonyl]-5,6,7,8-tetrahydro-imidazo[1,2-a]pyridin-2-yl}-benzamid |
| **176** | N-Methyl-2-phenyl-N-{3-[4-(3-phenyl-propyl)-piperazin-1-carbonyl]-5,6,7,8-tetrahydro-imidazo[1,2-a] pyridin-2-yl}-acetamid |
| **177** | 2-(Benzoyl-methyl-amino)-5,6,7,8-tetrahydro-imidazo[1,2-a]pyridin-3-carbonsäure 3-fluor-benzylamid |
| **178** | N-Butyl-N-{3-[4-(2-chlor-phenyl)-piperazin-1-carbonyl]-5,6,7,8-tetrahydro-imidazo[1,2-a]pyridin-2-yl}-3,4-difluor-benzamid |

(fortgesetzt)

| Beispiel | Name |
|---|---|
| **179** | 2-[Methyl-(naphthalen-1-carbonyl)-amino]-5,6,7,8-tetrahydro-imidazo[1,2-a]pyridin-3-carbonsäure benzo[1,3]dioxol-5-ylamid |
| **180** | 2-[Butyl-(3,4-difluor-benzoyl)-amino]-5,6,7,8-tetrahydro-imidazo[1,2-a]pyridin-3-carbonsäure (3,3-diphenyl-propyl)-amid |
| **181** | 2-[Butyl-(3-chlor-benzoyl)-amino]-5,6,7,8-tetrahydro-imidazo[1,2-a]pyridin-3-carbonsäure (2,3-dihydro-benzo[1,4]dioxin-6-yl)-amid |
| **182** | 2-(Benzoyl-methyl-amino)-5,6,7,8-tetrahydro-imidazo[1,2-a]pyridin-3-carbonsäure [2-(3,4-dimethoxy-phenyl)-ethyl]-amid |
| **183** | Naphthalen-1-carbonsäure [3-(4-benzoyl-piperidin-1-carbonyl)-5,6,7,8-tetrahydro-imidazo[1,2-a]pyridin-2-yl]-methyl-amid |
| **184** | 4-Methyl-2-({2-[methyl-(naphthalen-1-carbonyl)-amino]-5,6,7,8-tetrahydro-imidazo[1,2-a]pyridin-3-carbonyl}-amino)-valeriansäure tert-butylester |
| **185** | 2-[Methyl-(naphthalen-1-carbonyl)-amino]-5,6,7,8-tetrahydro-imidazo[1,2-a]pyridin-3-carbonsäure (4-phenoxy-phenyl)-amid |
| **186** | N-Methyl-2-phenyl-N-[3-(4-phenyl-piperazin-1-carbonyl)-5,6,7,8-tetrahydro-imidazo[1,2-a]pyridin-2-yl]-acetamid |
| **187** | N-Butyl-3,4-difluor-N-{3-[4-(5-trifluormethyl-pyridin-2-yl)-piperazin-1-carbonyl]-5,6,7,8-tetrahydro-imidazo[1,2-a]pyridin-2-yl}-benzamid |
| **188** | 2-(Benzoyl-methyl-amino)-5,6,7,8-tetrahydro-imidazo[1,2-a]pyridin-3-carbonsäure 2,4-dichlor-6-methyl-benzylamid |
| **189** | [4-({2-[Butyl-(3-chlor-benzoyl)-amino]-5,6,7,8-tetrahydro-imidazo[1,2-a]pyridin-3-carbonyl}-amino)-phenyl]-carbaminsäure tert-butyl ester |
| **190** | N-Methyl-2-phenyl-N-[3-(2-pyrrolidin-1-ylmethyl-pyrrolidin-1-carbonyl)-5,6,7,8-tetrahydro-imidazo[1,2-a]pyridin-2-yl]-acetamid |
| **191** | 4-[2-(Methyl-phenylacetyl-amino)-5,6,7,8-tetrahydro-imidazo[1,2-a]pyridin-3-carbonyl]-piperazin-1-carbonsäure tert-butyl ester |

**Pharmakologische Daten**

Die Affinität der erfindungsgemäßen substituierten 5,6,7,8-Tetrahydro-imidazo[1,2-a]pyridin-2-ylamin-Verbindungen für die Batrachotoxin-(BTX)-Bindungsstelle und den μ-Opioid-Rezeptor, sowie die agonistische bzw. antagonistische Wirkung der erfindungsgemäßen substituierten 5,6,7,8-Tetrahydro-imidazo[1,2-a]pyridin-2-ylamin-Verbindungen am Vanilloid-Rezeptor 1 (VR1/TRPV1-Rezeptor), wurden wie vorstehend beschrieben bestimmt.

Die untersuchten erfindungsgemäßen 5,6,7,8-Tetrahydro-imidazo[1,2-a]pyridin-2-ylamin-Verbindungen zeigen eine ausgezeichnete Wirkung auf den Vanilloid-Rezeptor 1 (VR1/TRPV1-Rezeptor).

Darüber hinaus zeigen diese erfindungsgemäßen Verbindungen auch ausgezeichnete Affinitäten für die Batrachotoxin-(BTX)-Bindungsstelle des Natriumkanals und den μ-Opioid-Rezeptor.

| Verbindung gemäß Beispiel | VR1 (Ratte) (% Stimulation im Vgl. zu 10 μM CP) | VR1 (Ratte) (% Hemmung im Vgl. zu 10 μM CP) | BTX Inhibierung (Ratte) (% Hemmung) | μ-Opiold Rezeptor (Mensch) (% Hemmung) |
|---|---|---|---|---|
| 2 | | | 59 | |
| 3 | | | 34 | |
| 4 | | | | |
| 5 | | | | |
| 9 | | | 36 | |

(fortgesetzt)

| Verbindung gemäß Beispiel | VR1 (Ratte) (% Stimulation im Vgl. zu 10 μM CP) | VR1 (Ratte) (% Hemmung im Vgl. zu 10 μM CP) | BTX Inhibierung (Ratte) (% Hemmung) | μ-Opiold Rezeptor (Mensch) (% Hemmung) |
|---|---|---|---|---|
| 11 | | | | |
| 12 | 48 | 73 | | |
| 14 | | | 41 | |
| 15 | | | 68 | 35 |
| 16 | 90 | 100 | 47 | |
| 17 | | | | |
| 19 | 49 | 87 | | |
| 20 | | | | |
| 21 | | | | |
| 22 | | | | |
| 26 | | | 93 | |
| 27 | | | 35 | |
| 28 | | 43 | 42 | |
| 30 | | | 85 | |
| 31 | | | 70 | |
| 33 | | | 49 | |
| 34 | | | 83 | |
| 38 | | 40 | | |
| 39 | 38 | 99 | 47 | |
| 40 | | | | |
| 42 | 43 | 90 | | |
| 43 | 59 | 104 | 40 | |
| 45 | | | 41 | |
| 46 | | | 67 | |
| 47 | 60 | 81 | 44 | |
| 48 | | | 89 | |
| 49 | | | 37 | |
| 51 | 47 | 96 | 35 | |
| 52 | 53 | 101 | 45 | |
| 53 | | | 64 | |
| 56 | | 53 | 65 | |
| 57 | 40 | 103 | 31 | |
| 58 | | | 50 | |
| 59 | | | 95 | |
| 61 | | | 38 | |
| 65 | | 30 | | |

(fortgesetzt)

| Verbindung gemäß Beispiel | VR1 (Ratte) (% Stimulation im Vgl. zu 10 µM CP) | VR1 (Ratte) (% Hemmung im Vgl. zu 10 µM CP) | BTX Inhibierung (Ratte) (% Hemmung) | µ-Opiold Rezeptor (Mensch) (% Hemmung) |
|---|---|---|---|---|
| 66 | | | | |
| 67 | | 52 | 51 | |
| 68 | | | 87 | |
| 69 | | 43 | 48 | |
| 71 | | | 36 | |
| 72 | | | 39 | |
| 73 | | 40 | | |
| 74 | 32 | 94 | | |
| 76 | | | 95 | |
| 77 | | 63 | 56 | |
| 79 | | 62 | 83 | |
| 81 | | | 33 | |
| 82 | | 77 | | |
| 83 | | | 80 | |
| 84 | | | 87 | |
| 85 | | | 30 | |
| 86 | 34 | 114 | 42 | |
| 87 | | | 93 | |
| 88 | 44 | 132 | 31 | |
| 91 | | 121 | 48 | |
| 93 | 51 | 171 | | |
| 95 | | 52 | 68 | 53 |
| 96 | | | 33 | |
| 99 | | | 33 | |
| 100 | | | 74 | |
| 101 | | | 54 | |
| 102 | 66 | 97 | | |
| 104 | | 45 | | |
| 105 | | 32 | | |
| 106 | | | 40 | 30 |
| 107 | | | 39 | |
| 108 | | | 41 | |
| 109 | 68 | 90 | | |
| 112 | | | | 51 |
| 113 | 39 | 89 | 31 | |
| 118 | | 66 | | |

(fortgesetzt)

| Verbindung gemäß Beispiel | VR1 (Ratte) (% Stimulation im Vgl. zu 10 μM CP) | VR1 (Ratte) (% Hemmung im Vgl. zu 10 μM CP) | BTX Inhibierung (Ratte) (% Hemmung) | μ-Opiold Rezeptor (Mensch) (% Hemmung) |
|---|---|---|---|---|
| 119 | | | 83 | |
| 120 | 67 | 97 | 61 | |
| 122 | | 62 | | |
| 123 | 36 | 98 | | |
| 124 | 37 | 93 | 48 | |
| 127 | | | 60 | |
| 128 | | 39 | 85 | |
| 129 | | | 76 | |
| 130 | | | 67 | |
| 132 | 67 | 99 | | |
| 133 | | 64 | 34 | |
| 134 | | | 93 | |
| 136 | | | 54 | |
| 137 | | | 72 | |
| 138 | | | 80 | |
| 139 | | 43 | 73 | |
| 140 | | | 81 | |
| 141 | | | 41 | |
| 144 | | | 31 | |
| 145 | 30 | 82 | 67 | |
| 146 | | | 70 | |
| 147 | | 53 | 56 | |
| 148 | | | 35 | |
| 149 | 43 | 72 | | |
| 150 | | | 69 | |
| 152 | | | 92 | |
| 154 | | | 92 | |
| 156 | | 34 | 40 | |
| 157 | | | 43 | |
| 158 | 33 | 53 | 33 | |
| 159 | | | 37 | |
| 161 | 32 | 91 | 49 | |
| 163 | 83 | 53 | 40 | |
| 165 | | 47 | 39 | |
| 166 | | | 72 | |
| 167 | | | 44 | |

(fortgesetzt)

| Verbindung gemäß Beispiel | VR1 (Ratte) (% Stimulation im Vgl. zu 10 μM CP) | VR1 (Ratte) (% Hemmung im Vgl. zu 10 μM CP) | BTX Inhibierung (Ratte) (% Hemmung) | μ-Opiold Rezeptor (Mensch) (% Hemmung) |
|---|---|---|---|---|
| 168 | | | 92 | |
| 170 | | | 80 | |
| 171 | 46 | 104 | 47 | |
| 172 | | | 96 | |
| 173 | | | 74 | |
| 174 | | | 47 | |
| 175 | | | 65 | |
| 176 | | | 71 | |
| 178 | | | 79 | |
| 179 | | | | |
| 180 | 56 | 102 | 62 | 47 |
| 181 | | | 82 | 58 |
| 184 | 35 | 41 | | |
| 185 | | 56 | 74 | |
| 186 | | | | 48 |
| 187 | | 37 | 41 | |
| 188 | | | | 59 |
| 189 | | | 93 | |

**Patentansprüche**

**1.** Substituierte 5,6,7,8-Tetrahydro-imidazo[1,2-a]pyridin-2-ylamin-Verbindungen der allgemeinen Formel I,

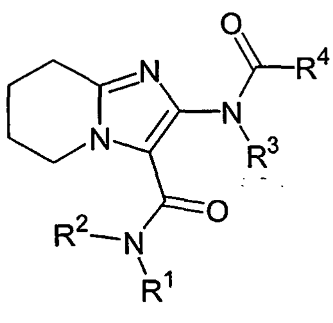

I

worin

$R^1$ und $R^2$, unabhängig voneinander, jeweils
für einen Wasserstoff-Rest;
-C(=O)-$OR^5$;
-($CHR^6$)-$(CH_2)_m$-C(=O)-$OR^7$ mit m = 0, 1, 2, 3, 4 oder 5;
-C(=O)-$R^8$;

$-(CH_2)_n-C(=O)-R^9$ mit n = 1, 2, 3, 4 oder 5;
$-C(=O)-NH-R^{10}$;
$-(CH_2)_o-C(=O)-NHR^{11}$ mit o = 0, 1, 2, 3, 4 oder 5;
$-C(=O)-NR^{12}R^{13}$;
$-(CH_2)_p-C(=O)-NR^{14}R^{15}$ mit p = 1, 2, 3, 4 oder 5;
$-(CHR^{16})-X_q-(CHR^{17})_r-Y_s-(CHR^{18})_t-Z_u-R^{19}$ mit q = 0 oder 1, r = 0 oder 1, s = 0 oder 1, t = 0 oder 1, u = 0 oder 1, worin X, Y und Z, unabhängig voneinander, jeweils für O, S, NH, $N(CH_3)$, N $(C_2H_5)$ oder $N[CH(CH_3)_2]$ stehen;
für einen linearen oder verzweigten, gesättigten oder ungesättigten, ggf. substituierten $C_{1-10}$ aliphatischen Rest;
für einen ungesättigten oder gesättigten, ggf. substituierten 3-, 4-, 5-, 6-, 7-, 8- oder 9-gliedrigen cycloaliphatischen Rest, der mit einer linearen oder verzweigten, ggf. substituierten $C_{1-5}$-Alkylen-Gruppe überbrückt und/ oder mit einem gesättigten, ungesättigten oder aromatischen, ggf. substituierten mono- oder polyzyklischen Ringsystem kondensiert sein kann;
oder für einen ggf. substituierten 5- bis 14-gliedrigen Aryl- oder Heteroaryl-Rest, der mit einem gesättigten oder ungesättigten, ggf. substituierten mono- oder polyzyklischen Ringsystem kondensiert sein kann, stehen; oder $R^1$ und $R^2$ zusammen mit dem sie verbindenden Stickstoffatom als Ringglied einen gesättigten oder ungesättigten, ggf. substituierten 4-, 5-, 6-, 7-, 8- oder 9-gliedrigen heterocycloaliphatischen Rest bilden, der mit einem gesättigten, ungesättigten oder aromatischen, ggf. substituierten mono- oder polyzyklischen Ringsystem kondensiert sein kann und/oder über ein gemeinsames Ringatom zusammen mit einem gesättigten oder ungesättigten, ggf. substituierten 5-, 6- oder 7-gliedrigen cycloaliphatischen Rest eine ggf. substituierte Spiro-Verbindung bilden kann,
wobei jeweils der heterocycloaliphatische Rest und ggf. der cycloaliphatische Rest der Spiro-Verbindung mit 1, 2, 3, 4 oder 5 Substituenten unabhängig voneinander ausgewählt aus der Gruppe bestehend aus $R^{20}$, $-(CHR^{21})-(CH_2)_v-(CH_2)_w-R^{22}$ mit v = 0 oder 1 und w = 0 oder 1, $-CH=CH-R^{23}$, $-(CH_2)_x-C(=O)-OR^{24}$ mit x = 0, 1, 2, 3, 4 oder 5; $-(CH_2)_y-C(=O)-R^{25}$ mit y = 0, 1, 2, 3, 4 oder 5; $-(CH_2)_z-C(=O)-NHR^{26}$ mit z = 0, 1, 2, 3, 4 oder 5; $-(CH_2)_{aa}-C(=O)-NR^{27}R^{28}$ mit aa = 0, 1, 2, 3, 4 oder 5; F; Cl; Br; -CN; $-CF_3$; $-NO_2$; Oxo (=O); Thioxo (=S); $-C_{1-5}$-Alkyl; -OH; $-O-C_{1-5}$-Alkyl; -SH; $-S-C_{1-5}$-Alkyl; $-NH_2$; $-NH-C_{1-5}$-Alkyl und $-N(C_{1-5}\text{-Alkyl})_2$ substituiert und/ oder jeweils weitere 1, 2, 3, 4 oder 5 Heteroatom(e) unabhängig voneinander ausgewählt aus der Gruppe bestehend aus Sauerstoff, Stickstoff und Schwefel als Ringglied(er) aufweisen kann;
$R^3$ für einen Wasserstoff-Rest;
für einen linearen oder verzweigten, gesättigten oder ungesättigten, ggf. substituierten $C_{1-10}$ aliphatischen Rest, für einen ungesättigten oder gesättigten, ggf. substituierten 3-, 4-, 5-, 6-, 7-, 8- oder 9-gliedrigen cycloaliphatischen Rest, der über eine lineare oder verzweigte, ggf. substituierte $C_{1-5}$-Alkylen-, $C_{2-5}$-Alkenylen- oder $C_{2-5}$-Alkinylen-Gruppe gebunden sein kann,
oder für einen ggf. substituierten 5- bis 14-gliedrigen Aryl- oder Heteroaryl-Rest, der mit einem gesättigten oder ungesättigten, ggf. substituierten mono- oder polyzyklischen Ringsystem kondensiert und/oder über eine lineare oder verzweigte, ggf. substituierte $C_{1-5}$-Alkylen-, $C_{2-5}$-Alkenylen- oder $C_{2-5}$-Alkinylen-Gruppe gebunden sein kann, steht;
$R^4$ für einen linearen oder verzweigten, gesättigten oder ungesättigten, ggf. substituierten $C_{1-10}$ aliphatischen Rest,
für einen ungesättigten oder gesättigten, ggf. substituierten 3-, 4-, 5-, 6-, 7-, 8- oder 9-gliedrigen cycloaliphatischen Rest, der über eine lineare oder verzweigte, ggf. substituierte $C_{1-5}$-Alkylen-, $C_{2-5}$-Alkenylen- oder $C_{2-5}$-Alkinylen-Gruppe gebunden sein kann,
oder für einen ggf. substituierten 5- bis 14-gliedrigen Aryl- oder Heteroaryl-Rest, der mit einem gesättigten oder ungesättigten, ggf. substituierten mono- oder polyzyklischen Ringsystem kondensiert und/oder über eine lineare oder verzweigte, ggf. substituierte $C_{1-5}$-Alkylen-, $C_{2-5}$-Alkenylen- oder $C_{2-5}$-Alkinylen-Gruppe gebunden sein kann, steht;
$R^5$, $R^7$ $R^8$, $R^9$, $R^{10}$, $R^{11}$, $R^{12}$, $R^{13}$, $R^{14}$, $R^{15}$, $R^{24}$, $R^{25}$, $R^{26}$, $R^{27}$ und $R^{28}$, unabhängig voneinander, jeweils für einen linearen oder verzweigten, gesättigten oder ungesättigten, ggf. substituierten $C_{1-10}$ aliphatischen Rest,
für einen ungesättigten oder gesättigten, ggf. substituierten 3-, 4-, 5-, 6-, 7-, 8- oder 9-gliedrigen cycloaliphatischen Rest, der mit einem gesättigten, ungesättigten oder aromatischen, ggf. substituierten mono- oder polyzyklischen Ringsystem kondensiert und/oder über eine lineare oder verzweigte, ggf. substituierte $C_{1-5}$-Alkylen-, $C_{2-5}$-Alkenylen- oder $C_{2-5}$-Alkinylen-Gruppe gebunden sein kann,
oder für einen ggf. substituierten 5- bis 14-gliedrigen Aryl- oder Heteroaryl-Rest, der mit einem gesättigten oder ungesättigten, ggf. substituierten mono- oder polyzyklischen Ringsystem kondensiert und/oder über eine lineare oder verzweigte, ggf. substituierte $C_{1-5}$-Alkylen-, $C_{2-5}$-Alkenylen- oder $C_{2-5}$-Alkinylen-Gruppe gebunden sein kann, stehen;
$R^6$ für einen Wasserstoff-Rest;

für einen linearen oder verzweigten, gesättigten oder ungesättigten, ggf. substituierten $C_{1-10}$ aliphatischen Rest, der ggf. 1, 2, 3, 4 oder 5 Heteroatom(e) unabhängig voneinander ausgewählt aus der Gruppe bestehend aus Sauerstoff, Schwefel und Stickstoff als Kettenglied(er) aufweisen kann;
oder für einen ggf. substituierten 5- bis 14-gliedrigen Aryl- oder Heteroaryl-Rest, der mit einem gesättigten oder ungesättigten, ggf. substituierten mono- oder polyzyklischen Ringsystem kondensiert und/oder über eine lineare oder verzweigte, ggf. substituierte $C_{1-5}$-Alkylen-, $C_{2-5}$-Alkenylen- oder $C_{2-5}$-Alkinylen-Gruppe gebunden sein kann, steht;
$R^{16}$, $R^{17}$ und $R^{18}$, unabhängig voneinander, jeweils
für einen Wasserstoff-Rest;
für einen linearen oder verzweigten, gesättigten oder ungesättigten, ggf. substituierten $C_{1-10}$ aliphatischen Rest, der 1, 2, 3, 4 oder 5 Heteroatom(e) unabhängig voneinander ausgewählt aus der Gruppe bestehend aus Sauerstoff, Schwefel und Stickstoff als Kettenglied(er) aufweisen kann
oder für einen ggf. substituierten 5- bis 14-gliedrigen Aryl- oder Heteroaryl-Rest, der mit einem gesättigten oder ungesättigten, ggf. substituierten mono- oder polyzyklischen Ringsystem kondensiert sein kann, stehen;
$R^{19}$ für einen ungesättigten oder gesättigten, ggf. substituierten 3-, 4-, 5-, 6-, 7-, 8- oder 9-gliedrigen cycloaliphatischen Rest, der mit 1, 2, 3, 4 oder 5 linearen oder verzweigten, ggf. substituierten $C_{1-5}$-Alkylen-Gruppen überbrückt und/oder mit einem gesättigten, ungesättigten oder aromatischen, ggf. substituierten mono- oder polyzyklischen Ringsystem kondensiert sein kann;
oder für einen ggf. substituierten 5- bis 14-gliedrigen Aryl- oder Heteroaryl-Rest, der mit einem gesättigten oder ungesättigten, ggf. substituierten mono- oder polyzyklischen Ringsystem kondensiert sein kann, steht;
$R^{20}$ für einen linearen oder verzweigten, gesättigten oder ungesättigten, ggf. substituierten $C_{1-10}$ aliphatischen Rest;
für einen ungesättigten oder gesättigten, ggf. substituierten 3-, 4-, 5-, 6-, 7-, 8- oder 9-gliedrigen cycloaliphatischen Rest
oder für einen ggf. substituierten 5- bis 14-gliedrigen Aryl- oder Heteroaryl-Rest, der mit einem gesättigten oder ungesättigten, ggf. substituierten mono- oder polyzyklischen Ringsystem kondensiert sein kann, steht;
$R^{21}$ für einen Wasserstoff-Rest;
für einen linearen oder verzweigten, gesättigten oder ungesättigten, ggf. substituierten $C_{1-10}$ aliphatischen Rest;
für einen ungesättigten oder gesättigten, ggf. substituierten 3-, 4-, 5-, 6-, 7-, 8- oder 9-gliedrigen cycloaliphatischen Rest
oder für einen ggf. substituierten 5- bis 14-gliedrigen Aryl- oder Heteroaryl-Rest, der mit einem gesättigten oder ungesättigten, ggf. substituierten mono- oder polyzyklischen Ringsystem kondensiert sein kann, steht;
und
$R^{22}$ und $R^{23}$, unabhängig voneinander, jeweils
für einen ungesättigten oder gesättigten, ggf. substituierten 3-, 4-, 5-, 6-, 7-, 8- oder 9-gliedrigen cycloaliphatischen Rest
oder für einen ggf. substituierten 5- bis 14-gliedrigen Aryl- oder Heteroaryl-Rest, der mit einem gesättigten oder ungesättigten, ggf. substituierten mono- oder polyzyklischen Ringsystem kondensiert sein kann, stehen;

wobei
die vorstehend genannten $C_{1-10}$ aliphatischen Reste jeweils ggf. mit 1, 2, 3, 4 oder 5 Substituenten unabhängig voneinander ausgewählt aus der Gruppe bestehend aus F, Cl, Br, I, -CN, $-NO_2$, -OH, -SH und $-NH_2$ substituiert sein können;
die vorstehend genannten cycloaliphatischen Reste jeweils ggf. mit 1, 2, 3, 4 oder 5 Substituenten unabhängig voneinander ausgewählt aus der Gruppe bestehend aus Oxo (=O), Thioxo (=S), F, Cl, Br, I, -CN, $-CF_3$, $-SF_5$, -OH, $-O-C_{1-5}$-Alkyl, $-NH_2$, $-NO_2$, $-O-CF_3$, $-S-CF_3$, -SH, $-S-C_{1-5}$-Alkyl, $-C_{1-5}$-Alkyl, -C(=O)-OH, $-C(=O)-O-C_{1-5}$-Alkyl, -NH-$C_{1-5}$-Alkyl, $-N(C_{1-5}\text{-Alkyl})_2$, $-(CH_2)$-Benzo[b]furanyl, -O-Phenyl, -O-Benzyl, Phenyl und Benzyl substituiert sein können, wobei jeweils der zyklische Teil der Reste -O-Phenyl, -O-Benzyl, Phenyl, $-(CH_2)$-Benzo[b]furanyl und Benzyl mit 1, 2, 3, 4 oder 5 Substituenten unabhängig voneinander ausgewählt aus der Gruppe bestehend aus F, Cl, Br, -OH, $-CF_3$, $-SF_5$, -CN, $-NO_2$, $-C_{1-5}$-Alkyl, $-O-C_{1-5}$-Alkyl, $-O-CF_3$, $-S-CF_3$, Phenyl und -O-Benzyl substituiert sein kann und die vorstehend genannten cycloaliphatischen Reste jeweils ggf. 1, 2, 3, 4 oder 5 Heteroatom(e) unabhängig voneinander ausgewählt aus der Gruppe bestehend aus Sauerstoff, Stickstoff und Schwefel als Ringglied(er) aufweisen können;
die vorstehend genannten $C_{1-5}$-Alkylen-, $C_{2-5}$-Alkenylen- oder $C_{2-5}$-Alkinylen-Gruppen jeweils ggf. mit 1, 2, 3, 4 oder 5 Substituenten unabhängig voneinander ausgewählt aus der Gruppe bestehend aus F, Cl, Br, -OH, -SH, $-NH_2$, -CN, $NO_2$ und Phenyl substituiert sein können;
die Ringe der vorstehend genannten mono- oder polyzyklischen Ringsysteme ggf. jeweils mit 1, 2, 3, 4 oder 5 Substituenten unabhängig voneinander ausgewählt aus der Gruppe bestehend aus Oxo (=O), Thioxo (=S), F, Cl,

Br, I, -CN, $-CF_3$, $-SF_5$, -OH, $-O-C_{1-5}$-Alkyl, $-NH_2$, $-NO_2$, $-O-CF_3$, $-S-CF_3$, -SH, $-S-C_{1-5}$-Alkyl, $-C_{1-5}$-Alkyl, -C(=O)-OH, $-C(=O)-O-C_{1-5}$-Alkyl, $-NH-C_{1-5}$-Alkyl, $-N(C_{1-5}\text{-Alkyl})_2$, $-(CH_2)$-Benzo[b]furanyl, -O-Phenyl, -O-Benzyl, Phenyl und Benzyl substituiert sein können, wobei jeweils der zyklische Teil der Reste -O-Phenyl, -O-Benzyl, Phenyl, $-(CH_2)$-Benzo[b]furanyl und Benzyl mit 1, 2, 3, 4 oder 5 Substituenten unabhängig voneinander ausgewählt aus der Gruppe bestehend aus F, Cl, Br, -OH, $-CF_3$, $-SF_5$, -CN, $-NO_2$, $-C_{1-5}$-Alkyl, $-O-C_{1-5}$-Alkyl, $-O-CF_3$, $-S-CF_3$, Phenyl und -O-Benzyl substituiert sein kann

und die Ringe der vorstehend genannten mono- oder polyzyklischen Ringsysteme jeweils 5-, 6- oder 7-gliedrig sind und jeweils ggf. 1, 2, 3, 4 oder 5 Heteroatom(e) als Ringglied(er) aufweisen können, die unabhängig voneinander aus der Gruppe bestehend aus Sauerstoff, Stickstoff und Schwefel ausgewählt sind;

und die vorstehend genannten Aryl- oder Heteroaryl-Reste jeweils ggf. mit 1, 2, 3, 4 oder 5 Substituenten unabhängig voneinander ausgewählt aus der Gruppe bestehend aus F, Cl, Br, I, -CN, $-CF_3$, $-SF_5$, -OH, $-O-C_{1-5}$-Alkyl, $-NH_2$, $-NO_2$, $-O-CF_3$, $-S-CF_3$, -SH, $-S-C_{1-5}$-Alkyl, $-C_{1-5}$-Alkyl, -C(=O)-OH, $-C(=O)-O-C_{1-5}$-Alkyl, $-NH-C_{1-5}$-Alkyl, $-N(C_{1-5}\text{-Alkyl})_2$, $-NH-C(=O)-O-C_{1-5}$-Alkyl, -C(=O)-H, $-C(=O)-C_{1-5}$-Alkyl, $-C(=O)-NH_2$, $-C(=O)-NH-C_{1-5}$-Alkyl, $C(=O)-N-(C_{1-5}\text{-Alkyl})_2$, $-(CH_2)$-Benzo[b]furanyl, -O-Phenyl, -O-Benzyl, Phenyl und Benzyl substituiert sein können, wobei jeweils der zyklische Teil der Reste -O-Phenyl, -O-Benzyl, Phenyl, $-(CH_2)$-Benzo[b]furanyl und Benzyl mit 1, 2, 3, 4 oder 5 Substituenten unabhängig voneinander ausgewählt aus der Gruppe bestehend aus F, Cl, Br, -OH, $-CF_3$, $-SF_5$, -CN, $-NO_2$, $-C_{1-5}$-Alkyl, $-O-C_{1-5}$-Alkyl, $-O-CF_3$, $-S-CF_3$, Phenyl und -O-Benzyl substituiert sein kann,

und

die vorstehend genannten Heteroaryl-Reste jeweils ggf. 1, 2, 3, 4 oder 5 Heteroatom(e) unabhängig voneinander ausgewählt aus der Gruppe bestehend aus Sauerstoff, Stickstoff und Schwefel als Ringglied(er) aufweisen können; jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form entsprechender Salze, oder jeweils in Form entsprechender Solvate.

**2.** Verbindungen gemäß Anspruch 1, **dadurch gekennzeichnet, dass**
$R^1$ für einen Wasserstoff-Rest; $-C(=O)-OR^5$; $-(CHR^6)-(CH^2)_m-C(=O)-OR^7$ mit m = 0, 1, 2, 3, 4 oder 5; $-C(=O)-NH-R^{10}$; $-(CH_2)_o-C(=O)-NHR^{11}$ mit o = 0, 1, 2, 3, 4 oder 5; $-(CHR^{16})-X_q-(CHR^{17})_r-Y_s-(CHR^{18})_t-Z_u-R^{19}$ mit q = 0 oder 1, r = 0 oder 1, s = 0 oder 1, t = 0 oder 1, u = 0 oder 1, worin X, Y und Z, unabhängig voneinander, jeweils für O, S, NH und $N(CH_3)$ stehen;

für einen ggf. substituierten Alkyl-Rest ausgewählt aus der Gruppe bestehend aus Methyl, Ethyl, n-Propyl, iso-Propyl, $-(CH_2)-(CH_2)-(CN)$, n-Butyl, sec-Butyl, iso-Butyl, tert-Butyl, $-C(H)(CH_3)-C(H)(CH_3)_2$ und $-(CH_2)-(CH_2)-(C(CH_3)_3)$,

für einen Alkenyl-Rest ausgewählt aus der Gruppe bestehend aus Vinyl, 1-Propenyl und 2-Propenyl,

für einen (hetero)cycloaliphatischen Rest ausgewählt aus der Gruppe bestehend aus Cyclopropyl, Cyclobutyl, Cyclopentyl, Cyclohexyl, Cycloheptyl, Cyclopentenyl, Cyclohexenyl, Cycloheptenyl, Imidazolidinyl, Tetrahydrofuranyl, Tetrahydrothiophenyl, Pyrrolidinyl, Piperidinyl, Morpholinyl, Piperazinyl, Thiomorpholinyl, Tetrahydropyranyl, Azepanyl, Diazepanyl, Dithiolanyl, (6,6)-Dimethyl-[3.1.1]-bicycloheptyl, Indanyl, Indenyl, (1,4)-Benzodioxanyl, (1,2,3,4)-Tetrahydronaphthyl, (1,2,3,4)-Tetrahydrochinolinyl und (1,2,3,4)-Tetrahydrochinazolinyl, wobei der (hetero)cycloaliphatische Rest jeweils ggf. mit 1, 2, 3, 4 oder 5 Substituenten unabhängig voneinander ausgewählt aus der Gruppe bestehend aus F, Cl, Br, I, -CN, $-CF_3$, $-SF_5$, -OH, $-NH_2$, $-O-CF_3$, -SH, $-O-CH_3$, $-O-C_2H_5$, Methyl, Ethyl, n-Propyl, iso-Propyl, n-Butyl, sec-Butyl, iso-Butyl, tert-Butyl, $-S-CH_3$, $-S-C_2H_5$, $-C(=O)-O-CH_3$, $-C(=O)-O-C_2H_5$, Oxo (=O), $-N(CH_3)_2$, $-N(C_2H_5)_2$, $-N(H)(CH_3)$, $-N(H)(C_2H_5)$, $-NO_2$, $-SCF_3$, -C(=O)-OH, $-(CH_2)$-Benzo[b]furanyl, -O-Phenyl, -O-Benzyl, Phenyl und Benzyl substituiert sein kann,

wobei jeweils der zyklische Teil der $-(CH_2)$-Benzo[b]furanyl, -O-Phenyl, -O-Benzyl-, Benzyl- und Phenyl-Reste mit 1, 2, 3, 4 oder 5 Substituenten unabhängig voneinander ausgewählt aus der Gruppe bestehend aus F, Cl, Br, -OH, $-CF_3$, $-SF_5$, -CN, $-NO_2$, Methyl, Ethyl, iso-Propyl, n-Propyl, $-O-CH_3$, $-O-C_2H_5$, $-O-CF_3$, Phenyl und -O-Benzyl substituiert sein kann,

oder für einen Rest ausgewählt aus der Gruppe bestehend aus Phenyl, Naphthyl, (1,3)-Benzodioxolyl, (1,4)-Benzodioxanyl, Thiophenyl, Furanyl, Pyrrolyl, Pyrazolyl, Pyrazinyl, Pyranyl, Triazolyl, Pyridinyl, Imidazolyl, Indolyl, Isoindolyl, Benzo[b]furanyl, Benzo[b]thiophenyl, Thiazolyl, Oxazolyl, Isoxazolyl, Pyridazinyl, Pyrazinyl, Pyrimidinyl, Indazolyl, Chinazolinyl, Chinolinyl und Isochinolinyl, wobei der Rest jeweils ggf. mit 1, 2, 3, 4 oder 5 Substituenten unabhängig voneinander ausgewählt aus der Gruppe bestehend aus F, Cl, Br, I, -CN, $-NO_2$, -OH, -SH, Methyl, Ethyl, n-Propyl, iso-Propyl, n-Butyl, sec-Butyl, iso-Butyl, tert-Butyl, $-CF_3$, $-O-CF_3$, $-S-CF_3$, $-SF_5$, $-O-CH_3$, $-O-C_2H_5$, -O-Phenyl, -O-Benzyl, $-NH_2$, $-N(CH_3)_2$, $-N(C_2H_5)_2$, $-N(H)(CH_3)$, $-N(H)(C_2H_5)$, $-NH-C(=O)-O-CH_3$, $-NH-C(=O)-O-C_2H_5$ und $-NH-C(=O)-O-C(CH_3)_3$ substituiert sein kann, steht.

**3.** Verbindungen gemäß Anspruch 1 oder 2, **dadurch gekennzeichnet, dass**

$R^2$ für einen Wasserstoff-Rest,
für $-(CHR^{16})-R^{19}$,
oder für einen Alkyl-Rest ausgewählt aus der Gruppe bestehend aus Methyl, Ethyl, n-Propyl, iso-Propyl, n-Butyl, sec-Butyl, iso-Butyl und tert-Butyl steht.

**4.** Verbindungen gemäß einem oder mehreren der Ansprüche 1 bis 3, **dadurch gekennzeichnet, dass**
$R^1$ und $R^2$ zusammen mit dem sie verbindenden Stickstoffatom als Ringglied einen heterocycloaliphatischen Rest bilden ausgewählt aus der Gruppe bestehend aus Pyrrolidinyl; Piperidinyl; (1,2,3,6)-Tetrahydropyridinyl; (1,2,3,4)-Tetrahydropyridinyl und (1,4)-Dioxa-8-aza-spiro[4.5]decan, wobei der heterocycloaliphatische Rest jeweils mit 1 oder 2 Substituenten unabhängig voneinander ausgewählt aus der Gruppe bestehend aus $R^{20}$, $-(CHR^{21})-(CH_2)_v-R^{22}$ mit v = 0 oder 1; $-C(=O)-OR^{24}$; $-C(=O)-R^{25}$; F; Cl; Br; -CN; $-CF_3$ und -OH substituiert sein kann,
oder
$R^1$ und $R^2$ zusammen mit dem sie verbindenden Stickstoffatom als Ringglied einen heterocycloaliphatischen Rest bilden ausgewählt aus der Gruppe bestehend aus (1,3,4,5)-Tetrahydropyrido[4,3-b]indolyl; (3,4)-Dihydro-1 H-isochinolinyl und (1,3,4,9)-Tetrahydro-[b]-carbolinyl, wobei der heterocycloaliphatische Rest jeweils mit 1 oder 2 Substituenten unabhängig voneinander ausgewählt aus der Gruppe bestehend aus F, Cl, Br, -CN, $-CF_3$ und -OH substituiert sein kann,
oder
$R^1$ und $R^2$ zusammen mit dem sie verbindenden Stickstoffatom als Ringglied einen heterocycloaliphatischen Rest bilden ausgewählt aus der Gruppe bestehend aus Imidazolidinyl, (1,3)-Thiazolidinyl, Piperazinyl, Morpholinyl und Thiomorpholinyl, wobei der heterocycloaliphatische Rest jeweils mit 1 oder 2 Substituenten unabhängig voneinander ausgewählt aus der Gruppe bestehend aus $R^{20}$, $-(CHR^{21})-(CH_2)_v-(CH_2)_w-R^{22}$ mit v = 0 oder 1 und w = 0 oder 1, $-CH=CH-R^{23}$,- $C(=O)-OR^{24}$; $-C(=O)-R^{25}$; $-(CH_2)_z-(=O)-NHR^{26}$ mit z = 1 und $-(CH_2)_{aa}-C(=O)-NR^{27}R^{28}$ mit aa = 1 substituiert sein kann.

**5.** Verbindungen gemäß einem oder mehreren der Ansprüche 1 bis 4, **dadurch gekennzeichnet, dass**
$R^3$ für einen Wasserstoff-Rest oder für einen Alkyl-Rest ausgewählt aus der Gruppe bestehend aus Methyl, Ethyl, n-Propyl, iso-Propyl, n-Butyl, sec-Butyl, iso-Butyl, tert-Butyl und n-Pentyl steht.

**6.** Verbindungen gemäß einem oder mehreren der Ansprüche 1 bis 5, **dadurch gekennzeichnet, dass**
$R^4$ für einen Phenyl- oder Naphthyl-Rest steht, der jeweils mit 1, 2, 3, 4 oder 5 Substituenten unabhängig voneinander ausgewählt aus der Gruppe bestehend aus Methyl, Ethyl, n-Propyl, iso-Propyl, n-Butyl, iso-Butyl, sec-Butyl, tert-Butyl, F, Cl, Br, -CN, $-SF_5$, $-S-CF_3$, $-NH_2$, $-N(CH_3)_2$, $-N(C_2H_5)_2$, $-N(H)(CH_3)$, $-N(H)(C_2H_5)$, -SH, $-NO_2$, $-CF_3$, $-OCF_3$, -OH, $-O-CH_3$ und $-O-C_2H_5$ substituiert und/oder über eine $-(CH_2)-$, $-(CH_2)_2-$ oder $-(CH_2)_3$-Gruppe gebunden sein kann.

**7.** Verbindungen gemäß einem oder mehreren der Ansprüche 1 bis 6, **dadurch gekennzeichnet, dass**
$R^5$, $R^7$, $R^8$, $R^9$, $R^{10}$, $R^{11}$, $R^{12}$, $R^{13}$, $R^{14}$ und $R^{15}$, unabhängig voneinander, jeweils für einen Alkyl-Rest ausgewählt aus der Gruppe bestehend aus Methyl, Ethyl, n-Propyl, iso-Propyl, n-Butyl, sec-Butyl, iso-Butyl und tert-Butyl, wobei der Alkyl-Rest jeweils ggf. mit 1, 2, 3, 4 oder 5 Substituenten unabhängig voneinander ausgewählt aus der Gruppe bestehend aus F, Cl, Br, I, -CN, $-NO_2$, -OH, -SH und $-NH_2$ substituiert sein kann,
oder für einen Rest ausgewählt aus der Gruppe bestehend aus Phenyl, Naphthyl, (1,3)-Benzodioxolyl, (1,4)-Benzodioxanyl, Thiophenyl, Furanyl, Pyrrolyl, Pyrazolyl, Pyrazinyl, Pyranyl, Triazolyl, Pyridinyl, Imidazolyl, Indolyl, Isoindolyl, Benzo[b]furanyl, Benzo[b]thiophenyl, Thiazolyl, Oxazolyl, Isoxazolyl, Pyridazinyl, Pyrazinyl, Pyrimidinyl, Indazolyl, Chinazolinyl, Chinolinyl und Isochinolinyl, wobei der Rest jeweils über eine $-(CH_2)-$, $-(CH_2)_2-$ oder $-(CH_2)_3$-Gruppe gebunden und/oder ggf. mit 1, 2, 3, 4 oder 5 Substituenten unabhängig voneinander ausgewählt aus der Gruppe bestehend aus F, Cl, Br, I, -CN, $-NO_2$, -OH, -SH, Methyl, Ethyl, n-Propyl, iso-Propyl, n-Butyl, sec-Butyl, iso-Butyl, tert-Butyl, $-CF_3$, $-O-CF_3$, $-S-CF_3$, $-SF_5$, $-O-CH_3$, $-O-C_2H_5$, -O-Phenyl, -O-Benzyl, $-NH_2$, $-N(CH_3)_2$, $-N(C_2H_5)_2$, $-N(H)(CH_3)$, $-N(H)(C_2H_5)$, $-NH-C(=O)-O-CH_3$, $-NH-C(=O)-O-C_2H_5$ und $-NH-C(=O)-O-C(CH_3)_3$ substituiert sein kann, stehen.

**8.** Verbindungen gemäß einem oder mehreren der Ansprüche 1 bis 7, **dadurch gekennzeichnet, dass**
$R^6$ für einen Wasserstoff-Rest,
für einen Rest ausgewählt aus der Gruppe bestehend aus Methyl, Ethyl, n-Propyl, iso-Propyl, n-Butyl, sec-Butyl, iso-Butyl, tert-Butyl, n-Pentyl, sec-Pentyl, $-C(H)(CH_3)-C(H)(CH_3)_2$, $-(CH_2)-(CH_2)-(C(CH_3)_3)$, $-C(H)(CH_3)(O(C(CH_3)_3))$ und n-Hexyl, wobei der Rest jeweils ggf. mit 1, 2, 3, 4 oder 5 Substituenten unabhängig voneinander ausgewählt aus der Gruppe bestehend aus F, Cl, Br, I, -CN, $-NO_2$, -OH, -SH und $-NH_2$ substituiert sein kann,
oder für einen Rest ausgewählt aus der Gruppe bestehend aus Phenyl, Naphthyl, (1,3)-Benzodioxolyl, (1,4)-Benzodioxanyl, Thiophenyl, Furanyl, Pyrrolyl, Pyrazolyl, Pyrazinyl, Pyranyl, Triazolyl, Pyridinyl, Imidazolyl, Indolyl, Isoindolyl, Benzo[b]furanyl, Benzo[b]thiophenyl, Thiazolyl, Oxazolyl, Isoxazolyl, Pyridazinyl, Pyrazinyl, Pyrimidinyl, Indazolyl, Chinazolinyl, Chinolinyl und Isochinolinyl, wobei der Rest jeweils über eine $-(CH_2)-$, $-(CH_2)_2-$ oder $-(CH_2)_3$-Gruppe gebunden und/oder ggf. mit 1, 2, 3, 4 oder 5 Substituenten unabhängig voneinander ausgewählt aus der Gruppe bestehend aus F, Cl, Br, I, -CN, $-NO_2$, -OH, -SH, Methyl, Ethyl, n-Propyl, iso-Propyl, n-Butyl, sec-Butyl, iso-Butyl, tert-Butyl, $-CF_3$, $-O-CF_3$, $-S-CF_3$, $-SF_5$, $-O-CH_3$, $-O-C_2H_5$, -O-Phenyl, -O-Benzyl, $-NH_2$, $-N(CH_3)_2$, $-N(C_2H_5)_2$, $-N(H)(CH_3)$, $-N(H)(C_2H_5)$, $-NH-C(=O)-O-CH_3$, $-NH-C(=O)-O-C_2H_5$ und $-NH-C(=O)-O-C(CH_3)_3$ substituiert sein kann, steht.

9. Verbindungen gemäß einem oder mehreren der Ansprüche 1 bis 8, **dadurch gekennzeichnet, dass** $R^{16}$, $R^{17}$ und $R^{18}$, unabhängig voneinander, jeweils für einen Wasserstoff-Rest,
für einen Rest ausgewählt aus der Gruppe bestehend aus Methyl, Ethyl, n-Propyl, iso-Propyl, n-Butyl, sec-Butyl, iso-Butyl, tert-Butyl, n-Pentyl, sec-Pentyl, $-C(H)(CH_3)-C(H)(CH_3)_2$, $-(CH_2)-(CH_2)-(C(CH_3)_3)$, $-(CH_2)-O-(CH_3)$ und n-Hexyl,
wobei der Rest jeweils ggf. mit 1, 2, 3, 4 oder 5 Substituenten unabhängig voneinander ausgewählt aus der Gruppe bestehend aus F, Cl, Br, I, -CN, $-NO_2$, -OH, -SH und $-NH_2$ substituiert sein kann,
oder für einen Rest ausgewählt aus der Gruppe bestehend aus Phenyl und Naphthyl, wobei der Rest jeweils mit 1, 2, 3, 4 oder 5 Substituenten unabhängig voneinander ausgewählt aus der Gruppe bestehend aus F, Cl, Br, I, -CN, $-NO_2$, -OH, -SH, Methyl, Ethyl, n-Propyl, iso-Propyl, n-Butyl, sec-Butyl, iso-Butyl, tert-Butyl, $-CF_3$, $-O-CF_3$, $-S-CF_3$, $-SF_5$, $-O-CH_3$, $-O-C_2H_5$, -O-Phenyl, -O-Benzyl, $-NH_2$, $-N(CH_3)_2$, $-N(C_2H_5)_2$, $-N(H)(CH_3)$, $-N(H)(C_2H_5)$, $-NH-C(=O)-O-CH_3$, $-NH-C(=O)-O-C_2H_5$ und $-NH-C(=O)-O-C(CH_3)_3$ substituiert sein kann, stehen.

10. Verbindungen gemäß einem oder mehreren der Ansprüche 1 bis 9, **dadurch gekennzeichnet, dass** $R^{19}$ für einen (hetero)cycloaliphatischen Rest ausgewählt aus der Gruppe bestehend aus Cyclopropyl, Cyclobutyl, Cyclopentyl, Cyclohexyl, Cycloheptyl, Cyclopentenyl, Cyclohexenyl, Cycloheptenyl, Imidazolidinyl, Tetrahydrofuranyl, Tetrahydrothiophenyl, Pyrrolidinyl, Piperidinyl, Morpholinyl, Piperazinyl, Thiomorpholinyl, Tetrahydropyranyl, Azepanyl, Diazepanyl, Dithiolanyl, (6,6)-Dimethyl-[3.1.1]-bicycloheptyl, Adamantyl (Tricyclo-[3.3.1.1$^{3,7}$]-decanyl), Indanyl, Indenyl, (1,4)-Benzodioxanyl, (1,2,3,4)-Tetrahydronaphthyl, (1,2,3,4)-Tetrahydrochinolinyl und (1,2,3,4)-Tetrahydrochinazolinyl, wobei der (hetero)cycloaliphatische Rest jeweils ggf. mit 1, 2, 3, 4 oder 5 Substituenten unabhängig voneinander ausgewählt aus der Gruppe bestehend aus F, Cl, Br, I, -CN, $-CF_3$, $-SF_5$, -OH, $-NH_2$, $-O-CF_3$, -SH, $-O-CH_3$, $-O-C_2H_5$, Methyl, Ethyl, n-Propyl, iso-Propyl, n-Butyl, sec-Butyl, iso-Butyl, tert-Butyl, $-S-CH_3$, $-S-C_2H_5$, $-C(=O)-O-CH_3$, $-C(=O)-O-C_2H_5$, Oxo (=O), $-N(CH_3)_2$, $-N(C_2H_5)_2$, $-N(H)(CH_3)$, $-N(H)(C_2H_5)$, $-NO_2$, $-SCF_3$, -C(=O)-OH, $-(CH_2)$-Benzo[b]furanyl, -O-Phenyl, -O-Benzyl, Phenyl und Benzyl substituiert sein kann,
oder für einen Rest ausgewählt aus der Gruppe bestehend aus Phenyl, Naphthyl, (1,3)-Benzodioxolyl, (1,4)-Benzodioxanyl, Thiophenyl, Furanyl, Pyrrolyl, Pyrazolyl, Pyrazinyl, Pyranyl, Triazolyl, Pyridinyl, Imidazolyl, Indolyl, Isoindolyl, Benzo[b]furanyl, Benzo[b]thiophenyl, Thiazolyl, Oxazolyl, Isoxazolyl, Pyridazinyl, Pyrazinyl, Pyrimidinyl, Indazolyl, Chinazolinyl, Chinolinyl und Isochinolinyl, wobei der Rest jeweils ggf. mit 1, 2, 3, 4 oder 5 Substituenten unabhängig voneinander ausgewählt aus der Gruppe bestehend aus F, Cl, Br, I, -CN, $-NO_2$, -OH, -SH, Methyl, Ethyl, n-Propyl, iso-Propyl, n-Butyl, sec-Butyl, iso-Butyl, tert-Butyl, $-CF_3$, $-O-CF_3$, $-S-CF_3$, $-SF_5$, $-O-CH_3$, $-O-C_2H_5$, -O-Phenyl, -O-Benzyl, $-NH_2$, $-N(CH_3)_2$, $-N(C_2H_5)_2$, $-N(H)(CH_3)$, $-N(H)(C_2H_5)$, $-NH-C(=O)-O-CH_3$, $-NH-C(=O)-O-C_2H_5$ und $-NH-C(=O)-O-C(CH_3)_3$ substituiert sein kann, steht.

11. Verbindungen gemäß einem oder mehreren der Ansprüche 1 bis 10, **dadurch gekennzeichnet, dass** $R^{20}$ für einen Alkyl-Rest ausgewählt aus der Gruppe bestehend aus Methyl, Ethyl, n-Propyl, iso-Propyl, n-Butyl, iso-Butyl, sec-Butyl, tert-Butyl und n-Pentyl,
für einen (hetero)cycloaliphatischen Rest ausgewählt aus der Gruppe bestehend aus Cyclopentyl, Cyclohexyl, Piperidinyl und Cycloheptyl, wobei der (hetero)cycloaliphatische Rest jeweils ggf. mit 1, 2, 3, 4 oder 5 Substituenten unabhängig voneinander ausgewählt aus der Gruppe bestehend aus F, Cl, Br, I, -CN, $-CF_3$, $-SF_5$, -OH, $-NH_2$, $-O-CF_3$, -SH, $-O-CH_3$, $-O-C_2H_5$, Methyl, Ethyl, n-Propyl, iso-Propyl, n-Butyl, sec-Butyl, iso-Butyl und tert-Butyl substituiert sein kann,
oder für einen Rest ausgewählt aus der Gruppe bestehend aus Phenyl, Naphthyl, (1,3)-Benzodioxolyl, Thiophenyl, Furanyl, Pyridinyl, Imidazolyl, Indolyl, Benzo[b]furanyl, Benzo[b]thiophenyl, Thiazolyl, Oxazolyl, Isoxazolyl, Pyridazinyl, Pyrazinyl, Pyrimidinyl und Chinolinyl, wobei der Rest jeweils ggf. mit 1, 2, 3, 4 oder 5 Substituenten unabhängig voneinander ausgewählt aus der Gruppe bestehend aus Methyl, Ethyl, n-Propyl, iso-Propyl, n-Butyl, iso-Butyl, sec-Butyl, tert-Butyl, F, Cl, Br, -CN, $-SF_5$, $-O-CF_3$, $-S-CF_3$, $-NH_2$, $-N(CH_3)_2$, $-N(C_2H_5)_2$, $-N(H)(CH_3)$, $-N(H)(C_2H_5)$, -SH, $-NO_2$, $-CF_3$, $-OCF_3$, -OH, $-O-CH_3$ und $-O-C_2H_5$ substituiert sein kann, steht.

12. Verbindungen gemäß einem oder mehreren der Ansprüche 1 bis 11, **dadurch gekennzeichnet, dass**

$R^{21}$ für einen Wasserstoff-Rest
oder für einen Phenyl- oder Naphthyl-Rest, der mit 1, 2 oder 3 Substituenten unabhängig voneinander ausgewählt aus der Gruppe bestehend aus Methyl, Ethyl, n-Propyl, iso-Propyl, n-Butyl, iso-Butyl, sec-Butyl, tert-Butyl, F, Cl und Br substituiert sein kann, steht.

**13.** Verbindungen gemäß einem oder mehreren der Ansprüche 1 bis 12, **dadurch gekennzeichnet, dass**
$R^{22}$ und $R^{23}$, unabhängig voneinander, jeweils für einen (hetero)cycloaliphatischen Rest ausgewählt aus der Gruppe bestehend aus Pyrrolidinyl, Morpholinyl und Thiomorpholinyl, wobei der (hetero)cycloaliphatische Rest jeweils ggf. mit 1, 2, 3, 4 oder 5 Substituenten unabhängig voneinander ausgewählt aus der Gruppe bestehend aus F, Cl, Br, I, -CN, $-CF_3$, $-SF_5$, -OH, $-NH_2$, $-O-CF_3$, -SH, $-O-CH_3$, $-O-C_2H_5$, Methyl, Ethyl, n-Propyl, iso-Propyl, n-Butyl, sec-Butyl, iso-Butyl und tert-Butyl substituiert sein kann,
oder für einen Rest ausgewählt aus der Gruppe bestehend aus Phenyl, Naphthyl, (1,3)-Benzodioxolyl, Thiophenyl, Furanyl, Pyridinyl, Imidazolyl, Indolyl, Benzo[b]furanyl, Benzo[b]thiophenyl, Thiazolyl, Oxazolyl, Isoxazolyl, Pyridazinyl, Pyrazinyl, Pyrimidinyl und Chinolinyl, wobei der Rest jeweils ggf. mit 1, 2, 3, 4 oder 5 Substituenten unabhängig voneinander ausgewählt aus der Gruppe bestehend aus Methyl, Ethyl, n-Propyl, iso-Propyl, n-Butyl, iso-Butyl, sec-Butyl, tert-Butyl, F, Cl, Br, -CN, $-SF_5$, $-O-CF_3$, $-S-CF_3$, $-NH_2$, $-N(CH_3)_2$, $-N(C_2H_5)_2$, $-N(H)(CH_3)$, $-N(H)(C_2H_5)$, -SH, $-NO_2$, $-CF_3$, $-OCF_3$, -OH, $-O-CH_3$ und $-O-C_2H_5$ substituiert sein kann, stehen.

**14.** Verbindungen gemäß einem oder mehreren der Ansprüche 1 bis 13, **dadurch gekennzeichnet, dass**
$R^{24}$ für einen Alkyl-Rest ausgewählt aus der Gruppe bestehend aus Methyl, Ethyl, n-Propyl, iso-Propyl, n-Butyl, iso-Butyl, sec-Butyl und tert-Butyl steht,
$R^{25}$ für einen Rest ausgewählt aus der Gruppe bestehend aus Phenyl, Naphthyl, Furanyl, Pyrazinyl und Pyrimidinyl, der über eine $-(CH_2)-$, $-(CH_2)_2-$ oder $-(CH_2)_3$-Gruppe gebunden und/oder mit 1, 2 oder 3 Substituenten unabhängig voneinander ausgewählt aus der Gruppe bestehend aus Methyl, Ethyl, n-Propyl und iso-Propyl substituiert sein kann, steht,
$R^{26}$ für einen Alkyl-Rest ausgewählt aus der Gruppe bestehend aus Methyl, Ethyl, n-Propyl und iso-Propyl steht,
$R^{27}$ für einen Alkyl-Rest ausgewählt aus der Gruppe bestehend aus Methyl, Ethyl, iso-Propyl und n-Propyl oder für einen Phenyl-Rest steht,
und
$R^{28}$ für einen Alkyl-Rest ausgewählt aus der Gruppe bestehend aus Methyl, Ethyl, iso-Propyl und n-Propyl oder für einen Phenyl-Rest steht.

**15.** Verbindungen gemäß einem oder mehreren der Ansprüche 1 bis 14, **dadurch gekennzeichnet, dass**

$R^1$ für $-C(=O)-OR^5$; $-(CHR^6)-C(=O)-OR^7$;
$-C(=O)-NHR^{11}$; $-(CH_2)-C(=O)-NHR^{11}$; $-(CHR^{16})-R^{19}$; $-(CHR^{16})-(CHR^{17})-R^{19}$;
$-(CHR^{16})-(CHR^{17})-O-R^{19}$; $-(CHR^{16})-(CHR^{17})-(CHR^{18})-R^{19}$; $-(CHR^{16})-(CHR^{17})-S-(CHR^{18})-R^{19}$; $-(CHR^{16})-(CHR^{17})-(CHR^{18})-N(CH_3)-R^{19}$,
für einen ggf. substituierten Alkyl-Rest ausgewählt aus der Gruppe bestehend aus Methyl, Ethyl, n-Propyl, iso-Propyl, $-CH_2-CH_2-CN$, n-Butyl, sec-Butyl, iso-Butyl, tert-Butyl, $-CH(CH_3)-CH(CH_3)_2$ und $-CH_2-CH_2-C(CH_3)_3$,
für einen Alkenyl-Rest ausgewählt aus der Gruppe bestehend aus 1-Propenyl und 2-Propenyl,
für einen Rest ausgewählt aus der Gruppe bestehend aus Cyclopentyl, Cyclohexyl, Cycloheptyl, (6,6)-Dimethyl-[3.1.1]-bicycloheptyl, Indanyl und Indenyl, wobei der Rest jeweils mit 1, 2 oder 3 Substituenten unabhängig voneinander ausgewählt aus der Gruppe bestehend aus F, Cl, Br, Methyl, Ethyl, iso-Propyl, n-Propyl und -O-Benzyl substituiert sein kann,
für einen Pyrrolidinyl-Rest, der mit einem $-(CH_2)$-Benzo[b]furanyl- oder Benzyl-Rest substituiert sein kann, wobei der zyklische Teil des Benzyl-Rests jeweils mit 1, 2 oder 3 Substituenten unabhängig voneinander ausgewählt aus der Gruppe bestehend aus F, Cl, Br, -OH, Methyl, Ethyl, iso-Propyl, n-Propyl, $-CF_3$, $-O-CH_3$, $-O-C_2H_5$, Phenyl und -O-Benzyl substituiert sein kann,
oder für einen Rest ausgewählt aus der Gruppe bestehend aus Phenyl, Naphthyl, (1,3)-Benzodioxolyl und (1,4)-Benzodioxanyl steht, wobei der Rest jeweils mit 1, 2 oder 3 Substituenten unabhängig voneinander ausgewählt aus der Gruppe bestehend aus F, Cl, Br, I, Methyl, Ethyl, n-Propyl, iso-Propyl, n-Butyl, sec-Butyl, iso-Butyl, tert-Butyl, -O-Phenyl, -O-Benzyl, $-NH-C(=O)-O-CH_3$, $-NH-C(=O)-O-C_2H_5$ und $-NH-C(=O)-O-C(CH_3)_3$ substituiert sein kann;
$R^2$ für einen Wasserstoff-Rest,
$-(CHR^{16})-R^{19}$,
oder für einen Alkyl-Rest ausgewählt aus der Gruppe bestehend aus Methyl, Ethyl, n-Propyl, iso-Propyl, n-Butyl, sec-Butyl, iso-Butyl und tert-Butyl steht;

oder

$R^1$ und $R^2$ zusammen mit dem sie verbindenden Stickstoffatom als Ringglied einen Rest bilden, der ausgewählt ist aus der Gruppe bestehend aus

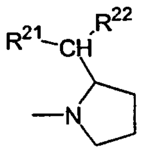 , 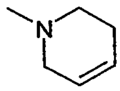 , 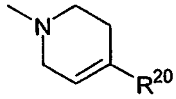 , 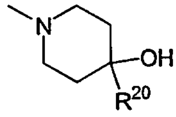 ,

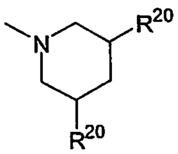 , 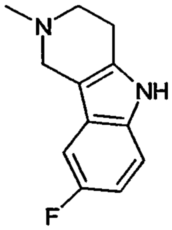 , 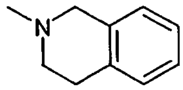 , 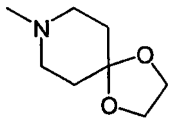 ,

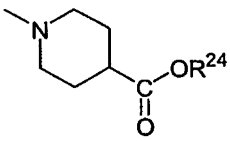 , 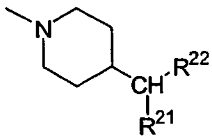 , 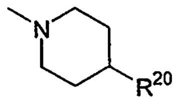 , 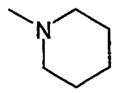 ,

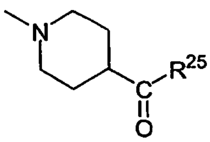 , 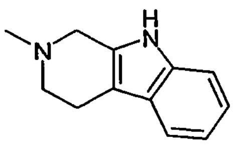 , 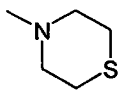 , 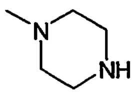 ,

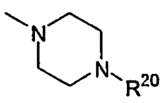 , 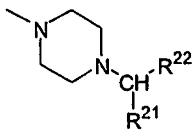 , 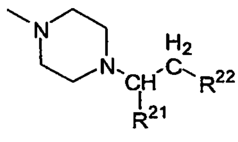 ,

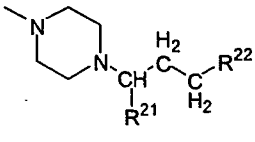 , 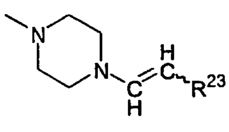 , 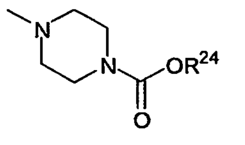 ,

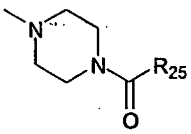, 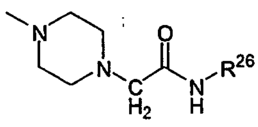 und 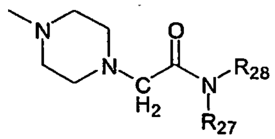,

$R^3$ für einen Alkyl-Rest ausgewählt aus der Gruppe bestehend aus Methyl, Ethyl, n-Propyl, iso-Propyl, n-Butyl, sec-Butyl, iso-Butyl und tert-Butyl;

$R^4$ für einen Rest steht, der ausgewählt ist aus der Gruppe bestehend aus

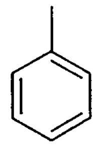, 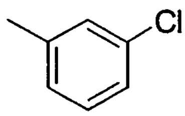, 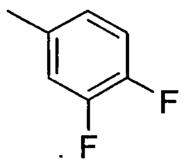, 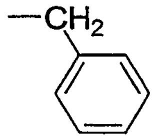

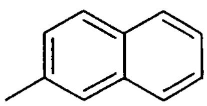 und 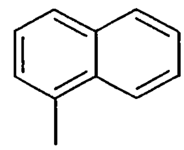,

$R^5$, $R^7$ und $R^{11}$, unabhängig voneinander, jeweils
für einen Alkyl-Rest ausgewählt aus der Gruppe bestehend aus Methyl, Ethyl, n-Propyl, iso-Propyl, n-Butyl, sec-Butyl, iso-Butyl und tert-Butyl;
oder für einen Benzyl- oder Naphthyl-Rest stehen;
$R^6$ für einen Wasserstoff-Rest,
für einen Rest ausgewählt aus der Gruppe bestehend aus Methyl, Ethyl, n-Propyl, iso-Propyl, n-Butyl, sec-Butyl, iso-Butyl, tert-Butyl, n-Pentyl, sec-Pentyl, neo-Pentyl und $-C(H)(CH_3)(O(C(CH_3)_3))$,
oder für einen Indolyl-Rest, der über eine $-(CH_2)$-Gruppe gebunden ist, steht;
$R^{16}$ für einen Wasserstoff-Rest,
für einen Rest ausgewählt aus der Gruppe bestehend aus Methyl, Ethyl, iso-Propyl, n-Propyl, n-Butyl, sec-Butyl, iso-Butyl, tert.-Butyl und $-(CH_2)-O-(CH_3)$,
oder für einen Phenyl-Rest steht;
$R^{17}$ für einen Wasserstoff-Rest,
für einen Alkyl-Rest ausgewählt aus der Gruppe bestehend aus Methyl, Ethyl, iso-Propyl, n-Propyl, n-Butyl, iso-Butyl, sec-Butyl und tert.-Butyl oder für einen Phenyl-Rest steht;
$R^{18}$ für einen Wasserstoff-Rest
oder für einen Phenyl-Rest steht;
$R^{19}$ für einen (hetero)cycloaliphatischen Rest ausgewählt aus der Gruppe bestehend aus Cyclopentyl, Cyclohexyl, Cycloheptyl, Tetrahydrofuranyl, Tetrahydrothiophenyl, Pyrrolidinyl, Piperidinyl, Adamantyl (Tricyclo-[3.3.1.1$^{3,7}$]-decanyl) und (1,4)-Benzodioxanyl, wobei der (hetero)cycloaliphatische Rest jeweils mit 1 oder 2 Substituenten unabhängig voneinander ausgewählt aus der Gruppe bestehend Methyl, Ethyl, iso-Propyl und n-Propyl substituiert sein kann,
oder für einen Rest ausgewählt aus der Gruppe bestehend aus Phenyl, Naphthyl, (1,3)-Benzodioxolyl, (1,4)-Benzodioxanyl, Thiophenyl, Furanyl, Pyridinyl, Imidazolyl, Indolyl und Isoindolyl, wobei der Rest jeweils mit 1, 2, 3, 4 oder 5 Substituenten unabhängig voneinander ausgewählt aus der Gruppe bestehend aus F, Cl, Br, I, Methyl, Ethyl, n-Propyl, iso-Propyl, n-Butyl, sec-Butyl, iso-Butyl, tert-Butyl, $-CF_3$, $-O-CH_3$, $-O-C_2H_5$, $-O-CF_3$, -O-Phenyl, -O-Benzyl, $-NH_2$, $-N(CH_3)_2$, $-N(C_2H_5)_2$, $-N(H)(CH_3)$ und $-N(H)(C_2H_5)$ substituiert sein kann, steht;
$R^{20}$ für einen Methyl- oder Ethyl-Rest,
für einen (hetero)cycloaliphatischen Rest ausgewählt aus der Gruppe bestehend aus Cyclopentyl, Cyclohexyl, Piperidinyl und Cycloheptyl,

oder für einen Phenyl- oder Pyridinyl-Rest, der mit der mit 1, 2 oder 3 Substituenten unabhängig voneinander ausgewählt aus der Gruppe bestehend aus Methyl, Ethyl, n-Propyl, iso-Propyl, n-Butyl, iso-Butyl, sec-Butyl, tert-Butyl, F, Cl, Br, $-CF_3$, -OH, $-O-CH_3$ und $-O-C_2H_5$ substituiert sein kann, steht;
$R^{21}$ für einen Wasserstoff-Rest
oder für einen Phenyl-Rest, der mit 1, 2 oder 3 Substituenten unabhängig voneinander ausgewählt aus der Gruppe bestehend aus Methyl, Ethyl, n-Propyl, iso-Propyl, n-Butyl, iso-Butyl, sec-Butyl, tert-Butyl, F, Cl und Br substituiert sein kann, steht;
$R^{22}$ für einen Pyrrolidinyl- oder Morpholinyl-Rest
oder für einen Rest ausgewählt aus der Gruppe bestehend aus Phenyl, Naphthyl, (1,3)-Benzodioxolyl, Thiophenyl, Benzo[b]furanyl, Benzo[b]thiophenyl und Chinolinyl, wobei der Rest jeweils mit 1, 2 oder 3 Substituenten unabhängig voneinander ausgewählt aus der Gruppe bestehend aus Methyl, Ethyl, n-Propyl, F, Cl, Br, -OH, $-O-CH_3$ und $-O-C_2H_5$ substituiert sein kann, steht;
$R^{23}$ für einen Phenyl-Rest steht;
$R^{24}$ für einen Alkyl-Rest ausgewählt aus der Gruppe bestehend aus Methyl, Ethyl, n-Propyl, iso-Propyl, n-Butyl, iso-Butyl, sec-Butyl und tert-Butyl steht;
$R^{25}$ für einen Rest ausgewählt aus der Gruppe bestehend aus Phenyl, Naphthyl, Furanyl, Pyrazinyl und Pyrimidinyl steht, der über eine $-(CH_2)$-Gruppe gebunden und/oder mit 1, 2 oder 3 Substituenten unabhängig voneinander ausgewählt aus der Gruppe bestehend aus Methyl, Ethyl, n-Propyl und iso-Propyl substituiert sein kann;
$R^{26}$ für einen Alkyl-Rest ausgewählt aus der Gruppe bestehend aus Methyl, Ethyl, n-Propyl und iso-Propyl steht;
$R^{27}$ für einen Alkyl-Rest ausgewählt aus der Gruppe bestehend aus Methyl, Ethyl, iso-Propyl und n-Propyl steht und
$R^{28}$ für einen Phenyl-Rest steht;

jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form entsprechender Salze, oder jeweils in Form entsprechender Solvate.

**16.** Verbindungen gemäß einem oder mehreren der Ansprüche 1 bis 15 ausgewählt aus der Gruppe bestehend aus

[1] 2-[(3-Chlor-benzoyl)-methyl-amino]-5,6,7,8-tetrahydro-imidazo[1,2-a]pyridin-3-carbonsäure (2-thiophen-2-yl-ethyl)-amid,
[2] 2-[Butyl-(3-chlor-benzoyl)-amino]-5,6,7,8-tetrahydro-imidazo[1,2-a]pyridin-3-carbonsäure [2-(2,5-dimethoxy-phenyl)-ethyl]-amid,
[3] N-{3-[4-(2-Ethyl-phenyl)-piperazin-1-carbonyl]-5,6,7,8-tetrahydro-imidazo[1,2-a]pyridin-2-yl}-N-methyl-benzamid,
[4] 2-(Methyl-phenylacetyl-amino)-5,6,7,8-tetrahydro-imidazo[1,2-a]pyridin-3-carbonsäure [3-(2-methyl-piperidin-1-yl)-propyl]-amid,
[5] 2-[(3-Chlor-benzoyl)-methyl-amino]-5,6,7,8-tetrahydro-imidazo[1,2-a]pyridin-3-carbonsäure (2-phenyl-propyl)-amid,
[6] 2-[(3-Chlor-benzoyl)-methyl-amino]-5,6,7,8-tetrahydro-imidazo[1,2-a]pyridin-3-carbonsäure (2-pyrrolidin-1-yl-ethyl)-amid,
[7] 2-[Methyl-(naphthalen-1-carbonyl)-amino]-5,6,7,8-tetrahydro-imidazo[1,2-a]pyridin-3-carbonsäure ethyl-pyridin-4-ylmethyl-amid,
[8] 2-(Benzoyl-methyl-amino)-5,6,7,8-tetrahydro-imidazo[1,2-a]pyridin-3-carbonsäure (2-thiophen-2-yl-ethyl)-amid,
[9] 2-[Butyl-(3-chlor-benzoyl)-amino]-5,6,7,8-tetrahydro-imidazo[1,2-a]pyridin-3-carbonsäure (3-imidazol-1-yl-propyl)-amid,
[10] 3-Chlor-N-{3-[4-(3-chlor-phenyl)-piperazin-1-carbonyl]-5,6,7,8-tetrahydro-imidazo[1,2-a]pyridin-2-yl}-N-methyl-benzamid,
[11] 3-Chlor-N-methyl-N-[3-(4-phenyl-piperazin-1-carbonyl)-5,6,7,8-tetrahydro-imidazo[1,2-a]pyridin-2-yl]-benzamid,
[12] 2-[Butyl-(3,4-difluor-benzoyl)-amino]-5,6,7,8-tetrahydro-imidazo[1,2-a]pyridin-3-carbonsäure indan-1-ylamid,
[13] N-Butyl-3-chlor-N-{3-[4-(2-morpholin-4-yl-ethyl)-piperazin-1-carbonyl]-5,6,7,8-tetrahydro-imidazo[1,2-a] pyridin-2-yl}-benzamid,
[14] 3-Chlor-N-methyl-N-{3-[4-(3-trifluormethyl-phenyl)-piperazin-1-carbonyl]-5,6,7,8-tetrahydro-imidazo[1,2-a]pyridin-2-yl}-benzamid,
[15] 2-[Methyl-(naphthalen-1-carbonyl)-amino]-5,6,7,8-tetrahydro-imidazo[1,2-a]pyridin-3-carbonsäure 3,5-bis-trifluormethyl-benzylamid,
[16] 2-[Butyl-(3,4-difluor-benzoyl)-amino]-5,6,7,8-tetrahydro-imidazo[1,2-a]pyridin-3-carbonsäure (4-methyl-cyclohexyl)-amid,
[17] 2-(Benzoyl-methyl-amino)-5,6,7,8-tetrahydro-imidazo[1,2-a]pyridin-3-carbonsäure 3-methoxy-benzylamid,
[18] 2-[Methyl-(naphthalen-1-carbonyl)-amino]-5,6,7,8-tetrahydro-imidazo[1,2-a]pyridin-3-carbonsäure (1-p-tolyl-ethyl)-amid,
[19] 2-[Butyl-(3-chlor-benzoyl)-amino]-5,6,7,8-tetrahydro-imidazo[1,2-a]pyridin-3-carbonsäure (1,2-dimethyl-propyl)-amid,
[20] 2-(Benzoyl-methyl-amino)-5,6,7,8-tetrahydro-imidazo[1,2-a]pyridin-3-carbonsäure (4-methyl-cyclohexyl)-amid,
[21] 2-(Benzoyl-methyl-amino)-5,6,7,8-tetrahydro-imidazo[1,2-a]pyridin-3-carbonsäure (2-phenyl-propyl)-amid,
[22] 4-[2-(Benzoyl-methyl-amino)-5,6,7,8-tetrahydro-imidazo[1,2-a]pyridin-3-carbonyl]-piperazin-1-carbonsäure ethyl ester,
[23] 3-Chlor-N-methyl-N-(3-{4-[(methyl-phenyl-carbamoyl)-methyl]-piperazin-1-carbonyl}-5,6,7,8-tetrahydro-imidazo[1,2-a]pyridin-2-yl)-benzamid,
[24] 3-Chlor-N-{3-[4-(furan-2-carbonyl)-piperazin-1-carbonyl]-5,6,7,8-tetrahydro-imidazo[1,2-a]pyridin-2-yl}-N-methyl-benzamid,
[25] N-Methyl-N-[3-(4-p-tolyl-piperazin-1-carbonyl)-5,6,7,8-tetrahydro-imidazo[1,2-a]pyridin-2-yl]-benzamid,
[26] N-Butyl-3-chlor-N-{3-[4-(3-phenyl-propyl)-piperazin-1-carbonyl]-5,6,7,8-tetrahydro-imidazo[1,2-a]pyridin-2-yl}-benzamid,
[27] 2-[(3-Chlor-benzoyl)-methyl-amino]-5,6,7,8-tetrahydro-imidazo[1,2-a]pyridin-3-carbonsäure (2,6,6-trimethyl-bicyclo[3.1.1]hept-3-yl)-amid,
[28] 2-[Butyl-(3,4-difluor-benzoyl)-amino]-5,6,7,8-tetrahydro-imidazo[1,2-a]pyridin-3-carbonsäure (3-phenyl-propyl)-amid,
[29] Naphthalen-1-carbonsäure {3-[4-(4-methoxy-phenyl)-piperazin-1-carbonyl]-5,6,7,8-tetrahydro-imidazo [1,2-a]pyridin-2-yl}-methyl-amid,
[30] 2-[Methyl-(naphthalen-1-carbonyl)-amino]-5,6,7,8-tetrahydro-imidazo[1,2-a]pyridin-3-carbonsäure [1-(4-trifluormethyl-benzyl)-pyrrolidin-3-yl]-amid,
[31] 2-[(3-Chlor-benzoyl)-methyl-amino]-5,6,7,8-tetrahydro-imidazo[1,2-a]pyridin-3-carbonsäure (4-ethyl-phenyl)-amid,
[32] 2-[Methyl-(naphthalen-1-carbonyl)-amino]-5,6,7,8-tetrahydro-imidazo[1,2-a]pyridin-3-carbonsäure (3-methoxy-benzyl)-(tetrahydrofuran-2-ylmethyl)-amid,
[33] 2-[Methyl-(naphthalen-1-carbonyl)-amino]-5,6,7,8-tetrahydro-imidazo[1,2-a]pyridin-3-carbonsäure 2,3-dichlor-benzylamid,
[34] 2-[Butyl-(3,4-difluor-benzoyl)-amino]-5,6,7,8-tetrahydro-imidazo[1,2-a]pyridin-3-carbonsäure (1-benzofuran-2-ylmethyl-pyrrolidin-3-yl)-methyl-amid,
[35] 3-Chlor-N-{3-[4-(4-chlor-phenyl)-piperazin-1-carbonyl]-5,6,7,8-tetrahydro-imidazo[1,2-a]pyridin-2-yl}-N-methyl-benzamid,
[36] 2-({2-[(3-Chlor-benzoyl)-methyl-amino]-5,6,7,8-tetrahydro-imidazo[1,2-a]pyridin-3-carbonyl}-amino)-3-(1H-indol-3-yl)-propionsäure methyl ester,
[37] N-Butyl-3-chlor-N-[3-(4-phenylacetyl-piperazin-1-carbonyl)-5,6,7,8-tetrahydro-imidazo[1,2-a]pyridin-2-yl]-benzamid,
[38] 2-({2-[(3-Chlor-benzoyl)-methyl-amino]-5,6,7,8-tetrahydro-imidazo[1,2-a]pyridin-3-carbonyl}-amino)-3-methyl-pentansäure-tert-butyl ester,
[39] 2-[Butyl-(3-chlor-benzoyl)-amino]-5,6,7,8-tetrahydro-imidazo[1,2-a]pyridin-3-carbonsäure (1-naphthalen-1-yl-ethyl)-amid,
[40] 2-[Methyl-(naphthalen-1-carbonyl)-amino]-5,6,7,8-tetrahydro-imidazo[1,2-a]pyridin-3-carbonsäure allyl-methyl-amid,
[41] N-Butyl-N-[3-(3,6-dihydro-2H-pyridin-1-carbonyl)-5,6,7,8-tetrahydro-imidazo[1,2-a]pyridin-2-yl]-3,4-difluor-benzamid,
[42] 2-({2-[(3-Chlor-benzoyl)-methyl-amino]-5,6,7,8-tetrahydro-imidazo[1,2-a]pyridin-3-carbonyl}-amino)-4-methyl-pentansäure-benzyl ester,
[43] 2-[Butyl-(3-chlor-benzoyl)-amino]-5,6,7,8-tetrahydro-imidazo[1,2-a]pyridin-3-carbonsäure 2-ethoxy-benzylamid,

[44] N-Butyl-3-chlor-N-{3-[4-(5-methyl-pyrazin-2-carbonyl)-piperazin-1-carbonyl]-5,6,7,8-tetrahydro-imidazo [1,2-a]pyridin-2-yl}-benzamid,
[45] N-[3-(4-Benzo[1,3]dioxol-5-ylmethyl-piperazin-1-carbonyl)-5,6,7,8-tetrahydro-imidazo[1,2-a]pyridin-2-yl]-3-chlor-N-methyl-benzamid,
[46] 2-[Methyl-(naphthalen-1-carbonyl)-amino]-5,6,7,8-tetrahydro-imidazo[1,2-a]pyridin-3-carbonsäure (2,2-diphenyl-ethyl)-amid,
[47] 2-[Butyl-(3-chlor-benzoyl)-amino]-5,6,7,8-tetrahydro-imidazo[1,2-a]pyridin-3-carbonsäure 2,4-difluor-benzylamid,
[48] N-Butyl-3-chlor-N-{3-[4-(2-fluor-phenyl)-piperazin-1-carbonyl]-5,6,7,8-tetrahydro-imidazo[1,2-a]pyridin-2-yl}-benzamid,
[49] 2-[Methyl-(naphthalen-1-carbonyl)-amino]-5,6,7,8-tetrahydro-imidazo[1,2-a]pyridin-3-carbonsäure (2-p-tolyl-ethyl)-amid,
[50] 2-[Methyl-(naphthalen-1-carbonyl)-amino]-5,6,7,8-tetrahydro-imidazo[1,2-a]pyridin-3-carbonsäure (pyridin-2-ylmethyl)-amid,
[51] 2-[Butyl-(3-chlor-benzoyl)-amino]-5,6,7,8-tetrahydro-imidazo[1,2-a]pyridin-3-carbonsäure 2-trifluormethyl-benzylamid,
[52] 2-[Butyl-(3-chlor-benzoyl)-amino]-5,6,7,8-tetrahydro-imidazo[1,2-a]pyridin-3-carbonsäure indan-1-ylamid,
[53] 3-Chlor-N-methyl-N-[3-(4-chinolin-2-ylmethyl-piperazin-1-carbonyl)-5,6,7,8-tetrahydro-imidazo[1,2-a]pyridin-2-yl]-benzamid,
[54] N-Butyl-3,4-difluor-N-[3-(4-methyl-piperazin-1-carbonyl)-5,6,7,8-tetrahydro-imidazo[1,2-a]pyridin-2-yl]-benzamid,
[55] 2-[(3-Chlor-benzoyl)-methyl-amino]-5,6,7,8-tetrahydro-imidazo[1,2-a]pyridin-3-carbonsäure 2,4-difluor-benzylamid,
[56] N-[3-(4-Benzhydryl-piperazin-1-carbonyl)-5,6,7,8-tetrahydro-imidazo[1,2-a]pyridin-2-yl]-N-butyl-3,4-difluor-benzamid,
[57] 4-Methyl-2-({2-[methyl-(naphthalen-1-carbonyl)-amino]-5,6,7,8-tetrahydro-imidazo[1,2-a]pyridin-3-carbonyl}-amino)-pentansäure-benzyl ester,
[58] 2-[Butyl-(3-chlor-benzoyl)-amino]-5,6,7,8-tetrahydro-imidazo[1,2-a]pyridin-3-carbonsäure (2-pyrrolidin-1-yl-ethyl)-amid,
[59] 2-[(3-Chlor-benzoyl)-methyl-amino]-5,6,7,8-tetrahydro-imidazo[1,2-a]pyridin-3-carbonsäure [1-(2-benzyloxy-benzyl)-pyrrolidin-3-yl]-amid,
[60] 2-[Butyl-(3,4-difluor-benzoyl)-amino]-5,6,7,8-tetrahydro-imidazo[1,2-a]pyridin-3-carbonsäure (pyridin-3-ylmethyl)-amid,
[61] 3-Chlor-N-{3-[4-(4-fluor-phenyl)-3,6-dihydro-2H-pyridin-1-carbonyl]-5,6,7,8-tetrahydro-imidazo[1,2-a]pyridin-2-yl}-N-methyl-benzamid,
[62] 2-[(3-Chlor-benzoyl)-methyl-amino]-5,6,7,8-tetrahydro-imidazo[1,2-a]pyridin-3-carbonsäure cyclohexylamid,
[63] N-[3-(4-Cycloheptyl-piperazin-1-carbonyl)-5,6,7,8-tetrahydro-imidazo[1,2-a]pyridin-2-yl]-N-methyl-benzamid,
[64] Naphthalen-1-carbonsäure methyl-[3-(4-thiophen-3-ylmethyl-piperazin-1-carbonyl)-5,6,7,8-tetrahydro-imidazo[1,2-a]pyridin-2-yl]-amid,
[65] 2-[Butyl-(3-chlor-benzoyl)-amino]-5,6,7,8-tetrahydro-imidazo[1,2-a]pyridin-3-carbonsäure 2,4-dimethoxy-benzylamid,
[66] 1-[2-(Benzoyl-methyl-amino)-5,6,7,8-tetrahydro-imidazo[1,2-a]pyridin-3-carbonyl]-piperidin-4-carbonsäure ethyl ester,
[67] 2-[(3-Chlor-benzoyl)-methyl-amino]-5,6,7,8-tetrahydro-imidazo[1,2-a]pyridin-3-carbonsäure (2-benzyloxy-cyclohexyl)-amid,
[68] 2-[Methyl-(naphthalen-1-carbonyl)-amino]-5,6,7,8-tetrahydro-imidazo[1,2-a]pyridin-3-carbonsäure [2-(4-phenoxy-phenyl)-ethyl]-amid,
[69] 2-[Butyl-(3-chlor-benzoyl)-amino]-5,6,7,8-tetrahydro-imidazo[1,2-a]pyridin-3-carbonsäure [2-(7-methyl-1 H-indol-3-yl)-ethyl]-amid,
[70] Naphthalen-1-carbonsäure methyl-[3-(4-phenyl-piperazin-1-carbonyl)-5,6,7,8-tetrahydro-imidazo[1,2-a] pyridin-2-yl]-amid,
[71] 2-[(3-Chlor-benzoyl)-methyl-amino]-5,6,7,8-tetrahydro-imidazo[1,2-a]pyridin-3-carbonsäure [2-(3-trifluormethyl-phenyl)-ethyl]-amid,
[72] N-Butyl-3-chlor-N-[3-(4-pyridin-2-yl-piperazin-1-carbonyl)-5,6,7,8-tetrahydro-imidazo[1,2-a]pyridin-2-yl]-benzamid,
[73] 2-[Methyl-(naphthalen-1-carbonyl)-amino]-5,6,7,8-tetrahydro-imidazo[1,2-a]pyridin-3-carbonsäure 3-methoxy-benzylamid,
[74] 2-[Butyl-(3,4-difluor-benzoyl)-amino]-5,6,7,8-tetrahydro-imidazo[1,2-a]pyridin-3-carbonsäure 2,4-difluor-benzylamid,
[75] 3-Chlor-N-methyl-N-[3-(4-pyridin-2-yl-piperazin-1-carbonyl)-5,6,7,8-tetrahydro-imidazo[1,2-a]pyridin-2-yl]-benzamid,
[76] 2-[Methyl-(naphthalen-1-carbonyl)-amino]-5,6,7,8-tetrahydro-imidazo[1,2-a]pyridin-3-carbonsäure (1-biphenyl-4-ylmethyl-pyrrolidin-3-yl)-methyl-amid,
[77] 2-[Butyl-(3-chlor-benzoyl)-amino]-5,6,7,8-tetrahydro-imidazo[1,2-a]pyridin-3-carbonsäure (2,3-dihydro-benzo[1,4]dioxin-2-ylmethyl)-amid,
[78] 2-[Butyl-(3-chlor-benzoyl)-amino]-5,6,7,8-tetrahydro-imidazo[1,2-a]pyridin-3-carbonsäure [2-(1H-indol-3-yl)-ethyl]-methyl-amid,
[79] N-Butyl-3-chlor-N-{3-[4-(3-trifluormethyl-phenyl)-piperazin-1-carbonyl]-5,6,7,8-tetrahydro-imidazo[1,2-a]pyridin-2-yl}-benzamid,
[80] 2-(Benzoyl-methyl-amino)-5,6,7,8-tetrahydro-imidazo[1,2-a]pyridin-3-carbonsäure [2-(1H-indol-3-yl)-ethyl]-methyl-amid,
[81] 3-Chlor-N-{3-[4-hydroxy-4-(3-trifluormethyl-phenyl)-piperidin-1-carbonyl]-5,6,7,8-tetrahydro-imidazo[1,2-a]pyridin-2-yl}-N-methyl-benzamid,
[82] 2-[Butyl-(3,4-difluor-benzoyl)-amino]-5,6,7,8-tetrahydro-imidazo[1,2-a]pyridin-3-carbonsäure (1-methoxymethyl-2-phenyl-ethyl)-amid,
[83] 2-[(3-Chlor-benzoyl)-methyl-amino]-5,6,7,8-tetrahydro-imidazo[1,2-a]pyridin-3-carbonsäure [1-(2,6-dichlor-benzyl)-pyrrolidin-3-yl]-amid,
[84] 2-[(3-Chlor-benzoyl)-methyl-amino]-5,6,7,8-tetrahydro-imidazo[1,2-a]pyridin-3-carbonsäure [1-(2-brom-4,5-dimethoxy-benzyl)-pyrrolidin-3-yl]-amid,
[85] 2-(Benzoyl-methyl-amino)-5,6,7,8-tetrahydro-imidazo[1,2-a]pyridin-3-carbonsäure benzo[1,3]dioxol-5-ylamid,
[86] 2-[Butyl-(3-chlor-benzoyl)-amino]-5,6,7,8-tetrahydro-imidazo[1,2-a]pyridin-3-carbonsäure (1-methoxymethyl-2-phenyl-ethyl)-amid,
[87] 2-[Butyl-(3-chlor-benzoyl)-amino]-5,6,7,8-tetrahydro-imidazo[1,2-a]pyridin-3-carbonsäure [1-(2,6-dichlor-benzyl)-pyrrolidin-3-yl]-methyl-amid,
[88] 2-[Butyl-(3,4-difluor-benzoyl)-amino]-5,6,7,8-tetrahydro-imidazo[1,2-a]pyridin-3-carbonsäure 2-trifluormethoxy-benzylamid,
[89] N-Butyl-3,4-difluor-N-{3-[4-(isopropylcarbamoyl-methyl)-piperazin-1-carbonyl]-5,6,7,8-tetrahydro-imidazo[1,2-a]pyridin-2-yl}-benzamid,
[90] 2-[(3-Chlor-benzoyl)-methyl-amino]-5,6,7,8-tetrahydro-imidazo[1,2-a]pyridin-3-carbonsäure (1-methyl-3-phenyl-propyl)-amid,
[91] 2-({2-[Butyl-(3-chlor-benzoyl)-amino]-5,6,7,8-tetrahydro-imidazo[1,2-a]pyridin-3-carbonyl}-amino)-3-methyl-buttersäurebenzylester,
[92] 2-[(3-Chlor-benzoyl)-methyl-amino]-5,6,7,8-tetrahydro-imidazo[1,2-a]pyridin-3-carbonsäure (thiophen-2-ylmethyl)-amid,
[93] 2-[Butyl-(3,4-difluor-benzoyl)-amino]-5,6,7,8-tetrahydro-imidazo[1,2-a]pyridin-3-carbonsäure (2-methyl-cyclohexyl)-amid,
[94] 2-(Benzoyl-methyl-amino)-5,6,7,8-tetrahydro-imidazo[1,2-a]pyridin-3-carbonsäure benzyl-methyl-amid,
[95] 2-[Butyl-(3-chlor-benzoyl)-amino]-5,6,7,8-tetrahydro-imidazo[1,2-a]pyridin-3-carbonsäure [2-(2,6-dichlor-benzylsulfanyl)-ethyl]-amid,
[96] 2-[Butyl-(3,4-difluor-benzoyl)-amino]-5,6,7,8-tetrahydro-imidazo[1,2-a]pyridin-3-carbonsäure (2,3-dihydro-benzo[1,4]dioxin-2-ylmethyl)-amid,
[97] 2-[(3-Chlor-benzoyl)-methyl-amino]-5,6,7,8-tetrahydro-imidazo[1,2-a]pyridin-3-carbonsäure 4-trifluormethyl-benzylamid,
[98] N-[3-(4-Benzyl-piperidin-1-carbonyl)-5,6,7,8-tetrahydro-imidazo[1,2-a]pyridin-2-yl]-N-methyl-benzamid,
[99] 2-[Methyl-(naphthalen-1-carbonyl)-amino]-5,6,7,8-tetrahydro-imidazo[1,2-a]pyridin-3-carbonsäure 2-trifluormethoxy-benzylamid,
[100] 3-Chlor-N-{3-[4-(2,5-dimethyl-phenyl)-piperazin-1-carbonyl]-5,6,7,8-tetrahydro-imidazo[1,2-a]pyridin-2-yl}-N-methyl-benzamid,
[101] 2-(Benzoyl-methyl-amino)-5,6,7,8-tetrahydro-imidazo[1,2-a]pyridin-3-carbonsäure [1-(2,6-dichlor-benzyl)-pyrrolidin-3-yl]-amid,
[102] 2-[Butyl-(3,4-difluor-benzoyl)-amino]-5,6,7,8-tetrahydro-imidazo[1,2-a]pyridin-3-carbonsäure 2-chlor-6-methyl-benzylamid,
[103] N-Butyl-3-chlor-N-[3-(3,5-dimethyl-piperidin-1-carbonyl)-5,6,7,8-tetrahydro-imidazo[1,2-a]pyridin-2-yl]-benzamid,
[104] 2-({2-[(3-Chlor-benzoyl)-methyl-amino]-5,6,7,8-tetrahydro-imidazo[1,2-a]pyridin-3-carbonyl}-amino)-3,3-dimethyl-buttersäure tert-butylester,
[105] 2-[Methyl-(naphthalen-1-carbonyl)-amino]-5,6,7,8-tetrahydro-imidazo[1,2-a]pyridin-3-carbonsäure (1-cyclohexyl-ethyl)-amid,
[106] 3-Chlor-N-methyl-N-[3-(4-phenethyl-piperazin-1-carbonyl)-5,6,7,8-tetrahydro-imidazo[1,2-a]pyridin-2-yl]-benzamid,
[107] 2-[Butyl-(3-chlor-benzoyl)-amino]-5,6,7,8-tetrahydro-imidazo[1,2-a]pyridin-3-carbonsäure (2-phenoxy-ethyl)-amid,
[108] Naphthalen-1-carbonsäure [3-(4-benzofuran-2-ylmethyl-piperazin-1-carbonyl)-5,6,7,8-tetrahydro-imidazo[1,2-a]pyridin-2-yl]-methyl-amid,
[109] N-[3-([1,4']Bipiperidinyl-1'-carbonyl)-5,6,7,8-tetrahydro-imidazo[1,2-a]pyridin-2-yl]-N-butyl-3-chlor-benzamid,
[110] N-Butyl-3-chlor-N-[3-(8-fluor-1,3,4,5-tetrahydro-pyrido[4,3-b]indol-2-carbonyl)-5,6,7,8-tetrahydro-imidazo[1,2-a]pyridin-2-yl]-benzamid,
[111] 2-[Methyl-(naphthalen-1-carbonyl)-amino]-5,6,7,8-tetrahydro-imidazo[1,2-a]pyridin-3-carbonsäure 4-dimethylamino-benzylamid,
[112] 2-[Methyl-(naphthalen-1-carbonyl)-amino]-5,6,7,8-tetrahydro-imidazo[1,2-a]pyridin-3-carbonsäure (1-methyl-3-phenyl-propyl)-amid,
[113] 2-[Butyl-(3-chlor-benzoyl)-amino]-5,6,7,8-tetrahydro-imidazo[1,2-a]pyridin-3-carbonsäure 3-methyl-benzylamid,
[114] 2-[(3-Chlor-benzoyl)-methyl-amino]-5,6,7,8-tetrahydro-imidazo[1,2-a]pyridin-3-carbonsäure (2,3-dihydro-benzo[1,4]dioxin-6-yl)-amid,
[115] Naphthalen-1-carbonsäure methyl-[3-(4-chinolin-2-ylmethyl-piperazin-1-carbonyl)-5,6,7,8-tetrahydro-imidazo[1,2-a]pyridin-2-yl]-amid,
[116] 2-[Butyl-(3,4-difluor-benzoyl)-amino]-5,6,7,8-tetrahydro-imidazo[1,2-a]pyridin-3-carbonsäure (2-cyano-ethyl)-methyl-amid,
[117] 2-(Methyl-phenylacetyl-amino)-5,6,7,8-tetrahydro-imidazo[1,2-a]pyridin-3-carbonsäure (2-p-tolyl-ethyl)-amid,
[118] 2-[Butyl-(3,4-difluor-benzoyl)-amino]-5,6,7,8-tetrahydro-imidazo[1,2-a]pyridin-3-carbonsäure [1-(4-fluor-phenyl)-ethyl]-amid,
[119] N-Butyl-N-{3-[4-(5-chlor-2-methyl-phenyl)-piperazin-1-carbonyl]-5,6,7,8-tetrahydro-imidazo[1,2-a]pyridin-2-yl}-3,4-difluor-benzamid,
[120] 2-[Butyl-(3-chlor-benzoyl)-amino]-5,6,7,8-tetrahydro-imidazo[1,2-a]pyridin-3-carbonsäure cyclohexylamid,
[121] 2-[(3-Chlor-benzoyl)-methyl-amino]-5,6,7,8-tetrahydro-imidazo[1,2-a]pyridin-3-carbonsäure (benzo[1,3]dioxol-5-ylmethyl)-amid,
[122] 2-[Butyl-(3,4-difluor-benzoyl)-amino]-5,6,7,8-tetrahydro-imidazo[1,2-a]pyridin-3-carbonsäure 3-methoxy-benzylamid,
[123] 3-tert-Butoxy-2-({2-[(3-chlor-benzoyl)-methyl-amino]-5,6,7,8-tetrahydro-imidazo[1,2-a]pyridin-3-carbonyl}-amino)-buttersäure methyl ester,
[124] 2-[Butyl-(3-chlor-benzoyl)-amino]-5,6,7,8-tetrahydro-imidazo[1,2-a]pyridin-3-carbonsäure (2-benzyloxy-cyclohexyl)-amid,
[125] N-{3-[4-(4-Fluor-phenyl)-piperazin-1-carbonyl]-5,6,7,8-tetrahydro-imidazo[1,2-a]pyridin-2-yl}-N-methyl-benzamid,
[126] 2-(Benzoyl-methyl-amino)-5,6,7,8-tetrahydro-imidazo[1,2-a]pyridin-3-carbonsäure (naphthalen-2-ylcarbamoylmethyl)-amid,
[127] 2-[Butyl-(3-chlor-benzoyl)-amino]-5,6,7,8-tetrahydro-imidazo[1,2-a]pyridin-3-carbonsäure 4-trifluormethoxy-benzylamid,
[128] 2-[(3-Chlor-benzoyl)-methyl-amino]-5,6,7,8-tetrahydro-imidazo[1,2-a]pyridin-3-carbonsäure (3,3-diphenyl-propyl)-amid,
[129] 2-[(3-Chlor-benzoyl)-methyl-amino]-5,6,7,8-tetrahydro-imidazo[1,2-a]pyridin-3-carbonsäure (4-benzyloxy-phenyl)-amid,
[130] N-{3-[4-(5-Brom-2-ethoxy-benzyl)-piperazin-1-carbonyl]-5,6,7,8-tetrahydro-imidazo[1,2-a]pyridin-2-yl}-N-methyl-2-phenyl-acetamid,
[131] N-[3-(3,4-Dihydro-1H-isochinolin-2-carbonyl)-5,6,7,8-tetrahydro-imidazo[1,2-a]pyridin-2-yl]-N-methyl-benzamid,
[132] 2-[Butyl-(3-chlor-benzoyl)-amino]-5,6,7,8-tetrahydro-imidazo[1,2-a]pyridin-3-carbonsäure (1-phenyl-propyl)-amid,
[133] 2-[Butyl-(3-chlor-benzoyl)-amino]-5,6,7,8-tetrahydro-imidazo[1,2-a]pyridin-3-carbonsäure 2,3-dimethylbenzylamid,
[134] 2-[Butyl-(3-chlor-benzoyl)-amino]-5,6,7,8-tetrahydro-imidazo[1,2-a]pyridin-3-carbonsäure [2-(4-phenoxyphenyl)-ethyl]-amid,
[135] N-{3-[4-(4-Ethoxy-phenyl)-piperazin-1-carbonyl]-5,6,7,8-tetrahydro-imidazo[1,2-a]pyridin-2-yl}-N-methyl-2-phenyl-acetamid,
[136] Naphthalen-1-carbonsäure {3-[4-(2-ethyl-phenyl)-piperazin-1-carbonyl]-5,6,7,8-tetrahydro-imidazo[1,2-a]pyridin-2-yl}-methyl-amid,
[137] N-[3-(4-Benzo[1,3]dioxol-5-ylmethyl-piperazin-1-carbonyl)-5,6,7,8-tetrahydro-imidazo[1,2-a]pyridin-2-yl]-N-butyl-3,4-difluor-benzamid,
[138] N-Butyl-N-{3-[4-(2,5-dimethyl-phenyl)-piperazin-1-carbonyl]-5,6,7,8-tetrahydro-imidazo[1,2-a]pyridin-2-yl}-3,4-difluor-benzamid,
[139] 2-[Methyl-(naphthalen-1-carbonyl)-amino]-5,6,7,8-tetrahydro-imidazo[1,2-a]pyridin-3-carbonsäure [(4-chlor-phenyl)-phenyl-methyl]-amid,
[140] N-Butyl-3-chlor-N-{3-[4-(4-chlor-benzyl)-piperazin-1-carbonyl]-5,6,7,8-tetrahydro-imidazo[1,2-a]pyridin-2-yl}-benzamid,
[141] 2-[Butyl-(3-chlor-benzoyl)-amino]-5,6,7,8-tetrahydro-imidazo[1,2-a]pyridin-3-carbonsäure (1-naphthalen-2-yl-ethyl)-amid,
[142] 2-[(ethyl-phenylacetyl-amino)-5,6,7,8-tetrahydro-imidazo[1,2-a]pyridin-3-carbonsäure 2-fluor-benzylamid,
[143] N-[3-(1,4-Dioxa-8-aza-spiro[4.5]decane-8-carbonyl)-5,6,7,8-tetrahydro-imidazo[1,2-a]pyridin-2-yl]-N-methyl-2-phenyl-acetamid,
[144] Naphthalen-1-carbonsäure methyl-[3-(1,3,4,9-tetrahydro-b-carbolin-2-carbonyl)-5,6,7,8-tetrahydro-imidazo[1,2-a]pyridin-2-yl]-amid,
[145] 2-[Butyl-(3-chlor-benzoyl)-amino]-5,6,7,8-tetrahydro-imidazo[1,2-a]pyridin-3-carbonsäure (1-methyl-3-phenyl-propyl)-amid,
[146] 3-Chlor-N-methyl-N-{3-[4-(2,4,6-trimethoxy-benzyl)-piperazin-1-carbonyl]-5,6,7,8-tetrahydro-imidazo [1,2-a]pyridin-2-yl}-benzamid,
[147] 2-[Butyl-(3-chlor-benzoyl)-amino]-5,6,7,8-tetrahydro-imidazo[1,2-a]pyridin-3-carbonsäure benzhydrylamid,
[148] Naphthalen-1-carbonsäure {3-[4-(2-chlor-phenyl)-piperazin-1-carbonyl]-5,6,7,8-tetrahydro-imidazo[1,2-a]pyridin-2-yl}-methyl-amid,
[149] 2-[Butyl-(3-chlor-benzoyl)-amino]-5,6,7,8-tetrahydro-imidazo[1,2-a]pyridin-3-carbonsäure (thiophen-2-ylmethyl)-amid,
[150] N-Butyl-N-{3-[4-(4-chlor-benzyl)-piperazin-1-carbonyl]-5,6,7,8-tetrahydro-imidazo[1,2-a]pyridin-2-yl}-3,4-difluor-benzamid,
[151] 2-(Benzoyl-methyl-amino)-5,6,7,8-tetrahydro-imidazo[1,2-a]pyridin-3-carbonsäure (3-phenyl-propyl)-amid,
[152] 2-[Butyl-(3-chlor-benzoyl)-amino]-5,6,7,8-tetrahydro-imidazo[1,2-a]pyridin-3-carbonsäure (4-tert-butylphenyl)-amid,
[153] 2-[Butyl-(3-chlor-benzoyl)-amino]-5,6,7,8-tetrahydro-imidazo[1,2-a]pyridin-3-carbonsäure (4-ethyl-phenyl)-amid,
[154] 2-[Methyl-(naphthalen-1-carbonyl)-amino]-5,6,7,8-tetrahydro-imidazo[1,2-a]pyridin-3-carbonsäure cyclohexylamid,
[155] 2-[Methyl-(naphthalen-1-carbonyl)-amino]-5,6,7,8-tetrahydro-imidazo[1,2-a]pyridin-3-carbonsäure [3-(methyl-phenyl-amino)-propyl]-amid,
[156] 2-(Methyl-phenylacetyl-amino)-5,6,7,8-tetrahydro-imidazo[1,2-a]pyridin-3-carbonsäure [2-(3-trifluormethyl-phenyl)-ethyl]-amid,
[157] 2-(Benzoyl-methyl-amino)-5,6,7,8-tetrahydro-imidazo[1,2-a]pyridin-3-carbonsäure 4-chlor-benzylamid,
[158] 2-[Butyl-(3-chlor-benzoyl)-amino]-5,6,7,8-tetrahydro-imidazo[1,2-a]pyridin-3-carbonsäure 3-fluor-benzylamid,
[159] 2-(Methyl-phenylacetyl-amino)-5,6,7,8-tetrahydro-imidazo[1,2-a]pyridin-3-carbonsäure p-tolylamid,
[160] 2-(Benzoyl-methyl-amino)-5,6,7,8-tetrahydro-imidazo[1,2-a]pyridin-3-carbonsäure (2-phenoxyethyl)-amid,
[161] 2-[Butyl-(3,4-difluor-benzoyl)-amino]-5,6,7,8-tetrahydro-imidazo[1,2-a]pyridin-3-carbonsäure (2-benzyloxy-cyclohexyl)-amid,
[162] Naphthalen-1-carbonsäure {3-[4-(4-fluor-phenyl)-piperazin-1-carbonyl]-5,6,7,8-tetrahydro-imidazo[1,2-a]pyridin-2-yl}-methyl-amid,
[163] 2-[Methyl-(naphthalen-1-carbonyl)-amino]-5,6,7,8-tetrahydro-imidazo[1,2-a]pyridin-3-carbonsäure [2-(4-chlor-phenyl)-propyl]-amid,
[164] N-Butyl-3,4-difluor-N-[3-(thiomorpholin-4-carbonyl)-5,6,7,8-tetrahydro-imidazo[1,2-a]pyridin-2-yl]-benzamid,
[165] 2-[(3-Chlor-benzoyl)-methyl-amino]-5,6,7,8-tetrahydro-imidazo[1,2-a]pyridin-3-carboxylsäure (1-adamantan-1-yl-ethyl)-amid,
[166] 2-(Benzoyl-methyl-amino)-5,6,7,8-tetrahydro-imidazo[1,2-a]pyridin-3-carbonsäure [(4-chlor-phenyl)-phenyl-methyl]-amid,
[167] N-Methyl-N-{3-[4-(4-trifluormethyl-phenyl)-piperazin-1-carbonyl]-5,6,7,8-tetrahydro-imidazo[1,2-a]pyridin-2-yl}-benzamid,
[168] 2-(Methyl-phenylacetyl-amino)-5,6,7,8-tetrahydro-imidazo[1,2-a]pyridin-3-carbonsäure (1-biphenyl-4-ylmethyl-pyrrolidin-3-yl)-methyl-amid,
[169] N-[3-(4-Benzoyl-piperidin-1-carbonyl)-5,6,7,8-tetrahydro-imidazo[1,2-a]pyridin-2-yl]-3-chlor-N-methylbenzamid,
[170] 2-[Methyl-(naphthalen-1-carbonyl)-amino]-5,6,7,8-tetrahydro-imidazo[1,2-a]pyridin-3-carbonsäure (4-tert-butyl-phenyl)-amid,
[171] 2-[Butyl-(3,4-difluor-benzoyl)-amino]-5,6,7,8-tetrahydro-imidazo[1,2-a]pyridin-3-carbonsäure (3,3-dimethyl-butyl)-amid,
[172] 2-[Butyl-(3-chlor-benzoyl)-amino]-5,6,7,8-tetrahydro-imidazo[1,2-a]pyridin-3-carbonsäure [1-(2-brom-4,5-dimethoxy-benzyl)-pyrrolidin-3-yl]-amid,
[173] Naphthalen-1-carbonsäure methyl-{3-[4-(3-phenyl-allyl)-piperazin-1-carbonyl]-5,6,7,8-tetrahydro-imidazo[1,2-a]pyridin-2-yl}-amid,
[174] Naphthalen-1-carbonsäure methyl-[3-(4-phenethyl-piperazin-1-carbonyl)-5,6,7,8-tetrahydro-imidazo[1,2-a]pyridin-2-yl]-amid,
[175] N-Butyl-3-chlor-N-{3-[4-hydroxy-4-(3-trifluormethyl-phenyl)-piperidin-1-carbonyl]-5,6,7,8-tetrahydro-imidazo[1,2-a]pyridin-2-yl}-benzamid,
[176] N-Methyl-2-phenyl-N-{3-[4-(3-phenyl-propyl)-piperazin-1-carbonyl]-5,6,7,8-tetrahydro-imidazo[1,2-a]pyridin-2-yl}-acetamid,
[177] 2-(Benzoyl-methyl-amino)-5,6,7,8-tetrahydro-imidazo[1,2-a]pyridin-3-carbonsäure 3-fluor-benzylamid,
[178] N-Butyl-N-{3-[4-(2-chlor-phenyl)-piperazin-1-carbonyl]-5,6,7,8-tetrahydro-imidazo[1,2-a]pyridin-2-yl}-3,4-difluor-benzamid,
[179] 2-[Methyl-(naphthalen-1-carbonyl)-amino]-5,6,7,8-tetrahydro-imidazo[1,2-a]pyridin-3-carbonsäure benzo [1,3]dioxol-5-ylamid,
[180] 2-[Butyl-(3,4-difluor-benzoyl)-amino]-5,6,7,8-tetrahydro-imidazo[1,2-a]pyridin-3-carbonsäure (3,3-diphenyl-propyl)-amid,
[181] 2-[Butyl-(3-chlor-benzoyl)-amino]-5,6,7,8-tetrahydro-imidazo[1,2-a]pyridin-3-carbonsäure (2,3-dihydrobenzo[1,4]dioxin-6-yl)-amid,
[182] 2-(Benzoyl-methyl-amino)-5,6,7,8-tetrahydro-imidazo[1,2-a]pyridin-3-carbonsäure [2-(3,4-dimethoxyphenyl)-ethyl]-amid,
[183] Naphthalen-1-carbonsäure [3-(4-benzoyl-piperidin-1-carbonyl)-5,6,7,8-tetrahydro-imidazo[1,2-a]pyridin-2-yl]-methyl-amid,
[184] 4-Methyl-2-({2-[methyl-(naphthalen-1-carbonyl)-amino]-5,6,7,8-tetrahydro-imidazo[1,2-a]pyridin-3-carbonyl}-amino)-valeriansäure tert-butylester,
[185] 2-[Methyl-(naphthalen-1-carbonyl)-amino]-5,6,7,8-tetrahydro-imidazo[1,2-a]pyridin-3-carbonsäure (4-phenoxy-phenyl)-amid,
[186] N-Methyl-2-phenyl-N-[3-(4-phenyl-piperazin-1-carbonyl)-5,6,7,8-tetrahydro-imidazo[1,2-a]pyridin-2-yl]-acetamid,
[187] N-Butyl-3,4-difluor-N-{3-[4-(5-trifluormethyl-pyridin-2-yl)-piperazin-1-carbonyl]-5,6,7,8-tetrahydro-imidazo[1,2-a]pyridin-2-yl}-benzamid,
[188] 2-(Benzoyl-methyl-amino)-5,6,7,8-tetrahydro-imidazo[1,2-a]pyridin-3-carbonsäure 2,4-dichlor-6-methylbenzylamid,
[189] [4-({2-[Butyl-(3-chlor-benzoyl)-amino]-5,6,7,8-tetrahydro-imidazo[1,2-a]pyridin-3-carbonyl}-amino)-phenyl]-carbaminsäure tert-butyl ester,
[190] N-Methyl-2-phenyl-N-[3-(2-pyrrolidin-1-ylmethyl-pyrrolidin-1-carbonyl)-5,6,7,8-tetrahydro-imidazo[1,2-a]pyridin-2-yl]-acetamid und
[191] 4-[2-(Methyl-phenylacetyl-amino)-5,6,7,8-tetrahydro-imidazo[1,2-a]pyridin-3-carbonyl]-piperazin-1-carbonsäure tert-butyl ester,

jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form entsprechender Salze, oder jeweils in Form entsprechender Solvate.

**17.** Verbindungen gemäß einem oder mehreren der Ansprüche 1 bis 16, **dadurch gekennzeichnet, dass** die Verbindungen im FLIPR-Assay in einer Konzentration von 10 μM eine Hemmung des $Ca^{2+}$-Ionen-Einstroms in Dorsalwurzelganglien von Ratten von wenigstens 30 %, bevorzugt von wenigstens 50 %, besonders bevorzugt von wenigstens 70 %, ganz besonders bevorzugt von wenigstens 80 %, noch weiter bevorzugt von wenigstens 90 %, im Vergleich zur maximal erreichbaren Hemmung des $Ca^{2+}$-Ionen-Einstroms mit Capsaicin in einer Konzentration von 10 μM aufweisen.

**18.** Verfahren zur Herstellung substituierter 5,6,7,8-Tetrahydro-imidazo[1,2-a]pyridin-2-ylamin-Verbindungen der allgemeinen Formel I gemäß einem oder mehreren der Ansprüche 1 bis 17, **dadurch gekennzeichnet, dass** wenigstens eine Verbindung der allgemeinen Formel II,

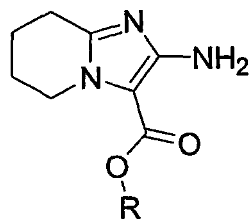

II

worin R für einen linearen oder verzweigten $C_{1-6}$-Alkyl-Rest steht, in einem Reaktionsmedium in Gegenwart wenigstens eines Reduktionsmittels, ggf. in Gegenwart einer organischen Säure, vorzugsweise in Gegenwart von Essigsäure, mit wenigstens einer Verbindung der allgemeinen Formel $R^3$-C(=O)-H, worin $R^3$ die Bedeutung gemäß einem oder mehreren der Ansprüche 1 bis 16 hat, zu einer Verbindung der allgemeinen Formel III,

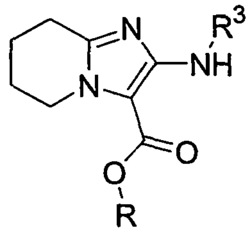

III

worin R und $R^3$ die vorstehend genannte Bedeutung haben, umgesetzt wird und diese ggf. gereinigt und/oder isoliert wird
und wenigstens eine Verbindung der allgemeinen Formel III in einem Reaktionsmedium ggf. in Gegenwart wenigstens einer Base mit wenigstens einer Verbindung der allgemeinen Formel $R^4$-C(=O)-X, worin $R^4$ die Bedeutung gemäß einem oder mehreren der Ansprüche 1 bis 16 hat und X für eine Abgangsgruppe, vorzugsweise für einen Halogen-Rest steht, oder in einem Reaktionsmedium in Gegenwart wenigstens eines Kupplungsreagenzes ggf. in Gegenwart wenigstens einer Base mit einer Verbindung der allgemeinen Formel $R^4$-C(=O)-OH, worin $R^4$ die Bedeutung gemäß einem oder mehreren der Ansprüche 1 bis 16 hat, zu einer Verbindung der allgemeinen Formel IV,

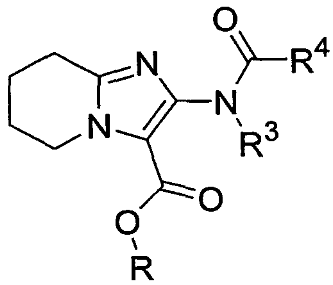

IV

worin R, $R^3$ und $R^4$ die vorstehend genannte Bedeutung haben und $R^3$ nicht gleich Wasserstoff ist, umgesetzt wird und diese ggf. gereinigt und/oder isoliert wird

oder

wenigstens eine Verbindung der allgemeinen Formel II, worin R die vorstehend genannte Bedeutung hat, in einem Reaktionsmedium ggf. in Gegenwart wenigstens einer Base mit wenigstens einer Verbindung der allgemeinen Formel $R^4$-C(=O)-X, worin $R^4$ die vorstehend genannte Bedeutung hat und X für eine Abgangsgruppe, vorzugsweise für einen Halogen-Rest steht, oder in einem Reaktionsmedium in Gegenwart wenigstens eines Kupplungsreagenzes ggf. in Gegenwart wenigstens einer Base mit einer Verbindung der allgemeinen Formel $R^4$-C(=O)-OH, worin $R^4$ vorstehend genannte Bedeutung hat, zu einer Verbindung der allgemeinen Formel V,

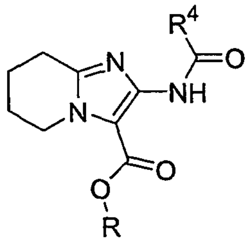

V

worin R und $R^4$ die vorstehend genannte Bedeutung haben, umgesetzt wird und diese ggf. gereinigt und/oder isoliert wird und wenigstens eine Verbindung der allgemeinen Formel V in einem Reaktionsmedium in Gegenwart wenigstens einer Base, vorzugsweise eines Metallhydridsalzes, mit wenigstens einer Verbindung der allgemeinen Formel $R^3$-X, worin $R^3$ die vorstehend genannte Bedeutung hat und nicht gleich Wasserstoff ist und X für eine Abgangsgruppe, vorzugsweise für einen Halogen-Rest steht, zu einer Verbindung der allgemeinen Formel IV, worin R, $R^3$ und $R^4$ die vorstehend genannte Bedeutung haben und $R^3$ nicht gleich Wasserstoff ist, umgesetzt wird und diese ggf. gereinigt und/oder isoliert wird

und wenigstens eine Verbindung der allgemeinen Formel IV in einem Reaktionsmedium in Gegenwart wenigstens einer Base, vorzugsweise in Gegenwart wenigstens eines Metallhydroxidsalzes, zu einer Verbindung der allgemeinen Formel VI,

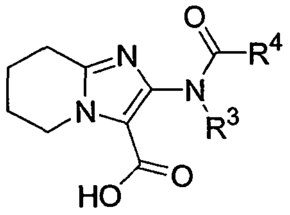

VI

worin $R^3$ und $R^4$ die vorstehend genannte Bedeutung haben und $R^3$ nicht gleich Wasserstoff ist, umgesetzt wird und diese ggf. gereinigt und/oder isoliert wird und wenigstens eine Verbindung der allgemeinen Formel VI in einem Reaktionsmedium in Gegenwart wenigstens eines Kupplungsreagenzes ggf. in Gegenwart wenigstens einer Base mit wenigstens einer Verbindung der allgemeinen Formel $HNR^1R^2$, worin $R^1$ die vorstehend genannte Bedeutung hat und $R^2$ für Wasserstoff steht, zu einer Verbindung der allgemeinen Formel I,

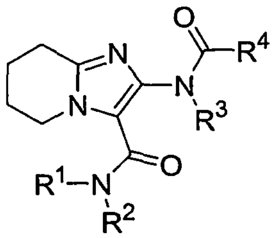

I

worin $R^1$, $R^3$ und $R^4$ die vorstehend genannte Bedeutung haben, $R^3$ nicht gleich Wasserstoff ist und $R^2$ für Wasserstoff steht, umgesetzt wird und diese ggf. gereinigt und/oder isoliert wird
und ggf. wenigstens eine Verbindung der allgemeinen Formel I, worin $R^1$, $R^3$ und $R^4$ die vorstehend genannte Bedeutung haben, $R^3$ nicht gleich Wasserstoff ist und $R^2$ für Wasserstoff steht, in einem Reaktionsmedium in Gegenwart wenigstens einer Base, vorzugsweise eines Metallhydridsalzes, mit wenigstens einer Verbindung der allgemeinen Formel $R^2$-X, worin $R^2$ die vorstehend genannte Bedeutung hat und nicht gleich Wasserstoff ist und X für eine Abgangsgruppe, vorzugsweise einen Halogen-Rest steht, zu einer Verbindung der allgemeinen Formel I, worin $R^1$, $R^2$, $R^3$ und $R^4$ die vorstehend genannte Bedeutung haben und $R^2$ und $R^3$ nicht gleich Wasserstoff sind, umgesetzt wird und diese ggf. gereinigt und/oder isoliert wird
oder
wenigstens eine Verbindung der allgemeinen Formel VI in einem Reaktionsmedium in Gegenwart wenigstens eines Kupplungsreagenzes ggf. in Gegenwart wenigstens einer Base mit wenigstens einer Verbindung der allgemeinen Formel $HNR^1R^2$, worin $R^1$ und $R^2$ die vorstehend genannte Bedeutung haben und nicht gleich Wasserstoff sind, zu einer Verbindung der allgemeinen Formel I, worin $R^1$, $R^2$, $R^3$ und $R^4$ die vorstehend genannte Bedeutung haben und $R^1$, $R^2$ und $R^3$ nicht gleich Wasserstoff sind, umgesetzt wird und diese ggf. gereinigt und/oder isoliert wird.

**19.** Verfahren zur Herstellung substituierter 5,6,7,8-Tetrahydro-imidazo[1,2-a]pyridin-2-ylamin-Verbindungen der allgemeinen Formel I gemäß einem oder mehreren der Ansprüche 1 bis 17, **dadurch gekennzeichnet, dass** wenigstens eine Verbindung der allgemeinen Formel II,

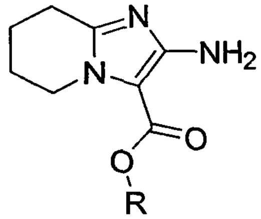

II

worin R für einen linearen oder verzweigten $C_{1-6}$-Alkyl-Rest steht, in einem Reaktionsmedium ggf. in Gegenwart wenigstens einer Base mit wenigstens einer Verbindung der allgemeinen Formel $R^4$-C(=O)-X, worin $R^4$ die Bedeutung gemäß einem oder mehreren der Ansprüche 1 bis 16 hat und X für eine Abgangsgruppe, vorzugsweise für einen Halogen-Rest steht, oder in einem Reaktionsmedium in Gegenwart wenigstens eines Kupplungsreagenzes ggf. in Gegenwart wenigstens einer Base mit einer Verbindung der allgemeinen Formel $R^4$-C(=O)-OH, worin $R^4$ die Bedeutung gemäß einem oder mehreren der Ansprüche 1 bis 16 hat, zu einer Verbindung der allgemeinen Formel IV,

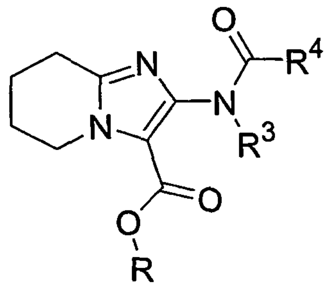

IV

worin R und und $R^4$ die vorstehend genannte Bedeutung haben und $R^3$ gleich Wasserstoff ist, umgesetzt wird und diese ggf. gereinigt und/oder isoliert wird, und wenigstens eine Verbindung der allgemeinen Formel IV in einem Reaktionsmedium in Gegenwart wenigstens einer Base, vorzugsweise in Gegenwart wenigstens eines Metallhydroxidsalzes, zu einer Verbindung der allgemeinen Formel VI,

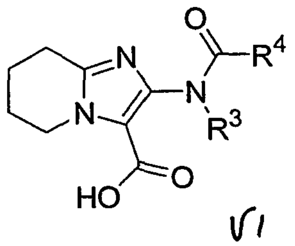

VI

worin $R^4$ die vorstehend genannte Bedeutung hat und $R^3$ gleich Wasserstoff ist, umgesetzt wird und diese ggf. gereinigt und/isoliert wird

wenigstens eine Verbindung der allgemeinen Formel VI in einem Reaktionsmedium in Gegenwart wenigstens eines Kupplungsreagenzes ggf. in Gegenwart wenigstens einer Base mit wenigstens einer Verbindung der allgemeinen Formel $HNR^1R^2$, worin $R^1$ und $R^2$ die Bedeutung gemäß einem oder mehreren der Ansprüche 1 bis 16 haben, zu einer Verbindung den allgemeinen Formel I,

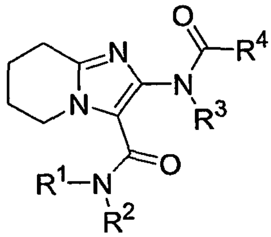

I

worin $R^1$, $R^2$ und $R^4$ die vorstehend genannte Bedeutung haben und $R^3$ gleich Wasserstoff ist, umgesetzt wird und diese ggf. gereinigt und/oder isoliert wird.

**20.** Arzneimittel enthaltend wenigstens eine Verbindung gemäß einem oder mehreren der Ansprüche 1 bis 17 und ggf. einen oder mehrere physiologisch verträgliche Hilfsstoffe.

**21.** Arzneimittel gemäß Anspruch 20 zur Prophylaxe und/oder Behandlung von Schmerz, vorzugsweise von Schmerz ausgewählt aus der Gruppe bestehend aus akutem Schmerz, chronischem Schmerz und neuropathischem Schmerz.

**22.** Arzneimittel gemäß Anspruch 20 zur Prophylaxe und/oder Behandlung von einer oder mehreren Erkrankungen ausgewählt aus der Gruppe bestehend aus Migräne; Depressionen; Harninkontinenz; Husten; neurodegenerativen Erkrankungen, vorzugsweise ausgewählt aus der Gruppe bestehend aus Morbus Parkinson, Morbus Huntington, Morbus Alzheimer und multipler Sklerose; Störungen der Nahrungsmittelaufnahme, vorzugsweise ausgewählt aus der Gruppe bestehend aus Bulimie, Anorexie, Fettsucht und Kachexie; Angstzuständen; kognitiven Dysfunktionen, vorzugsweise Gedächtnisstörungen; kognitive Mangelzustände (attention deficit syndrom, ADS); Epilepsie; Diarrhoe und Pruritus; oder zur Prophylaxe und/oder Behandlung von Alkohol- und/oder Drogen- und/oder Medikamentenmißbrauch und Alkohol- und/oder Drogen- und/oder Medikamentenabhängigkeit, vorzugsweise zur Prophylaxe und/oder Verminderung von Entzugserscheinungen bei Alkohol- und/oder Drogen- und/oder Medikamentenabhängigkeit; zur Prophylaxe und/oder Verminderung einer Toleranzentwicklung gegenüber Medikamenten, insbesondere Medikamenten auf Basis von Opioiden; zur Regulation der Nahrungsaufnahme; zur Modulation der Bewegungsaktivität; zur Regulation des kardiovaskulären Systems; zur Lokalanästhesie; zur Vigilanzsteigerung; zur Libidosteigerung; zur Diurese und/oder zur Antinatriurese.

**23.** Verwendung wenigstens einer Verbindung gemäß einem oder mehreren der Ansprüche 1 bis 17 zur Herstellung eines Arzneimittels zur Prophylaxe und/oder Behandlung von Schmerz, vorzugsweise von Schmerz ausgewählt aus der Gruppe bestehend aus akutem Schmerz, chronischem Schmerz und neuropathischem Schmerz.

**24.** Verwendung wenigstens einer Verbindung gemäß einem oder mehreren der Ansprüche 1 bis 17 zur Herstellung eines Arzneimittels zur Prophylaxe und/oder Behandlung von einer oder mehreren Erkrankungen ausgewählt aus der Gruppe bestehend aus Migräne; Depressionen; Harninkontinenz; Husten; neurodegenerativen Erkrankungen, vorzugsweise ausgewählt aus der Gruppe bestehend aus Morbus Parkinson, Morbus Huntington, Morbus Alzheimer und multipler Sklerose; Störungen der Nahrungsmittelaufnahme, vorzugsweise ausgewählt aus der Gruppe bestehend aus Bulimie, Anorexie, Fettsucht und Kachexie; Angstzuständen; kognitiven Dysfunktionen, vorzugsweise Gedächtnisstörungen; kognitive Mangelzustände (attention deficit syndrom, ADS); Epilepsie; Diarrhoe und Pruritus; zur Prophylaxe und/oder Behandlung von Alkohol- und/oder Drogen- und/oder Medikamentenmißbrauch und Alkohol- und/oder Drogen- und/oder Medikamentenabhängigkeit, vorzugsweise zur Prophylaxe und/oder Verminderung von Entzugserscheinungen bei Alkohol- und/oder Drogen- und/oder Medikamentenabhängigkeit; zur Prophylaxe und/oder Verminderung einer Toleranzentwicklung gegenüber Medikamenten, insbesondere Medikamenten auf Basis von Opioiden; zur Regulation der Nahrungsaufnahme; zur Modulation der Bewegungsaktivität; zur Regulation des kardiovaskulären Systems; zur Lokalanästhesie; zur Vigilanzsteigerung; zur Libidosteigerung; zur Diurese und/oder zur Antinatriurese.

## Claims

**1.** Substituted 5,6,7,8-tetrahydro-imidazo[1,2-a] pyridin-2-ylamine compounds of the general formula 1,

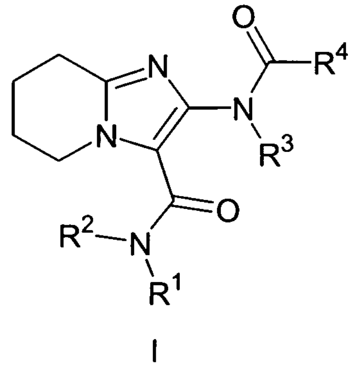

I

wherein

$R^1$ and $R^2$ each represent, independently of one another,
a hydrogen radical
$-C(=O)-OR^5$;
$-(CHR^6)-(CH_2)_m-C(=O)-OR^7$ in which m = 0, 1, 2, 3, 4 or 5;
$-C(=O)-R^8$;
$-(CH_2)_n-C(=O)-R^9$ in which n = 1, 2, 3, 4 or 5;
$-C(=O)-NH-R^{10}$;
$-(CH_2)_o-C(=O)-NHR^{11}$ in which o = 0, 1, 2, 3, 4 or 5;
$-C(=O)-NR^{12}R^{13}$;
$-(CH_2)_pC(=O)-NR^{14}R^{15}$ in which p = 1, 2, 3, 4 or 5;
$-(CHR^{16})-X_q-(CHR^{17})_r-Y_s-(CHR^{18})_t-Z_u-R^{19}$ in which q = 0 or 1, r = 0 or 1, s = 0 or 1, t = 0 or 1, u = 0 or 1 and in which X, Y and Z each represent, independently of one another, O, S, NH, $N(CH_3)$, $N(C_2H_5)$ or $N[CH(CH_3)_2]$;
or a linear or branched, saturated or unsaturated, optionally substituted $C_{1-10}$ aliphatic radical;
an unsaturated or saturated, optionally substituted 3, 4, 5, 6, 7, 8 or 9-member radical which can be bridged with a linear or branched, optionally substituted $C_{1-5}$ alkylene group and/or condensed with a saturated, unsaturated or aromatic, optionally substituted mono or polycyclic cycle compound;
or an optionally substituted 5 to 14-member aryl or heteroaryl radical which can be condensed with a saturated or unsaturated, optionally substituted mono or polycyclic cycle compound; or
$R^1$ and $R^2$, together with the nitrogen atom which binds them serving as a cyclic member, form a saturated or unsaturated, optionally substituted 4, 5, 6, 7, 8 or 9-member heterocycloaliphatic radical, which can be condensed with a saturated, unsaturated or aromatic, optionally substituted mono or polycyclic cycle compound and/or, together with a saturated or unsaturated, optionally substituted 5, 6 or 7-member cycloaliphatic radical, can form an optionally substituted spiro compound via a common cycle atom,
in which the heterocycloaliphatic radical and optionally the cycloaliphatic radical of the spiro compound can be substituted with 1, 2, 3, 4 or 5 substituents selected independently of one another from the group consisting of $R^{20}$, $-(CHR^{21})-(CH_2)_v-(CH_2)_w-R^{22}$ in which v = 0 or 1 and w = 0 or 1, $-CH=CH-R^{23}$, $-(CH_2)_x-C(=O)-OR^{24}$ in which x = 0, 1, 2, 3, 4 or 5; $-(CH_2)_y-C(=O)-R^{25}$ in which y = 0, 1, 2, 3, 4 or 5; $-(CH_2)_z-C(=O)-NHR^{26}$ in which z = 0, 1, 2, 3, 4 or 5; $-(CH_2)_{aa}-C(=O)-NR^{27}R^{28}$ in which aa = 0, 1, 2, 3, 4 or 5; F; Cl; Br; -CN; $-CF_3$; $-NO_2$; oxo (=O); thioxo (=S); $-C_{1-5}$-alkyl; -OH; $-O-C_{1-5}$-alkyl; -SH; $-S-C_{1-5}$-alkyl; $-NH_2$; $NH-C_{1-5}$-alkyl and $-N(C_{1-5}\text{-alkyl})_2$ and/or may have 1, 2, 3, 4 or 5 heteroatom(s) respectively as cycle members, the heteroatom(s) being selected independently of one another from the group consisting of oxygen, nitrogen and sulphur;
$R^3$ represents a hydrogen radical;
a linear or branched, saturated or unsaturated, optionally substituted $C_{1-10}$ aliphatic radical,
a saturated or unsaturated, optionally substituted 3, 4, 5, 6, 7, 8 or 9-member cycloaliphatic radical which can be bound by a linear or branched, optionally substituted $C_{1-5}$ alkylene, $C_{2-5}$ alkenylene or $C_{2-5}$ alkinylene group,
or an optionally substituted 5 to 14-member aryl or heteroaryl radical which can be condensed with a saturated or unsaturated, optionally substituted mono or polycyclic cycle compound and/or can be bound by a linear or branched, optionally substituted $C_{1-5}$ alkylene, $C_{2-5}$ alkenylene or $C_{2-5}$ alkinylene group;
$R^4$ represents a linear or branched, saturated or unsaturated, optionally substituted $C_{1-10}$ aliphatic radical,
a saturated or unsaturated, optionally substituted 3, 4, 5, 6, 7, 8 or 9-member cycloaliphatic radical which can be bound by a linear or branched, optionally substituted $C_{1-5}$ alkylene, $C_{2-5}$ alkenylene or $C_{2-5}$ alkinylene group,
or an optionally substituted 5 to 14-member aryl or heteroaryl radical which can be condensed with a saturated or unsaturated, optionally substituted mono or polycyclic cycle compound and/or can be bound by a linear or branched, optionally substituted $C_{1-5}$ alkylene, $C_{2-5}$ alkenylene or $C_{2-5}$ alkinylene group;
$R^5$, $R^7$, $R^8$, $R^9$, $R^{10}$, $R^{11}$, $R^{12}$, $R^{13}$, $R^{14}$, $R^{15}$, $R^{24}$, $R^{25}$, $R^{26}$ $R^{27}$ and $R^{28}$ each represent, independently of one another,
a linear or branched, saturated or unsaturated, optionally substituted $C_{1-10}$ aliphatic radical,
an unsaturated or saturated, optionally substituted 3, 4, 5, 6, 7, 8 or 9-member cycloaliphatic radical which can be condensed with a saturated, unsaturated or aromatic, optionally substituted mono or polycyclic cycle compound and/or can be bound by a linear or branched, optionally substituted $C_{1-5}$ alkylene, $C_{2-5}$ alkenylene- or $C_{2-5}$ alkinylene group,
or an optionally substituted 5 to 14-member aryl or heteroaryl radical which can be condensed with a saturated or unsaturated, optionally substituted mono or polycyclic cycle compound and/or can be bound by a linear or branched, optionally substituted $C_{1-5}$ alkylene, $C_{2-5}$ alkenylene or $C_{2-5}$ alkinylene group;
$R^6$ represents a hydrogen radical;
a linear or branched, saturated or unsaturated, optionally substituted $C_{1-10}$ aliphatic radical, which may optionally

have 1, 2, 3, 4 or 5 heteroatom(s) as (a) chain link(s) selected from the group consisting of oxygen, sulphur and nitrogen;
or an optionally substituted 5 to 14-member aryl or heteroaryl radical which can be condensed with a saturated or unsaturated, optionally substituted mono or polycyclic cycle compound and/or can be bound by a linear or branched, optionally substituted $C_{1-5}$ alkylene, $C_{2-5}$ alkenylene or $C_{2-5}$ alkinylene group;
$R^{16}$, $R^{17}$ and $R^{18}$ each represent, independently of one another,
a hydrogen radical;
a linear or branched, saturated or unsaturated, optionally substituted $C_{1-10}$ aliphatic radical, which may have 1, 2, 3, 4 or 5 heteroatom(s) as (a) chain link(s) selected from the group consisting of oxygen, sulphur and nitrogen;
or an optionally substituted 5 to 14-member aryl or heteroaryl radical which can be condensed with a saturated or unsaturated, optionally substituted mono or polycyclic cycle compound;
$R^{19}$ represents a saturated or unsaturated, optionally substituted 3, 4, 5, 6, 7, 8 or 9-member cycloaliphatic radical which can be bridged with 1, 2, 3, 4 or 5 linear or branched, optionally substituted $C_{1-5}$ alkylene groups and/or condensed with a saturated, unsaturated or aromatic, optionally substituted mono or polycyclic cycle compound;
or an optionally substituted 5 to 14-member aryl or heteroaryl radical which can be condensed with a saturated or unsaturated, optionally substituted mono or polycyclic cycle compound;
$R^{20}$ represents a linear or branched, saturated or unsaturated, optionally substituted $C_{1-10}$ aliphatic radical;
an unsaturated or saturated, optionally substituted 3, 4, 5, 6, 7, 8 or 9-member cycloaliphatic radical;
or an optionally substituted 5 to 14-member aryl or heteroaryl radical which can be condensed with a saturated or unsaturated, optionally substituted mono or polycyclic cycle compound;
$R^{21}$ represents a hydrogen radical;
a linear or branched, saturated or unsaturated, optionally substituted $C_{1-10}$ aliphatic radical;
an unsaturated or saturated, optionally substituted 3, 4, 5, 6, 7, 8 or 9-member cycloaliphatic radical;
and
or an optionally substituted 5 to 14-member aryl or heteroaryl radical which can be condensed with a saturated or unsaturated, optionally substituted mono or polycyclic cycle compound;
$R^{22}$ and $R^{23}$ each represent, independently of one another,
an unsaturated or saturated, optionally substituted 3, 4, 5, 6, 7, 8 or 9-member cycloaliphatic radical;
or an optionally substituted 5 to 14-member aryl or heteroaryl radical which can be condensed with a saturated or unsaturated, optionally substituted mono or polycyclic cycle compound;

in which
the aforementioned $C_{1-10}$aliphatic radicals may each be optionally substituted with 1, 2, 3, 4 or 5 substituents selected independently of one another from the group consisting of F, Cl, Br, I, -CN, $-NO_2$, -OH, -SH and $-NH_2$;
the aforementioned cycloaliphatic radicals may be optionally substituted with 1, 2, 3, 4 or 5 substituents respectively, the substituents being selected independently of one another from the group consisting of oxo (=O), thioxo (=S), F, Cl, Br, I, -CN, $-CF_3$, $-SF_5$, -OH, -O-$C_{1-5}$-alkyl, $-NH_2$, $-NO_2$, $-O-CF_3$, $-S-CF_3$, -SH, -S-$C_{1-5}$-alkyl, -$C_{1-5}$-alkyl, -C(=O)-OH, -C(=O)-O-$C_{1-5}$-alkyl, -NH-$C_{1-5}$-alkyl, -N($C_{1-5}$-alkyl)$_2$, -($CH_2$)-benzo[b]furanyl, -O-phenyl, -O-benzyl, phenyl and benzyl, in which the cyclic portion of the -O-phenyl, -O-benzyl, phenyl, -($CH_2$)-benzo[b]furanyl and benzyl radicals may be optionally substituted with 1, 2, 3, 4 or 5 substituents selected independently of one another from the group consisting of F, Cl, Br, -OH, $-CF_3$, $-SF_5$, -CN, $-NO_2$, -$C_{1-5}$-alkyl, -O-$C_{1-5}$-alkyl, $-O-CF_3$, $-S-CF_3$, phenyl und -O-benzyl and the aforementioned cycloaliphatic radicals may have 1, 2, 3, 4 or 5 heteroatom(s) respectively as (a) cycle member(s), the heteroatom(s) being selected independently of one another from the group consisting of oxygen, nitrogen and sulphur;
the aforementioned $C_{1-5}$ alkylene, $C_{2-5}$ alkenylene or $C_{2-5}$ alkinylene groups may optionally be substituted with 1, 2, 3, 4 or 5 substituents respectively, the substituents being selected independently of one another from the group consisting of F, Cl, Br, -OH, -SH, $-NH_2$, -CN, $NO_2$ and phenyl;
the cycles of the aforementioned mono or polycyclic cycle compounds may each be optionally substituted with 1, 2, 3, 4 or 5 substituents respectively, the substituents being selected independently of one another from the group consisting of oxo (=O), thioxo (=S), F, Cl, Br, I, -CN, $-CF_3$, $-SF_5$, -OH, -O-$C_{1-5}$-alkyl, $-NH_2$, $-NO_2$, $-O-CF_3$, $-S-CF_3$, -SH, -S-$C_{1-5}$-alkyl, -$C_{1-5}$-alkyl, -C(=O)-OH, -C(=O)-O-$C_{1-5}$-alkyl, -NH-$C_{1-5}$-alkyl, -N($C_{1-5}$-alkyl)$_2$, -($CH_2$)-benzo[b]furanyl, -O-phenyl, -O-benzyl, phenyl and benzyl, in which the cyclic portion of the -O-phenyl, -O-benzyl, phenyl, -($CH_2$)-benzo[b]furanyl and benzyl radicals respectively may be optionally substituted with 1, 2, 3, 4 or 5 substituents selected independently of one another from the group consisting of F, Cl, Br, -OH, $-CF_3$, $-SF_5$, -CN, $-NO_2$, -$C_{1-5}$-alkyl, -O-$C_{1-5}$-alkyl, $-O-CF_3$, $-S-CF_3$, phenyl und -O-benzyl
and the cycles of the aforementioned mono or polycyclic cycle compounds have 5, 6, or 7-members respectively and may optionally have 1, 2, 3, 4 or 5 heteroatom(s) respectively as (a) cycle member(s), the heteroatom(s) being

selected independently of one another from the group consisting of oxygen, nitrogen and sulphur;
and the aforementioned aryl or heteroaryl radicals may be optionally substituted with 1, 2 , 3 , 4 or 5 substituents respectively, the substituents being selected independently of one another from the group consisting of F, Cl, Br, I, -CN, $-CF_3$, $-SF_5$, -OH, -O-$C_{1-5}$-alkyl, $-NH_2$, $-NO_2$, $-O-CF_3$, $-S-CF_3$, -SH, -S-$C_{1-5}$-alkyl, -$C_{1-5}$-alkyl, -C(=O)-OH, -C(=O)-O-$C_{1-5}$-alkyl, -NH-$C_{1-5}$-alkyl, -N($C_{1-5}$-alkyl)$_2$, -NH-C(=O)-O-$C_{1-5}$-alkyl, C(=O)-H, -C(=O)-$C_{1-5}$-alkyl, -C(=O)-$NH_2$, -C(=O)-NH-$C_{1-5}$-alkyl, C(=O)-N-($C_{1-5}$-alkyl)$_2$, -($CH_2$)-benzo[b]furanyl, -O-phenyl, -O-benzyl, phenyl and benzyl, in which the cyclic portion of the -O-phenyl, -O-benzyl, phenyl, -($CH_2$)-benzo[b]furanyl and benzyl radicals may be optionally substituted with 1, 2, 3, 4 or 5 substituents selected independently of one another from the group consisting of F, Cl, Br, -OH, $-CF_3$, $-SF_5$, -CN, $-NO_2$, -$C_{1-5}$-alkyl, -O-$C_{1-5}$-alkyl, $-O-CF_3$, $-S-CF_3$, phenyl und -O-benzyl, and
the aforementioned heteroaryl radicals may have 1, 2, 3, 4 or 5 heteroatom(s) respectively as (a) cycle member(s), the heteroatom(s) being selected independently of one another from the group consisting of oxygen, nitrogen and sulphur;
each optionally in the form of one of the pure stereoisomers thereof, in particular enantiomers or diastereomers, the racemates thereof or in the form of a mixture of stereoisomers, in particular of enantiomers and/or diastereomers, in any desired mixture ratio, or in the form of appropriate salts or appropriate solvates respectively.

**2.** Compounds according to claim 1, **characterised in that**
$R^1$ represents a hydrogen radical; -C(=O)-$OR^5$; -($CHR^6$)-$(CH_2)_m$-C(=O)-$OR^7$ in which m = 0, 1, 2, 3, 4 or 5; -C(=O)-NH-$R^{10}$; -$(CH_2)_o$-C(=O)-$NHR^{11}$ in which o = 0, 1, 2, 3, 4 or 5; -($CHR^{16}$)-$X_q$-$(CHR^{17})_r$-$Y_s$-$(CHR^{18})_t$-$Z_u$-$R^{19}$ in which q = 0 or 1, r = 0 or 1, s = 0 or 1, t = 0 or 1, u = 0 or 1 and in which X, Y and Z represent, independently of one another, O, S, NH and N($CH_3$) respectively;
an optionally substituted alkyl radical selected from the group consisting of methyl, ethyl, n-propyl, isopropyl, -($CH_2$)-($CH_2$)-(CN), n-butyl, sec-butyl, isobutyl, tert-butyl, -C(H)($CH_3$)-C(H)$(CH_3)_2$ and -($CH_2$)-($CH_2$)-(C$(CH_3)_3$),
an alkenyl radical selected from the group consisting of vinyl, 1-propenyl and 2-propenyl,
a (hetero)cycloaliphatic radical selected from the group consisting of cyclopropyl, cyclobutyl, cyclopentyl, cyclohexyl, cycloheptyl, cyclopentenyl, cyclohexenyl, cycloheptenyl, imidazolidinyl, tetrahydrofuranyl, tetrahydrothiophenyl, pyrrolidinyl, piperidinyl, morpholinyl, piperazinyl, thiomorpholinyl, tetrahydropyranyl, azepanyl, diazepanyl, dithiolanyl, (6,6)-dimethyl-[3.1.1]-bicycloheptyl, indanyl, indenyl, (1,4)-benzodioxanyl, (1,2,3,4)-tetrahydronaphthyl, (1,2,3,4)-tetrahydroquinolinyl and (1,2,3,4)-tetrahydroquinazolinyl, in which the (hetero)cycloaliphatic radical can be optionally substituted with 1, 2, 3, 4 or 5 substituents respectively, the substituents being selected independently of one another from the group consisting of F, Cl, Br, I, -CN, $-CF_3$, $-SF_5$, -OH, $-NH_2$, $-O-CF_3$, -SH, $-O-CH_3$, $-O-C_2H_5$, methyl, ethyl, n-propyl, isopropyl, n-butyl, sec-butyl, isobutyl, tert-butyl, $-S-CH_3$, $-S-C_2H_5$, -C(=O)-O-$CH_3$, -C(=O)-O-$C_2H_5$, Oxo (=O), -N$(CH_3)_2$, -N$(C_2H_5)_2$, -N(H)($CH_3$), -N(H)($C_2H_5$), $-NO_2$, $-SCF_3$, -C(=O)-OH, -($CH_2$)-benzo[b]furanyl, -O-phenyl, -O-benzyl, phenyl and benzyl, in which the cyclic portion of the -($CH_2$)-benzo[b]furanyl, -O-phenyl, -O-benzyl-, benzyl- and phenyl radicals respectively may be substituted with 1, 2, 3, 4 or 5 substituents selected independently of one another from the group consisting of F, Cl, Br, -OH, $-CF_3$, $-SF_5$, -CN, $-NO_2$, methyl, ethyl, isopropyl, n-propyl, $-O-CH_3$, $-O-C_2H_5$, $-O-CF_3$, phenyl and -O-benzyl or a radical selected from the group consisting of phenyl, naphthyl, (1,3)-benzodioxolyl, (1,4)-benzodioxanyl, thiophenyl, furanyl, pyrrolyl, pyrazolyl, pyrazinyl, pyranyl, triazolyl, pyridinyl, imidazolyl, indolyl, isoindolyl, benzo[b]furanyl, benzo[b]thiophenyl, thiazolyl, oxazolyl, isoxazolyl, pyridazinyl, pyrazinyl, pyrimidinyl, indazolyl, quinazolinyl, quinolinyl and isoquinolinyl, in which the radical may optionally be substituted with 1, 2, 3, 4 or 5 substituents respectively, the substituents being selected independently of one another from the group consisting of F, Cl, Br, I, -CN, $-NO_2$, -OH, -SH, methyl, ethyl, n-propyl, isopropyl, n-butyl, sec-butyl, isobutyl, tert-butyl, $-CF_3$, $-O-CF_3$, $-S-CF_3$, $-SF_5$, $-O-CH_3$, $-O-C_2H_5$, -O-phenyl, -O-benzyl, $-NH_2$, -N$(CH_3)_2$, -N$(C_2H_5)_2$, -N(H)($CH_3$), -N(H)($C_2H_5$), -NH-C(=O)-O-$CH_3$,- NH-C(=O)-O-$C_2H_5$ and -NH-C(=O)-O-C$(CH_3)_3$.

**3.** Compounds according to either claim 1 or claim 2, **characterised in that**
$R^2$ represents a hydrogen radical,
a -($CHR^{16}$)-$R^{19}$,
or an alkyl radical selected from the group consisting of methyl, ethyl, n-propyl, isopropyl, n-butyl, sec-butyl, isobutyl and tert-butyl.

**4.** Compounds according to one or more of claims 1 to 3, **characterised in that**
$R^1$ and $R^2$, together with the nitrogen atom which binds them serving as a cycle member, form a heterocycloaliphatic radical selected from the group consisting of pyrrolidinyl; piperidinyl; (1,2,3,6)-tetrahydropyridinyl; (1,2,3,4)-tetrahydropyridinyl and (1,4)-dioxa-8-aza-spiro[4.5]decan, in which the heterocycloaliphatic radical may be substituted with 1 or 2 substituents respectively, the substituents being selected independently of one another from the group consisting of $R^{20}$, -($CHR^{21}$)-$(CH)_v$-$R^{22}$

in which v = 0 or 1; $-C(=O)-OR^{24}$; $-C(=O)-R^{25}$; F; Cl; Br; -CN; $-CF_3$ and -OH,

or

$R^1$ and $R^2$, together with the nitrogen atom which binds them serving as a cycle member, form a heterocycloaliphatic radical selected from the group consisting of (1,3,4,5)-tetrahydropyrido[4,3-b]indolyl; (3,4)-dihydro-1H-isoquinolinyl and (1,3,4,9)-tetrahydro-[b]-carbolinyl, in which the heterocycloaliphatic radical may be substituted with 1 or 2 substituents respectively, the substituents being selected independently of one another from the group consisting of F, Cl, Br, -CN, $-CF_3$ and -OH,

or

$R^1$ and $R^2$, together with the nitrogen atom which binds them serving as a cycle member, form a heterocycloaliphatic radical selected from the group consisting of imidazolidinyl, (1,3)-thiazolidinyl, piperazinyl, morpholinyl und thiomorpholinyl, in which the heterocycloaliphatic radical may be substituted with 1 or 2 substituents respectively selected independently of one another from the group consisting of $R^{20}$, $-(CHR^{21})-(CH_2)_v-(CH_2)_w-R^{22}$ in which v = 0 or 1 and w = 0 or 1, $-CH=CH-R^{23}$, $-C(=O)-OR^{24}$; $-C(=O)-R^{25}$; $-(CH_2)_z-C(=O)-NHR^{26}$ in which z = 1 and $-(CH_2)_{aa}-C(=O)-NR^{27}R^{28}$ in which aa = 1.

5. Compounds according to one or more of claims 1 to 4, **characterised in that** $R^3$ represents a hydrogen radical or an alkyl radical selected from the group consisting of methyl, ethyl, n-propyl, isopropyl, n-butyl, sec-butyl, isobutyl and tert-butyl and n-pentyl.

6. Compounds according to one or more of claims 1 to 5, **characterised in that** $R^4$ represents a phenyl or naphthyl radical which may be substituted with 1, 2, 3, 4 or 5 substituents respectively, selected independently of one another from the group consisting of methyl, ethyl, n-propyl, isopropyl, n-butyl, isobutyl, sec-butyl, tert-butyl, F, Cl, Br, -CN, $-SF_5$, $-S-CF_3$, $-NH_2$, $-N(CH_3)_2$, $-N(C_2H_5)_2$, $-N(H)(CH_3)$, $-N(H)(C_2H_5)$, -SH, $-NO_2$, $-CF_3$, $-OCF_3$, -OH, $-O-CH_3$ and $-O-C_2H_5$ and/or may be bound by a $-(CH_2)-$, $(CH_2)_2-$ or $-(CH_2)_3$ group.

7. Compounds according to one or more of claims 1 to 6, **characterised in that** $R^5$, $R^7$, $R^8$, $R^9$, $R^{10}$, $R^{11}$, $R^{12}$, $R^{13}$, $R^{14}$ and $R^{15}$ each represent, independently of one another, an alkyl radical selected from the group consisting of methyl, ethyl, n-propyl, isopropyl, n-butyl, sec-butyl, isobutyl und tert-butyl, in which the alkyl radical may be optionally substituted with 1, 2, 3, 4 or 5 substituents respectively, selected independently of one another from the group consisting of F, Cl, Br, I, -CN, $-NO_2$,- OH, -SH and $-NH_2$,
or a radical selected from the group consisting of phenyl, naphthyl, (1,3)-benzodioxolyl, (1,4)-benzodioxanyl, thiophenyl, furanyl, pyrrolyl, pyrazolyl, pyrazinyl, pyranyl, triazolyl, pyridinyl, imidazolyl, indolyl, isoindolyl, benzo[b]furanyl, benzo[b]thiophenyl, thiazolyl, oxazolyl, isoxazolyl, pyridazinyl, pyrazinyl, pyrimidinyl, indazolyl, quinazolinyl, quinolinyl and isoquinolinyl, in which the radical may be bound by a $-(CH_2)-$, $-(CH_2)_2-$ or $-(CH_2)_3$ group and/or optionally substituted with 1, 2 , 3, 4 or 5 substituents selected independently of one another from the group consisting of F, Cl, Br, I, -CN, $-NO_2$, -OH, -SH, methyl, ethyl, n-Propyl, isopropyl, n-butyl, sec-butyl, isobutyl, tert-butyl, $-CF_3$, $-O-CF_3$, $-S-CF_3$, $-SF_5$, $-O-CH_3$, $-O-C_2H_5$, -O-phenyl, -O-benzyl, $-NH_2$, $-N(CH_3)_2$, $-N(C_2H_5)_2$, $-N(H)(CH_3)$, $-N(H)(C_2H_5)$, $-NH-C(=O)-O-CH_3$, $-NH-C(=O)-O-C_2H_5$ and $-NH-C(=O)-O-C(CH_3)_3$.

8. Compounds according to one or more of claims 1 to 7, **characterised in that** $R^6$ represents a hydrogen radical,
a radical selected from the group consisting of methyl, ethyl, n-propyl, isopropyl, n-butyl, sec-butyl, isobutyl, tert-butyl, n-pentyl, sec-pentyl, $-C(H)(CH_3)-C(H)(CH_3)_2$, $-(CH_2)-(CH_2)-(C(CH_3)_3)$, $-C(H)(CH_3)(O(C(CH_3)_3))$ and n-hexyl, in which the radical may be substituted with 1, 2, 3, 4 or 5 substituents respectively, selected independently of one another from the group consisting of F, Cl, Br, I, -CN, $-NO_2$, -OH, -SH and $-NH_2$,
or a radical selected from the group consisting of phenyl, naphthyl, (1,3)-benzodioxolyl, (1,4)-benzodioxanyl, thiophenyl, furanyl, pyrrolyl, pyrazolyl, pyrazinyl, pyranyl, triazolyl, pyridinyl, imidazolyl, indolyl, isoindolyl, benzo[b]furanyl, benzo[b]thiophenyl, thiazolyl, oxazolyl, isoxazolyl, pyridazinyl, pyrazinyl, pyrimidinyl, indazolyl, quinazolinyl, quinolinyl and isoquinolinyl, in which the radical may be bound by a $-(CH_2)-$, $-(CH_2)_2-$ or $-(CH_2)_3$ group and/or optionally substituted with 1, 2 , 3, 4 or 5 substituents respectively, the substituents being selected independently of one another from the group consisting of F, Cl, Br, I, -CN, $-NO_2$, -OH, -SH, methyl, ethyl, n-propyl, isopropyl, n-butyl, sec-butyl, isobutyl, tert-butyl, $-CF_3$, $-O-CF_3$, $-S-CF_3$, $-SF_5$, $-O-CH_3$, $-O-C_2H_5$, -O-phenyl, -O-benzyl, $-NH_2$, $N-(CH_3)_2$, $-N(C_2H_5)_2$, $-N(H)(CH_3)$, $-N(H)(C_2H_5)$, $-NH-C(=O)-O-CH_3$, $-NH-C(=O)-O-C_2H_5$ and $-NH-C(=O)-O-C(CH_3)_3$.

9. Compounds according to one or more of claims 1 to 8, **characterised in that** $R^{16}$, $R^{17}$ and $R^{18}$, each represent, independently of one another, a hydrogen radical,
a radical selected from the group consisting of methyl, ethyl, n-propyl, isopropyl, n-butyl, sec-butyl, isobutyl, tert-butyl, n-pentyl, sec-pentyl, $-C(H)(CH_3)-C(H)(CH_3)_2$, $(-CH_2)-(CH_2)-(C(CH_3)_3)$, $-(CH_2)-O-(CH_3)$ and n-hexyl, in which

the radical may be substituted with 1, 2, 3, 4 or 5 substituents respectively, selected independently of one another from the group consisting of F, Cl, Br, I, -CN, $-NO_2$, -OH, -SH and $-NH_2$,
or a radical selected from the group consisting of phenyl and naphthyl, in which the radical may be substituted with 1, 2, 3, 4 or 5 substituents respectively, selected independently of one another from the group consisting of F, Cl, Br, I, -CN, $-NO_2$, -OH, -SH, methyl, ethyl, n-propyl, isopropyl, n-butyl, sec-butyl, isobutyl, tert-butyl, $-CF_3$, $-O-CF_3$, $-S-CF_3$, $-SF_5$, $-O-CH_3$, $-O-C_2H_5$, -O-phenyl, -O-benzyl, $-NH_2$, $-N(CH_3)_2$, $-N(C_2H_5)_2$, $-N(H)(CH_3)$, $-N(H)(C_2H_5)$, $-NH-C(=O)-O-CH_3$, $-NH-C(=O)-O-C_2H_5$ and $-NH-C(=O)-O-C(CH_3)_3$.

**10.** Compounds according to one or more of claims 1 to 9, **characterised in that**
$R^{19}$ represents a (hetero)cycloaliphatic radical selected from the group consisting of cyclopropyl, cyclobutyl, cyclopentyl, cyclohexyl, cycloheptyl, cyclopentenyl, cyclohexenyl, cycloheptenyl, imidazolidinyl, tetrahydrofuranyl, tetrahydrothiophenyl, pyrrolidinyl, piperidinyl, morpholinyl, piperazinyl, thiomorpholinyl, tetrahydropyranyl, azepanyl, diazepanyl, dithiolanyl, (6,6)-dimethyl-[3.1.1]-bicycloheptyl, adamantyl (tricyclo-[3.3.1.1$^{3,7}$]-decanyl), indanyl, indenyl, (1,4)-benzodioxanyl, (1,2,3,4)-tetrahydronaphthyl, (1,2,3,4)-tetrahydroquinolinyl and (1,2,3,4)-tetrahydroquinazolinyl, in which the (hetero)cycloaliphatic radical may be substituted with 1, 2, 3, 4 or 5 substituents respectively, selected independently of one another from the group consisting of F, Cl, Br, I, -CN, $-CF_3$, $-SF_5$, -OH, $-NH_2$, $-O-CF_3$, -SH, $-O-CH_3$, $-O-C_2H_5$, methyl, ethyl, n-propyl, isopropyl, n-butyl, sec-butyl, isobutyl, tert-butyl, $-S-CH_3$, $-S-C_2H_5$, $-C(=O)-O-CH_3$, $-C(=O)-O-C_2H_5$, oxo (=O), $-N(CH_3)_2$, $-N(C_2H_5)_2$, $-N(H)(CH_3)$, $-N(H)(C_2H_5)$, $-NO_2$, $-SCF_3$, -C(=O)-OH, $-(CH_2)$-benzo[b]furanyl, -O-phenyl, -O-benzyl, phenyl and benzyl,
or a radical selected from the group consisting of phenyl, naphthyl, (1,3)-benzodioxolyl, (1,4)-benzodioxanyl, thiophenyl, furanyl, pyrrolyl, pyrazolyl, pyrazinyl, pyranyl, triazolyl, pyridinyl, imidazolyl, indolyl, isoindolyl, benzo[b]furanyl, benzo[b]thiophenyl, thiazolyl, oxazolyl, isoxazolyl, pyridazinyl, pyrazinyl, pyrimidinyl, indazolyl, quinazolinyl, quinolinyl and isoquinolinyl, in which the radical may optionally respectively be substituted with 1, 2, 3, 4 or 5 substituents selected independently of one another from the group consisting of F, Cl, Br, I, -CN, $-NO_2$, -OH, -SH, methyl, ethyl, n-propyl, isopropyl, n-butyl, sec-butyl, isobutyl, tert-butyl, $-CF_3$, $-O-CF_3$, $-S-CF_3$, $-SF_5$, $-O-CH_3$, $-O-C_2H_5$, -O-phenyl, -O-benzyl, $-NH_2$, $-N(CH_3)_2$, $-N(C_2H_5)_2$, $-N(H)(CH_3)$, $-N(H)(C_2H_5)$, $-NH-C(=O)-O-CH_3$, $-NH-C(=O)-O-C_2H_5$ and $-NH-C(=O)-O-C(CH_3)_3$.

**11.** Compounds according to one or more of claims 1 to 10, **characterised in that**
$R^{20}$ represents a hydrogen radical or an alkyl radical selected from the group consisting of methyl, ethyl, n-propyl, isopropyl, n-butyl, isobutyl, sec-butyl, tert-butyl and n-pentyl,
a (hetero)cycloaliphatic radical selected from the group consisting of cyclopentyl, cyclohexyl, piperidinyl and cycloheptyl, in which the (hetero)cycloaliphatic radical may be substituted with 1, 2, 3, 4 or 5 substituents respectively, selected independently of one another from the group consisting of F, Cl, Br, I, -CN, $-CF_3$, $-SF_5$, -OH, $-NH_2$, $-O-CF_3$, -SH, $-O-CH_3$, $-O-C_2H_5$, methyl, ethyl, n-propyl, isopropyl, n-butyl, sec-butyl, isobutyl and tert-butyl,
or a radical selected from the group consisting of phenyl, naphthyl, (1,3)-benzodioxolyl, thiophenyl, furanyl, pyridinyl, imidazolyl, indolyl, benzo[b]furanyl, benzo[b]thiophenyl, thiazolyl, oxazolyl, isoxazolyl, pyridazinyl, pyrazinyl, pyrimidinyl, and quinolinyl, in which the radical may optionally be substituted with 1, 2, 3, 4 or 5 substituents respectively, selected independently of one another from the group consisting of methyl, ethyl, n-propyl, isopropyl, n-butyl, isobutyl, sec-butyl, tert-butyl, F, Cl, Br, -CN, $-SF_5$, $-O-CF_3$, $-S-CF_3$, $-NH_2$, $-N(CH_3)_2$, $-N(C_2H_5)_2$, $-N(H)(CH_3)$, $-N(H)(C_2H_5)$, -SH, $-NO_2$, $-CF_3$, $-OCF_3$, -OH, $-O-CH_3$ and $-O-C_2H_5$.

**12.** Compounds according to one or more of claims 1 to 11, **characterised in that**
$R^{21}$ represents a hydrogen radical
or a phenyl or naphthyl radical, which can be substituted with 1, 2 or 3 substituents selected independently of one another from the group consisting of methyl, ethyl, n-propyl, isopropyl, n-butyl, isobutyl, sec-butyl, tert-butyl, F, Cl and Br.

**13.** Compounds according to one or more of claims 1 to 12, **characterised in that**
$R^{22}$ and $R^{23}$ each represent, independently of one another, a (hetero)cycloaliphatic radical selected from the group consisting of pyrrolidinyl, morpholinyl and thiomorpholinyl, in which the (hetero)cycloaliphatic radical may be substituted with 1, 2, 3, 4 or 5 substituents respectively, selected independently of one another from the group consisting of F, Cl, Br, I, -CN, $-CF_3$, $-SF_5$, -OH, $-NH_2$, $-O-CF_3$, -SH, $-O-CH_3$, $-O-C_2H_5$, methyl, ethyl, n-propyl, isopropyl, n-butyl, sec-butyl, isobutyl and tert-butyl,
or a radical selected from the group consisting of phenyl, naphthyl, (1,3)-benzodioxolyl, thiophenyl, furanyl, pyridinyl, imidazolyl, indolyl, benzo[b]furanyl, benzo[b]thiophenyl, thiazolyl, oxazolyl, isoxazolyl, pyridazinyl, pyrazinyl, pyrimidinyl, and quinolinyl, in which the radical may optionally be substituted with 1, 2, 3, 4 or 5 substituents respectively, selected independently of one another from the group consisting of methyl, ethyl, n-propyl, isopropyl, n-butyl, isobutyl,

sec-butyl, tert-butyl, F, Cl, Br, -CN, $-SF_5$, $-O-CF_3$, $-S-CF_3$, $-NH_2$, $-N(CH_3)_2$, $-N(C_2H_5)_2$, $-N(H)(CH_3)$, $-N(H)(C_2H_5)$, -SH, $-NO_2$, $-CF_3$, $-OCF_3$, -OH, $-O-CH_3$ and $-O-C_2H_5$.

**14.** Compounds according to one or more of claims 1 to 13, **characterised in that**
$R^{24}$ represents an alkyl radical selected from the group consisting of methyl, ethyl, n-propyl, isopropyl, n-butyl, sec-butyl, isobutyl and tert-butyl,
$R^{25}$ represents a radical selected from the group consisting of phenyl, naphthyl, furanyl, pyrazinyl and pyrimidinyl, which may be bound by a $-(CH_2)-$, $-(CH_2)_2-$ or $-(CH_2)_3$ group and/or substituted with 1, 2 or 3 substituents selected independently of one another from the group consisting of methyl, ethyl, n-propyl and isopropyl,
$R^{26}$ represents an alkyl radical selected from the group consisting of methyl, ethyl, n-propyl, and isopropyl,
$R^{27}$ represents an alkyl radical selected from the group consisting of methyl, ethyl, isopropyl and n-propyl or a phenyl radical,
and
$R^{28}$ represents an alkyl radical selected from the group consisting of methyl, ethyl, isopropyl and n-propyl or a phenyl radical.

**15.** Compounds according to one or more of claims 1 to 14, **characterised in that**

$R^1$ represents $-C(=O)-OR^5$; $-(CHR^6)-C(=O)-OR^7$;
$-C(=O)-NHR^{11}$; $-(CH_2)-C(=O)-NHR^{11}$; $-(CHR^{16})-R^{19}$; $-(CHR^{16})-(CHR^{17})-R^{19}$;
$-(CHR^{16})-(CHR^{17})-O-R^{19}$; $-(CHR^{16})-(CHR^{17})-(CHR^{18})-R^{19}$; $-(CHR^{16})-(CHR^{17})-S-(CHR^{18})-R^{19}$;- $(CHR^{16})-(CHR^{17})-(CHR^{18})-N(CH_3)-R^{19}$,
an optionally substituted alkyl radical selected from the group consisting of methyl, ethyl, n-propyl, isopropyl, $-CH_2-CH_2-CN$, n-butyl, sec-butyl, isobutyl, tert-butyl, $-CH(CH_3)-CH(CH_3)_2$ and $-CH_2-CH_2-C(CH_3)_3$,
an alkenyl radical selected from the group consisting of 1-propenyl and 2-propenyl,
a radical selected from the group consisting of cyclopentyl, cyclohexyl, cycloheptyl, (6,6)-dimethyl-[3.1.1]-bicycloheptyl, indanyl and indenyl, in which the radical may be substituted with 1, 2 or 3 substituents respectively, selected independently from the group consisting of F, Cl, Br, methyl, ethyl, isopropyl, n-propyl and -O-benzyl,
a pyrrolidinyl radical which can be substituted with a $-(CH_2)$-benzo[b]furanyl or benzyl radical, in which the cyclic portion of the benzyl radical may be substituted with 1, 2 or 3 substituents respectively, selected independently of one another from the group consisting of F, Cl, Br, -OH, methyl, ethyl, isopropyl, n-propyl, $-CF_3$, $-O-CH_3$, $-O-C_2H_5$, phenyl and -O-benzyl,
or a radical selected from the group consisting of phenyl, naphthyl, (1,3)-benzodioxolyl and (1,4)-benzodioxanyl, in which the radical may be substituted with 1, 2 or 3 substituents respectively, selected independently of one another from the group consisting of F, Cl, Br, I, methyl, ethyl, n-propyl, isopropyl, n-butyl, sec-butyl, isobutyl, tert-butyl,-O-phenyl, -O-benzyl, $-NH-C(=O)-O-CH_3$, $-NH-C(=O)-O-C_2H_5$ and $-NH-C(=O)-O-C(CH_3)_3$;
$R^2$ represents a hydrogen radical,
$-(CHR^{16})-R^{19}$,
or an alkyl radical selected from the group consisting of methyl, ethyl, n-propyl, isopropyl, n-butyl, sec-butyl, isobutyl and tert-butyl;
or
$R^1$ and $R^2$, together with the nitrogen atom which binds them serving as a cycle member, form a radical selected from the group consisting of

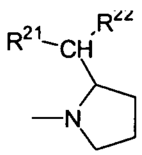 , 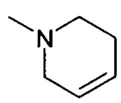 , 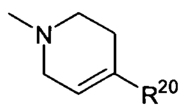 , 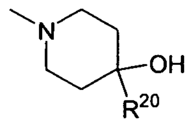 ,

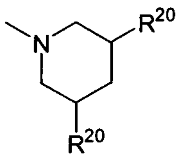 , 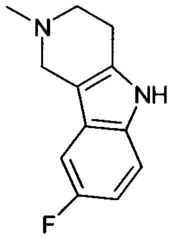 , 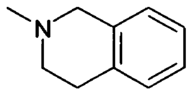 , 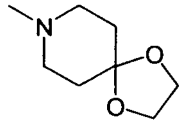 ,

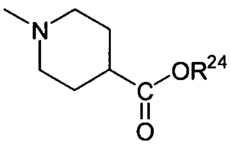 , 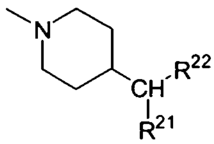 , 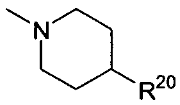 , 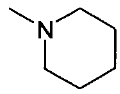 ,

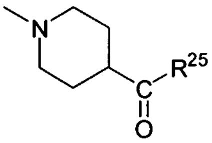 , 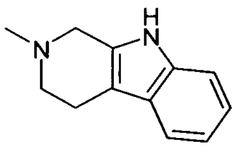 , 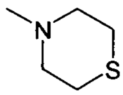 , 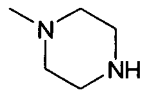 ,

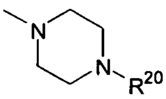 , 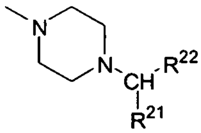 , 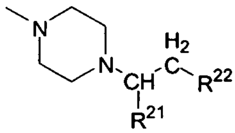 ,

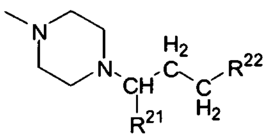 , 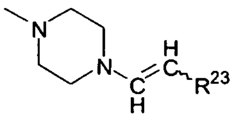 , 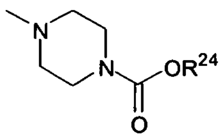 ,

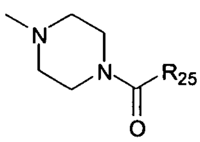 , 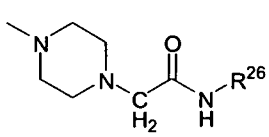 und 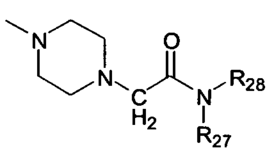 ,

$R^3$ represents an alkyl radical selected from the group consisting of methyl, ethyl, n-propyl, isopropyl, n-butyl, sec-butyl, isobutyl and tert-butyl;
$R^4$ represents a radical selected from the group consisting of

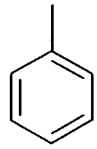, 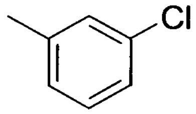, 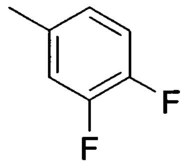, 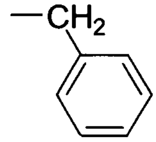

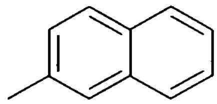 und 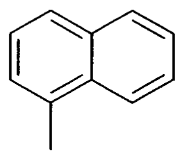 ,

$R^5$, $R^7$ and $R^{11}$ each represent, independently of one another,
an alkyl radical selected from the group consisting of methyl, ethyl, n-propyl, isopropyl, n-butyl, sec-butyl, isobutyl and tert-butyl;
or a benzyl or naphthyl radical;
$R^6$ represents a hydrogen radical,
a radical selected from the group consisting of methyl, ethyl, n-propyl, isopropyl, n-butyl, sec-butyl, isobutyl, tert-butyl, n-pentyl, sec-pentyl, neopentyl and $-C(H)(CH_3)(O(C(CH_3)_3))$,
or an indolyl radical bound by a $-(CH_2)$ group;
$R^{16}$ represents a hydrogen radical,
a radical selected from the group consisting of methyl, ethyl, isopropyl, n-propyl, n-butyl, sec-butyl, isobutyl, tert-butyl and $-(CH_2)-O-(CH_3)$,
or a phenyl radical;
$R^{17}$ represents a hydrogen radical,
an alkyl radical selected from the group consisting of methyl, ethyl, n-propyl, isopropyl, n-butyl, isobutyl, sec-butyl and tert-butyl or a phenyl radical;
$R^{18}$ represents a hydrogen radical
or a phenyl radical;
$R^{19}$ represents a (hetero)cycloaliphatic radical selected from the group consisting of cyclopentyl, cyclohexyl, cycloheptyl, tetrahydrofuranyl, tetrahydrothiophenyl, pyrrolidinyl, piperidinyl, adamantyl (tricyclo-[3.3.1.1$^{3,7}$]-decanyl) and (1,4)-benzodioxanyl, in which the (hetero)cycloaliphatic radical may be substituted with 1 or 2 substituents respectively, selected independently of one another from the group consisting of methyl, ethyl, isopropyl and n-propyl,
or a radical selected from the group consisting of phenyl, naphthyl, (1,3)-benzodioxolyl, (1,4)-benzodioxanyl, thiophenyl, furanyl, pyridinyl, imidazolyl, indolyl and isoindolyl, in which the radical may be substituted with 1, 2, 3, 4 or 5 substituents respectively, selected independently of one another from the group consisting of F, Cl, Br, I, methyl, ethyl, n-propyl, isopropyl, n-butyl, sec-butyl, isobutyl, tert-butyl, $-CF_3$, $-O-CH_3$, $-O-C_2H_5$, $-O-CF_3$, -O-Phenyl, -O-Benzyl, $-NH_2$, $-N(CH_3)_2$, $-N(C_2H_5)_2$, $-N(H)(CH_3)$ and $-N(H)(C_2H_5)$;
$R^{20}$ represents a methyl or ethyl radical,
a (hetero)cycloaliphatic radical selected from the group consisting of cyclopentyl, cyclohexyl, piperidinyl and cycloheptyl,
or a phenyl or pyridinyl radical, which can be substituted with 1, 2 or 3 substituents selected independently of one another from the group consisting of methyl, ethyl, n-propyl, isopropyl, n-butyl, isobutyl, sec-butyl, tert-butyl, F, Cl, Br, $-CF_3$, -OH, $-O-CH_3$ and $-O-C_2H_5$;
$R^{21}$ represents a hydrogen radical
or a phenyl radical, which can be substituted with 1, 2 or 3 substituents selected independently of one another from the group consisting of methyl, ethyl, n-propyl, isopropyl, n-butyl, isobutyl, sec-butyl, tert-butyl, F, Cl and Br;
$R^{22}$ represents a pyrrolidinyl or morpholinyl radical
or a radical selected from the group consisting of phenyl, naphthyl, (1,3)-benzodioxolyl, thiophenyl, benzo[b]furanyl, benzo[b]thiophenyl and quinolinyl, in which the radical may be substituted with 1, 2 or 3 substituents respectively, selected independently of one another from the group consisting of methyl, ethyl, n-propyl, F, Cl, Br, -OH, $-O-CH_3$ and $-O-C_2H_5$;

$R^{23}$ represents a phenyl radical;
$R^{24}$ represents an alkyl radical selected from the group consisting of methyl, ethyl, n-propyl, isopropyl, n-butyl, isobutyl, sec-butyl and tert-butyl;
$R^{25}$ represents a radical selected from the group consisting of phenyl, naphthyl, furanyl, pyrazinyl and pyrimidinyl, which may be bound by a -($CH_2$) group and/or substituted with 1, 2 or 3 substituents selected independently of one another from the group consisting of methyl, ethyl, n-propyl and isopropyl;
$R^{26}$ represents an alkyl radical selected from the group consisting of methyl, ethyl, n-propyl and isopropyl;
$R^{27}$ represents an alkyl radical selected from the group consisting of methyl, ethyl, isopropyl and n-propyl and
$R^{28}$ represents a phenyl radical;

respectively optionally in the form of one of the pure stereoisomers thereof, in particular enantiomers or diastereomers, the racemates thereof or in the form of a mixture of stereoisomers, in particular of enantiomers and/or diastereomers, in any desired mixture ratio, or in the form of appropriate salts or appropriate solvates respectively.

**16.** Compounds according to one or more of claims 1 to 15 selected from the group consisting of

[1] 2-[(3-chlorobenzoyl)-methyl-amino]-5,6,7,8-tetrahydro-imidazo[1,2-a]pyridine-3-carboxylic acid (2-thiophen-2-yl-ethyl)-amide,
[2] 2-[butyl-(3-chlorobenzoyl)-amino]-5,6,7,8-tetrahydro-imidazo[1,2-a]pyridine-3-carboxylic acid [2-(2,5-dimethoxy-phenyl)-ethyl]-amide,
[3] N-{3-[4-(2-ethyl-phenyl)-piperazine-1-carbonyl]-5,6,7,8-tetrahydro-imidazo[1,2-a]pyridin-2-yl}-N-methyl-benzamide,
[4] 2-(methyl-phenylacetyl-amino)-5,6,7,8-tetrahydro-imidazo[1,2-a]pyridine-3-carboxylic acid [3-(2-methyl-piperidin-1-yl)-propyl]-amide,
[5] 2-[(3-chlorobenzoyl)-methyl-amino]-5,6,7,8-tetrahydro-imidazo[1,2-a]pyridine-3-carboxylic acid (2-phenyl-propyl)-amide,
[6] 2-[(3-chlorobenzoyl)-methyl-amino]-5,6,7,8-tetrahydro-imidazo[1,2-a]pyridine-3-carboxylic acid (2-pyrrolidin-1-yl-ethyl)-amide,
[7] 2-[methyl-(naphthalene-1-carbonyl)-amino]-5,6,7,8-tetrahydro-imidazo[1,2-a]pyridine-3-carboxylic acid ethyl-pyridin-4-ylmethyl-amide,
[8] 2-(benzoyl-methyl-amino)-5,6,7,8-tetrahydro-imidazo[1,2-a]pyridine-3-carboxylic acid (2-thiophen-2-yl-ethyl)-amide,
[9] 2-[butyl-(3-chlorobenzoyl)-amino]-5,6,7,8-tetrahydro-imidazo[1,2-a]pyridine-3-carboxylic acid (3-imidazol-1-yl-propyl)-amide,
[10] 3-chloro-N-{3-[4-(3-chlorophenyl)-piperazine-1-carbonyl]-5,6,7,8-tetrahydro-imidazo[1,2-a]pyridin-2-yl}-N-methyl-benzamide,
[11] 3-chloro-N-methyl-N-[3-(4-phenyl-piperazine-1-carbonyl)-5,6,7,8-tetrahydro-imidazo[1,2-a]pyridin-2-yl]-benzamide,
[12] 2-[butyl-(3,4-difluorobenzoyl)-amino]-5,6,7,8-tetrahydro-imidazo[1,2-a]pyridine-3-carboxylic acid indan-1-ylamide,
[13] N-butyl-3-chloro-N-{3-[4-(2-morpholin-4-yl-ethyl)-piperazine-1-carbonyl]-5,6,7,8-tetrahydro-imidazo[1,2-a]pyridin-2-yl}-benzamide,
[14] 3-chloro-N-methyl-N-{3-[4-(3-trifluoromethyl-phenyl)-piperazine-1-carbonyl]-5,6,7,8-tetrahydro-imidazo[1,2-a]pyridin-2-yl}-benzamide,
[15] 2-[methyl-(naphthalene-1-carbonyl)-amino]-5,6,7,8-tetrahydro-imidazo[1,2-a]pyridine-3-carboxylic acid 3,5-bis-trifluoromethyl-benzylamide,
[16] 2-[butyl-(3,4-difluorobenzoyl)-amino]-5,6,7,8-tetrahydro-imidazo[1,2-a]pyridine-3-carboxylic acid (4-methyl-cyclohexyl)-amide,
[17] 2-(benzoyl-methyl-amino)-5,6,7,8-tetrahydro-imidazo[1,2-a]pyridine-3-carboxylic acid 3-methoxy-benzylamide,
[18] 2-[methyl-(naphthalene-1-carbonyl)-amino]-5,6,7,8-tetrahydro-imidazo[1,2-a]pyridine-3-carboxylic acid (1-p-tolyl-ethyl)-amide,
[19] 2-[butyl-(3-chlorobenzoyl)-amino]-5,6,7,8-tetrahydro-imidazo[1,2-a]pyridine-3-carboxylic acid (1,2-dimethyl-propyl)-amide,
[20] 2-(benzoyl-methyl-amino)-5,6,7,8-tetrahydro-imidazo[1,2-a]pyridine-3-carboxylic acid (4-methyl-cyclohexyl)-amide,
[21] 2-(benzoyl-methyl-amino)-5,6,7,8-tetrahydro-imidazo[1,2-a]pyridine-3-carboxylic acid (2-phenyl-propyl)-amide,

[22] 4-[2-(benzoyl-methyl-amino)-5,6,7,8-tetrahydro-imidazo[1,2-a]pyridine-3-carbonyl]-piperazine-1-carboxylic acid ethyl ester,

[23] 3-chloro-N-methyl-N-(3-{4-[(methyl-phenyl-carbamoyl)-methyl]-piperazine-1-carbonyl}-5,6,7,8-tetrahydro-imidazo[1,2-a]pyridin-2-yl)-benzamide,

[24] 3-chloro-N-{3-[4-(furan-2-carbonyl)-piperazine-1-carbonyl]-5,6,7,8-tetrahydro-imidazo[1,2-a]pyridin-2-yl}-N-methyl-benzamide,

[25] N-methyl-N-[3-(4-p-tolyl-piperazine-1-carbonyl)-5,6,7,8-tetrahydro-imidazo[1,2-a]pyridin-2-yl]-benzamide,

[26] N-butyl-3-chloro-N-{3-[4-(3-phenyl-propyl)-piperazine-1-carbonyl]-5,6,7,8-tetrahydro-imidazo[1,2-a]pyridin-2-yl}-benzamide,

[27] 2-[(3-chlorobenzoyl)-methyl-amino]-5,6,7,8-tetrahydro-imidazo[1,2-a]pyridine-3-carboxylic acid (2,6,6-trimethyl-bicyclo[3.1.1]hept-3-yl)-amide,

[28] 2-[butyl-(3,4-difluorobenzoyl)-amino]-5,6,7,8-tetrahydro-imidazo[1,2-a]pyridine-3-carboxylic acid (3-phenyl-propyl)-amide,

[29] naphthalene-1-carboxylic acid {3-[4-(4-methoxy-phenyl)-piperazine-1-carbonyl]-5,6,7,8-tetrahydro-imidazo[1,2-a]pyridin-2-yl}-methyl-amide,

[30] 2-[methyl-(naphthalene-1-carbonyl)-amino]-5,6,7,8-tetrahydro-imidazo[1,2-a]pyridine-3-carboxylic acid [1-(4-trifluoromethyl-benzyl)-pyrrolidin-3-yl]-amide,

[31] 2-[(3-chlorobenzoyl)-methyl-amino]-5,6,7,8-tetrahydro-imidazo[1,2-a]pyridine-3-carboxylic acid (4-ethyl-phenyl)-amide,

[32] 2-[methyl-(naphthalene-1-carbonyl)-amino]-5,6,7,8-tetrahydro-imidazo[1,2-a]pyridine-3-carboxylic acid (3-methoxy-benzyl)-(tetrahydro-furan-2-ylmethyl)-amide,

[33] 2-[methyl-(naphthalene-1-carbonyl)-amino]-5,6,7,8-tetrahydro-imidazo[1,2-a]pyridine-3-carboxylic acid 2,3-dichloro-benzylamide,

[34] 2-[butyl-(3,4-difluorobenzoyl)-amino]-5,6,7,8-tetrahydro-imidazo[1,2-a]pyridine-3-carboxylic acid (1-benzofuran-2-ylmethyl-pyrrolidin-3-yl)-methyl-amide,

[35] 3-chloro-N-{3-[4-(4-chlorophenyl)-piperazine-1-carbonyl]-5,6,7,8-tetrahydro-imidazo[1,2-a]pyridin-2-yl}-N-methyl-benzamide,

[36] 2-({2-[(3-chlorobenzoyl)-methyl-amino]-5,6,7,8-tetrahydro-imidazo[1,2-a]pyridine-3-carbonyl}-amino)-3-(1H-indol-3-yl)-propionic acid methyl ester,

[37] N-butyl-3-chloro-N-[3-(4-phenylacetyl-piperazine-1-carbonyl)-5,6,7,8-tetrahydro-imidazo[1,2-a]pyridin-2-yl]-benzamide,

[38] 2-({2-[(3-chlorobenzoyl)-methyl-amino]-5,6,7,8-tetrahydro-imidazo[1,2-a]pyridine-3-carbonyl}-amino)-3-methyl-valeric acid-tert-butyl ester,

[39] 2-[butyl-(3-chlorobenzoyl)-amino]-5,6,7,8-tetrahydro-imidazo[1,2-a]pyridine-3-carboxylic acid (1-naphthalen-1-yl-ethyl)-amide,

[40] 2-[methyl-(naphthalene-1-carbonyl)-amino]-5,6,7,8-tetrahydro-imidazo[1,2-a]pyridine-3-carboxylic acid allyl-methyl-amide,

[41] N-butyl-N-[3-(3,6-dihydro-2H-pyridine-1-carbonyl)-5,6,7,8-tetrahydro-imidazo[1,2-a]pyridin-2-yl]-3,4-difluoro-benzamide,

[42] 2-({2-[(3-chlorobenzoyl)-methyl-amino]-5,6,7,8-tetrahydro-imidazo[1,2-a]pyridine-3-carbonyl}-amino)-4-methyl-valeric acid-benzyl ester,

[43] 2-[butyl-(3-chlorobenzoyl)-amino]-5,6,7,8-tetrahydro-imidazo[1,2-a]pyridine-3-carboxylic acid 2-ethoxy-benzylamide,

[44] N-butyl-3-chloro-N-{3-[4-(5-methyl-pyrazine-2-carbonyl)-piperazine-1-carbonyl]-5,6,7,8-tetrahydro-imidazo[1,2-a]pyridin-2-yl}-benzamide,

[45] N-[3-(4-benzo[1,3]dioxol-5-ylmethyl-piperazine-1-carbonyl)-5,6,7,8-tetrahydro-imidazo[1,2-a]pyridin-2-yl]-3-chloro-N-methyl-benzamide,

[46] 2-[methyl-(naphthalene-1-carbonyl)-amino]-5,6,7,8-tetrahydro-imidazo[1,2-a]pyridine-3-carboxylic acid (2,2-diphenyl-ethyl)-amide,

[47] 2-[butyl-(3-chlorobenzoyl)-amino]-5,6,7,8-tetrahydro-imidazo[1,2-a]pyridine-3-carboxylic acid 2,4-difluoro-benzylamide,

[48] N-butyl-3-chloro-N-{3-[4-(2-fluoro-phenyl)-piperazine-1-carbonyl]-5,6,7,8-tetrahydro-imidazo[1,2-a]pyridin-2-yl}-benzamide,

[49] 2-[methyl-(naphthalene-1-carbonyl)-amino]-5,6,7,8-tetrahydro-imidazo[1,2-a]pyridine-3-carboxylic acid (2-p-tolyl-ethyl)-amide,

[50] 2-[methyl-(naphthalene-1-carbonyl)-amino]-5,6,7,8-tetrahydro-imidazo[1,2-a]pyridine-3-carboxylic acid (pyridin-2-ylmethyl)-amide,

[51] 2-[butyl-(3-chlorobenzoyl)-amino]-5,6,7,8-tetrahydro-imidazo[1,2-a]pyridine-3-carboxylic acid 2-trifluoromethyl-benzylamide,
[52] 2-[butyl-(3-chlorobenzoyl)-amino]-5,6,7,8-tetrahydro-imidazo[1,2-a]pyridine-3-carboxylic acid indan-1-ylamide,
[53] 3-chloro-N-methyl-N-[3-(4-quinolin-2-ylmethyl-piperazine-1-carbonyl)-5,6,7,8-tetrahydro-imidazo[1,2-a]pyridin-2-yl]-benzamide,
[54] N-butyl-3,4-difluoro-N-[3-(4-methyl-piperazine-1-carbonyl)-5,6,7,8-tetrahydro-imidazo[1,2-a]pyridin-2-yl]-benzamide,
[55] 2-[(3-chlorobenzoyl)-methyl-amino]-5,6,7,8-tetrahydro-imidazo[1,2-a]pyridine-3-carboxylic acid 2,4-difluoro-benzylamide,
[56] N-[3-(4-benzhydryl-piperazine-1-carbonyl)-5,6,7,8-tetrahydro-imidazo[1,2-a]pyridin-2-yl]-N-butyl-3,4-difluoro-benzamide,
[57] 4-methyl-2-({2-[methyl-(naphthalene-1-carbonyl)-amino]-5,6,7,8-tetrahydro-imidazo[1,2-a]pyridine-3-carbonyl}-amino)-valeric acid-benzyl ester,
[58] 2-[butyl-(3-chlorobenzoyl)-amino]-5,6,7,8-tetrahydro-imidazo[1,2-a]pyridine-3-carboxylic acid (2-pyrrolidin-1-yl-ethyl)-amide,
[59] 2-[(3-chlorobenzoyl)-methyl-amino]-5,6,7,8-tetrahydro-imidazo[1,2-a]pyridine-3-carboxylic acid [1-(2-benzyloxy-benzyl)-pyrrolidin-3-yl]-amide,
[60] 2-[butyl-(3,4-difluorobenzoyl)-amino]-5,6,7,8-tetrahydro-imidazo[1,2-a]pyridine-3-carboxylic acid (pyridin-3-ylmethyl)-amide,
[61] 3-chloro-N-{3-[4-(4-fluorophenyl)-3,6-dihydro-2H-pyridine-1-carbonyl]-5,6,7,8-tetrahydro-imidazo[1,2-a]pyridin-2-yl}-N-methyl-benzamide,
[62] 2-[(3-chlorobenzoyl)-methyl-amino]-5,6,7,8-tetrahydro-imidazo[1,2-a]pyridine-3-carboxylic acid cyclohexylamide, c[63] N-[3-(4-Cycloheptyl-piperazine-1-carbonyl)-5,6,7,8-tetrahydro-imidazo[1,2-a]pyridin-2-yl]-N-methyl-benzamide,
[64] naphthalene-1-carboxylic acid methyl-[3-(4-thiophen-3-ylmethyl-piperazine-1-carbonyl)-5,6,7,8-tetrahydro-imidazo[1,2-a]pyridin-2-yl]-amide,
[65] 2-[butyl-(3-chlorobenzoyl)-amino]-5,6,7,8-tetrahydro-imidazo[1,2-a]pyridine-3-carboxylic acid 2,4-dimethoxy-benzylamide,
[66] 1-[2-(benzoyl-methyl-amino)-5,6,7,8-tetrahydro-imidazo[1,2-a]pyridine-3-carbonyl]-piperidine-4-carboxylic acid ethyl ester,
[67] 2-[(3-chlorobenzoyl)-methyl-amino]-5,6,7,8-tetrahydro-imidazo[1,2-a]pyridine-3-carboxylic acid (2-benzyloxy-cyclohexyl)-amide,
[68] 2-[methyl-(naphthalene-1-carbonyl)-amino]-5,6,7,8-tetrahydro-imidazo[1,2-a]pyridine-3-carboxylic acid [2-(4-phenoxy-phenyl)-ethyl]-amide,
[69] 2-[butyl-(3-chlorobenzoyl)-amino]-5,6,7,8-tetrahydro-imidazo[1,2-a]pyridine-3-carboxylic acid [2-(7-methyl-1H-indol-3-yl)-ethyl]-amide,
[70] naphthalene-1-carboxylic acid methyl-[3-(4-phenyl-piperazine-1-carbonyl)-5,6,7,8-tetrahydro-imidazo[1,2-a]pyridin-2-yl]-amide,
[71] 2-[(3-chloro-benzoyl)-methyl-amino]-5,6,7,8-tetrahydro-imidazo[1,2-a]pyridine-3-carboxylic acid [2-(3-trifluoromethyl-phenyl)-ethyl]-amide,
[72] N-butyl-3-chloro-N-[3-(4-pyridin-2-yl-piperazine-1-carbonyl)-5,6,7,8-tetrahydro-imidazo[1,2-a]pyridin-2-yl]-benzamide,
[73] 2-[methyl-(naphthalene-1-carbonyl)-amino]-5,6,7,8-tetrahydro-imidazo[1,2-a]pyridine-3-carboxylic acid 3-methoxy-benzylamide,
[74] 2-[butyl-(3,4-difluorobenzoyl)-amino]-5,6,7,8-tetrahydro-imidazo[1,2-a]pyridine-3-carboxylic acid 2,4-difluoro-benzylamide,
[75] 3-chloro-N-methyl-N-[3-(4-pyridin-2-yl-piperazine-1-carbonyl)-5,6,7,8-tetrahydro-imidazo[1,2-a]pyridin-2-yl]-benzamide,
[76] 2-[methyl-(naphthalene-1-carbonyl)-amino]-5,6,7,8-tetrahydro-imidazo[1,2-a]pyridine-3-carboxylic acid (1-biphenyl-4-ylmethyl-pyrrolidin-3-yl)-methyl-amide,
[77] 2-[butyl-(3-chlorobenzoyl)-amino]-5,6,7,8-tetrahydro-imidazo[1,2-a]pyridine-3-carboxylic acid (2,3-dihydro-benzo[1,4]dioxin-2-ylmethyl)-amide,
[78] 2-[butyl-(3-chlorobenzoyl)-amino]-5,6,7,8-tetrahydro-imidazo[1,2-a]pyridine-3-carboxylic acid [2-(1H-indol-3-yl)-ethyl]-methyl-amide,
[79] N-butyl-3-chloro-N-{3-[4-(3-trifluoromethyl-phenyl)-piperazine-1-carbonyl]-5,6,7,8-tetrahydro-imidazo[1,2-a]pyridin-2-yl}-benzamide,
[80] 2-(benzoyl-methyl-amino)-5,6,7,8-tetrahydro-imidazo[1,2-a]pyridine-3-carboxylic acid [2-(1H-indol-3-yl)-ethyl]-methyl-amide,

[81] 3-chloro-N-{3-[4-hydroxy-4-(3-trifluoromethyl-phenyl)-piperidine-1-carbonyl]-5,6,7,8-tetrahydro-imidazo [1,2-a]pyridin-2-yl}-N-methyl-benzamide,

[82] 2-[butyl-(3,4-difluorobenzoyl)-amino]-5,6,7,8-tetrahydro-imidazo[1,2-a]pyridine-3-carboxylic acid (1-methoxymethyl-2-phenyl-ethyl)-amide,

[83] 2-[(3-chlorobenzoyl)-methyl-amino]-5,6,7,8-tetrahydro-imidazo[1,2-a]pyridine-3-carboxylic acid [1-(2,6-dichloro-benzyl)-pyrrolidin-3-yl]-amide,

[84] 2-[(3-chlorobenzoyl)-methyl-amino]-5,6,7,8-tetrahydro-imidazo[1,2-a]pyridine-3-carboxylic acid [1-(2-bromo-4,5-dimethoxy-benzyl)-pyrrolidin-3-yl]-amide,

[85] 2-(benzoyl-methyl-amino)-5,6,7,8-tetrahydro-imidazo[1,2-a]pyridine-3-carboxylic acid benzo[1,3]dioxol-5-ylamide,

[86] 2-[butyl-(3-chlorobenzoyl)-amino]-5,6,7,8-tetrahydro-imidazo[1,2-a]pyridine-3-carboxylic acid (1-methoxymethyl-2-phenyl-ethyl)-amide,

[87] 2-[butyl-(3-chlorobenzoyl)-amino]-5,6,7,8-tetrahydro-imidazo[1,2-a]pyridine-3-carboxylic acid [1-(2,6-dichloro-benzyl)-pyrrolidin-3-yl]-methyl-amide,

[88] 2-[butyl-(3,4-difluorobenzoyl)-amino]-5,6,7,8-tetrahydro-imidazo[1,2-a]pyridine-3-carboxylic acid 2-trifluoromethoxy-benzylamide,

[89] N-butyl-3,4-difluoro-N-{3-[4-(isopropylcarbamoyl-methyl)-piperazine-1-carbonyl]-5,6,7,8-tetrahydro-imidazo[1,2-a]pyridin-2-yl}-benzamide,

[90] 2-[(3-Chloro-benzoyl)-methyl-amino]-5,6,7,8-tetrahydro-imidazo[1,2-a]pyridine-3-carboxylic acid (1-methyl-3-phenyl-propyl)-amide,

[91] 2-({2-[butyl-(3-chlorobenzoyl)-amino]-5,6,7,8-tetrahydro-imidazo[1,2-a]pyridine-3-carbonyl}-amino)-3-methyl-ethyl butyrate,

[92] 2-[(3-chloro-benzoyl)-methyl-amino]-5,6,7,8-tetrahydro-imidazo[1,2-a]pyridine-3-carboxylic acid (thiophen-2-ylmethyl)-amide,

[93] 2-[butyl-(3,4-difluorobenzoyl)-amino]-5,6,7,8-tetrahydro-imidazo[1,2-a]pyridine-3-carboxylic acid (2-methyl-cyclohexyl)-amide,

[94] 2-(benzoyl-methyl-amino)-5,6,7,8-tetrahydro-imidazo[1,2-a]pyridine-3-carboxylic acid benzyl-methyl-amide,

[95] 2-[butyl-(3-chlorobenzoyl)-amino]-5,6,7,8-tetrahydro-imidazo[1,2-a]pyridine-3-carboxylic acid [2-(2,6-dichlor-benzylsulphanyl)-ethyl]-amide,

[96] 2-[butyl-(3,4-difluorobenzoyl)-amino]-5,6,7,8-tetrahydro-imidazo[1,2-a]pyridine-3-carboxylic acid (2,3-dihydro-benzo[1,4]dioxin-2-ylmethyl)-amide,

[97] 2-[(3-chlorobenzoyl)-methyl-amino]-5,6,7,8-tetrahydro-imidazo[1,2-a]pyridine-3-carboxylic acid 4-trifluoromethyl-benzylamide,

[98] N-[3-(4-benzyl-piperidine-1-carbonyl)-5,6,7,8-tetrahydro-imidazo[1,2-a]pyridin-2-yl]-N-methyl-benzamide,

[99] 2-[methyl-(naphthalene-1-carbonyl)-amino]-5,6,7,8-tetrahydro-imidazo[1,2-a]pyridine-3-carboxylic acid 2-trifluoromethoxy-benzylamide,

[100] 3-chloro-N-{3-[4-(2,5-dimethyl-phenyl)-piperazine-l-carbonyl]-5,6,7,8-tetrahydro-imidazo[1,2-a]pyridin-2-yl}-N-methyl-benzamide,

[101] 2-(benzoyl-methyl-amino)-5,6,7,8-tetrahydro-imidazo[1,2-a]pyridine-3-carboxylic acid [1-(2,6-dichloro-benzyl)-pyrrolidin-3-yl]-amide,

[102] 2-[butyl-(3,4-difluorobenzoyl)-amino]-5,6,7,8-tetrahydro-imidazo[1,2-a]pyridine-3-carboxylic acid 2-chloro-6-methyl-benzylamide,

[103] N-butyl-3-chloro-N-[3-(3,5-dimethyl-piperidine-1-carbonyl)-5,6,7,8-tetrahydro-imidazo[1,2-a]pyridin-2-yl]-benzamide,

[104] 2-({2-[(3-chlorobenzoyl)-methyl-amino]-5,6,7,8-tetrahydro-imidazo[1,2-a]pyridine-3-carbonyl}-amino)-3,3-dimethyl-butyric acid tert-butylester,

[105] 2-[methyl-(naphthalene-1-carbonyl)-amino]-5,6,7,8-tetrahydro-imidazo[1,2-a]pyridine-3-carboxylic acid (1-cyclohexyl-ethyl)-amide,

[106] 3-chloro-N-methyl-N-[3-(4-phenethyl-piperazine-1-carbonyl)-5,6,7,8-tetrahydro-imidazo[1,2-a]pyridin-2-yl]-benzamide,

[107] 2-[butyl-(3-chlorobenzoyl)-amino]-5,6,7,8-tetrahydro-imidazo[1,2-a]pyridine-3-carboxylic acid (2-phenoxy-ethyl)-amide,

[108] naphthalene-1-carboxylic acid [3-(4-benzofuran-2-ylmethyl-piperazine-1-carbonyl)-5,6,7,8-tetrahydro-imidazo[1,2-a]pyridin-2-yl]-methyl-amide,

[109] N-[3-([1,4']bipiperidinyl-1'-carbonyl)-5,6,7,8-tetrahydro-imidazo[1,2-a]pyridin-2-yl]-N-butyl-3-chloro-benzamide,

[110] N-butyl-3-chloro-N-[3-(8-fluoro-1,3,4,5-tetrahydro-pyrido[4,3-b]indol-2-carbonyl)-5,6,7,8-tetrahydro-imidazo[1,2-a]pyridin-2-yl]-benzamide,
[111] 2-[methyl-(naphthalene-1-carbonyl)-amino]-5,6,7,8-tetrahydro-imidazo[1,2-a]pyridine-3-carboxylic acid 4-dimethylamino-benzylamide,
[112] 2-[methyl-(naphthalene-1-carbonyl)-amino]-5,6,7,8-tetrahydro-imidazo[1,2-a]pyridine-3-carboxylic acid (1-methyl-3-phenyl-propyl)-amide,
[113] 2-[butyl-(3-chlorobenzoyl)-amino]-5,6,7,8-tetrahydro-imidazo[1,2-a]pyridine-3-carboxylic acid 3-methyl-benzylamide,
[114] 2-[(3-chlorobenzoyl)-methyl-amino]-5,6,7,8-tetrahydro-imidazo[1,2-a]pyridine-3-carboxylic acid (2,3-dihydro-benzo[1,4]dioxin-6-yl)-amide,
[115] naphthalene-1-carboxylic acid methyl-[3-(4-quinolin-2-ylmethyl-piperazine-1-carbonyl)-5,6,7,8-tetrahydro-imidazo[1,2-a]pyridin-2-yl]-amide,
[116] 2-[butyl-(3,4-difluorobenzoyl)-amino]-5,6,7,8-tetrahydro-imidazo[1,2-a]pyridine-3-carboxylic acid (2-cyano-ethyl)-methyl-amide,
[117] 2-(methyl-phenylacetyl-amino)-5,6,7,8-tetrahydro-imidazo[1,2-a]pyridine-3-carboxylic acid (2-p-tolyl-ethyl)-amide,
[118] 2-[butyl-(3,4-difluorobenzoyl)-amino]-5,6,7,8-tetrahydro-imidazo[1,2-a]pyridine-3-carboxylic acid [1-(4-fluoro-phenyl)-ethyl]-amide,
[119] N-butyl-N-{3-[4-(5-chloro-2-methyl-phenyl)-piperazine-1-carbonyl]-5,6,7,8-tetrahydro-imidazo[1,2-a]pyridin-2-yl}-3,4-difluoro-benzamide,
[120] 2-[butyl-(3-chlorobenzoyl)-amino]-5,6,7,8-tetrahydro-imidazo[1,2-a]pyridine-3-carboxylic acid cyclohexylamide,
[121] 2-[(3-chlorobenzoyl)-methyl-amino]-5,6,7,8-tetrahydro-imidazo[1,2-a]pyridine-3-carboxylic acid (benzo[1,3]dioxol-5-ylmethyl)-amide,
[122] 2-[butyl-(3,4-difluorobenzoyl)-amino]-5,6,7,8-tetrahydro-imidazo[1,2-a]pyridine-3-carboxylic acid 3-methoxy-benzylamide,
[123] 3-tert-butoxy-2-({2-[(3-chlorobenzoyl)-methyl-amino]-5,6,7,8-tetrahydro-imidazo[1,2-a]pyridine-3-carbonyl}-amino)-butyric acid methyl ester,
[124] 2-[butyl-(3-chlorobenzoyl)-amino]-5,6,7,8-tetrahydro-imidazo[1,2-a]pyridine-3-carboxylic acid (2-benzyloxy-cyclohexyl)-amide,
[125] N-{3-[4-(4-fluoro-phenyl)-piperazine-1-carbonyl]-5,6,7,8-tetrahydro-imidazo[1,2-a]pyridin-2-yl}-N-methyl-benzamide,
[126] 2-(benzoyl-methyl-amino)-5,6,7,8-tetrahydro-imidazo[1,2-a]pyridine-3-carboxylic acid (naphthalen-2-ylcarbamoylmethyl)-amide,
[127] 2-[butyl-(3-chlorobenzoyl)-amino]-5,6,7,8-tetrahydro-imidazo[1,2-a]pyridine-3-carboxylic acid 4-trifluoromethoxy-benzylamide,
[128] 2-[(3-chlorobenzoyl)-methyl-amino]-5,6,7,8-tetrahydro-imidazo[1,2-a]pyridine-3-carboxylic acid (3,3-diphenyl-propyl)-amide,
[129] 2-[(3-chlorobenzoyl)-methyl-amino]-5,6,7,8-tetrahydro-imidazo[1,2-a]pyridine-3-carboxylic acid (4-benzyloxy-phenyl)-amide,
[130] N-{3-[4-(5-bromo-2-ethoxy-benzyl)-piperazine-1-carbonyl]-5,6,7,8-tetrahydro-imidazo[1,2-a]pyridin-2-yl}-N-methyl-2-phenyl-acetamide,
[131] N-[3-(3,4-dihydro-1H-isoquinolin-2-carbonyl)-5,6,7,8-tetrahydro-imidazo[1,2-a]pyridin-2-yl]-N-methyl-benzamide,
[132] 2-[butyl-(3-chlorobenzoyl)-amino]-5,6,7,8-tetrahydro-imidazo[1,2-a]pyridine-3-carboxylic acid (1-phenyl-propyl)-amide,
[133] 2-[butyl-(3-chlorobenzoyl)-amino]-5,6,7,8-tetrahydro-imidazo[1,2-a]pyridine-3-carboxylic acid 2,3-dimethyl-benzylamide,
[134] 2-[butyl-(3-chlorobenzoyl)-amino]-5,6,7,8-tetrahydro-imidazo[1,2-a]pyridine-3-carboxylic acid [2-(4-phenoxy-phenyl)-ethyl]-amide,
[135] N-{3-[4-(4-ethoxy-phenyl)-piperazine-1-carbonyl]-5,6,7,8-tetrahydro-imidazo[1,2-a]pyridin-2-yl}-N-methyl-2-phenyl-acetamide,
[136] naphthalene-1-carboxylic acid {3-[4-(2-ethyl-phenyl)-piperazine-1-carbonyl]-5,6,7,8-tetrahydro-imidazo[1,2-a]pyridin-2-yl}-methyl-amide,
[137] N-[3-(4-benzo[1,3]dioxol-5-ylmethyl-piperazine-1-carbonyl)-5,6,7,8-tetrahydro-imidazo[1,2-a]pyridin-2-yl]-N-butyl-3,4-difluoro-benzamide,
[138] N-butyl-N-{3-[4-(2,5-dimethyl-phenyl)-piperazine-1-carbonyl]-5,6,7,8-tetrahydro-imidazo[1,2-a]pyridin-2-yl}-3,4-difluoro-benzamide,

[139] 2-[methyl-(naphthalene-1-carbonyl)-amino]-5,6,7,8-tetrahydro-imidazo[1,2-a]pyridine-3-carboxylic acid [(4-chloro-phenyl)-phenyl-methyl]-amide,
[140] N-butyl-3-chloro-N-{3-[4-(4-chloro-benzyl)-piperazine-1-carbonyl]-5,6,7,8-tetrahydro-imidazo[1,2-a]pyridin-2-yl}-benzamide,
[141] 2-[butyl-(3-chlorobenzoyl)-amino]-5,6,7,8-tetrahydro-imidazo[1,2-a]pyridine-3-carboxylic acid (1-naphthalen-2-yl-ethyl)-amide,
[142] 2-(methyl-phenylacetyl-amino)-5,6,7,8-tetrahydro-imidazo[1,2-a]pyridine-3-carboxylic acid 2-fluoro-benzylamide,
[143] N-[3-(1,4-dioxa-8-aza-spiro[4.5]decane-8-carbonyl)-5,6,7,8-tetrahydro-imidazo[1,2-a]pyridin-2-yl]-N-methyl-2-phenyl-acetamide,
[144] naphthalene-1-carboxylic acid methyl-[3-(1,3,4,9-tetrahydro-b-carboline-2-carbonyl)-5,6,7,8-tetrahydro-imidazo[1,2-a]pyridin-2-yl]-amide,
[145] 2-[butyl-(3-chlorobenzoyl)-amino]-5,6,7,8-tetrahydro-imidazo[1,2-a]pyridine-3-carboxylic acid (1-methyl-3-phenyl-propyl)-amide,
[146] 3-chloro-N-methyl-N-{3-[4-(2,4,6-trimethoxy-benzyl)-piperazine-1-carbonyl]-5,6,7,8-tetrahydro-imidazo [1,2-a]pyridin-2-yl}-benzamide,
[147] 2-[butyl-(3-chlorobenzoyl)-amino]-5,6,7,8-tetrahydro-imidazo[1,2-a]pyridine-3-carboxylic acid benzhydryl-amide,
[148] naphthalene-1-carboxylic acid {3-[4-(2-chloro-phenyl)-piperazine-1-carbonyl]-5,6,7,8-tetrahydro-imidazo [1,2-a]pyridin-2-yl}-methyl-amide,
[149] 2-[butyl-(3-chlorobenzoyl)-amino]-5,6,7,8-tetrahydro-imidazo[1,2-a]pyridine-3-carboxylic acid (thiophen-2-ylmethyl)-amide,
[150] N-butyl-N-{3-[4-(4-chlorobenzyl)-piperazine-1-carbonyl]-5,6,7,8-tetrahydro-imidazo[1,2-a]pyridin-2-yl}-3,4-difluoro-benzamide,
[151] 2-(benzoyl-methyl-amino)-5,6,7,8-tetrahydro-imidazo[1,2-a]pyridine-3-carbonic acid (3-phenyl-propyl)-amide,
[152] 2-[butyl-(3-chlorobenzoyl)-amino]-5,6,7,8-tetrahydro-imidazo[1,2-a]pyridine-3-carboxylic acid (4-tert-butyl-phenyl)-amide,
[153] 2-[butyl-(3-chlorobenzoyl)-amino]-5,6,7,8-tetrahydro-imidazo[1,2-a]pyridine-3-carboxylic acid (4-ethyl-phenyl)-amide,
[154] 2-[methyl-(naphthalene-1-carbonyl)-amino]-5,6,7,8-tetrahydro-imidazo[1,2-a]pyridine-3-carboxylic acid cyclohexylamide,
[155] 2-[methyl-(naphthalene-1-carbonyl)-amino]-5,6,7,8-tetrahydro-imidazo[1,2-a]pyridine-3-carboxylic acid [3-(methyl-phenyl-amino)-propyl]-amide ,
[156] 2-(methyl-phenylacetyl-amino)-5,6,7,8-tetrahydro-imidazo[1,2-a]pyridine-3-carboxylic acid [2-(3-trifluormethyl-phenyl)-ethyl]-amide,
[157] 2-(benzoyl-methyl-amino)-5,6,7,8-tetrahydro-imidazo[1,2-a]pyridine-3-carboxylic acid 4-chloro-benzylamide,
[158] 2-[butyl-(3-chlorobenzoyl)-amino]-5,6,7,8-tetrahydro-imidazo[1,2-a]pyridine-3-carboxylic acid 3-fluoro-benzylamide,
[159] 2-(methyl-phenylacetyl-amino)-5,6,7,8-tetrahydro-imidazo[1,2-a]pyridine-3-carboxylic acid p-tolylamide,
[160] 2-(benzoyl-methyl-amino)-5,6,7,8-tetrahydro-imidazo[1,2-a]pyridine-3-carboxylic acid (2-phenoxy-ethyl)-amide,
[161] 2-[butyl-(3,4-difluorobenzoyl)-amino]-5,6,7,8-tetrahydro-imidazo[1,2-a]pyridine-3-carboxylic acid (2-benzyloxy-cyclohexyl)-amide,
[162] naphthalene-1-carboxylic acid {3-[4-(4-fluoro-phenyl)-piperazine-1-carbonyl]-5,6,7,8-tetrahydro-imidazo [1,2-a]pyridin-2-yl}-methyl-amide,
[163] 2-[methyl-(naphthalene-1-carbonyl)-amino]-5,6,7,8-tetrahydro-imidazo[1,2-a]pyridine-3-carboxylic acid [2-(4-chloro-phenyl)-propyl]-amide,
[164] N-butyl-3,4-difluoro-N-[3-(thiomorpholine-4-carbonyl)-5,6,7,8-tetrahydro-imidazo[1,2-a]pyridin-2-yl]-benzamide,
[165] 2-[(3-chlorobenzoyl)-methyl-amino]-5,6,7,8-tetrahydro-imidazo[1,2-a]pyridine-3-carboxylic acid (1-adamantan-1-yl-ethyl)-amide,
[166] 2-(benzoyl-methyl-amino)-5,6,7,8-tetrahydro-imidazo[1,2-a]pyridine-3-carboxylic acid [(4-chloro-phenyl)-phenyl-methyl]-amide,
[167] N-methyl-N-{3-[4-(4-trifluoromethyl-phenyl)-piperazine-1-carbonyl]-5,6,7,8-tetrahydro-imidazo[1,2-a]pyridin-2-yl}-benzamide,
[168] 2-(methyl-phenylacetyl-amino)-5,6,7,8-tetrahydro-imidazo[1,2-a]pyridine-3-carboxylic acid (1-biphenyl-4-ylmethyl-pyrrolidin-3-yl)-methyl-amide,
[169] N-[3-(4-benzoyl-piperidine-1-carbonyl)-5,6,7,8-tetrahydro-imidazo[1,2-a]pyridin-2-yl]-3-chloro-N-methyl-benzamide,
[170] 2-[methyl-(naphthalene-1-carbonyl)-amino]-5,6,7,8-tetrahydro-imidazo[1,2-a]pyridine-3-carboxylic acid (4-tert-butyl-phenyl)-amide,
[171] 2-[butyl-(3,4-difluoro-benzoyl)-amino]-5,6,7,8-tetrahydro-imidazo[1,2-a]pyridine-3-carboxylic acid (3,3-dimethyl-butyl)-amide,
[172] 2-[butyl-(3-chlorobenzoyl)-amino]-5,6,7,8-tetrahydro-imidazo[1,2-a]pyridine-3-carboxylic acid [1-(2-bromo-4,5-dimethoxy-benzyl)-pyrrolidin-3-yl]-amide,
[173] naphthalene-1-carboxylic acid methyl-{3-[4-(3-phenyl-allyl)-piperazine-1-carbonyl]-5,6,7,8-tetrahydro-imidazo[1,2-a]pyridin-2-yl}-amide,
[174] naphthalene-1-carboxylic acid methyl-[3-(4-phenethyl-piperazine-1-carbonyl)-5,6,7,8-tetrahydro-imidazo [1,2-a]pyridin-2-yl]-amide,
[175] N-butyl-3-chloro-N-{3-[4-hydroxy-4-(3-trifluormethyl-phenyl)-piperidine-1-carbonyl]-5,6,7,8-tetrahydro-imidazo[1,2-a]pyridin-2-yl}-benzamide,
[176] N-methyl-2-phenyl-N-{3-[4-(3-phenyl-propyl)-piperazine-1-carbonyl]-5,6,7,8-tetrahydro-imidazo[1,2-a] pyridin-2-yl}-acetamide,
[177] 2-(benzoyl-methyl-amino)-5,6,7,8-tetrahydro-imidazo[1,2-a]pyridine-3-carboxylic acid 3-fluoro-benzylamide,
[178] N-butyl-N-{3-[4-(2-chlorophenyl)-piperazine-l-carbonyl]-5,6,7,8-tetrahydro-imidazo[1,2-a]pyridin-2-yl}-3,4-difluoro-benzamide,
[179] 2-[methyl-(naphthalene-1-carbonyl)-amino]-5,6,7,8-tetrahydro-imidazo[1,2-a]pyridine-3-carboxylic acid benzo[1,3]dioxol-5-ylamide,
[180] 2-[butyl-(3,4-difluorobenzoyl)-amino]-5,6,7,8-tetrahydro-imidazo[1,2-a]pyridine-3-carboxylic acid (3,3-diphenyl-propyl)-amide,
[181] 2-[butyl-(3-chlorobenzoyl)-amino]-5,6,7,8-tetrahydro-imidazo[1,2-a]pyridine-3-carboxylic acid (2,3-dihydro-benzo[1,4]dioxin-6-yl)-amide,
[182] 2-(benzoyl-methyl-amino)-5,6,7,8-tetrahydro-imidazo[1,2-a]pyridin-3-carboxylic acid [2-(3,4-dimethoxy-phenyl)-ethyl]-amide,
[183] naphthalene-1-carboxylic acid [3-(4-benzoyl-piperidine-1-carbonyl)-5,6,7,8-tetrahydro-imidazo[1,2-a]pyridin-2-yl]-methyl-amide,
[184] 4-methyl-2-({2-[methyl-(naphthalene-1-carbonyl)-amino]-5,6,7,8-tetrahydro-imidazo[1,2-a]pyridine-3-carbonyl}-amino)-valeric acid tert-butylester,
[185] 2-[methyl-(naphthalene-1-carbonyl)-amino]-5,6,7,8-tetrahydro-imidazo[1,2-a]pyridine-3-carboxylic acid (4-phenoxy-phenyl)-amide,
[186] N-methyl-2-phenyl-N-[3-(4-phenyl-piperazine-1-carbonyl)-5,6,7,8-tetrahydro-imidazo[1,2-a]pyridin-2-yl]-acetamide,
[187] N-butyl-3,4-difluoro-N-{3-[4-(5-trifluoromethyl-pyridin-2-yl)-piperazine-1-carbonyl]-5,6,7,8-tetrahydro-imidazo[1,2-a]pyridin-2-yl}-benzamide,
[188] 2-(benzoyl-methyl-amino)-5,6,7,8-tetrahydro-imidazo[1,2-a]pyridine-3-carboxylic acid 2,4-dichloro-6-methyl-benzylamide,
[189] [4-({2-[butyl-(3-chlorobenzoyl)-amino]-5,6,7,8-tetrahydro-imidazo[1,2-a]pyridine-3-carbonyl}-amino)-phenyl]-carbamic acid tert-butyl ester,
[190] N-methyl-2-phenyl-N-[3-(2-pyrrolidin-1-ylmethyl-pyrrolidine-1-carbonyl)-5,6,7,8-tetrahydro-imidazo[1,2-a]pyridin-2-yl]-acetamide and
[191] 4-[2-(methyl-phenylacetyl-amino)-5,6,7,8-tetrahydro-imidazo[1,2-a]pyridine-3-carbonyl]-piperazine-1-carboxylic acid tert-butyl ester,

optionally in the form of one of the pure stereoisomers thereof, in particular enantiomers or diastereomers, the racemates thereof or in the form of a mixture of stereoisomers, in particular of enantiomers and/or diastereomers, in any desired mixture ratio, or in the form of appropriate salts or appropriate solvates respectively.

**17.** Compounds according to one or more of claims 1 to 16, **characterised in that** the compounds inhibit the $Ca^{2+}$ ion influx in the dorsal root ganglia of rats by at least 30 %, preferably by at least 50 %, particularly preferably by 70 %, more particularly preferably by 80 % and most particularly preferably by 90 % in the FLIPR assay at a concentration of 10 $\mu$M, in contrast to the maximum level of inhibition of the of the $Ca^{2+}$ ion influx of capsaicin at a concentration of 10 $\mu$M.

**18.** Method for preparing substituted 5,6,7,8-tetrahydro-imidazo[1,2-a]pyridin-2-ylamine compounds of the general formula I according to one or more of claims 1 to 17, **characterised in that** at least one compound of the general formula II,

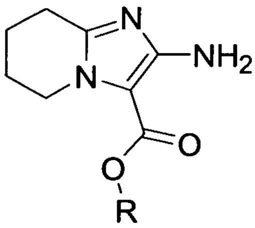

II

in which R represents a linear or branched $C_{1-6}$ alkyl radical, is converted, in a reaction medium in the presence of a reducing agent, optionally in the presence of an organic acid, preferably in the presence of acetic acid, with at least one compound of the general formula $R^3$-C(=O)-H, in which $R^3$ is defined according to one or more of claims 1 to 16 to a compound of the general formula III,

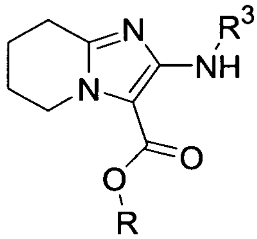

III

in which R and R.$^3$ are as defined hereinbefore, and said compound is optionally purified and/or isolated and at least one compound of the general formula III is converted, in a reaction medium, optionally in the presence of a base with at least one compound of the general formula $R^4$-C(=O)-X, in which $R^4$ is defined according to one or more of claims 1 to 16 and X represents a leaving group, preferably a halogen radical, or in a reaction medium in the presence of at least one coupling reagent, optionally in the presence of a base with a compound of the general formula $R^4$-C(=O)-OH, in which $R^4$ is defined according to one or more of claims 1 to 16, to a compound of the general formula IV,

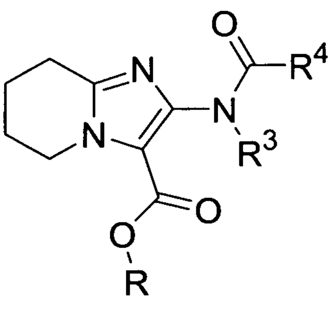

IV

in which R, $R^3$ and $R^4$ are as defined hereinbefore and $R^3$ is not hydrogen, and said compound is optionally purified and/or isolated

or

at least one compound of the general formula II, in which R is as defined hereinbefore, is converted, in a reaction medium, optionally in the presence of a base with at least one compound of the general formula $R^4$-C(=O)-X, in which $R^4$ is defined as hereinbefore and X represents a leaving group, preferably a halogen radical, or in a reaction medium in the presence of at least one coupling reagent, optionally in the presence of a base with a compound of the general formula $R^4$-C(=O)-OH, in which $R^4$ is defined as hereinbefore, to a compound of the general formula V,

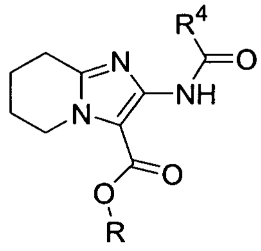

V

in which R and $R^4$ are as defined hereinbefore, and said compound is optionally purified and/or isolated and at least one compound of the general formula V is converted, in a reaction medium in the presence of at least one base, preferably a metal hydride salt, with at least one compound of the general formula $R^3$-X, in which $R^3$ is as defined hereinbefore and is not hydrogen and X represents a leaving group, preferably a halogen radical, to a compound of the general formula IV, in which R, $R^3$ and $R^4$ are as defined hereinbefore and $R^3$ is not hydrogen, and said compound is optionally purified and/or isolated
and at least one compound of the general formula IV is converted, in a reaction medium in the presence of at least one base, preferably in the presence of at least one metal hydroxide salt, to a compound of the general formula VI,

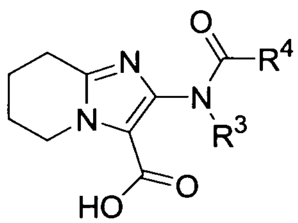

VI

in which $R^3$ and $R^4$ are as defined hereinbefore and $R^3$ is not hydrogen, and said compound is optionally purified and/or isolated and at least one compound of the general formula VI is converted, in a reaction medium in the presence of at least one coupling reagent, optionally in the presence of at least one base with at least one compound of the general formula $HNR^1R^2$, in which $R^1$ is as defined hereinbefore and $R^2$ represents hydrogen, to a compound of the general formula I,

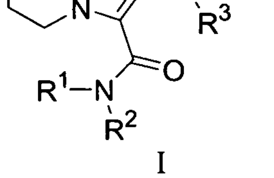

I

in which $R^1$, $R^3$ und $R^4$ are as defined hereinbefore, $R^3$ is not hydrogen and $R^2$ represents hydrogen, and said compound is purified and/or isolated

and optionally at least one compound of the general formula I, in which $R^1$, $R^3$ and $R^4$ are as defined hereinbefore, $R^3$ is not hydrogen and $R^2$ represents hydrogen, is converted, in a reaction medium in the presence of at least one base, preferably a metal hydride salt, with at least one compound of the general formula $R^2$-X, in which $R^2$ is as defined hereinbefore and is not hydrogen and X represents a leaving group, preferably a halogen radical, to a compound of the general formula I,
in which $R^1$, $R^2$, $R^3$ and $R^4$ are as defined hereinbefore and $R^2$ and $R^3$ are not hydrogen, and said compound is purified and/or isolated
or
at least one compound of the general formula VI is converted, in a reaction medium in the presence of at least one coupling reagent, optionally in the presence of at least one base, with at least one compound of the general formula $HNR^1R^2$, in which $R^1$ and $R^2$ are as defined hereinbefore and are not hydrogen, to a compound of the general formula I, in which $R^1$, $R^2$, $R^3$ and $R^4$ are as defined hereinbefore and $R^1$, $R^2$ and $R^3$ are not hydrogen, and said compound is optionally purified and/or isolated.

**19.** Method for the preparation of substituted 5,6,7,8-tetrahydro-imidazo[1,2-a]pyridin-2-ylamine compounds of the general formula I according to one or more of claims 1 to 17, **characterised in that** at least one compound of the general formula II,

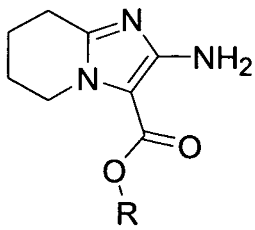

II

in which R represents a linear or branched $C_{1-6}$ alkyl radical, is converted, in a reaction medium, optionally in the presence of at least one base with at least one compound of the general formula $R^4$-C(=O)-X, in which $R^4$ is defined according to one or more of claims 1 to 16 and X represents a leaving group, preferably a halogen radical, or in a reaction medium in the presence of at least one coupling reagent, optionally in the presence of at least one base with a compound of the general formula $R^4$-C(=O)-OH, in which $R^4$ is defined according to one or more of claims 1 to 16, to a compound of the general formula IV,

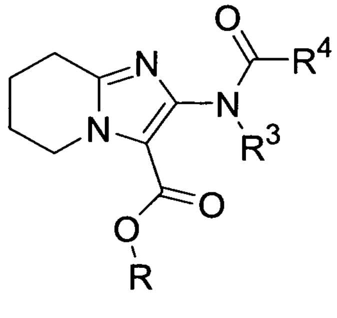

VI

in which R and $R^4$ are as defined hereinbefore and $R^3$ is hydrogen, and said compound is optionally purified and/or isolated,
and at least one compound of the general formula IV is converted, in a reaction medium in the presence of at least one base, preferably in the presence of at least one metal hydroxide salt, to a compound of the general formula VI,

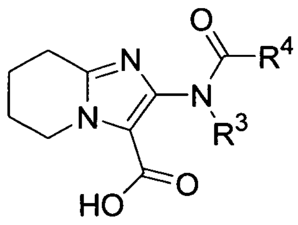

IV

in which $R^4$ is as defined hereinbefore and $R^3$ is hydrogen, and said compound is optionally purified and/or isolated at least one compound of the general formula VI is converted, in a reaction medium in the presence of at least one coupling reagent, optionally in the presence of at least one base, with at least one compound of the general formula $HNR^1R^2$, in which $R^1$ and $R^2$ are defined according to one or more of claims 1 to 16, to a compound of the general formula I,

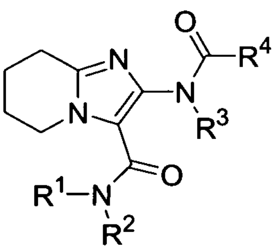

I

in which $R^1$, $R^2$ and $R^4$ are as defined hereinbefore and $R^3$ is hydrogen, and said compound is optionally purified and/or isolated.

**20.** Pharmaceutical drug comprising at least one compound according to one or more of claims 1 to 17 and optionally one or more physiologically compatible additives.

**21.** Pharmaceutical drug according to claim 20 for prophylaxis and/or the treatment of pain, preferably for pain selected from the group consisting of acute pain, chronic pain and neuropathic pain.

**22.** Pharmaceutical drug according to claim 20 for prophylaxis and/or the treatment of one or more diseases selected from the group consisting of migraines; depression; urinary incontinence; cough; neurodegenerative diseases, preferably selected from the group consisting of Parkinson's disease, Huntington's disease, Alzheimer's disease and multiple sclerosis; eating-related disorders, preferably selected from the group consisting of bulimia, anorexia, obesity and cachexia; state of anxiety; cognitive dysfunction, preferably memory impairment; cognitive deficiencies (attention deficit syndrome, ADS); epilepsy; diarrhoea and pruritis; or for prophylaxis and/or the treatment of alcohol and/or drug and/or medicine abuse and/or addiction to alcohol and/or drugs and/or medicines, preferably for prophylaxis and/or for reducing withdrawal symptoms for those with addictions to alcohol and/or drugs and/or medicines; for prophylaxis and/or reduction in the development of tolerance in relation to medicines, in particular opioid-based medicines; for regulation of food intake; for modulation of movement; for regulation of the cardiovascular system; as a local anaesthetic; for increasing vigilance; for increasing libido; for diuresis and/or antinatriuresis.

**23.** Use of at least one compound according to one or more of claims 1 to 17 for preparing a pharmaceutical drug for prophylaxis and/or the treatment of pain, preferably for pain selected from the group consisting of acute pain, chronic pain and neuropathic pain.

**24.** Use of at least one compound according to one or more of claims 1 to 17 for preparing a pharmaceutical drug for prophylaxis and/or the treatment of one or more diseases selected from the group consisting of migraines; depression; urinary incontinence; cough; neurodegenerative diseases, preferably selected from the group consisting of Parkinson's disease, Huntington's disease, Alzheimer's disease and multiple sclerosis; eating-related disorders, preferably selected from the group consisting of bulimia, anorexia, obesity and cachexia; state of anxiety; cognitive dysfunction, preferably memory impairment; cognitive deficiencies (attention deficit syndrome, ADS); epilepsy; diarrhoea and pruritis; for prophylaxis and/or the treatment of alcohol and/or drug and/or medicine abuse and/or addiction to alcohol and/or drugs and/or medicines, preferably for prophylaxis and/or for reducing withdrawal symptoms for those with addictions to alcohol and/or drugs and/or medicines; for prophylaxis and/or reduction in the development of tolerance in relation to medicines, in particular opioid-based medicines; for regulation of food intake; for modulation of movement; for regulation of the cardiovascular system; as a local anaesthetic; for increasing vigilance; for increasing libido; for diuresis and/or antinatriuresis.

## Revendications

**1.** Composés de 5,6,7,8-tétrahydro-imidazo[1,2-a]pyridin-2-ylamine substitués de formule générale I,

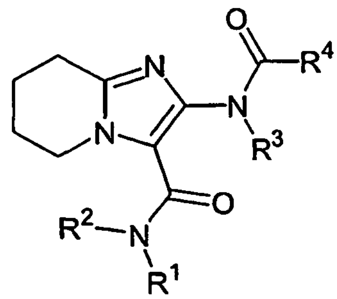

I

dans laquelle

$R^1$ et $R^2$ représentent, indépendamment l'un de l'autre, à chaque fois
un radical hydrogène ;
$-C(=O)-OR^5$;
$-(CHR^6)-(CH_2)_m-C(=O)-OR^7$ avec m = 0, 1, 2, 3, 4 ou 5 ;
$-C(=O)-R^8$ ;
$-(CH_2)_n-C(=O)-R^9$ avec n = 1, 2, 3, 4 ou 5 ;
$-C(=O)-NH-R^{10}$ ;
$-(CH_2)_o-C(=O)-NHR^{11}$ avec o = 0, 1, 2, 3, 4 ou 5 ;
$-C(=O)-NR^{12}R^{13}$ ;
$-(CH_2)_p-C(=O)-NR^{14}R^{15}$ avec p = 1, 2, 3, 4 ou 5 ;
$-(CHR^{16})-X_q-(CHR^{17})_r-Y_s-(CHR^{18})_t-Zu-R^{19}$ avec q = 0 ou 1, r = 0 ou 1, s = 0 ou 1, t = 0 ou 1, u = 0 ou 1, X, Y et Z représentant, indépendamment les uns des autres, à chaque fois 0, S, NH, $N(CH_3)$, $N(C_2H_5)$ ou $N[CH(CH_3)_2]$ ;
un radical aliphatique en $C_{1-10}$ linéaire ou ramifié, saturé ou insaturé, éventuellement substitué ;
un radical cycloaliphatique à 3, 4, 5, 6, 7, 8 ou 9 éléments, insaturé ou saturé, éventuellement substitué, qui peut être ponté avec un groupe alkylène en $C_{1-5}$ linéaire ou ramifié, éventuellement substitué, et/ou condensé avec un système cyclique mono- ou polycyclique saturé, insaturé ou aromatique, éventuellement substitué ;
ou un radical aryle ou hétéroaryle ayant 5 à 14 éléments, éventuellement substitué, qui peut être condensé avec un système cyclique mono- ou polycyclique saturé ou insaturé, éventuellement substitué ;
$R^1$ et $R^2$ forment ensemble avec l'atome d'azote auquel ils sont reliés en tant qu'élément de cycle un radical hétérocycloaliphatique à 4, 5, 6, 7, 8 ou 9 éléments, saturé ou insaturé, éventuellement substitué, qui peut être condensé avec un système cyclique mono- ou polycyclique saturé, insaturé ou aromatique, éventuellement substitué, et/ou former par le biais d'un atome de cycle commun, avec un radical cycloaliphatique à 5, 6 ou 7 éléments, saturé ou insaturé, éventuellement substitué, un composé spiro éventuellement substitué,
le radical hétérocycloaliphatique et éventuellement le radical cycloaliphatique du composé spiro pouvant à

chaque fois être substitués par 1, 2, 3, 4 ou 5 substituants choisis indépendamment les uns des autres dans le groupe constitué par $R^{20}$, $-(CHR^{21})-(CH_2)_v-(CH_2)_w-R^{22}$ avec v = 0 ou 1 et w = 0 ou 1, $-CH=CH-R^{23}$, $-(CH_2)_X-C(=O)-OR^{24}$ avec x = 0, 1, 2, 3, 4 ou 5 ; $-(CH_2)_y-C(=O)-R^{25}$ avec y = 0, 1, 2, 3, 4 ou 5 ; $-(CH_2)_z-C(=O)-NHR^{26}$ avec z = 0, 1, 2, 3, 4 ou 5 ; $-(CH_2)_{aa}-C(=O)-NR^{27}R^{28}$ avec aa = 0, 1, 2, 3, 4 ou 5 ; F ; Cl ; Br ; -CN ; $-CF_3$ ; $-NO_2$ ; oxo (=O) ; thioxo (=S) ; alkyle en $C_{1-5}$ ; -OH ; -O-$C_{1-5}$-alkyle ; -SH ; -S-$C_{1-5}$-alkyle ; $-NH_2$ ; -NH-$C_{1-5}$-alkyle et -N($C_{1-5}$-alkyle)$_2$ et/ou à chaque fois comporter 1, 2, 3, 4 ou 5 hétéroatomes supplémentaires choisis indépendamment les uns des autres dans le groupe constitué par l'oxygène, l'azote et le soufre en tant qu'éléments de cycle ;

$R^3$ représente un radical hydrogène ;

un radical aliphatique en $C_{1-10}$ linéaire ou ramifié, saturé ou insaturé, éventuellement substitué ;

un radical cycloaliphatique à 3, 4, 5, 6, 7, 8 ou 9 éléments, insaturé ou saturé, éventuellement substitué, qui peut être relié par un groupe alkylène en $C_{1-5}$, alcénylène en $C_{2-5}$ ou alcynylène en $C_{2-5}$ linéaire ou ramifié, éventuellement substitué,

ou un radical aryle ou hétéroaryle ayant 5 à 14 éléments éventuellement substitué, qui peut être condensé avec un système cyclique mono- ou polycyclique saturé ou insaturé, éventuellement substitué et/ou relié par un groupe alkylène en $C_{1-5}$, alcénylène en $C_{2-5}$ ou alcynylène en $C_{2-5}$ linéaire

ou ramifié, éventuellement substitué ;

$R^4$ représente un radical aliphatique en $C_{1-10}$ linéaire ou ramifié, saturé ou insaturé, éventuellement substitué ;

un radical cycloaliphatique à 3, 4, 5, 6, 7, 8 ou 9 éléments, insaturé ou saturé, éventuellement substitué, qui peut être relié par un groupe alkylène en $C_{1-5}$, alcénylène en $C_{2-5}$ ou alcynylène en $C_{2-5}$ linéaire ou ramifié, éventuellement substitué,

ou un radical aryle ou hétéroaryle ayant 5 à 14 éléments éventuellement substitué, qui peut être condensé avec un système cyclique mono- ou polycyclique saturé ou insaturé, éventuellement substitué et/ou relié par un groupe alkylène en $C_{1-5}$, alcénylène en $C_{2-5}$ ou alcynylène en $C_{2-5}$ linéaire

ou ramifié, éventuellement substitué ;

$R^5$, $R^8$, $R^9$, $R^{10}$, $R^{11}$, $R^{12}$, $R^{13}$, $R^{14}$, $R^{15}$, $R^{24}$, $R^{25}$, $R^{26}$, $R^{27}$ und $R^{28}$ représentent, indépendamment les uns des autres, à chaque fois un radical aliphatique en $C_{1-10}$ linéaire ou ramifié, saturé ou insaturé, éventuellement substitué ;

un radical cycloaliphatique à 3, 4, 5, 6, 7, 8 ou 9 éléments insaturé ou saturé, éventuellement substitué, qui peut être condensé avec un système cyclique mono- ou polycyclique saturé, insaturé ou aromatique, éventuellement substitué et/ou relié par un groupe alkylène en $C_{1-5}$, alcénylène en $C_{2-5}$

ou alcynylène en $C_{2-5}$ linéaire ou ramifié, éventuellement substitué ;

ou un radical aryle ou hétéroaryle ayant 5 à 14 éléments éventuellement substitué, qui peut être condensé avec un système cyclique mono- ou polycyclique saturé ou insaturé, éventuellement substitué et/ou relié par un groupe alkylène en $C_{1-5}$, alcénylène en $C_{2-5}$ ou alcynylène en $C_{2-5}$ linéaire ou ramifié, éventuellement substitué ;

$R^6$ représente un radical hydrogène ;

un radical aliphatique en $C_{1-10}$ linéaire ou ramifié, saturé ou insaturé, éventuellement substitué, qui peut éventuellement comporter 1, 2, 3, 4 ou 5 hétéroatomes choisis indépendamment les uns des autres dans le groupe constitué par l'oxygène, le soufre et l'azote en tant qu'éléments de chaîne ;

ou un radical aryle ou hétéroaryle ayant 5 à 14 éléments éventuellement substitué, qui peut être condensé avec un système cyclique mono- ou polycyclique saturé ou insaturé, éventuellement substitué et/ou relié par un groupe alkylène en $C_{1-5}$, alcénylène en $C_{2-5}$ ou alcynylène en $C_{2-5}$ linéaire ou ramifié, éventuellement substitué ;

$R^{16}$, $R^{17}$ et $R^{18}$ représentent, indépendamment les uns des autres, à chaque fois

un radical hydrogène ;

un radical aliphatique en $C_{1-10}$ linéaire ou ramifié, saturé ou insaturé, éventuellement substitué, qui peut éventuellement comporter 1, 2, 3, 4 ou 5 hétéroatomes choisis indépendamment les uns des autres dans le groupe constitué par l'oxygène, le soufre et l'azote en tant qu'éléments de chaîne ;

ou un radical aryle ou hétéroaryle ayant 5 à 14 éléments, éventuellement substitué, qui peut être condensé avec un système cyclique mono- ou polycyclique saturé ou insaturé, éventuellement substitué ;

$R^{19}$ représente un radical cycloaliphatique à 3, 4, 5, 6, 7, 8 ou 9 éléments, insaturé ou saturé, éventuellement substitué, qui peut être ponté avec 1, 2, 3, 4 ou 5 groupes alkylène en $C_{1-5}$ linéaires ou ramifiés, éventuellement substitués, et/ou condensé avec un système cyclique mono- ou polycyclique saturé, insaturé ou aromatique, éventuellement substitué ;

ou un radical aryle ou hétéroaryle ayant 5 à 14 éléments, éventuellement substitué, qui peut être condensé avec un système cyclique mono- ou polycyclique saturé ou insaturé, éventuellement substitué ;

$R^{20}$ représente un radical aliphatique en $C_{1-10}$ linéaire ou ramifié, saturé ou insaturé, éventuellement substitué ;

un radical cycloaliphatique à 3, 4, 5, 6, 7, 8 ou 9 éléments, insaturé ou saturé, éventuellement substitué

ou un radical aryle ou hétéroaryle ayant 5 à 14 éléments, éventuellement substitué, qui peut être condensé

avec un système cyclique mono- ou polycyclique saturé ou insaturé, éventuellement substitué ;
$R^{21}$ représente un radical hydrogène ;
un radical aliphatique en $C_{1-10}$ linéaire ou ramifié, saturé ou insaturé, éventuellement substitué ;
un radical cycloaliphatique à 3, 4, 5, 6, 7, 8 ou 9 éléments, insaturé ou saturé, éventuellement substitué ou un radical aryle ou hétéroaryle ayant 5 à 14 éléments, éventuellement substitué, qui peut être condensé avec un système cyclique mono- ou polycyclique saturé ou insaturé, éventuellement substitué ;
et
$R^{22}$ et $R^{23}$ représentent, indépendamment l'un de l'autre, à chaque fois
un radical cycloaliphatique à 3, 4, 5, 6, 7, 8 ou 9 éléments, insaturé ou saturé, éventuellement substitué ou un radical aryle ou hétéroaryle ayant 5 à 14 éléments, éventuellement substitué, qui peut être condensé avec un système cyclique mono- ou polycyclique saturé ou insaturé, éventuellement substitué ;

les radicaux aliphatiques en $C_{1-10}$ mentionnés précédemment pouvant à chaque fois éventuellement être substitués avec 1, 2, 3, 4 ou 5 substituants choisis indépendamment les uns des autres dans le groupe constitué par F, Cl, Br, I, -CN, $-NO_2$, -OH, -SH et - $NH_2$ ;
les radicaux cycloaliphatiques mentionnés précédemment pouvant à chaque fois éventuellement être substitués avec 1, 2, 3, 4 ou 5 substituants choisis indépendamment les uns des autres dans le groupe constitué par oxo (=O), thioxo (=S), F, Cl, Br, I, -CN, $-CF_3$, $-SF_5$, -OH, -O-$C_{1-5}$-alkyle, $-NH_2$, $-NO_2$, $-O-CF_3$, $-S-CF_3$, -SH, -S-$C_{1-5}$-alkyle, -$C_{1-5}$-alkyle, -C(=O)-OH) -C(=O)-O-$C_{1-5}$-alkyle, -NH-$C_{1-5}$-alkyle, -N($C_{1-5}$-alkyle)$_2$, -($CH_2$)-benzo[b]furanyle, -O-phényle, -O-benzyle, phényle et benzyle, la partie cyclique des radicaux -O-phényle, -O-benzyle, phényle, -($CH_2$)-benzo [b]furanyle et benzyle pouvant à chaque fois être substituée avec 1, 2, 3, 4 ou 5 substituants choisis indépendamment les uns des autres dans le groupe constitué par F, Cl, Br, -OH, $-CF_3$, $-SF_5$, -CN, $-NO_2$, -$C_{1-5}$-alkyle, -O-$C_{1-5}$-alkyle, $-O-CF_3$, $-S-CF_3$, phényle et -O-benzyle
et les radicaux cycloaliphatiques mentionnés précédemment pouvant à chaque fois éventuellement comporter 1, 2, 3, 4 ou 5 hétéroatomes choisis indépendamment les uns des autres dans le groupe constitué par l'oxygène, l'azote et le soufre en tant qu'éléments de cycle ;
les groupes alkylène en $C_{1-5}$, alcénylène en $C_{2-5}$ ou alcynylène en $C_{2-5}$ mentionnés précédemment pouvant à chaque fois éventuellement être substitués avec 1, 2, 3, 4 ou 5 substituants choisis indépendamment les uns des autres dans le groupe constitué par F, Cl, Br, -OH, -SH, $-NH_2$, -CN, $NO_2$ et phényle ;
les cycles des systèmes cycliques mono- ou polycycliques mentionnés précédemment pouvant à chaque fois éventuellement être substitués avec 1, 2, 3, 4 ou 5 substituants choisis indépendamment les uns des autres dans le groupe constitué par oxo (=O), thioxo (=S), F, Cl, Br, I, -CN, $-CF_3$, $-SF_5$, -OH, -O-$C_{1-5}$-alkyle, $-NH_2$, $-NO_2$, $-O-CF_3$, $-S-CF_3$, -SH, -S-$C_{1-5}$-alkyle, -$C_{1-5}$-alkyle, -C(=O)-OH, -C(=O)-O-$C_{1-5}$-alkyle, -NH-$C_{1-5}$-alkyle, -N($C_{1-5}$-alkyle)$_2$, -($CH_2$)-benzo[b]furanyle, -O-phényle, -O-benzyle, phényle et benzyle, la partie cyclique des radicaux -O-phényle, -O-benzyle, phényle, -($CH_2$)-benzo[b]furanyle et benzyle pouvant à chaque fois être substituée avec 1, 2, 3, 4 ou 5 substituants choisis indépendamment les uns des autres dans le groupe constitué par F, Cl, Br, -OH, $-CF_3$, $-SF_5$, -CN, $-NO_2$, -$C_{1-5}$-alkyle, -O-$C_{1-5}$-alkyle, $-O-CF_3$, $-S-CF_3$, phényle et -O-benzyle
et les cycles des systèmes cycliques mono- ou polycycliques mentionnés précédemment ayant à chaque fois 5, 6 ou 7 éléments et pouvant à chaque fois éventuellement comporter 1, 2, 3, 4 ou 5 hétéroatomes en tant qu'éléments de cycle, choisis indépendamment les uns des autres dans le groupe constitué par l'oxygène, l'azote et le soufre ;
et les radicaux aryles ou hétéroaryles mentionnés précédemment pouvant à chaque fois éventuellement être substitués avec 1, 2, 3, 4 ou 5 substituants choisis indépendamment les uns des autres dans le groupe constitué par F, Cl, Br, I, -CN, $-CF_3$, $-SF_5$, -OH, -O-$C_{1-5}$-alkyle, $-NH_2$, $-NO_2$, $-O-CF_3$, $-S-CF_3$, -SH, -S-$C_{1-5}$-alkyle, -$C_{1-5}$-alkyle, -C (=O)-OH, -C(=O)-O-$C_{1-5}$-alkyle, -NH-$C_{1-5}$-alkyle, -N($C_{1-5}$-alkyle)$_2$, -NH-C(=O)-O-$C_{1-5}$-alkyle, -C(=O)-H, -C (=O)-$C_{1-5}$-alkyle, -C(=O)-$NH_2$, -C(=O)-NH-$C_{1-5}$-alkyle, C(=O)-N-($C_{1-5}$-alkyle)$_2$, -($CH_2$)-benzo[b]furanyle, -O-phényle, -O-benzyle, phényle et benzyle, la partie cyclique des radicaux -O-phényle, -O-benzyle, phényle, -($CH_2$)-benzo [b]furanyle et benzyle pouvant à chaque fois être substituée avec 1, 2, 3, 4 ou 5 substituants choisis indépendamment les uns des autres dans le groupe constitué par F, Cl, Br, -OH, $-CF_3$, $-SF_5$, -CN, $-NO_2$, -$C_{1-5}$-alkyle, -O-$C_{1-5}$-alkyle, $-O-CF_3$, $-S-CF_3$, phényle et -O-benzyle,
et
les radicaux hétéroaryles mentionnés précédemment pouvant à chaque fois éventuellement comporter 1, 2, 3, 4 ou 5 hétéroatomes choisis indépendamment les uns des autres dans le groupe constitué par l'oxygène, l'azote et le soufre en tant qu'éléments de cycle ;
à chaque fois éventuellement sous la forme d'un de leurs stéréoisomères purs, notamment énantiomères ou diastéréoisomères, leurs racémates ou sous la forme d'un mélange de stéréoisomères, notamment les énantiomères et/ou les diastéréoisomères, en un rapport de mélange quelconque, ou à chaque fois sous la forme des sels correspondants, ou à chaque fois sous la forme des solvates correspondants.

**2.** Composés selon la revendication 1, **caractérisés en ce que**

$R^1$ représente un radical hydrogène ; -C(=O)-$OR^5$; -($CHR^6$)-$(CH_2)_m$-C(=O)-$OR^7$ avec m = 0, 1, 2, 3, 4 ou 5 ; -C (=O)-NH-$R^{10}$; -$(CH_2)_o$-C(=O)-$NHR^{11}$ avec O = 0, 1, 2, 3, 4 ou 5 ; -($CHR^{16}$)-$X_q$-($CHR^{17}$)$_r$-$Y_s$-($CHR^{18}$)$_t$-$Z_u$-$R^{19}$ avec q = 0 ou 1, r = 0 ou 1, s = 0 ou 1, t = 0 ou 1, u = 0 ou 1, X, Y et Z, représentant, indépendamment les uns des autres, à chaque fois 0, S, NH et N($CH_3$);
un radical alkyle éventuellement substitué choisi dans le groupe constitué par méthyle, éthyle, n-propyle, iso-propyle, - ($CH_2$)-($CH_2$)-(CN), n-butyle, sec-butyle, iso-butyle, tert-butyle, -C(H)($CH_3$)-C(H)$(CH_3)_2$ et -($CH_2$)-($CH_2$)-(C$(CH_3)_3$),
un radical alcényle choisi dans le groupe constitué par vinyle, 1-propényle et 2-propényle,
un radical (hétéro)cycloaliphatique choisi dans le groupe constitué par cyclopropyle, cyclobutyle, cyclopentyle, cyclohexyle, cycloheptyle, cyclopentényle, cyclohexényle, cycloheptényle, imidazolidinyle, tétrahydrofuranyle, tétrahydrothiophényle, pyrrolidinyle, pipéridinyle, morpholinyle, pipérazinyle, thiomorpholinyle, tétrahydropyranyle, azépanyle, diazépanyle, dithiolanyle, (6,6)-diméthyl-[3.1.1]-bicycloheptyle, indanyle, indényle, (1,4)-benzodioxanyle, (1,2,3,4)-tétrahydronaphtyle, (1,2,3,4)-tétrahydroquinolinyle et (1,2,3,4)-tétrahydroquinazolinyle, le radical (hétéro)cycloaliphatique pouvant à chaque fois éventuellement être substitué avec 1, 2, 3, 4 ou 5 substituants choisis indépendamment les uns des autres dans le groupe constitué par F, Cl, Br, I, -CN, -$CF_3$, -$SF_5$, -OH, -$NH_2$, -O-$CF_3$, -SH, -O-$CH_3$, -O-$C_2H_5$, méthyle, éthyle, n-propyle, iso-propyle, n-butyle, sec-butyle, iso-butyle, tert-butyle, -S-$CH_3$, -S-$C_2H_5$, -C(=O)-O-$CH_3$, -C(=O)-O-$C_2H_5$, oxo (=O),- N$(CH_3)_2$, -N$(C_2H_5)_2$, -N(H) ($CH_3$), -N(H)($C_2H_5$), -$NO_2$, -$SCF_3$, -C (=0) -OH, - ($CH_2$) -benzo [b] furanyle, -O-phényle, -O-benzyle, phényle et benzyle, la partie cyclique des radicaux -($CH_2$)-benzo[b]furanyle, -O-phényle, -O-benzyle, benzyle et phényle pouvant à chaque fois être substituée avec 1, 2, 3, 4 ou 5 substituants choisis indépendamment les uns des autres dans le groupe constitué par F, Cl, Br, -OH, -$CF_3$, -$SF_5$, -CN, -$NO_2$, méthyle, éthyle, iso-propyle, n-propyle, -O-$CH_3$, -O-$C_2H_5$, -O-$CF_3$, phényle et -O-benzyle,
ou un radical choisi dans le groupe constitué par phényle, naphtyle, (1,3)-benzodioxolyle, (1,4)-benzodioxanyle, thiophényle, furanyle, pyrrolyle, pyrazolyle, pyrazinyle, pyranyle, triazolyle, pyridinyle, imidazolyle, indolyle, isoindolyle, benzo[b]furanyle, benzo[b]thiophényle, thiazolyle, oxazolyle, isoxazolyle, pyridazinyle, pyrazinyle, pyrimidinyle, indazolyle, quinazolinyle, quinolinyle et isoquinolinyle, le radical pouvant à chaque fois éventuellement être substitué avec 1, 2, 3, 4 ou 5 substituants choisis indépendamment les uns des autres dans le groupe constitué par F, Cl, Br, I, -CN, -$NO_2$, -OH, -SH, méthyle, éthyle, n-propyle, iso-propyle, n-butyle, sec-butyle, iso-butyle, tert-butyle, -$CF_3$, -O-$CF_3$, -S-$CF_3$, -$SF_5$, -O-$CH_3$, -O-$C_2H_5$, -O-phényle, -O-benzyle, -$NH_2$, -N $(CH_3)_2$, -N$(C_2H_5)_2$, -N(H)($CH_3$), -N(H)($C_2H_5$), -NH-C(=O)-O-$CH_3$, -NH-C(=O)-O-$C_2H_5$ et -NH-C(=O)-O-C$(CH_3)_3$.

**3.** Composés selon la revendication 1 ou 2, **caractérisés en ce que**

$R^2$ représente un radical hydrogène,
-($CHR^{16}$)-$R^{19}$,
ou un radical alkyle choisi dans le groupe constitué par méthyle, éthyle, n-propyle, iso-propyle, n-butyle, sec-butyle, iso-butyle et tert-butyle.

**4.** Composés selon une ou plusieurs des revendications 1 à 3, **caractérisés en ce que**
$R^1$ et $R^2$ forment ensemble avec l'atome d'azote auquel ils sont reliés en tant qu'élément de cycle un radical hétérocycloaliphatique choisi dans le groupe constitué par pyrrolidinyle ; pipéridinyle ; (1,2,3,6)-tétrahydropyridinyle ; (1,2,3,4)-tétrahydropyridinyle et (1,4)-dioxa-8-aza-spiro[4.5]décane, le radical hétérocycloaliphatique pouvant à chaque fois être substitué avec 1 ou 2 substituants choisis indépendamment les uns des autres dans le groupe constitué par $R^{20}$, -($CHR^{21}$)-$(CH_2)_v$-$R^{22}$ avec v = 0 ou 1 ; -C(=O)-$OR^{24}$; -C(=O)-$R^{25}$; F ; Cl ; Br ; -CN ; -$CF_3$ et -OH,
ou
$R^1$ et $R^2$ forment ensemble avec l'atome d'azote auquel ils sont reliés en tant qu'élément de cycle un radical hétérocycloaliphatique choisi dans le groupe constitué par (1,3,4,5)-tétrahydropyrido[4,3-b]indolyle ; (3,4)-dihydro-1H-isoquinolinyle et (1,3,4,9)-tétrahydro-[b]-carbolinyle, le radical hétérocycloaliphatique pouvant à chaque fois être substitué avec 1 ou 2 substituants choisis indépendamment les uns des autres dans le groupe constitué par F, Cl, Br, -CN, -$CF_3$ et -OH,
ou
$R^1$ et $R^2$ forment ensemble avec l'atome d'azote auquel ils sont reliés en tant qu'élément de cycle un radical hétérocycloaliphatique choisi dans le groupe constitué par imidazolidinyle, (1,3)-thiazolidinyle, pipérazinyle, morpholinyle et thiomorpholinyle, le radical hétérocycloaliphatique pouvant à chaque fois être substitué avec 1 ou 2 substituants choisis indépendamment les uns des autres dans le groupe constitué par $R^{20}$, -($CHR^{21}$)-$(CH_2)_v$-$(CH_2)_w$-$R^{22}$ avec v = 0 ou 1 et w = 0 ou 1, -CH=CH-$R^{23}$, -C(=O)-$OR^{24}$; -C(=O)-$R^{25}$; -$(CH_2)_z$-C

(=O)-$NHR^{26}$ avec z = 1 et -$(CH_2)_{aa}$-C(=O)-$NR^{27}R^{28}$ avec aa = 1.

5. Composés selon une ou plusieurs des revendications 1 à 4, **caractérisés en ce que** $R^3$ représente un radical hydrogène ou un radical alkyle choisi dans le groupe constitué par méthyle, éthyle, n-propyle, iso-propyle, n-butyle, sec-butyle, iso-butyle, tert-butyle et n-pentyle.

6. Composés selon une ou plusieurs des revendications 1 à 5, **caractérisés en ce que** $R^4$ représente un radical phényle ou naphtyle, pouvant à chaque fois être substitué avec 1, 2, 3, 4 ou 5 substituants choisis indépendamment les uns des autres dans le groupe constitué par méthyle, éthyle, n-propyle, iso-propyle, n-butyle, iso-butyle, sec-butyle, tert-butyle, F, Cl, Br, -CN, -$SF_5$, -S-$CF_3$, -$NH_2$, -$N(CH_3)_2$, -$N(C_2H_5)_2$, -N(H)($CH_3$), -N(H)($C_2H_5$), -SH, -$NO_2$, -$CF_3$, -$OCF_3$, -OH, -O-$CH_3$ et -O-$C_2H_5$ et/ou relié par un groupe -($CH_2$)-, -$(CH_2)_2$- ou -$(CH_2)_3$-.

7. Composés selon une ou plusieurs des revendications 1 à 6, **caractérisés en ce que**

$R^5$, $R^7$, $R^8$, $R^9$, $R^{10}$, $R^{11}$, $R^{12}$ $R^{13}$, $R^{14}$ et $R^{15}$ représentent, indépendamment les uns des autres, à chaque fois un radical alkyle choisi dans le groupe constitué par méthyle, éthyle, n-propyle, iso-propyle, n-butyle, sec-butyle, iso-butyle et tert-butyle, le radical alkyle pouvant éventuellement à chaque fois être substitué avec 1, 2, 3, 4 ou 5 substituants choisis indépendamment les uns des autres dans le groupe constitué par F, Cl, Br, I, -CN, -$NO_2$, -OH, -SH et -$NH_2$,
ou un radical choisi dans le groupe constitué par phényle, naphtyle, (1,3)-benzodioxolyle, (1,4)-benzodioxanyle, thiophényle, furanyle, pyrrolyle, pyrazolyle, pyrazinyle, pyranyle, triazolyle, pyridinyle, imidazolyle, indolyle, isoindolyle, benzo[b]furanyle, benzo[b]thiophényle, thiazolyle, oxazolyle, isoxazolyle, pyridazinyle, pyrazinyle, pyrimidinyle, indazolyle, quinazolinyle, quinolinyle et isoquinolinyle, le radical pouvant à chaque fois être relié par un groupe -($CH_2$)-, -$(CH_2)_2$ ou -$(CH_2)_3$- et/ou éventuellement être substitué avec 1, 2, 3, 4 ou 5 substituants choisis indépendamment les uns des autres dans le groupe constitué par F, Cl, Br, I, -CN, -$NO_2$, -OH, -SH, méthyle, éthyle, n-propyle, iso-propyle, n-butyle, sec-butyle, iso-butyle, tert-butyle, -$CF_3$, -O-$CF_3$, -S-$CF_3$, -$SF_5$, -O-$CH_3$, -O-$C_2H_5$, -O-phényle, -O-benzyle, -$NH_2$, -N($CH_3$)2, -$N(C_2H_5)_2$, -N(H)($CH_3$), -N(H)($C_2H_5$), -NH-C(=O)-O-$CH_3$, -NH-C(=O)-O-$C_2H_5$ et -NH-C(=O)-O-$C(CH_3)_3$.

8. Composés selon une ou plusieurs des revendications 1 à 7, **caractérisés en ce que**

$R^6$ représente un radical hydrogène,
un radical choisi dans le groupe constitué par méthyle, éthyle, n-propyle, iso-propyle, n-butyle, sec-butyle, iso-butyle, tert-butyle, n-pentyle, sec-pentyle, -C(H)($CH_3$) -C(H)$(CH_3)_2$, -($CH_2$)-($CH_2$)-(C$(CH_3)_3$), -C(H)($CH_3$)(O(C$(CH_3)_3$)) et n-hexyle, le radical pouvant à chaque fois éventuellement être substitué avec 1, 2, 3, 4 ou 5 substituants choisis indépendamment les uns des autres dans le groupe constitué par F, Cl, Br, I, -CN, -$NO_2$, -OH, -SH et -$NH_2$,
ou un radical choisi dans le groupe constitué par phényle, naphtyle, (1,3)-benzodioxolyle, (1,4)-benzodioxanyle, thiophényle, furanyle, pyrrolyle, pyrazolyle, pyrazinyle, pyranyle, triazolyle, pyridinyle, imidazolyle, indolyle, isoindolyle, benzo[b]furanyle, benzo[b]thiophényle, thiazolyle, oxazolyle, isoxazolyle, pyridazinyle, pyrazinyle, pyrimidinyle, indazolyle, quinazolinyle, quinolinyle et isoquinolinyle, le radical pouvant à chaque fois être relié par un groupe -($CH_2$)-, -$(CH_2)_2$ ou -$(CH_2)_3$- et/ou éventuellement être substitué avec 1, 2, 3, 4 ou 5 substituants choisis indépendamment les uns des autres dans le groupe constitué par F, Cl, Br, I, -CN, -$NO_2$, -OH, -SH, méthyle, éthyle, n-propyle, iso-propyle, n-butyle, sec-butyle, iso-butyle, tert-butyle, -$CF_3$, -O-$CF_3$, -S-$CF_3$, -$SF_5$, -O-$CH_3$, -O-$C_2H_5$, -O-phényle, -O-benzyle, -$NH_2$, -$N(CH_3)_2$, -$N(C_2H_5)_2$, -N(H)($CH_3$), -N(H)($C_2H_5$), -NH-C(=O)-O-$CH_3$, -NH-C(=O)-O-$C_2H_5$ et -NH-C(=O)-O-$C(CH_3)_3$.

9. Composés selon une ou plusieurs des revendications 1 à 8, **caractérisés en ce que**

$R^{16}$, $R^{17}$ et $R^{18}$ représentent, indépendamment les uns des autres, à chaque fois un radical hydrogène,
un radical choisi dans le groupe constitué par méthyle, éthyle, n-propyle, iso-propyle, n-butyle, sec-butyle, iso-butyle, tert-butyle, n-pentyle, sec-pentyle, -C(H)($CH_3$)-C(H)$(CH_3)_2$, -($CH_2$)-($CH_2$)-(C$(CH_3)_3$), -($CH_2$)-O-($CH_3$) et n-hexyle, le radical pouvant à chaque fois éventuellement être substitué avec 1, 2, 3, 4 ou 5 substituants choisis indépendamment les uns des autres dans le groupe constitué par F, Cl, Br, I, -CN, -$NO_2$, -OH, -SH et -$NH_2$,
ou un radical choisi dans le groupe constitué par phényle et naphtyle, le radical pouvant à chaque fois être substitué avec 1, 2, 3, 4 ou 5 substituants choisis indépendamment les uns des autres dans le groupe constitué par F, Cl, Br, I, -CN, -$NO_2$, -OH, -SH, méthyle, éthyle, n-propyle, iso-propyle, n-butyle, sec-butyle, iso-butyle,

tert-butyle, $-CF_3$, $-O-CF_3$, $-S-CF_3$, $-SF_5$, $-O-CH_3$, $-O-C_2H_5$, -O-phényle, -O-benzyle, $-NH_2$, $-N(CH_3)_2$, $-N(C_2H_5)_2$, $-N(H)(CH_3)$, $-N(H)(C_2H_5)$, $-NH-C(=O)-O-CH_3$, $-NH-C(=O)-O-C_2H_5$ et $-NH-C(=O)-O-C(CH_3)_3$.

**10.** Composés selon une ou plusieurs des revendications 1 à 9, **caractérisés en ce que**

$R^{19}$ représente un radical (hétéro)cycloaliphatique choisi dans le groupe constitué par cyclopropyle, cyclobutyle, cyclopentyle, cyclohexyle, cycloheptyle, cyclopentényle, cyclohexényle, cycloheptényle, imidazolidinyle, tétrahydrofuranyle, tétrahydrothiophényle, pyrrolidinyle, pipéridinyle, morpholinyle, pipérazinyle, thiomorpholinyle, tétrahydropyranyle, azépanyle, diazépanyle, dithiolanyle, (6,6)-diméthyl-[3.1.1]-bicycloheptyle, adamantyle (tricyclo-[3.3.1.1$^{3,7}$]-décanyle), indanyle, indényle, (1,4)-benzodioxanyle, (1,2,3,4)-tétrahydronaphtyle, (1,2,3,4)-tétrahydroquinolinyle et (1,2,3,4)-tétrahydroquinazolinyle, le radical (hétéro)cycloaliphatique pouvant à chaque fois éventuellement être substitué avec 1, 2, 3, 4 ou 5 substituants choisis indépendamment les uns des autres dans le groupe constitué par F, Cl, Br, I, -CN, $-CF_3$, $-SF_5$, -OH, $-NH_2$, $-O-CF_3$, -SH, $-O-CH_3$, $-O-C_2H_5$, méthyle, éthyle, n-propyl, iso-propyle, n-butyle, sec-butyle, iso-butyle, tert-butyle, $-S-CH_3$, $-S-C_2H_5$, $-C(=O)-O-CH_3$, $-C(=O)-O-C_2H_5$, oxo (=0), $-N(CH_3)_2$, $-N(C_2H_5)_2$, $-N(H)(CH_3)$, $-N(H)(C_2H_5)$, $-NO_2$, $-SCF_3$, $-C(=O)-OH$, $-(CH_2)$-benzo[b]furanyle, -O-phényle, -O-benzyle, phényle et benzyle,
ou un radical choisi dans le groupe constitué par phényle, naphtyle, (1,3)-benzodioxolyle, (1,4)-benzodioxanyle, thiophényle, furanyle, pyrrolyle, pyrazolyle, pyrazinyle, pyranyle, triazolyle, pyridinyle, imidazolyle, indolyle, isoindolyle, benzo[b]furanyle, benzo[b]thiophényle, thiazolyle, oxazolyle, isoxazolyle, pyridazinyle, pyrazinyle, pyrimidinyle, indazolyle, quinazolinyle, quinolinyle et isoquinolinyle, le radical pouvant à chaque fois éventuellement être substitué avec 1, 2, 3, 4 ou 5 substituants choisis indépendamment les uns des autres dans le groupe constitué par F, Cl, Br, I, -CN, $-NO_2$, -OH, -SH, méthyle, éthyle, n-propyle, iso-propyle, n-butyle, sec-butyle, iso-butyle, tert-butyle, $-CF_3$, $-O-CF_3$, $-S-CF_3$, $-SF_5$, $-O-CH_3$, $-O-C_2H_5$, -O-phényle, -O-benzyle, $-NH_2$, $-N(CH_3)_2$, $-N(C_2H_5)_2$, $-N(H)(CH_3)$, $-N(H)(C_2H_5)$, $-NH-C(=O)-O-CH_3$, $-NH-C(=O)-O-C_2H_5$ et $-NH-C(=O)-O-C(CH_3)_3$.

**11.** Composés selon une ou plusieurs des revendications 1 à 10, **caractérisés en ce que**

$R^{20}$ représente un radical alkyle choisi dans le groupe constitué par méthyle, éthyle, n-propyle, iso-propyle, n-butyle, iso-butyle, sec-butyle, tert-butyle et n-pentyle,
un radical (hétéro)cycloaliphatique choisi dans le groupe constitué par cyclopentyle, cyclohexyle, pipéridinyle et cycloheptyle, le radical (hétéro)cycloaliphatique pouvant à chaque fois éventuellement être substitué avec 1, 2, 3, 4 ou 5 substituants choisis indépendamment les uns des autres dans le groupe constitué par F, Cl, Br, I, -CN, $-CF_3$, $-SF_5$, -OH, $-NH_2$, $-O-CF_3$, -SH, $-O-CH_3$, $-O-C_2H_5$, méthyle, éthyle, n-propyle, iso-propyle, n-butyle, sec-butyle, iso-butyle et tert-butyle,
ou un radical choisi dans le groupe constitué par phényle, naphtyle, (1,3)-benzodioxolyle, thiophényle, furanyle, pyridinyle, imidazolyle, indolyle, benzo[b]furanyle, benzo[b]thiophényle, thiazolyle, oxazolyle, isoxazolyle, pyridazinyle, pyrazinyle, pyrimidinyle et quinolinyle, le radical pouvant à chaque fois éventuellement être substitué avec 1, 2, 3, 4 ou 5 substituants choisis indépendamment les uns des autres dans le groupe constitué par méthyle, éthyle, n-propyle, iso-propyle, n-butyle, iso-butyle, sec-butyle, tert-butyle, F, Cl, Br, -CN, $-SF_5$, $-O-CF_3$, $-S-CF_3$, $-NH_2$, $-N(CH_3)_2$, $-N(C_2H_5)_2$, $-N(H)(CH_3)$, $-N(H)(C_2H_5)$, SH, $-NO_2$, $-CF_3$, $-OCF_3$, -OH, $-O-CH_3$ et $-O-C_2H_5$ .

**12.** Composés selon une ou plusieurs des revendications 1 à 11, **caractérisés en ce que**

$R^{21}$ représente un radical hydrogène

ou un radical phényle ou naphtyle qui peut être substitué avec 1, 2 ou 3 substituants choisis indépendamment les uns des autres dans le groupe constitué par méthyle, éthyle, n-propyle, iso-propyle, n-butyle, iso-butyle, sec-butyle, tert-butyle, F, Cl et Br.

**13.** Composés selon une ou plusieurs des revendications 1 à 12, **caractérisés en ce que**

$R^{22}$ et $R^{23}$ représentent, indépendamment l'un de l'autre, à chaque fois un radical (hétéro)cycloaliphatique choisi dans le groupe constitué par pyrrolidinyle, morpholinyle et thiomorpholinyle, le radical (hétéro)cycloaliphatique pouvant à chaque fois éventuellement être substitué avec 1, 2, 3, 4 ou 5 substituants choisis indépendamment les uns des autres dans le groupe constitué par F, Cl. Br, I, -CN, $-CF_3$, $-SF_5$, -OH, $-NH_2$, $-O-CF_3$, -SH, $-O-CH_3$, $-O-C_2H_5$, méthyle, éthyle, n-propyle, iso-propyle, n-butyle, sec-butyle, iso-butyle et tert-butyle,
ou un radical choisi dans le groupe constitué par phényle, naphtyle, (1,3)-benzodioxolyle, thiophényle, furanyle, pyridinyle, imidazolyle, indolyle, benzo[b]furanyle, benzo[b]thiophényle, thiazolyle, oxazolyle, isoxazolyle, pyridazinyle, pyrazinyle, pyrimidinyle et quinolinyle, le radical pouvant à chaque fois éventuellement être substitué avec 1, 2, 3, 4 ou 5 substituants choisis indépendamment les uns des autres dans le groupe constitué par méthyle, éthyle, n-propyle, iso-propyle, n-butyle, iso-butyle, sec-butyle, tert-butyle, F, Cl, Br, -CN, $-SF_5$, $-O-CF_3$, $-S-CF_3$, $-NH_2$, $-N(CH_3)_2$, $-N(C_2H_5)_2$, $-N(H)(CH_3)$, $-N(H)(C_2H_5)$, -SH, $-NO_2$, $-CF_3$, $-OCF_3$, -OH, $-O-CH_3$ et $-O-C_2H_5$.

**14.** Composés selon une ou plusieurs des revendications 1 à 13, **caractérisés en ce que**

$R^{24}$ représente un radical alkyle choisi dans le groupe constitué par méthyle, éthyle, n-propyle, iso-propyle, n-butyle, iso-butyle, sec-butyle et tert-butyle.
$R^{25}$ représente un radical alkyle choisi dans le groupe constitué par phényle, naphtyle, furanyle, pyrazinyle et pyrimidinyle, qui peut être relié par un groupe $-(CH_2)-$, $-(CH_2)_2-$ ou $-(CH_2)_3-$ et/ou substitué avec 1, 2 ou 3 substituants choisis indépendamment les uns des autres dans le groupe constitué par méthyle, éthyle, n-propyle et iso-propyle,
$R^{26}$ représente un radical alkyle choisi dans le groupe constitué par méthyle, éthyle, n-propyle et iso-propyle,
$R^{27}$ représente un radical alkyle choisi dans le groupe constitué par méthyle, éthyle, iso-propyle et n-propyle ou un radical phényle,
et
$R^{28}$ représente un radical alkyle choisi dans le groupe constitué par méthyle, éthyle, iso-propyle et n-propyle ou un radical phényle.

**15.** Composés selon une ou plusieurs des revendications 1 à 14, **caractérisés en ce que**

$R^1$ représente $-C(=O)-OR^5$ ; $-(CHR^6)-C(=O)-OR^7$ ; $-C(=O)-NHR^{11}$ ; $-(CH^2)-C(=O)-NHR^{11}$ ; $-(CHR^{16})-R^{19}$ ; $-(CHR^{16})-(CHR^{17})-R^{19}$ ; $-(CHR^{16})-(CHR^{17})-O-R^{19}$ ; $-(CHR^{16})-(CHR^{17})-(CHR^{18})-R^{19}$ ; $-(CHR^{16})-(CHR^{17})-S-(CHR^{18})-R^{19}$ ; $-(CHR^{16})-(CHR^{17})-(CHR^{18})-N(CH_3)-R^{19}$,
un radical alkyle éventuellement substitué choisi dans le groupe constitué par méthyle, éthyle, n-propyle, iso-propyle, $-CH_2-CH_2-CN$, n-butyle, sec-butyle, iso-butyle, tert-butyle, $-CH(CH_3)-CH(CH_3)_2$ et $-CH_2-CH_2-C(CH_3)_3$,
un radical alcényle choisi dans le groupe constitué par 1-propényle et 2-propényle,
un radical choisi dans le groupe constitué par cyclopentyle, cyclohexyle, cycloheptyle, (6,6)-diméthyl-[3.1.1]-bicycloheptyle, indanyle et indényle, le radical pouvant à chaque fois être substitué avec 1, 2 ou 3 substituants choisis indépendamment les uns des autres dans le groupe constitué par F, Cl, Br, méthyle, éthyle, iso-propyle, n-propyle et -O-benzyle,
un radical pyrrolidinyle qui peut être substitué avec un radical $-(CH_2)$-benzo[b]furanyle ou benzyle, la partie cyclique du radical benzyle pouvant à chaque fois être substituée avec 1, 2 ou 3 substituants choisis indépendamment les uns des autres dans le groupe constitué par F, Cl, Br, -OH, méthyle, éthyle, iso-propyle, n-propyle, $-CF_3$, $-O-CH_3$, $-O-C_2H_5$, phényle et -O-benzyle,
ou un radical choisi dans le groupe constitué par phényle, naphtyle, (1,3)-benzodioxolyle et (1,4)-benzodioxanyle, le radical pouvant à chaque fois être substitué avec 1, 2 ou 3 substituants choisis indépendamment les uns des autres dans le groupe constitué par F, Cl, Br, I, méthyle, éthyle, n-propyle, iso-propyl, n-butyle, sec-butyle, iso-butyle, tert-butyle, -O-phényle, -O-benzyle, $-NH-C(=O)-O-CH_3$, $-NH-C(=O)-O-C_2H_5$ et $-NH-C(=O)-O-C(CH_3)_3$ ;
$R^2$ représente un radical hydrogène,
$-(CHR^{16})-R^{19}$,
ou un radical alkyle choisi dans le groupe propyle, n-butyle, sec-butyle, iso-butyle et tert-butyle ;
ou
$R^1$ et $R^2$ forment ensemble avec l'atome d'azote auquel ils sont reliés en tant qu'élément de cycle un radical qui est choisi dans le groupe constitué par

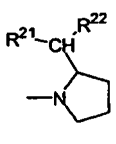 , 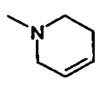 , 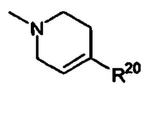 , 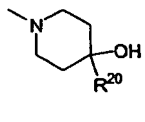 ,

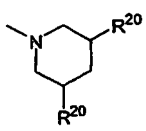, 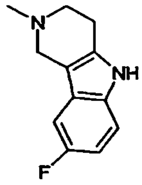, 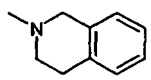, 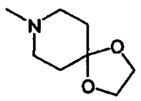,

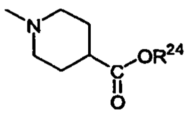, 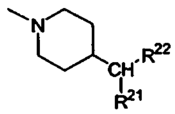, 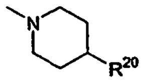, 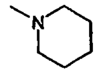,

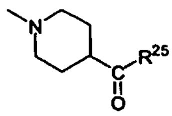, 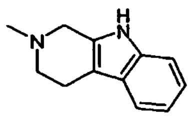, 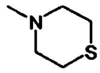, 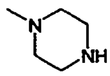,

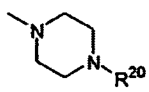, 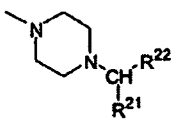, 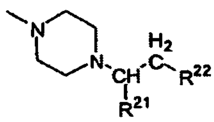,

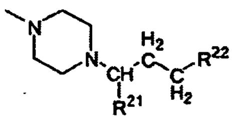, 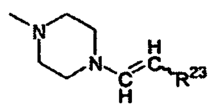, 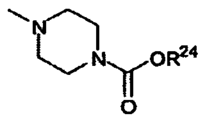,

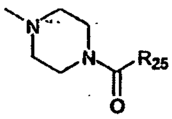, 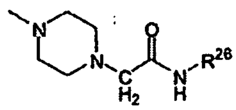 et 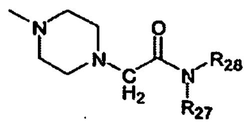

$R^3$ représente un radical alkyle choisi dans le groupe constitué par méthyle, éthyle, n-propyle, iso-propyle, n-butyle, sec-butyle, iso-butyle et tert-butyle ;

$R^4$ représente un radical choisi dans le groupe constitué par

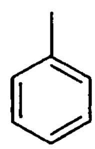, 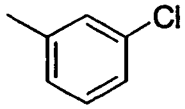 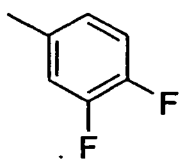, 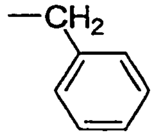

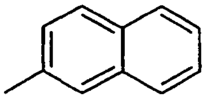

et

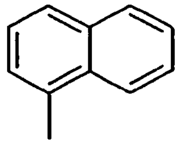

,

$R^5$, $R^7$ et $R^{11}$ représentent, indépendamment les uns des autres, à chaque fois un radical alkyle choisi dans le groupe constitué par méthyle, éthyle, n-propyle, iso-propyle, n-butyle, sec-butyle, iso-butyle et tert-butyle ;
ou un radical benzyle ou naphtyle ;
$R^6$ représente un radical hydrogène,
un radical choisi dans le groupe constitué par méthyle, éthyle, n-propyle, iso-propyle, n-butyle, sec-butyle, iso-butyle, tert-butyle, n-pentyle, sec-pentyle, néo-pentyle et $-C(H)(CH_3)(O(C(CH_3)_3))$,
ou un radical indolyle relié par un groupe $-(CH_2)-$ ;
$R^{16}$ représente un radical hydrogène,
un radical choisi dans le groupe constitué par méthyle, éthyle, iso-propyle, n-propyle, n-butyle, sec-butyle, iso-butyle, tert-butyle et $-(CH_2)-O-(CH_3)$,
ou un radical phényle ;
$R^{17}$ représente un radical hydrogène,
un radical alkyle choisi dans le groupe constitué par méthyle, éthyle, iso-propyle, n-propyle, n-butyle, iso-butyle, sec-butyle et tert-butyle ou un radical phényle ;
$R^{18}$ représente un radical hydrogène
ou un radical phényle ;
$R^{19}$ représente un radical (hétéro)cycloaliphatique choisi dans le groupe constitué par cyclopentyle, cyclohexyle, cycloheptyle, tétrahydrofuranyle, tétrahydrothiophényle, pyrrolidinyle, pipéridinyle, adamantyle (tricyclo-[3.3.1.1$^{3,7}$]-décanyle) et (1,4)-benzodioxanyle, le radical (hétéro)cycloaliphatique pouvant à chaque fois être substitué avec 1 ou 2 substituants choisis indépendamment les uns des autres dans le groupe constitué par méthyle, éthyle, iso-propyle et n-propyle,
ou un radical choisi dans le groupe constitué par phényle, naphtyle, (1,3)-benzodioxolyle, (1,4)-benzodioxanyle, thiophényle, furanyle, pyridinyle, imidazolyle, indolyle et isoindolyle, le radical pouvant à chaque fois être substitué avec 1, 2, 3, 4 ou 5 substituants choisis indépendamment les uns des autres dans le groupe constitué par F, Cl, Br, I, méthyle, éthyle, n-propyle, iso-propyle, n-butyle, sec-butyle, iso-butyle, tert-butyle, $-CF_3$, $-O-CH_3$, $-O-C_2H_5$, $-O-CF_3$, -O-phényl, -O-benzyle, $-NH_2$, $-N(CH_3)_2$, $-N(C_2H_5)_2$, $-N(H)(CH_3)$ et $-N(H)(C_2H_5)$;
$R^{20}$ représente un radical méthyle ou éthyle,
un radical (hétéro)cycloaliphatique choisi dans le groupe constitué par cyclopentyle, cyclohexyle, pipéridinyle et cycloheptyle,
ou un radical phényle ou pyridinyle qui peut être substitué avec 1, 2 ou 3 substituants choisis indépendamment les uns des autres dans le groupe constitué par méthyle, éthyle, n-propyle, iso-propyle, n-butyle, iso-butyle, sec-butyle, tert-butyle, F, Cl, Br, $-CF_3$, -OH, $-O-CH_3$ et $-O-C_2H_5$ ;
$R^{21}$ représente un radical hydrogène
ou un radical phényle qui peut être substitué avec 1, 2 ou 3 substituants choisis indépendamment les uns des autres dans le groupe constitué par méthyle, éthyle, n-propyle, iso-propyle, n-butyle, iso-butyle, sec-butyle, tert-butyle, F, Cl et Br ;
$R^{22}$ représente un radical pyrrolidinyle ou morpholinyle
ou un radical choisi dans le groupe constitué par phényle, naphtyle, (1,3)-benzodioxolyle, thiophényle, benzo[b]furanyle, benzo[b]thiophényle et quinolinyle, le radical pouvant à chaque fois être substitué avec 1, 2 ou 3 substituants choisis indépendamment les uns des autres dans le groupe constitué par méthyle, éthyle, n-propyle, F, Cl, Br, -OH, $-O-CH_3$ et $-O-C_2H_5$ ;
$R^{23}$ représente un radical phényle ;
$R^{24}$ représente un radical alkyle choisi dans le groupe constitué par méthyle, éthyle, n-propyle, iso-propyle, n-butyle, iso-butyle, sec-butyle et tert-butyle ;
$R^{25}$ représente un radical choisi dans le groupe constitué par phényle, naphtyle, furanyle, pyrazinyle et pyrimidinyle, qui peut être relié par un groupe $-(CH_2)-$ et/ou substitué avec 1, 2 ou 3 substituants choisis indépendamment les uns des autres dans le groupe constitué par méthyle, éthyle, n-propyle et iso-propyle ;
$R^{26}$ représente un radical alkyle choisi dans le groupe constitué par méthyle, éthyle, n-propyle et iso-propyle ;
$R^{27}$ représente un radical alkyle choisi dans le groupe constitué par méthyle, éthyle, iso-propyle et n-propyle

et
$R^{28}$ représente un radical phényle ;

à chaque fois éventuellement sous la forme d'un de leurs stéréoisomères purs, notamment énantiomères ou diastéréoisomères, leurs racémates ou sous la forme d'un mélange de stéréoisomères, notamment les énantiomères et/ou les diastéréoisomères, en un rapport de mélange quelconque, ou à chaque fois sous la forme des sels correspondants, ou à chaque fois sous la forme des solvates correspondants.

**16.** Composés selon une ou plusieurs des revendications 1 à 15, choisis dans le groupe constitué par

[1] (2-thiophén-2-yl-éthyl)-amide de l'acide 2-[(3-chloro-benzoyl)-méthyl-amino]-5,6,7,8-tétrahydro-imidazo [1,2-a]pyridin-3-carboxylique,
[2] [2-(2,5-diméthoxy-phényl)-éthyl]-amide de l'acide 2-[butyl-(3-chloro-benzoyl)-amino]-5,6,7,8-tétrahydro-imidazo[1,2-a]pyridin-3-carboxylique,
[3] N-{3-[4-(2-éthyl-phényl)-pipérazin-1-carbonyl]-5,6,7,8-tétrahydro-imidazo[1,2-a]pyridin-2-yl}-N-méthyl-benzamide,
[4] [3-(2-méthyl-pipéridin-1-yl)-propyl]-amide de l'acide 2-(méthyl-phénylacétyl-amino)-5,6,7,8-tétrahydro-imidazo[1,2-a]pyridin-3-carboxylique,
[5] (2-phényl-propyl)-amide de l'acide 2-[(3-chloro-benzoyl)-méthyl-amino]-5,6,7,8-tétrahydro-imidazo[1,2-a] pyridin-3-carboxylique,
[6] (2-pyrrolidin-1-yl-éthyl)-amide de l'acide 2-[(3-chloro-benzoyl)-méthyl-amino]-5,6,7,8-tétrahydro-imidazo [1,2-a]pyridin-3-carboxylique,
[7] éthyl-pyridin-4-ylméthyl-amide de l'acide 2-[méthyl-(naphtalèn-1-carbonyl)-amino]-5,6,7,8-tétrahydro-imidazo[1,2-a]pyridin-3-carboxylique,
[8] (2-thiophén-2-yl-éthyl)-amide de l'acide 2-(benzoyl-méthyl-amino)-5,6,7,8-tétrahydro-imidazo[1,2-a]pyridin-3-carboxylique,
[9] (3-imidazol-1-yl-propyl)-amide de l'acide 2-[butyl-(3-chloro-benzoyl)-amino]-5,6,7,8-tétrahydro-imidazo[1,2-a]pyridin-3-carboxylique,
[10] 3-chloro-N-{3-[4-(3-chloro-phényl)-pipérazin-1-carbonyl]-5,6,7,8-tétrahydro-imidazo[1,2-a]pyridin-2-yl}-N-méthyl-benzamide,
[11] 3-chloro-N-méthyl-N-[3-(4-phényl-pipérazin-1-carbonyl)-5,6,7,8-tétrahydro-imidazo[1,2-a]pyridin-2-yl]-benzamide,
[12] indan-1-ylamide de l'acide 2-[butyl-(3,4-difluoro-benzoyl)-amino]-5,6,7,8-tétrahydro-imidazo[1,2-a]pyridin-3-carboxylique,
[13] N-butyl-3-chloro-N-{3-[4-(2-morpholin-4-yl-éthyl)-pipérazin-1-carbonyl]-5,6,7,8-tétrahydro-imidazo[1,2-a] pyridin-2-yl}-benzamide,
[14] 3-chloro-N-méthyl-N-{3-[4-(3-trifluorométhyl-phényl)-pipérazin-1-carbonyl]-5,6,7,8-tétrahydro-imidazo [1,2-a]pyridin-2-yl}-benzamide,
[15] 3,5-bis-trifluorométhyl-benzylamide de l'acide 2-[méthyl-(naphtalèn-1-carbonyl)-amino]-5,6,7,8-tétrahydro-imidazo[1,2-a]pyridin-3-carboxylique,
[16] (4-méthyl-cyclohexyl)-amide de l'acide 2-[butyl-(3,4-difluoro-benzoyl)-amino]-5,6,7,8-tétrahydro-imidazo [1,2-a]pyridin-3-carboxylique,
[17] 3-méthoxy-benzylamide de l'acide 2-(benzoyl-méthyl-amino)-5,6,7,8-tétrahydro-imidazo[1,2-a]pyridin-3-carboxylique,
[18] (1-p-tolyl-éthyl)-amide de l'acide 2-[méthyl-(naphtalèn-1-carbonyl)-amino]-5,6,7,8-tétrahydro-imidazo[1,2-a]pyridin-3-carboxylique,
[19] (1,2-diméthyl-propyl)-amide de l'acide 2-[butyl-(3-chloro-benzoyl)-amino]-5,6,7,8-tétrahydro-imidazo[1,2-a]pyridin-3-carboxylique,
[20] (4-méthyl-cyclohexyl)-amide de l'acide 2-(benzoyl-méthyl-amino)-5,6,7,8-tétrahydro-imidazo[1,2-a]pyridin-3-carboxylique,
[21] (2-phényl-propyl)-amide de l'acide 2-(benzoyl-méthyl-amino)-5,6,7,8-tétrahydro-imidazo[1,2-a]pyridin-3-carboxylique,
[22] ester éthylique de l'acide 4-[2-(benzoyl-méthyl-amino)-5,6,7,8-tétrahydro-imidazo[1,2-a]pyridin-3-carbonyl]-pipérazin-1-carboxylique,
[23] 3-chloro-N-méthyl-N-(3-{4-[(méthyl-phényl-carbamoyl)-méthyl]-pipérazin-1-carbonyl}-5,6,7,8-tétrahydro-imidazo[1,2-a]pyridin-2-yl)-benzamide,
[24] 3-chloro-N-{3-[4-(furan-2-carbonyl)-pipérazin-1-carbonyl]-5,6,7,8-tétrahydro-imidazo[1,2-a]pyridin-2-yl}-N-méthyl-benzamide,

[25] N-méthyl-N-[3-(4-p-tolyl-pipérazin-1-carbonyl)-5,6,7,8-tétrahydro-imidazo[1,2-a]pyridin-2-yl]-benzamide,
[26] N-butyl-3-chloro-N-{3-[4-(3-phényl-propyl)-pipérazin-1-carbonyl]-5,6,7,8-tétrahydro-imidazo[1,2-a]pyridin-2-yl}-benzamide,
[27] (2,6,6-triméthyl-bicyclo[3.1.1]hept-3-yl)-amide de l'acide 2-[(3-chloro-benzoyl)-méthyl-amino]-5,6,7,8-tétrahydro-imidazo[1,2-a]pyridin-3-carboxylique,
[28] (3-phényl-propyl)-amide de l'acide 2-[butyl-(3,4-difluoro-benzoyl)-amino]-5,6,7,8-tétrahydro-imidazo[1,2-a]pyridin-3-carboxylique,
[29] {3-[4-(4-méthoxy-phényl)-pipérazin-1-carbonyl]-5,6,7,8-tétrahydro-imidazo[1,2-a]pyridin-2-yl}-méthyl-amide de l'acide naphtalèn-1-carboxylique,
[30] [1-(4-trifluorométhyl-benzyl)-pyrrolidin-3-yl]-amide de l'acide 2-[méthyl-(naphtalèn-1-carbonyl)-amino]-5,6,7,8-tétrahydro-imidazo[1,2-a]pyridin-3-carboxylique,
[31] (4-éthyl-phényl)-amide de l'acide 2-[(3-chlorobenzoyl)-méthyl-amino]-5,6,7,8-tétrahydro-imidazo[1,2-a]pyridin-3-carboxylique,
[32] (3-méthoxy-benzyl)-(tétrahydro-furan-2-ylméthyl)-amide de l'acide 2-[méthyl-(naphtalèn-1-carbonyl)-amino]-5,6,7,8-tétrahydro-imidazo[1,2-a]pyridin-3-carboxylique,
[33] 2,3-dichloro-benzylamide de l'acide 2-[méthyl-(naphtalèn-1-carbonyl)-amino]-5,6,7,8-tétrahydro-imidazo[1,2-a]pyridin-3-carboxylique,
[34] (1-benzofuran-2-ylméthyl-pyrrolidin-3-yl)-méthyl-amide de l'acide 2-[butyl-(3,4-difluoro-benzoyl)-amino]-5,6,7,8-tétrahydro-imidazo[1,2-a]pyridin-3-carboxylique,
[35] 3-chloro-N-{3-[4-(4-chloro-phényl)-pipérazin-1-carbonyl]-5,6,7,8-tétrahydro-imidazo[1,2-a]pyridin-2-yl}-N-méthyl-benzamide,
[36] ester méthylique de l'acide 2-({2-[(3-chlorobenzoyl)-méthyl-amino]-5,6,7,8-tétrahydro-imidazo[1,2-a]pyridin-3-carbonyl}-amino)-3-(1H-indol-3-yl)-propionique,
[37] N-butyl-3-chloro-N-[3-(4-phénylacétyl-pipérazin-1-carbonyl)-5,6,7,8-tétrahydro-imidazo[1,2-a]pyridin-2-yl]-benzamide,
[38] ester tert-butylique de l'acide 2-({2-[(3-chloro-benzoyl)-méthyl-amino]-5,6,7,8-tétrahydro-imidazo[1,2-a]pyridin-3-carbonyl}-amino)-3-méthyl-pentanoïque,
[39] (1-naphtalèn-1-yl-éthyl)-amide de l'acide 2-[butyl-(3-chloro-benzoyl)-amino]-5,6,7,8-tétrahydro-imidazo[1,2-a]pyridin-3-carboxylique,
[40] allyl-méthyl-amide de l'acide 2-[méthyl-(naphtalèn-1-carbonyl)-amino]-5,6,7,8-tétrahydro-imidazo[1,2-a]pyridin-3-carboxylique,
[41] N-butyl-N-[3-(3,6-dihydro-2H-pyridin-1-carbonyl)-5,6,7,8-tétrahydro-imidazo[1,2-a]pyridin-2-yl]-3,4-difluoro-benzamide,
[42] ester benzylique de l'acide 2-({2-[(3-chlorobenzoyl)-méthyl-amino]-5,6,7,8-tétrahydro-imidazo[1,2-a]pyridin-3-carbonyl}-amino)-4-méthyl-pentanoïque,
[43] 2-éthoxy-benzylamide de l'acide 2-[butyl-(3-chloro-benzoyl)-amino]-5,6,7,8-tétrahydro-imidazo[1,2-a]pyridin-3-carboxylique,
[44] N-butyl-3-chloro-N-{3-[4-(5-methyl-pyrazin-2-carbonyl)-pipérazin-1-carbonyl]-5,6,7,8-tétrahydro-imidazo[1,2-a]pyridin-2-yl}-benzamide,
[45] N-[3-(4-benzo[1,3]dioxol-5-ylméthyl-pipérazin-1-carbonyl)-5,6,7,8-tétrahydro-imidazo[1,2-a]pyridin-2-yl]-3-chloro-N-méthyl-benzamide,
[46] (2,2-diphényl-éthyl)-amide de l'acide 2-[méthyl-(naphtalèn-1-carbonyl)-amino]-5,6,7,8-tétrahydro-imidazo[1,2-a]pyridin-3-carboxylique,
[47] 2,4-difluoro-benzylamide de l'acide 2-[butyl-(3-chloro-benzoyl)-amino]-5,6,7,8-tétrahydro-imidazo[1,2-a]pyridin-3-carboxylique,
[48] N-butyl-3-chloro-N-{3-[4-(2-fluoro-phényl)-pipérazin-1-carbonyl]-5,6,7,8-tétrahydro-imidazo[1,2-a]pyridin-2-yl}-benzamide,
[49] (2-p-tolyl-éthyl)-amide de l'acide 2-[méthyl-(naphtalèn-1-carbonyl)-amino]-5,6,7,8-tétrahydro-imidazo[1,2-a]pyridin-3-carboxylique,
[50] (pyridin-2-ylméthyl)-amide de l'acide 2-[méthyl-(naphtalèn-1-carbonyl)-amino]-5,6,7,8-tétrahydro-imidazo[1,2-a]pyridin-3-carboxylique,
[51] 2-trifluorométhyl-benzylamide de l'acide 2-[butyl-(3-chloro-benzoyl)-amino]-5,6,7,8-tétrahydro-imidazo[1,2-a]pyridin-3-carboxylique,
[52] indan-1-ylamide de l'acide 2-[butyl-(3-chlorobenzoyl)-amino]-5,6,7,8-tétrahydro-imidazo[1,2-a]pyridin-3-carboxylique,
[53] 3-chloro-N-méthyl-N-[3-(4-quinolin-2-ylméthyl-pipérazin-1-carbonyl)-5,6,7,8-tétrahydro-imidazo[1,2-a]pyridin-2-yl]-benzamide,
[54] N-butyl-3,4-difluoro-N-[3-(4-méthyl-pipérazin-1-carbonyl)-5,6,7,8-tétrahydro-imidazo[1,2-a]pyridin-2-yl]-benzamide,

[55] 2,4-difluoro-benzylamide de l'acide 2-[(3-chloro-benzoyl)-méthyl-amino]-5,6,7,8-tétrahydro-imidazo[1,2-a] pyridin-3-carboxylique,

[56] N-[3-(4-benzhydryl-pipérazin-1-carbonyl)-5,6,7,8-tétrahydro-imidazo[1,2a]pyridin-2-yl]-N-butyl-3,4-difluoro-benzamide,

[57] ester benzylique de l'acide 4-méthyl-2-({2-[méthyl-(naphtalèn-1-carbonyl)-amino]-5,6,7,8-tétrahydro-imidazo[1,2-a]pyridin-3-carbonyl}-amino)-pentanoïque,

[58] (2-pyrrolidin-1-yl-éthyl)-amide de l'acide 2-[butyl-(3-chloro-benzoyl)-amino]-5,6,7,8-tétrahydro-imidazo [1,2-a]pyridin-3-carboxylique,

[59] [1-(2-benzyloxy-benzyl)-pyrrolidin-3-yl]-amide de l'acide 2-[(3-chloro-benzoyl)-méthyl-amino]-5,6,7,8-tétrahydro-imidazo[1,2-a]pyridin-3-carboxylique,

[60] (pyridin-3-ylméthyl)-amide de l'acide 2-[butyl-(3,4-difluoro-benzoyl)-amino]-5,6,7,8-tétrahydro-imidazo[1,2-a]pyridin-3-carboxylique,

[61] 3-chloro-N-{3-[4-(4-fluoro-phényl)-3,6-dihydro-2H-pyridin-1-carbonyl]-5,6,7,8-tétrahydro-imidazo[1,2-a] pyridin-2-yl}-N-méthyl-benzamide,

[62] cyclohexylamide de l'acide 2-[(3-chlorobenzoyl)-méthyl-amino]-5,6,7,8-tétrahydro-imidazo[1,2-a]pyridin-3-carboxylique,

[63] N-[3-(4-cycloheptyl-pipérazin-1-carbonyl)-5,6,7,8-tétrahydro-imidazo[1,2-a]pyridin-2-yl]-N-méthyl-benzamide,

[64] méthyl-[3-(4-thiophén-3-ylméthyl-pipérazin-1-carbonyl)-5,6,7,8-tétrahydro-imidazo[1,2-a]pyridin-2-yl]-amide de l'acide naphtalèn-1-carboxylique,

[65] 2,4-diméthoxy-benzylamide de l'acide 2-[butyl-(3-chloro-benzoyl)-amino]-5,6,7,8-tétrahydro-imidazo[1,2-a]pyridin-3-carboxylique,

[66] ester éthylique de l'acide 1-[2-(benzoyl-méthyl-amino)-5,6,7,8-tétrahydro-imidazo[1,2-a]pyridin-3-carbonyl]-pipéridin-4-carboxylique,

[67] (2-benzyloxy-cyclohexyl)-amide de l'acide 2-[(3-chloro-benzoyl)-méthyl-amino]-5,6,7,8-tétrahydro-imidazo [1,2-a]pyridin-3-carboxylique,

[68] [2-(4-phénoxy-phényl)-éthyl]-amide de l'acide 2-[méthyl-(naphtalèn-1-carbonyl)-amino]-5,6,7,8-tétrahydro-imidazo[1,2-a]pyridin-3-carboxylique,

[69] [2-(7-méthyl-1H-indol-3-yl)-éthyl]-amide de l'acide 2-[butyl-(3-chloro-benzoyl)-amino]-5,6,7,8-tétrahydro-imidazo[1,2-a]pyridin-3-carboxylique,

[70] méthyl-[3-(4-phényl-pipérazin-1-carbonyl)-5,6,7,8-tétrahydro-imidazo[1,2-a]pyridin-2-yl]-amide de l'acide naphtalèn-1-carboxylique,

[71] [2-(3-trifluorométhyl-phényl)-éthyl]-amide de l'acide 2-[(3-chloro-benzoyl)-méthyl-amino]-5,6,7,8-tétrahydro-imidazo[1,2-a]pyridin-3-carboxylique,

[72] N-butyl-3-chloro-N-[3-(4-pyridin-2-yl-pipérazin-1-carbonyl)-5,6,7,8-tétrahydro-imidazo[1,2-a]pyridin-2-yl]-benzamide,

[73] 3-méthoxy-benzylamide de l'acide 2-[méthyl-(naphtalèn-1-carbonyl)-amino]-5,6,7,8-tétrahydro-imidazo [1,2-a]pyridin-3-carboxylique,

[74] 2,4-difluoro-benzylamide de l'acide 2-[butyl-(3,4-difluoro-benzoyl)-aminol]-5,6,7,8-tétrahydro-imidazo[1,2-a]pyridin-3-carboxylique,

[75] 3-chloro-N-méthyl-N-[3-(4-pyridin-2-yl-pipérazin-1-carbonyl)-5,6,7,8-tétrahydro-imidazo[1,2-a]pyridin-2-yl]-benzamide,

[76] (1-biphényl-4-ylméthyl-pyrrolidin-3-yl)-méthyl-amide de l'acide 2-[méthyl-(naphtalèn-1-carbonyl)-amino]-5,6,7,8-tétrahydro-imidazo[1,2-a]pyridin-3-carboxylique,

[77] (2,3-dihydro-benzo[1,4]dioxin-2-ylméthyl)-amide de l'acide 2-[butyl-(3-chloro-benzoyl)-amino]-5,6,7,8-tétrahydro-imidazo[1,2-a]pyridin-3-carboxylique,

[78] [2-(1H-indol-3-yl)-éthyl]-méthyl-amide de l'acide 2-[butyl-(3-chloro-benzoyl)-amino]-5,6,7,8-tétrahydro-imidazo[1,2-a]pyridin-3-carboxylique,

[79] N-butyl-3-chloro-N-{3-[4-(3-trifluorométhyl-phényl)-pipérazin-1-carbonyl]-5,6,7,8-tétrahydro-imidazo[1,2-a]pyridin-2-yl}-benzamide,

[80] [2-(1H-indol-3-yl)-éthyl]-méthyl-amide de l'acide 2-(benzoyl-méthyl-amino)-5,6,7,8-tétrahydro-imidazo[1,2-a]pyridin-3-carboxylique,

[81] 3-chloro-N-{3-[4-hydroxy-4-(3-trifluorométhyl-phényl)-pipéridin-1-carbonyl]-5,6,7,8-tétrahydro-imidazo [1,2-a]pyridin-2-yl}-N-méthyl-benzamide,

[82] (1-méthoxyméthyl-2-phenyl-éthyl)-amide de l'acide 2-[butyl-(3,4-difluoro-benzoyl)-amino]-5,6,7,8-tétrahydro-imidazo[1,2-a]pyridin-3-carboxylique,

[83] [1-(2,6-dichloro-benzyl)-pyrrolidin-3-yl]-amide de l'acide 2-[(3-chloro-benzoyl)-méthyl-amino]-5,6,7,8-tétrahydro-imidazo[1,2-a]pyridin-3-carboxylique,
[84] [1-(2-bromo-4,5-diméthoxy-benzyl)-pyrrolidin-3-yl]-amide de l'acide 2-[(3-chloro-benzoyl)-méthyl-amino]-5,6,7,8-tétrahydro-imidazo[1,2-a]pyridin-3-carboxylique,
[85] benzo[1,3]dioxol-5-ylamide de l'acide 2-(benzoyl-méthyl-amino)-5,6,7,8-tétrahydro-imidazo[1,2-a]pyridin-3-carboxylique,
[86] (1-méthoxyméthyl-2-phényl-éthyl)-amide de l'acide 2-[butyl-(3-chloro-benzoyl)-amino]-5,6,7,8-tétrahydro-imidazo[1,2-a]pyridin-3-carboxylique,
[87] [1-(2,6-dichloro-benzyl)-pyrrolidin-3-yl]-méthyl-amide de l'acide 2-[butyl-(3-chlorobenzoyl)-amino]-5,6,7,8-tétrahydro-imidazo[1,2-a]pyridin-3-carboxylique,
[88] 2-trifluorométhoxy-benzylamide de l'acide 2-[butyl-(3,4-difluoro-benzoyl)-amino]-5,6,7,8-tétrahydro-imidazo[1,2-a]pyridin-3-carboxylique,
[89] N-butyl-3,4-difluoro-N-{3-[4-(isopropylcarbamoyl-méthyl)-pipérazin-1-carbonyl]-5,6,7,8-tétrahydro-imidazo[1,2-a]pyridin-2-yl}-benzamide,
[90] (1-méthyl-3-phényl-propyl)-amide de l'acide 2-[(3-chloro-benzoyl)-méthyl-amino]-5,6,7,8-tétrahydro-imidazo[1,2-a]pyridin-3-carboxylique,
[91] ester benzylique de l'acide 2-({2-[butyl-(3-chloro-benzoyl)-amino]-5,6,7,8-tétrahydro-imidazo[1,2-a]pyridin-3-carbonyl}-amino)-3-méthyl-butyrique,
[92] (thiophén-2-ylméthyl)-amide de l'acide 2-[(3-chloro-benzoyl)-méthyl-amino]-5,6,7,8-tétrahydro-imidazo [1,2-a]pyridin-3-carboxylique,
[93] (2-méthyl-cyclohexyl)-amide de l'acide 2-[butyl-(3,4-difluoro-benzoyl)-amino]-5,6,7,8-tétrahydro-imidazo [1,2-a]pyridin-3-carboxylique,
[94] benzyl-méthyl-amide de l'acide 2-(benzoyl-méthyl-amino)-5,6,7,8-tétrahydro-imidazo[1,2-a]pyridin-3-carboxylique,
[95] [2-(2,6-dichloro-benzylsulfanyl)-éthyl]-amide de l'acide 2-[butyl-(3-chloro-benzoyl)-amino]-5,6,7,8-tétrahydro-imidazo[1,2-a]pyridin-3-carboxylique,
[96] (2,3-dihydro-benzo[1,4]dioxin-2-ylméthyl)-amide de l'acide 2-[butyl-(3,4-difluoro-benzoyl)-amino]-5,6,7,8-tétrahydro-imidazo[1,2-a]pyridin-3-carboxylique,
[97] 4-trifluorométhyl-benzylamide de l'acide 2-[(3-chloro-benzoyl)-méthyl-amino]-5,6,7,8-tétrahydro-imidazo [1,2-a]pyridin-3-carboxylique,
[98] N-[3-(4-benzyl-pipéridin-1-carbonyl)-5,6,7,8-tétrahydro-imidazo[1,2-a]pyridin-2-yl]-N-méthyl-benzamide,
[99] 2-trifluorométhoxy-benzylamide de l'acide 2-[méthyl-(naphtalèn-1-carbonyl)-amino]-5,6,7,8-tétrahydro-imidazo[1,2-a]pyridin-3-carboxylique,
[100] 3-chloro-N-{3-[4-(2,5-diméthyl-phényl)-pipérazin-1-carbonyl]-5,6,7,8-tétrahydro-imidazo[1,2-a]pyridin-2-yl}-N-méthyl-benzamide,
[101] [1-(2,6-dichloro-benzyl)-pyrrolidin-3-yl]-amide de l'acide 2-(benzoyl-méthyl-amino)-5,6,7,8-tétrahydro-imidazo[1,2-a]pyridin-3-carboxylique,
[102] 2-chloro-6-méthyl-benzylamide de l'acide 2-[butyl-(3,4-difluoro-benzoyl)-amino]-5,6,7,8-tétrahydro-imidazo[1,2-a]pyridin-3-carboxylique,
[103] N-butyl-3-chloro-N-[3-(3,5-diméthyl-pipéridin-1-carbonyl)-5,6,7,8-tétrahydro-imidazo[1,2-a]pyridin-2-yl]-benzamide,
[104] ester tert-butylique de l'acide 2-({2-[(3-chloro-benzoyl)-méthyl-amino]-5,6,7,8-tétrahydro-imidazo[1,2-a] pyridin-3-carbonyl}-amino)-3,3-diméthyl-butyrique,
[105] (1-cyclohexyl-éthyl)-amide de l'acide 2-[méthyl-(naphtalèn-1-carbonyl)-amino]-5,6,7,8-tétrahydro-imidazo[1,2-a]pyridin-3-carboxylique,
[106] 3-chloro-N-méthyl-N-[3-(4-phenéthyl-pipérazin-1-carbonyl)-5,6,7,8-tétrahydro-imidazo[1,2-a]pyridin-2-yl]-benzamide,
[107] (2-phénoxy-éthyl)-amide de l'acide 2-[butyl-(3-chloro-benzoyl)-amino]-5,6,7,8-tétrahydro-imidazo[1,2-a] pyridin-3-carboxylique,
[108] [3-(4-benzofuran-2-ylméthyl-pipérazin-1-carbonyl)-5,6,7,8-tétrahydro-imidazo[1,2-a]pyridin-2-yl]-méthyl-amide de l'acide naphtalèn-1-carboxylique,
[109] N-[3-([1,4']bipipéridinyl-1'-carbonyl)-5,6,7,8-tétrahydro-imidazo[1,2-a]pyridin-2-yl]-N-butyl-3-chloro-benzamide,
[110] N-butyl-3-chloro-N-[3-(8-fluoro-1,3,4,5-tétrahydro-pyrido[4,3-b]indol-2-carbonyl)-5,6,7,8-tétrahydro-imidazo[1,2-a]pyridin-2-yl]-benzamide,
[111] 4-diméthylamino-benzylamide de l'acide 2-[méthyl-(naphtalèn-1-carbonyl)-amino]-5,6,7,8-tétrahydro-imidazo[1,2-a]pyridin-3-carboxylique,
[112] (1-méthyl-3-phényl-propyl)-amide de l'acide 2-[méthyl-(naphtalèn-1-carbonyl)-amino]-5,6,7,8-tétrahydro-imidazo[1,2-a]pyridin-3-carboxylique,

[113] 3-méthyl-benzylamide de l'acide 2-[butyl-(3-chloro-benzoyl)-amino]-5,6,7,8-tétrahydro-imidazo[1,2-a]pyridin-3-carboxylique,
[114] (2,3-dihydro-benzo[1,4]dioxin-6-yl)-amide de l'acide 2-[(3-chloro-benzoyl)-méthyl-amino]-5,6,7,8-tétrahydro-imidazo[1,2-a]pyridin-3-carboxylique,
[115] méthyl-[3-(4-quinolin-2-ylméthyl-pipérazin-1-carbonyl)-5,6,7,8-tétrahydro-imidazo[1,2-a]pyridin-2-yl]-amide de l'acide naphtalèn-1-carboxylique,
[116] (2-cyano-éthyl)-méthyl-amide de l'acide 2-[butyl-(3,4-difluoro-benzoyl)-amino]-5,6,7,8-tétrahydro-imidazo [1,2-a]pyridin-3-carboxylique,
[117] (2-p-tolyl-éthyl)-amide de l'acide 2-(méthyl-phénylacétyl-amino)-5,6,7,8-tétrahydro-imidazo[1,2-a]pyridin-3-carboxylique,
[118] [1-(4-fluoro-phényl)-éthyl]-amide de l'acide 2-[butyl-(3,4-difluoro-benzoyl)-amino]-5,6,7,8-tétrahydro-imidazo[1,2-a]pyridin-3-carboxylique,
[119] N-butyl-N-{3-[4-(5-chloro-2-méthyl-phényl)-pipérazin-1-carbonyl]-5,6,7,8-tétrahydro-imidazol[1,2-a]pyridin-2-yl}-3,4-difluoro-benzamide,
[120] cyclohexylamide de l'acide 2-[butyl-(3-chlorobenzoyl)-amino]-5,6,7,8-tétrahydro-imidazo[1,2-a]pyridin-3-carboxylique,
[121] (benzo[1,3]dioxol-5-ylmethyl)-amide de l'acide 2-[(3-chloro-benzoyl)-méthyl-amino]-5,6,7,8-tétrahydro-imidazo[1,2-a]pyridin-3-carboxylique,
[122] 3-méthoxy-benzylamide de l'acide 2-[butyl-(3,4-difluoro-benzoyl)-amino]-5,6,7,8-tétrahydro-imidazo[1,2-a]pyridin-3-carboxylique,
[123] ester méthylique de l'acide 3-tert-butoxy-2-({2-[(3-chloro-benzoyl)-méthyl-amino]-5,6,7,8-tétrahydro-imidazo[1,2-a]pyridin-3-carbonyl}-amino)-butyrique,
[124] (2-benzyloxy-cyclohexyl)-amide de l'acide 2-[butyl-(3-chloro-benzoyl)-amino]-5,6,7,8-tétrahydro-imidazo [1,2-a]pyridin-3-carboxylique,
[125] N-{3-[4-(4-fluoro-phényl)-pipérazin-1-carbonyl]-5,6,7,8-tétrahydro-imidazo[1,2-a]pyridin-2-yl}-N-méthyl-benzamide,
[126] (naphtalèn-2-ylcarbamoylméthyl)-amide de l'acide 2-(benzoyl-méthyl-amino)-5,6,7,8-tétrahydro-imidazo [1,2-a]pyridin-3-carboxylique,
[127] 4-trifluorométhoxy-benzylamide de l'acide 2-[butyl-(3-chloro-benzoyl)-amino]-5,6,7,8-tétrahydro-imidazo [1,2-a]pyridin-3-carboxylique,
[128] (3,3-diphényl-propyl)-amide de l'acide 2-[(3-chloro-benzoyl)-méthyl-amino]-5,6,7,8-tétrahydro-imidazo [1,2-a]pyridin-3-carboxylique,
[129] (4-benzyloxy-phényl)-amide de l'acide 2-[(3-chloro-benzoyl)-méthyl-amino]-5,6,7,8-tétrahydro-imidazo [1,2-a]pyridin-3-carboxylique,
[130] N-{3-[4-(5-bromo-2-éthoxy-benzyl)-pipérazin-1-carbonyl]-5,6,7,8-tétrahydro-imidazo[1,2-a]pyridin-2-yl}-N-méthyl-2-phényl-acétamide,
[131] N-[3-(3,4-dihydro-1H-isoquinolin-2-carbonyl)-5,6,7,8-tétrahydro-imidazo[1,2-a]pyridin-2-yl]-N-méthyl-benzamide,
[132] (1-phényl-propyl)-amide de l'acide 2-[butyl-(3-chloro-benzoyl)-amino]-5,6,7,8-tétrahydro-imidazo[1,2-a] pyridin-3-carboxylique,
[133] 2,3-diméthyl-benzylamide de l'acide 2-[butyl-(3-chloro-benzoyl)-amino]-5,6,7,8-tétrahydro-imidazo[1,2-a] pyridin-3-carboxylique,
[134] [2-(4-phénoxy-phényl)-éthyl]-amide de l'acide 2-[butyl-(3-chloro-benzoyl)-amino]-5,6,7,8-tétrahydro-imidazo[1,2-a]pyridin-3-carboxylique,
[135] N-{3-[4-(4-éthoxy-phényl)-pipérazin-1-carbonyl]-5,6,7,8-tétrahydro-imidazo[1,2-a]pyridin-2-yl}-N-méthyl-2-phényl-acétamide,
[136] {3-[4-(2-éthyl-phényl)-pipérazin-1-carbonyl]-5,6,7,8-tétrahydro-imidazo[1,2-a]pyridin-2-yl}-méthyl-amide de l'acide naphtalèn-1-carboxylique,
[137] N-[3-(4-benzo[1,3]dioxol-5-ylméthyl-pipérazin-1-carbonyl)-5,6,7,8-tétrahydro-imidazo[1,2-a]pyridin-2-yl]-N-butyl-3,4-difluoro-benzamide,
[138] N-butyl-N-{3-[4-(2,5-diméthyl-phényl)-pipérazin-1-carbonyl]-5,6,7,8-tétrahydro-imidazo[1,2-a]pyridin-2-yl}-3,4-difluoro-benzamide,
[139] [(4-chloro-phényl)-phényl-méthyl]-amide de l'acide 2-[méthyl-(naphtalèn-1-carbonyl)-amino]-5,6,7,8-tétrahydro-imidazo[1,2-a]pyridin-3-carboxylique,
[140] N-butyl-3-chloro-N-{3-[4-(4-chloro-benzyl)-pipérazin-1-carbonyl]-5,6,7,8-tétrahydro-imidazo[1,2-a]pyridin-2-yl}-benzamide,
[141] (1-naphtalèn-2-yl-éthyl)-amide de l'acide 2-[butyl-(3-chloro-benzoyl)-amino]-5,6,7,8-tétrahydro-imidazo [1,2-a]pyridin-3-carboxylique,

[142] 2-fluoro-benzylamide de l'acide 2-(méthyl-phénylacétyl-amino)-5,6,7,8-tétrahydro-imidazo[1,2-a]pyridin-3-carboxylique,
[143] N-[3-(1,4-dioxa-8-aza-spiro[4.5]décane-8-carbonyl)-5,6,7,8-tétrahydro-imidazo[1,2-a]pyridin-2-yl]-N-méthyl-2-phényl-acétamide,
[144] méthyl-[3-(1,3,4,9-tétrahydro-b-carbolin-2-carbonyl)-5,6,7,8-tétrahydro-imidazo[1,2-a]pyridin-2-yl]-amide de l'acide naphtalèn-1-carboxylique,
[145] (1-méthyl-3-phényl-propyl)-amide de l'acide 2-[butyl-(3-chloro-benzoyl)-amino]-5,6,7,8-tétrahydro-imidazo[1,2-a]pyridin-3-carboxylique,
[146] 3-chloro-N-méthyl-N-{3-[4-(2,4,6-triméthoxy-benzyl)-pipérazin-1-carbonyl]-5,6,7,8-tétrahydro-imidazo [1,2-a]pyridin-2-yl}-benzamide,
[147] benzhydryl-amide de l'acide 2-[butyl-(3-chlorobenzoyl)-amino]-5,6,7,8-tétrahydro-imidazo[1,2-a]pyridin-3-carboxylique,
[148] {3-[4-(2-chloro-phényl)-pipérazin-1-carbonyl]-5,6,7,8-tétrahydro-imidazo[1,2-a]pyridin-2-yl}-méthyl-amide naphtalèn-1-carboxylique,
[149] 2-[butyl-(3-chloro-benzoyl)-amino]-5,6,7,8-tétrahydro-imidazo[1,2-a]pyridin-3-carboxylique(thiophén-2-ylméthyl)-amide,
[150] N-butyl-N-{3-[4-(4-chloro-benzyl)-pipérazin-1-carbonyl]-5,6,7,8-tétrahydro-imidazo[1,2-a]pyridin-2-yl}-3,4-difluoro-benzamide,
[151] (3-phényl-propyl)-amide de l'acide 2-(benzoyl-méthyl-amino)-5,6,7,8-tétrahydro-imidazo[1,2-a]pyridin-3-carboxylique,
[152] (4-tert-butyl-phényl)-amide de l'acide 2-[butyl-(3-chloro-benzoyl)-amino]-5,6,7,8-tétrahydro-imidazo[1,2-a]pyridin-3-carboxylique,
[153] (4-éthyl-phényl)-amide de l'acide 2-[butyl-(3-chloro-benzoyl)-amino]-5,6,7,8-tétrahydro-imidazo[1,2-a]pyridin-3-carboxylique,
[154] cyclohexylamide de l'acide 2-[méthyl-(naphtalèn-1-carbonyl)-amino]-5,6,7,8-tétrahydro-imidazo[1,2-a]pyridin-3-carboxylique,
[155] [3-(méthyl-phényl-amino)-propyl]-amide de l'acide 2-[méthyl-(naphtalèn-1-carbonyl)-amino]-5,6,7,8-tétrahydro-imidazo[1,2-a]pyridin-3-carboxylique,
[156] [2-(3-trifluorométhyl-phényl)-éthyl]-amide de l'acide 2-(méthyl-phénylacétyl-amino)-5,6,7,8-tétrahydro-imidazo[1,2-a]pyridin-3-carboxylique,
[157] 4-chloro-benzylamide de l'acide 2-(benzoyl-méthyl-amino)-5,6,7,8-tétrahydro-imidazo[1,2-a]pyridin-3-carboxylique,
[158] 3-fluoro-benzylamide de l'acide 2-[butyl-(3-chloro-benzoyl)-amino]-5,6,7,8-tétrahydro-imidazo[1,2-a]pyridin-3-carboxylique,
[159] p-tolylamide de l'acide 2-(méthyl-phénylacétyl-amino)-5,6,7,8-tétrahydro-imidazo[1,2-a]pyridin-3-carboxylique,
[160] (2-phénoxy-éthyl)-amide de l'acide 2-(benzoyl-méthyl-amino)-5,6,7,8-tétrahydro-imidazo[1,2-a]pyridin-3-carboxylique,
[161] (2-benzyloxy-cyclohexyl)-amide de l'acide 2-[butyl-(3,4-difluoro-benzoyl)-amino]-5,6,7,8-tétrahydro-imidazo[1,2-a]pyridin-3-carboxylique,
[162] {3-[4-(4-fluoro-phényl)-pipérazin-1-carbonyl]-5,6,7,8-tétrahydro-imidazo[1,2-a]pyridin-2-yl}-méthyl-amide de l'acide naphtalèn-1-carboxylique,
[163] [2-(4-chloro-phényl)-propyl]-amide de l'acide 2-[méthyl-(naphtalèn-1-carbonyl)-amino]-5,6,7,8-tétrahydro-imidazo[1,2-a]pyridin-3-carboxylique,
[164] N-butyl-3,4-difluoro-N-[3-(thiomorpholin-4-carbonyl)-5,6,7,8-tétrahydro-imidazo[1,2-a]pyridin-2-yl]-benzamide,
[165] (1-adamantan-1-yl-éthyl)-amide de l'acide 2-[(3-chloro-benzoyl)-méthyl-amino]-5,6,7,8-tétrahydro-imidazo[1,2-a]pyridin-3-carboxylique,
[166] [(4-chloro-phényl)-phényl-méthyl]-amide de l'acide 2-(benzoyl-méthyl-amino)-5,6,7,8-tétrahydro-imidazo [1,2-a]pyridin-3-carboxylique,
[167] N-méthyl-N-{3-[4-(4-trifluorométhyl-phényl)-pipérazin-1-carbonyl]-5,6,7,8-tétrahydro-imidazo[1,2-a]pyridin-2-yl}-benzamide,
[168] (1-biphényl-4-ylméthyl-pyrrolidin-3-yl)-méthyl-amide de l'acide 2-(méthyl-phénylacétyl-amino)-5,6,7,8-tétrahydro-imidazo[1,2-a]pyridin-3-carboxylique,
[169] N-[3-(4-benzoyl-pipéridin-1-carbonyl)-5,6,7,8-tétrahydro-imidazo[1,2-a]pyridin-2-yl]-3-chloro-N-méthyl-benzamide,
[170] (4-tert-butyl-phényl)-amide de l'acide 2-[méthyl-(naphtalèn-1-carbonyl)-amino]-5,6,7,8-tétrahydro-imidazo[1,2-a]pyridin-3-carboxylique,

[171] (3,3-diméthyl-butyl)-amide de l'acide 2-[butyl-(3,4-difluoro-benzoyl)-amino]-5,6,7,8-tétrahydro-imidazo [1,2-a]pyridin-3-carboxylique,
[172] [1-(2-brom-4,5-diméthoxy-benzyl)-pyrrolidin-3-yl]-amide de l'acide 2-[butyl-(3-chlorobenzoyl)-amino]-5,6,7,8-tétrahydro-imidazo[1,2-a]pyridin-3-carboxylique,
[173] méthyl-{3-[4-(3-phényl-allyl)-pipérazin-1-carbonyl]-5,6,7,8-tétrahydro-imidazo[1,2-a]pyridin-2-yl}-amide de l'acide naphtalèn-1-carboxylique,
[174] méthyl-[3-(4-phenéthyl-pipérazin-1-carbonyl)-5,6,7,8-tétrahydro-imidazo[1,2-a]pyridin-2-yl]-amide de l'acide naphtalèn-1-carboxylique,
[175] N-butyl-3-chloro-N-{3-[4-hydroxy-4-(3-trifluorométhyl-phényl)-pipéridin-1-carbonyl]-5,6,7,8-tétrahydro-imidazo[1,2-a]pyridin-2-yl}-benzamide,
[176] N-méthyl-2-phényl-N-{3-[4-(3-phényl-propyl)-pipérazin-1-carbonyl]-5,6,7,8-tétrahydro-imidazo[1,2-a]pyridin-2-yl}-acétamide,
[177] 3-fluoro-benzylamide de l'acide 2-(benzoyl-méthyl-amino)-5,6,7,8-tétrahydro-imidazo[1,2-a]pyridin-3-carboxylique,
[178] N-butyl-N-{3-[4-(2-chloro-phényl)-pipérazin-1-carbonyl]-5,6,7,8-tétrahydro-imidazo[1,2-a]pyridin-2-yl}-3,4-difluoro-benzamide,
[179] benzo[1,3]dioxol-5-ylamide de l'acide 2-[méthyl-(naphtalèn-1-carbonyl)-amino]-5,6,7,8-tétrahydro-imidazo[1,2-a]pyridin-3-carboxylique,
[180] (3,3-diphényl-propyl)-amide de l'acide 2-[butyl-(3,4-difluoro-benzoyl)-amino]-5,6,7,8-tétrahydro-imidazo [1,2-a]pyridin-3-carboxylique,
[181] (2,3-dihydro-benzo[1,4]dioxin-6-yl)-amide de l'acide 2-[butyl-(3-chloro-benzoyl)-amino]-5,6,7,8-tétrahydro-imidazo[1,2-a]pyridin-3-carboxylique,
[182] [2-(3,4-diméthoxy-phényl)-éthyl]-amide de l'acide 2-(benzoyl-méthyl-amino)-5,6,7,8-tétrahydro-imidazo [1,2-a]pyridin-3-carboxylique,
[183] [3-(4-benzoyl-pipéridin-1-carbonyl)-5,6,7,8-tétrahydro-imidazo[1,2-a]pyridin-2-yl]-méthyl-amide de l'acide naphtalèn-1-carboxylique,
[184] ester tert-butylique de l'acide 4-méthyl-2-({2-[méthyl-(naphtalèn-1-carbonyl)-amino]-5,6,7,8-tétrahydro-imidazo[1,2-a]pyridin-3-carbonyl}-amino)-valérianique,
[185] (4-phénoxy-phényl)-amide de l'acide 2-[méthyl-(naphtalèn-1-carbonyl)-amino]-5,6,7,8-tétrahydro-imidazo[1,2-a]pyridin-3-carboxylique,
[186] N-méthyl-2-phényl-N-[3-(4-phényl-pipérazin-1-carbonyl)-5,6,7,8-tétrahydro-imidazo[1,2-a]pyridin-2-yl]-acétamide,
[187] N-butyl-3,4-difluoro-N-{3-[4-(5-trifluorométhyl-pyridin-2-yl)-pipérazin-1-carbonyl]-5,6,7,8-tétrahydro-imidazo[1,2-a]pyridin-2-yl}-benzamide,
[188] 2,4-dichloro-6-méthyl-benzylamide de l'acide 2-(benzoyl-méthyl-amino)-5,6,7,8-tétrahydro-imidazo[1,2-a]pyridin-3-carboxylique,
[189] ester tert-butylique de l'acide [4-({2-[butyl-(3-chloro-benzoyl)-amino]-5,6,7,8-tétrahydro-imidazo[1,2-a]pyridin-3-carbonyl}-amino)-phényl]-carbamique,
[190] N-méthyl-2-phényl-N-[3-(2-pyrrolidin-1-ylméthyl-pyrrolidin-1-carbonyl)-5,6,7,8-tétrahydro-imidazo[1,2-a] pyridin-2-yl]-acétamide
et
[191] ester tert-butylique de l'acide 4-[2-(méthyl-phénylacétyl-amino)-5,6,7,8-tétrahydro-imidazo[1,2-a]pyridin-3-carbonyl]-pipérazin-1-carboxylique,

à chaque fois éventuellement sous la forme d'un de leurs stéréoisomères purs, notamment énantiomères ou diastéréoisomères, leurs racémates ou sous la forme d'un mélange de stéréoisomères, notamment les énantiomères et/ou les diastéréoisomères, en un rapport de mélange quelconque, ou à chaque fois sous la forme des sels correspondants, ou à chaque fois sous la forme des solvates correspondants.

**17.** Composés selon une ou plusieurs des revendications 1 à 16, **caractérisés en ce que** les composés présentent lors du dosage FLIPR en une concentration de 10 μM une inhibition de l'afflux d'ions $Ca^{2+}$ dans les ganglions de la racine dorsale de rats d'au moins 30 %, de préférence d'au moins 50 %, de manière particulièrement préférée d'au moins 70 %, de manière toute particulièrement préférée d'au moins 80 %, de manière encore davantage préférée d'au moins 90 %, par rapport à l'inhibition maximale atteignable de l'afflux d'ions $Ca^{2+}$ avec la capsaïcine en une concentration de 10 μM.

**18.** Procédé de fabrication de composés de 5,6,7,8-tétrahydro-imidazo[1,2-a]pyridin-2-ylamine substitués de formule générale I selon une ou plusieurs des revendications 1 à 17, **caractérisé en ce qu'**au moins un composé de formule

générale II,

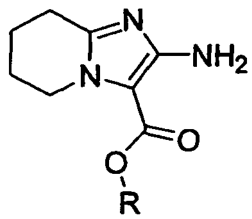

II

dans laquelle R représente un radical alkyle en $C_{1-6}$ linéaire ou ramifié, est mis en réaction dans un milieu réactionnel en présence d'au moins un agent réducteur, éventuellement en présence d'un acide organique, de préférence en présence d'acide acétique, avec au moins un composé de formule générale $R^3$-C(=O)-H, dans laquelle $R^3$ a la signification donnée dans une ou plusieurs des revendications 1 à 16, pour former un composé de formule générale III,

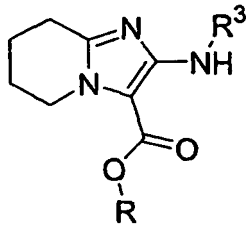

III

dans laquelle R et $R^3$ ont la signification mentionnée précédemment, et celui-ci est éventuellement purifié et/ou isolé et au moins un composé de formule générale III est mis en réaction dans un milieu réactionnel, éventuellement en présence d'au moins une base, avec au moins un composé de formule générale $R^4$-C(=O)-X, dans laquelle $R^4$ a la signification donnée dans une ou plusieurs des revendications 1 à 16 et X représente un groupe partant, de préférence un radical halogène, ou dans un milieu réactionnel en présence d'au moins un réactif de couplage, éventuellement en présence d'au moins une base, avec un composé de formule générale $R^4$-C(=O)-OH,
dans laquelle $R^4$ a la signification donnée dans une ou plusieurs des revendications 1 à 16, pour former un composé de formule générale IV,

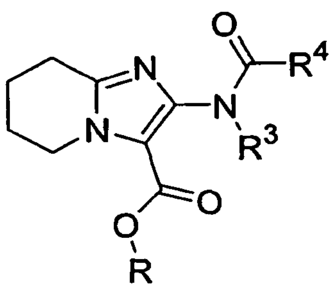

IV

dans laquelle R, $R^3$ et $R^4$ ont la signification mentionnée précédemment et $R^3$ ne représente pas l'hydrogène, et celui-ci est éventuellement purifié et/ou isolé
ou
au moins un composé de formule générale II, dans laquelle R a la signification mentionnée précédemment, est mis

en réaction dans un milieu réactionnel éventuellement en présence d'au moins une base, avec au moins un composé de formule générale $R^4$-C(=O)-X, dans laquelle $R^4$ a la signification mentionnée précédemment et X représente un groupe partant, de préférence un radical halogène, ou dans un milieu réactionnel en présence d'au moins un réactif de couplage, éventuellement en présence d'au moins une base, avec un composé de formule générale $R^4$-C (=O)-OH, dans laquelle $R^4$ a la signification mentionnée précédemment, pour former un composé de formule générale V,

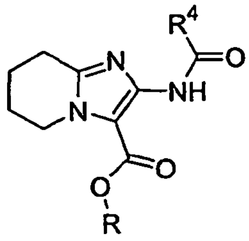

V

dans laquelle R et $R^4$ ont la signification mentionnée précédemment, et celui-ci est éventuellement purifié et/ou isolé, et au moins un composé de formule générale V est mis en réaction dans un milieu réactionnel en présence d'au moins une base, de préférence un sel d'hydrure métallique, avec au moins un composé de formule générale $R^3$-X, dans laquelle $R^3$ a la signification mentionnée précédemment et ne représente pas l'hydrogène et X représente un groupe partant, de préférence un radical halogène, pour former un composé de formule générale IV, dans laquelle R, $R^3$ et $R^4$ ont la signification mentionnée précédemment et $R^3$ ne représente pas l'hydrogène, et celui-ci est éventuellement purifié et/ou isolé,
et au moins un composé de formule générale IV est mis en réaction dans un milieu réactionnel en présence d'au moins une base, de préférence en présence d'au moins un sel d'hydroxyde métallique, pour former un composé de formule générale VI,

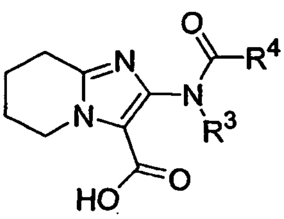

VI

dans laquelle $R^3$ et $R^4$ ont la signification mentionnée précédemment et $R^3$ ne représente pas l'hydrogène, et celui-ci est éventuellement purifié et/ou isolé, et au moins un composé de formule générale VI est mis en réaction dans un milieu réactionnel en présence d'au moins un réactif de couplage, éventuellement en présence d'au moins une base, avec au moins un composé de formule générale $HNR^1R^2$, dans laquelle $R^1$ a la signification mentionnée précédemment et $R^2$ représente l'hydrogène, pour former un composé de formule générale I,

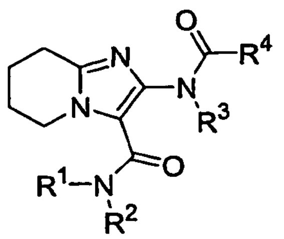

I

dans laquelle $R^1$, $R^3$ et $R^4$ ont la signification mentionnée précédemment, $R^3$ ne représente pas l'hydrogène et $R^2$ représente l'hydrogène, et celui-ci est éventuellement purifié et/ou isolé,
et éventuellement au moins un composé de formule générale I, dans laquelle $R^1$, $R^3$ et $R^4$ ont la signification mentionnée précédemment, $R^3$ ne représente pas l'hydrogène et $R^2$ représente l'hydrogène, est mis en réaction dans un milieu réactionnel en présence d'au moins une base, de préférence un sel d'hydrure métallique, avec au moins un composé de formule générale $R^2$-X, dans laquelle $R^2$ a la signification donnée précédemment et ne représente pas l'hydrogène et X représente un groupe partant, de préférence un radical halogène, pour former un composé de formule générale I,
dans laquelle $R^1$, $R^2$, $R^3$ et $R^4$ ont la signification mentionnée précédemment et $R^2$ et $R^3$ ne représentent pas l'hydrogène, et celui-ci est éventuellement purifié et/ou isolé,
ou
au moins un composé de formule générale VI est mis en réaction dans un milieu réactionnel en présence d'au moins un réactif de couplage, éventuellement en présence d'au moins une base, avec au moins un composé de formule générale $HNR^1R^2$, dans laquelle $R^1$ et $R^2$ ont la signification donnée précédemment et ne représentent pas l'hydrogène, pour former un composé de formule générale I, dans laquelle $R^1$, $R^2$, $R^3$ et $R^4$ ont la signification donnée précédemment et $R^1$, $R^2$ et $R^3$ ne représentent pas l'hydrogène, et celui-ci est éventuellement purifié et/ou isolé.

**19.** Procédé de fabrication de composés de 5,6,7,8-tétrahydro-imidazo[1,2-a]pyridin-2-ylamine substitués de formule générale I selon une ou plusieurs des revendications 1 à 17, **caractérisé en ce qu'**au moins un composé de formule générale II,

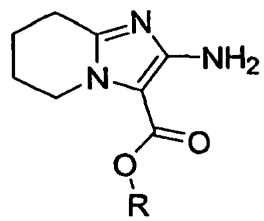

II

dans laquelle R représente un radical alkyle en $C_{1-6}$ linéaire ou ramifié, est mis en réaction dans un milieu réactionnel éventuellement en présence d'au moins une base, avec au moins un composé de formule générale $R^4$-C(=O)-X, dans laquelle $R^4$ a la signification donnée dans une ou plusieurs des revendications 1 à 16 et X représente un groupe partant, de préférence un radical halogène, ou dans un milieu réactionnel en présence d'au moins un réactif de couplage, éventuellement en présence d'au moins une base, avec un composé de formule générale $R^4$-C(=O)-OH, dans laquelle $R^4$ a la signification donnée dans une ou plusieurs des revendications 1 à 16, pour former un composé de formule générale IV,

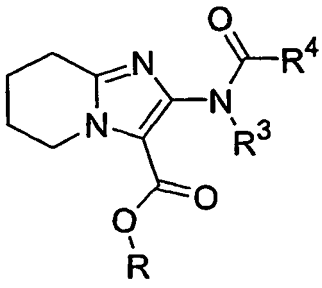

IV

dans laquelle R et $R^4$ ont la signification mentionnée précédemment et $R^3$ représente l'hydrogène, et celui-ci est éventuellement purifié et/ou isolé,
et au moins un composé de formule générale IV est mis en réaction dans un milieu réactionnel en présence d'au moins une base, de préférence en présence d'au moins un sel d'hydroxyde métallique, pour former un composé de formule générale VI,

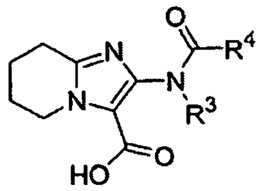

VI

dans laquelle $R^4$ a la signification mentionnée précédemment et $R^3$ représente l'hydrogène, et celui-ci est éventuellement purifié et/ou isolé,
au moins un composé de formule générale VI est mis en réaction dans un milieu réactionnel en présence d'au moins un réactif de couplage, éventuellement en présence d'au moins une base, avec au moins un composé de formule générale $HNR^1R^2$, dans laquelle $R^1$ et $R^2$ ont la signification donnée dans une ou plusieurs des revendications 1 à 16, pour former un composé de formule générale I,

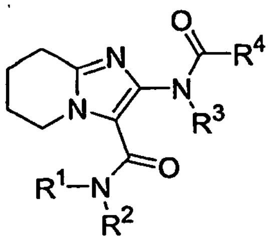

I

dans laquelle $R^1$, $R^2$ et $R^4$ ont la signification mentionnée précédemment et $R^3$ représente l'hydrogène, et celui-ci est éventuellement purifié et/ou isolé.

**20.** Médicament contenant au moins un composé selon une ou plusieurs des revendications 1 à 17 et éventuellement un ou plusieurs adjuvants physiologiquement acceptables.

**21.** Médicament selon la revendication 20 pour la prophylaxie et/ou le traitement de la douleur, notamment de la douleur

choisie dans le groupe constitué par la douleur aiguë, la douleur chronique et la douleur neuropathique.

**22.** Médicament selon la revendication 20 pour la prophylaxie et/ou le traitement d'une ou de plusieurs maladies choisies dans le groupe constitué par la migraine ; les dépressions ; l'incontinence urinaire ; la toux ; les maladies neurodégénératives, de préférence choisies dans le groupe constitué par la maladie de Parkinson, la maladie d'Huntington, la maladie d'Alzheimer et la sclérose en plaques ; les perturbations de l'alimentation, de préférence choisies dans le groupe constitué par la boulimie, l'anorexie, l'obésité et la cachexie ; l'anxiété ; les dysfonctionnements cognitifs, de préférence les troubles de la mémoire ; les déficiences cognitives (attention deficit syndrom, ADS) ; l'épilepsie ; la diarrhée et le prurit ; ou pour la prophylaxie et/ou le traitement de l'abus d'alcool et/ou de drogues et/ou de médicaments et de la dépendance à l'alcool et/ou aux drogues et/ou aux médicaments, de préférence pour la prophylaxie et/ou la réduction des symptômes de sevrage lors de la dépendance à l'alcool et/ou aux drogues et/ou aux médicaments ; pour la prophylaxie et/ou la réduction d'un développement de tolérance envers des médicaments, notamment des médicaments à base d'opioïdes ; pour la régulation de l'alimentation ; pour la modulation de l'activité motrice ; pour la régulation du système cardiovasculaire ; pour l'anesthésie locale ; pour l'augmentation de la vigilance ; pour l'augmentation de la libido ; pour la diurèse et/ou pour l'antinatriurèse.

**23.** Utilisation d'au moins un composé selon une ou plusieurs des revendications 1 à 17 pour la fabrication d'un médicament pour la prophylaxie et/ou le traitement de la douleur, de préférence de la douleur choisie dans le groupe constitué par la douleur aiguë, la douleur chronique et la douleur neuropathique.

**24.** Utilisation d'au moins un composé selon une ou plusieurs des revendications 1 à 17 pour la fabrication d'un médicament pour la prophylaxie et/ou le traitement d'une ou de plusieurs maladies choisies dans le groupe constitué par la migraine ; les dépressions ; l'incontinence urinaire ; la toux ; les maladies neurodégénératives, de préférence choisies dans le groupe constitué par la maladie de Parkinson, la maladie d'Huntington, la maladie d'Alzheimer et la sclérose en plaques ; les perturbations de l'alimentation, de préférence choisies dans le groupe constitué par la boulimie, l'anorexie, l'obésité et la cachexie ; l'anxiété ; les dysfonctionnements cognitifs, de préférence les troubles de la mémoire ; les déficiences cognitives (attention déficit syndrom, ADS) ; l'épilepsie ; la diarrhée et le prurit ; ou pour la prophylaxie et/ou le traitement de l'abus d'alcool et/ou de drogues et/ou de médicaments et de la dépendance à l'alcool et/ou aux drogues et/ou aux médicaments, de préférence pour la prophylaxie et/ou la réduction des symptômes de sevrage lors de la dépendance à l'alcool et/ou aux drogues et/ou aux médicaments ; pour la prophylaxie et/ou la réduction d'un développement de tolérance envers des médicaments, notamment des médicaments à base d'opioïdes ; pour la régulation de l'alimentation ; pour la modulation de l'activité motrice ; pour la régulation du système cardiovasculaire ; pour l'anesthésie locale ; pour l'augmentation de la vigilance ; pour l'augmentation de la libido ; pour la diurèse et/ou pour l'antinatriurèse.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Nicht-Patentliteratur


- **Sako, M.** *Science of Synthesis,* 2004, 1109-1153
- **Wada, A. et al.** *Chemical and Pharmaceutical Bulletin,* 1991, 1189-1192
- **Takahata, H. et al.** *Fukusokan Kagaku Toronkai Koen Yoshishu,* 1979, 296-300
- Remington's Pharmaceutical Sciences. Mack Publishing Company, 1985
- **Gray ; Whittaker.** *J. Anat.,* 1962, vol. 76, 79-88
- **Pauwels, Leysen ; Laduron durchgeführt.** *Eur. J. Pharmacol.,* vol. 124, 291-298